Europäisches Patentamt

European Patent Office

Office européen des brevets

(19)

(11) **EP 1 484 405 A1**

(12) **EUROPEAN PATENT APPLICATION**

(43) Date of publication:
**08.12.2004 Bulletin 2004/50**

(51) Int Cl.⁷: **C12N 15/31**, A61K 39/04,
C07K 14/35, C12N 15/62,
A61K 38/16, G01N 33/569,
C12Q 1/68, C07K 16/12

(21) Application number: **04077071.1**

(22) Date of filing: **08.10.1998**

(84) Designated Contracting States:
**AT BE CH CY DE DK ES FI FR GB GR IE IT LI LU MC NL PT SE**

(30) Priority: **10.11.1997 DK 127797**
**05.01.1998 US 70488 P**
**01.04.1998 WOPCT/DK98/00132**

(62) Document number(s) of the earlier application(s) in accordance with Art. 76 EPC:
**98947412.7 / 1 029 053**

(71) Applicant: **Statens Serum Institut**
**2300 Copenhagen S (DK)**

(72) Inventors:
• **Andersen, Peter**
**2700 Brönshöj (DK)**
• **Skjot, Rikke Louise Vinther**
**2640 Hedehusene (DK)**

(74) Representative: **Plougmann & Vingtoft a/s**
**Sundkrogsgade 9,**
**P.O. Box 831**
**2100 Copenhagen (DK)**

Remarks:
This application was filed on 19 - 07 - 2004 as a divisional application to the application mentioned under INID code 62.

(54) **Nucleic acid fragments and polypeptide fragments derived from M. Tuberculosis**

(57)    The present invention is based on the identification and characterisation of novel protein and protein fragments derived from *M. tuberculosis* (SEQ ID NO: 92) The invention is directed to the polypeptide and immunologically active fragments and analogues thereof, the genes encoding them, immunological compositions such as vaccines and skin test reagents containing the polypeptides.

**EP 1 484 405 A1**

**Description**

FIELD OF THE INVENTION

The present invention relates to a number of immunologically active, novel polypeptide fragments derived from the *Mycobacterium tuberculosis,* vaccines and other immunologic compositions containing the fragments as immunogenic components, and methods of production and use of the polypeptides. The invention also relates to novel nucleic acid fragments derived from *M. tuberculosis* which are useful in the preparation of the polypeptide fragments of the invention or in the diagnosis of infection with *M. tuberculosis.*

BACKGROUND OF THE INVENTION

Human tuberculosis (hereinafter designated "TB") caused by *Mycobacterium tuberculosis* is a severe global health problem responsible for approximately 3 million deaths annually, according to the WHO. The worldwide incidence of new TB cases has been progressively falling for the last decade but the recent years has markedly changed this trend due to the advent of AIDS and the appearance of multidrug resistant strains of *M. tuberculosis.*

The only vaccine presently available for clinical use is BCG, a vaccine which efficacy remains a matter of controversy. BCG generally induces a high level of acquired resistance in animal models of TB, but several human trials in developing countries have failed to demonstrate significant protection. Notably, BCG is not approved by the FDA for use in the United States.

This makes the development of a new and improved vaccine against TB an urgent matter which has been given a very high priority by the WHO. Many attempts to define protective mycobacterial substances have been made, and from 1950 to 1970 several investigators reported an increased resistance after experimental vaccination. However, the demonstration of a specific long-term protective immune response with the potency of BCG has not yet been achieved by administration of soluble proteins or cell wall fragments, although progress is currently being made by relying on polypeptides derived from short term-culture filtrate, cf. the discussion below.

Immunity to *M. tuberculosis* is characterized by three basic features; i) Living bacilli efficiently induces a protective immune response in contrast to killed preparations; ii) Specifically sensitized T lymphocytes mediate this protection; iii) The most important mediator molecule seems to be interferon gamma (INF-γ).

Short term-culture filtrate (ST-CF) is a complex mixture of proteins released from *M. tuberculosis* during the first few days of growth in a liquid medium (Andersen *et al*., 1991). Culture filtrates has been suggested to hold protective antigens recognized by the host in the first phase of TB infection (Andersen *et al*. 1991, Orme *et al*. 1993). Recent data from several laboratories have demonstrated that experimental subunit vaccines based on culture filtrate antigens can provide high levels of acquired resistance to TB (Pal and Horwitz, 1992; Roberts *et al*., 1995; Andersen, 1994; Lindblad *et al*., 1997). Culture filtrates are, however, complex protein mixtures and until now very limited information has been available on the molecules responsible for this protective immune response. In this regard, only two culture filtrate antigens have been described as involved in protective immunity, the low mass antigen ESAT-6 (Andersen *et al*., 1995 and EP-A-0 706 571) and the 31 kDa molecule Ag85B (EP-0 432 203).

There is therefore a need for the identification of further antigens involved in the induction of protective immunity against TB in order to eventually produce an effective subunit vaccine.

OBJECT OF THE INVENTION

It is an object of the invention to provide novel antigens which are effective as components in a subunit vaccine against TB or which are useful as components in diagnostic compositions for the detection of infection with mycobacteria, especially virulence-associated mycobacteria. The novel antigens may also be important drug targets.

SUMMARY OF THE INVENTION

The present invention is *i.a.* based on the identification and characterization of a number of previously uncharacterized culture filtrate antigens from *M. tuberculosis.* In animal models of TB, T cells mediating immunity are focused predominantly to antigens in the regions 6-12 and 17-30 kDa of ST-CF. In the present invention 6 antigens in the low molecular weight region (ORF7-1, ORF7-2, ORF11-1, ORF11-2, ORF11-3, ORF11-4) have been identified.

Furthermore immunological and biological data on several important antigens are presented.

The encoding genes for 8 antigens have been determined. The panel hold antigens with potential for vaccine purposes as well as for diagnostic purposes, since the antigens are all secreted by metabolizing mycobacteria.

The following table lists the antigens of the invention by the names used herein as well as by reference to relevant SEQ ID NOs of N-terminal sequences, full amino acid sequences and sequences of DNA encoding the antigens:

| Antigen | N-terminal sequence SEQ ID NO: | Nucleotide sequence SEQ ID NO: | Amino acid sequence SEQ ID NO: |
|---|---|---|---|
| CFP7 | | 1 | 2 |
| CFP7A | 81 | 47 | 48 |
| CFP7B | 168 | 146 | 147 |
| CFP8A | 73 | 148 | 149 |
| CFP8B | 74 | 150 | 151 |
| CFP9 | | 3 | 4 |
| CFP10A | 169 | 140 | 141 |
| CFP11 | 170 | 142 | 143 |
| CFP16 | 79 | 63 | 64 |
| CFP17 | 17 | 5 | 6 |
| CFP19 | 82 | 49 | 50 |
| CFP19A | | 51 | 52 |
| CFP19B | 80 | | |
| CFP20 | 18 | 7 | 8 |
| CFP21 | 19 | 9 | 10 |
| CFP22 | 20 | 11 | 12 |
| CFP22A | 83 | 53 | 54 |
| CFP23 | | 55 | 56 |
| CFP23A | 76 | | |
| CFP23B | 75 | | |
| CFP25 | 21 | 13 | 14 |
| CFP25A | 78 | 65 | 66 |
| CFP27 | 84 | 57 | 58 |
| CFP28 | 22 | | |
| CFP29 | 23 | 15 | 16 |
| CFP30A | 85 | 59 | 60 |
| CFP30B | 171 | 144 | 145 |
| CFP50 | 86 | 61 | 62 |
| MPT51 | | 41 | 42 |
| CWP32 | 77 | 152 | 153 |
| RD1-ORF8 | | 67 | 68 |
| RD1-ORF2 | | 71 | 72 |
| RD1-ORF9B | | 69 | 70 |
| RD1-ORF3 | | 87 | 88 |
| RD1-ORF9A | | 93 | 94 |
| RD1-ORF4 | | 89 | 90 |
| RD1-ORF5 | | 91 | 92 |
| MPT59-ESAT6 | | | 172 |
| ESAT6-MPT59 | | | 173 |
| ORF7-1 | | 174 | 175 |
| ORF7-2 | | 176 | 177 |
| ORF11-1 | | 178 | 179 |
| ORF11-2 | | 180 | 181 |
| ORF11-3 | | 182 | 183 |
| ORF11-4 | | 184 | 185 |

It is well-known in the art that T-cell epitopes are responsible for the elicitation of the acquired immunity against TB, whereas B-cell epitopes are without any significant influence on acquired immunity and recognition of mycobacteria *in vivo.* Since such T-cell epitopes are linear and are known to have a minimum length of 6 amino acid residues, the present invention is especially concerned with the identification and utilisation of such T-cell epitopes.

Hence, in its broadest aspect the invention relates to a substantially pure polypeptide fragment which

a) comprises an amino acid sequence selected from the sequences shown in SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, and 185

b) comprises a subsequence of the polypeptide fragment defined in a) which has a length of at least 6 amino acid residues, said subsequence being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex, or

c) comprises an amino acid sequence having a sequence identity with the polypeptide defined in a) or the subsequence defined in b) of at least 70% and at the same time being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex,

with the proviso that

i) the polypeptide fragment is in essentially pure form when consisting of the amino acid sequence 1-96 of SEQ ID NO: 2 or when consisting of the amino acid sequence 87-108 of SEQ ID NO: 4 fused to β-galactosidase,

ii) the degree of sequence identity in c) is at least 95 % when the polypeptide comprises a homologue of a polypeptide which has the amino acid sequence SEQ ID NO: 12 or a subsequence thereof as defined in b), and

iii) the polypeptide fragment contains a threonine residue corresponding to position 213 in SEQ ID NO: 42 when comprising an amino acid sequence of at least 6 amino acids in SEQ ID NO: 42.

Other parts of the invention pertains to the DNA fragments encoding a polypeptide with the above definition as well as to DNA fragments useful for determining the presence of DNA encoding such polypeptides.

DETAILED DISCLOSURE OF THE INVENTION

In the present specification and claims, the term "polypeptide fragment" denotes both short peptides with a length of at least two amino acid residues and at most 10 amino acid residues, oligopeptides (11-100 amino acid residues), and longer peptides (the usual interpretation of "polypeptide", *i.e.* more than 100 amino acid residues in length) as well as proteins (the functional entity comprising at least one peptide, oligopeptide, or polypeptide which may be chemically modified by being glycosylated, by being lipidated, or by comprising prosthetic groups). The definition of polypeptides also comprises native forms of peptides/proteins in mycobacteria as well as recombinant proteins or peptides in any type of expression vectors transforming any kind of host, and also chemically synthesized peptides.

In the present context the term "substantially pure polypeptide fragment" means a polypeptide preparation which contains at most 5% by weight of other polypeptide material with which it is natively associated (lower percentages of other polypeptide material are preferred, e.g. at most 4%, at most 3%, at most 2%, at most 1 %, and at most ½ %). It is preferred that the substantially pure polypeptide is at least 96% pure, *i.e.* that the polypeptide constitutes at least 96% by weight of total polypeptide material present in the preparation, and higher percentages are preferred, such as at least 97%, at least 98%, at least 99%, at least 99,25%, at least 99,5%, and at least 99,75%. It is especially preferred that the polypeptide fragment is in "essentially pure form", *i.e.* that the polypeptide fragment is essentially free of any other antigen with which it is natively associated, *i.e.* free of any other antigen from bacteria belonging to the tuberculosis complex. This can be accomplished by preparing the polypeptide fragment by means of recombinant methods in a non-mycobacterial host cell as will be described in detail below, or by synthesizing the polypeptide fragment by the well-known methods of solid or liquid phase peptide synthesis, e.g. by the method described by Merrifield or variations thereof.

The term "subsequence" when used in connection with a polypeptide of the invention having a SEQ ID NO selected from 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, and 185 denotes any continuous stretch of at least 6 amino acid residues taken from the *M. tuberculosis* derived polypeptides in SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, and 185 and being immunological equivalent thereto with respect to the ability of conferring increased resistance to infections with bacteria belonging to the tuberculosis complex. Thus, included is also a polypeptide from different sources, such as other bacteria or even from eukaryotic cells.

When referring to an "immunologically equivalent" polypeptide is herein meant that the polypeptide, when formulated in a vaccine or a diagnostic agent (*i.e.* together with a pharmaceutically acceptable carrier or vehicle and optionally an adjuvant), will

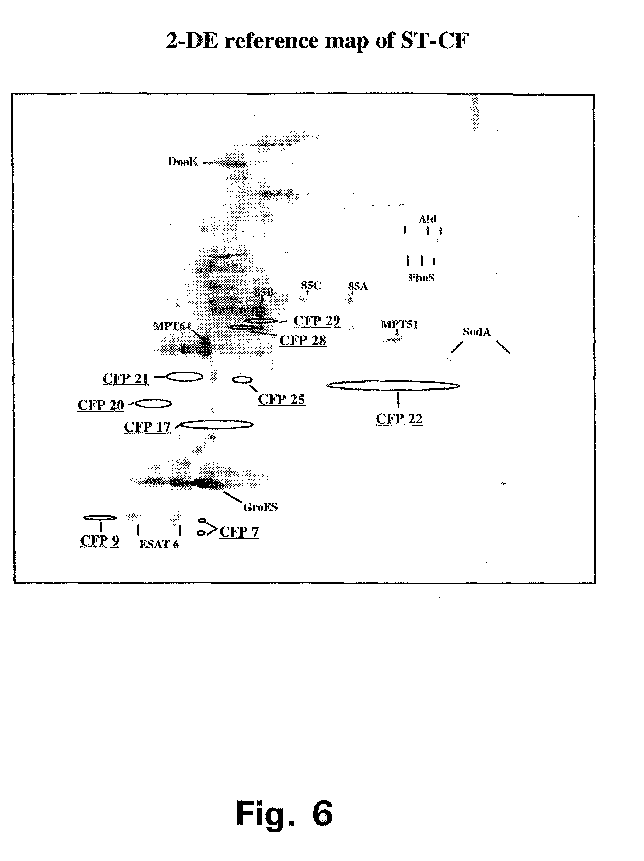

I) confer, upon administration (either alone or as an immunologically active constituent together with other antigens), an acquired increased specific resistance in a mouse and/or in a guinea pig and/or in a primate such as a human being against infections with bacteria belonging to the tuberculosis complex which is at least 20% of the acquired increased resistance conferred by *Mycobacterium bovis* BCG and also at least 20% of the acquired increased resistance conferred by the parent polypeptide comprising SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185 (said parent polypeptide having substantially the same relative location and pattern in a 2DE gel prepared as the 2DE gel shown in Fig. 6, cf. the examples), the acquired increased resistance being assessed by the observed reduction in mycobacterial counts from spleen, lung or other organ homogenates isolated from the mouse or guinea pig receiving a challenge infection with a virulent strain of *M. tuberculosis,* or, in a primate such as a human being, being assessed by determining the protection against development of clinical tuberculosis in a vaccinated group versus that observed in a control group receiving a placebo or BCG (preferably the increased resistance is higher and corresponds to at least 50% of the protective immune response elicited by *M. bovis* BCG, such as at least 60%, or even more preferred to at least 80% of the protective immune response elicited by *M. bovis* BCG, such as at least 90%; in some cases it is expected that the increased resistance will supersede that conferred by *M. bovis* BCG, and hence it is preferred that the resistance will be at least 100%, such as at least 110% of said increased resistance); and/or

II) elicit a diagnostically significant immune response in a mammal indicating previous or ongoing sensitization with antigens derived from mycobacteria belonging to the tuberculosis complex; this diagnostically significant immune response can be in the form of a delayed type hypersensitivity reaction which can e.g. be determined by a skin test, or can be in the form of IFN-γ release determined e.g. by an IFN-γ assay as described in detail below. A diagnostically significant response in a skin test setup will be a reaction which gives rise to a skin reaction which is at least 5 mm in diameter and which is at least 65% (preferably at least 75% such as at the least 85%) of the skin reaction (assessed as the skin reaction diameter) elicited by the parent polypeptide comprising SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185.

The ability of the polypeptide fragment to confer increased immunity may thus be assessed by measuring in an experimental animal, e.g. a mouse or a guinea pig, the reduction in mycobacterial counts from the spleen, lung or other organ homogenates isolated from the experimental animal which have received a challenge infection with a virulent strain of mycobacteria belonging to the tuberculosis complex after previously having been immunized with the polypeptide, as compared to the mycobacterial counts in a control group of experimental animals infected with the same virulent strain, which experimental animals have not previously been immunized against tuberculosis. The comparison of the mycobacterial counts may also be carried out with mycobacterial counts from a group of experimental animals receiving a challenge infection with the same virulent strain after having been immunized with *Mycobacterium bovis* BCG.

The mycobacterial counts in homogenates from the experimental animals immunized with a polypeptide fragment according to the present invention must at the most be 5 times the counts in the mice or guinea pigs immunized with *Mycobacterium bovis* BCG, such as at the most 3 times the counts, and preferably at the most 2 times the counts.

A more relevant assessment of the ability of the polypeptide fragment of the invention to confer increased resistance is to compare the incidence of clinical tuberculosis in two groups of individuals (e.g. humans or other primates) where one group receives a vaccine as described herein which contains an antigen of the invention and the other group receives either a placebo or an other known TB vaccine (e.g. BCG). In such a setup, the antigen of the invention should give rise to a protective immunity which is significantly higher than the one provided by the administration of the placebo (as determined by statistical methods known to the skilled artisan).

In the context of the present application, the term "wide genetically" should be understood in a meaning of at least two strains. That is, if a polypeptide is recognised by at least two different strains, it is considered to have a wide genetically recognition. A subunit vaccine component is defined as a reagent which stimulates protective immunity in an animal model of infection with an organism of the M. tuberculosis complex, when given prior to infection and which also generates a significant immune responses in human volunteers.

The "tuberculosis-complex" has its usual meaning, *i.e.* the complex of mycobacteria causing TB which are *Mycobacterium tuberculosis, Mycobacterium bovis, Mycobacterium bovis* BCG, and *Mycobacterium africanum*.

In the present context the term "metabolizing mycobacteria" means live mycobacteria that are multiplying logarithmically and releasing polypeptides into the culture medium wherein they are cultured.

The term "sequence identity" indicates a quantitative measure of the degree of homology between two amino acid sequences or between two nucleotide sequences of equal length, of if not of equal length aligned to best possible fit: The sequence identity can be calculated as $\frac{(N_{ref}-N_{dif})\,100}{N_{ref}}$ 1, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC ($N_{dif}$ = 2 and $N_{ref}$ = 8). Sequence identity can alternatively be calculated by the BLASTP program ((Pearson W.R and D.J. Lipman (1988) PNAS USA 85:2444-2448) in the EMBL database (www.ncbi.nlm.gov/cgi-bin/BLAST). Generally, the default settings with respect to e.g. "scoring matrix" and "gap penalty" will be used for alignment.

The sequence identity is used here to illustrate the degree of identity between the amino acid sequence of a given polypeptide and the amino acid sequence shown in SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185. The amino acid sequence to be compared with the amino acid sequence shown in SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185 may be deduced from a DNA sequence, e.g. obtained by hybridization as defined below, or may be obtained by conventional amino acid sequencing methods. The sequence identity is preferably determined on the amino acid sequence of a mature polypeptide, *i.e.* without taking any leader sequence into consideration.

As appears from the above disclosure, polypeptides which are not identical to the polypeptides having SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185 are embraced by the present invention. The invention allows for minor variations which do not have an adverse effect on immunogenicity compared to the parent sequences and which may give interesting and useful novel binding properties or biological functions and immunogenicities etc.

Each polypeptide fragment may thus be characterized by specific amino acid and nucleic acid sequences. It will be understood that such sequences include analogues and variants produced by recombinant methods wherein such nucleic acid and polypeptide sequences have been modified by substitution, insertion, addition and/or deletion of one or more nucleotides in said nucleic acid sequences to cause the substitution, insertion, addition or deletion of one or more amino acid residues in the recombinant polypeptide. When the term DNA is used in the following, it should be understood that for the number of purposes where DNA can be substituted with RNA, the term DNA should be read to include RNA embodiments which will be apparent for the man skilled in the art. For the purposes of hybridization, PNA or LNA may be used instead of DNA. PNA has been shown to exhibit a very dynamic hybridization profile (PNA is described in Nielsen P E *et al*., 1991, Science 254: 1497-1500). LNA (Locked Nucleic Acids) is a recently introduced oligonucleotide analogue containing bicyclo nucleoside monomers (Koshkin et al., 1998, 54, 3607-3630;Nielsen, N.K. et al. J.Am.Chem.Soc 1998, 120, 5458-5463).

In both immunodiagnostics and vaccine preparation, it is often possible and practical to prepare antigens from segments of a known immunogenic protein or polypeptide.

Certain epitopic regions may be used to produce responses similar to those produced by the entire antigenic polypeptide. Potential antigenic or immunogenic regions may be identified by any of a number of approaches, e.g., Jameson-Wolf or Kyte-Doolittle antigenicity analyses or Hopp and Woods (1981) hydrophobicity analysis (see, e.g., Jameson and Wolf, 1988; Kyte and Doolittle, 1982; or U.S. Patent No. 4,554,101). Hydrophobicity analysis assigns average hydrophilicity values to each amino acid residue from these values average hydrophilicities can be calculated and regions of greatest hydrophilicity determined. Using one or more of these methods, regions of predicted antigenicity may be derived from the amino acid sequence assigned to the polypeptides of the invention.

Alternatively, in order to identify relevant T-cell epitopes which are recognized during an immune response, it is also possible to use a "brute force" method: Since T-cell epitopes are linear, deletion mutants of polypeptides having SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185 will, if constructed systematically, reveal what regions of the polypeptides are essential in immune recognition, e.g. by subjecting these deletion mutants to the IFN-γ assay described herein. Another method utilises overlapping oligomers (preferably synthetic having a length of e.g. 20 amino acid residues) derived from polypeptides having SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185. Some of these will give a positive response in the IFN-γ assay whereas others will not.

In a preferred embodiment of the invention, the polypeptide fragment of the invention comprises an epitope for a T-helper cell.

Although the minimum length of a T-cell epitope has been shown to be at least 6 amino acids, it is normal that such epitopes are constituted of longer stretches of amino acids. Hence it is preferred that the polypeptide fragment of the invention has a length of at least 7 amino acid residues, such as at least 8, at least 9, at least 10, at least 12, at least 14, at least 16, at least 18, at least 20, at least 22, at least 24, and at least 30 amino acid residues.

As will appear from the examples, a number of the polypeptides of the invention are natively translation products which include a leader sequence (or other short peptide sequences), whereas the product which can be isolated from short-term culture filtrates from bacteria belonging to the tuberculosis complex are free of these sequences. Although it may in some applications be advantageous to produce these polypeptides recombinantly and in this connection facilitate export of the polypeptides from the host cell by including information encoding the leader sequence in the gene for the polypeptide, it is more often preferred to either substitute the leader sequence with one which has been shown to be superior in the host system for effecting export, or to totally omit the leader sequence (e.g. when producing the polypeptide by peptide synthesis. Hence, a preferred embodiment of the invention is a polypeptide which is free from amino acid residues -30 to -1 in SEQ ID NO: 6 and/or -32 to -1 in SEQ ID NO: 10 and/or -8 to -1 in SEQ ID NO: 12 and/or -32 to -1 in SEQ ID NO: 14 and/or - 33 to -1 in SEQ ID NO: 42 and/or -38 to -1 in SEQ ID NO: 52 and/or -33 to -1 in SEQ ID NO: 56 and/or -56 to -1 in SEQ ID NO: 58 and/or -28 to -1 in SEQ ID NO: 151.

In another preferred embodiment, the polypeptide fragment of the invention is free from any signal sequence; this is especially interesting when the polypeptide fragment is produced synthetically but even when the polypeptide fragments are produced recombinantly it is normally acceptable that they are not exported by the host cell to the periplasm or the extracellular space; the polypeptide fragments can be recovered by traditional methods (cf. the discussion below) from the cytoplasm after disruption of the host cells, and if there is need for refolding of the polypeptide fragments, general refolding schemes can be employed, cf. e.g. the disclosure in WO 94/18227 where such a general applicable refolding method is described.

A suitable assay for the potential utility of a given polypeptide fragment derived from SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185 is to assess the ability of the polypeptide fragment to effect IFN-γ release from primed memory T-lymphocytes. Polypeptide fragments which have this capability are according to the invention especially interesting embodiments of the invention: It is contemplated that polypeptide fragments which stimulate T lymphocyte immune response shortly after the onset of the infection are important in the control of the mycobacteria causing the infection before the mycobacteria have succeeded in multiplying up to the number of bacteria that would have resulted in fulminant infection.

It is presently contemplated that when this application refers to IFN-γ release as a measure of immunogenicity, other cytokines could be relevant, such as IL-12, TNF-α, IL-4, IL-5, IL-10, IL-6, TGF-β. Usually one or more cytokines will be measured utilising for example the PCR technique or ELISA. It will be appreciated by the person skilled in the art, that a significant increase or decrease in any of these cytokines will be indicative of an immunological effective polypeptide or polypeptide fragment.

Thus, an important embodiment of the invention is a polypeptide fragment defined above which

1) induces a release of IFN-γ from primed memory T-lymphocytes withdrawn from a mouse within 2 weeks of primary infection or within 4 days after the mouse has been re-challenge infected with mycobacteria belonging to the tuberculosis complex, the induction performed by the addition of the polypeptide to a suspension comprising about 200,000 spleen cells per ml, the addition of the polypeptide resulting in a concentration of 1-4 µg polypeptide per ml suspension, the release of IFN-γ being assessable by determination of IFN-γ in supernatant harvested 2 days after the addition of the polypeptide to the suspension, and/or

2) induces a release of IFN-γ of at least 1,500 pg/ml above background level from about 1,000,000 human PBMC (peripheral blood mononuclear cells) per ml isolated from TB patients in the first phase of infection, or from healthy BCG vaccinated donors, or from healthy contacts to TB patients, the induction being performed by the addition of the polypeptide to a suspension comprising the about 1,000,000 PBMC per ml, the addition of the polypeptide resulting in a concentration of 1-4 μg polypeptide per ml suspension, the release of IFN-γ being assessable by determination of IFN-γ in supernatant harvested 2 days after the addition of the polypeptide to the suspension; and/or

3) induces an IFN-γ release from bovine PBMC derived from animals previously sensitized with mycobacteria belonging to the tuberculosis complex, said release being at least two times the release observed from bovine PBMC derived from animals not previously sensitized with mycobacteria belonging to the tuberculosis complex.

Preferably, in alternatives 1 and 2, the release effected by the polypeptide fragment gives rise to at least 1,500 pg/ml IFN-γ in the supernatant but higher concentrations are preferred, e.g. at least 2,000 pg/ml and even at least 3,000 pg/ml IFN-γ in the supernatant. The IFN-γ release from bovine PBMC can e.g. be measured as the optical density (OD) index over background in a standard cytokine ELISA and should thus be at least two, but higher numbers such as at least 3, 5, 8, and 10 are preferred.

The polypeptide fragments of the invention preferably comprises an amino acid sequence of at least 6 amino acid residues in length which has a higher sequence identity than 70 percent with SEQ ID NO: 2, 4, 6, 8, 10, 12, 14, 16, any one of 17-23, 42, 48, 50, 52, 54, 56, 58, 60, 62, 64, 66, 68, 70, any one of 72-86, 88, 90, 92, 94, 141, 143, 145, 147, 149, 151, 153, any one of 168-171, 175, 177, 179, 181, 183, or 185. A preferred minimum percentage of sequence identity is at least 80%, such as at least 85%, at least 90%, at least 91 %, at least 92%, at least 93%, at least 94%, at least 95%, at least 96%, at least 97%, at least 98%, at least 99%, and at least 99.5%.

As mentioned above, it will normally be interesting to omit the leader sequences from the polypeptide fragments of the invention. However, by producing fusion polypeptides, superior characteristics of the polypeptide fragments of the invention can be achieved. For instance, fusion partners which facilitate export of the polypeptide when produced recombinantly, fusion partners which facilitate purification of the polypeptide, and fusion partners which enhance the immunogenicity of the polypeptide fragment of the invention are all interesting possibilities. Therefore, the invention also pertains to a fusion polypeptide comprising at least one polypeptide fragment defined above and at least one fusion partner. The fusion partner can, in order to enhance immunogenicity, e.g. be selected from the group consisting of another polypeptide fragment as defined above (so as to allow for multiple expression of relevant epitopes), and an other polypeptide derived from a bacterium belonging to the tuberculosis complex, such as ESAT-6, CFP7, CFP10, CFP17, CFP21, CFP25, CFP29, MPB59, MPT59, MPB64, and MPT64 or at least one T-cell epitope of any of these antigens. Other immunogenicity enhancing polypeptides which could serve as fusion partners are T-cell epitopes (e.g. derived from the polypeptides ESAT-6, MPB64, MPT64, or MPB59) or other immunogenic epitopes enhancing the immunogenicity of the target gene product, e.g. lymphokines such as INF-γ, IL-2 and IL-12. In order to facilitate expression and/or purification the fusion partner can e.g. be a bacterial fimbrial protein, e.g. the pilus components pilin and papA; protein A; the ZZ-peptide (ZZ-fusions are marketed by Pharmacia in Sweden); the maltose binding protein; gluthatione S-transferase; β-galactosidase; or poly-histidine.

Other interesting fusion partners are polypeptides which are lipidated and thereby effect that the immunogenic polypeptide is presented in a suitable manner to the immune system. This effect is e.g. known from vaccines based on the *Borrelia burgdorferi* OspA polypeptide, wherein the lipidated membrane anchor in the polypeptide confers a self-adjuvating effect to the polypeptide (which is natively lipidated) when isolated from cells producing it. In contrast, the OspA polypeptide is relatively silent immunologically when prepared without the lipidation anchor.

As evidenced in Example 6A, the fusion polypeptide consisting of MPT59 fused directly N-terminally to ESAT-6 enhances the immunogenicity of ESAT-6 beyond what would be expected from the immunogenicities of MPT59 and ESAT-6 alone. The precise reason for this surprising finding is not yet known, but it is expected that either the presence of both antigens lead to a synergistic effect with respect to immunogenicity or the presence of a sequence N-terminally to the ESAT-6 sequence protects this immune dominant protein from loss of important epitopes known to be present in the N-terminus. A third, alternative, possibility is that the presence of a sequence C-terminally to the MPT59 sequence enhances the immunologic properties of this antigen.

Hence, one part of the invention pertains to a fusion polypeptide fragment which comprises a first amino acid sequence including at least one stretch of amino acids constituting a T-cell epitope derived from the *M. tuberculosis* protein ESAT-6 or MPT59, and a second amino acid sequence including at least one T-cell epitope derived from a *M. tuberculosis* protein different from ESAT-6 (if the first stretch of amino acids are derived from ESAT-6) or MPT59 (if the first stretch of amino acids are derived from MPT59) and/or including a stretch of amino acids which protects the first amino acid sequence from *in vivo* degradation or post-translational processing. The first amino acid sequence may be situated N- or C-terminally to the second amino acid sequence, but in line with the above considerations regarding protection of the ESAT-6 N-terminus it is preferred that the first amino acid sequence is C-terminal to the second when the first amino acid sequence is derived from ESAT-6.

Although only the effect of fusion between MPT59 and ESAT6 has been investigated at present, it is believed that ESAT6 and MPT59 or epitopes derived therefrom could be advantageously be fused to other fusion partners having substantially the same effect on overall immunogenicity of the fusion construct. Hence, it is preferred that such a fusion polypeptide fragment according of the invention is one, wherein the at least one T-cell epitope included in the second amino acid sequence is derived from a M. *tuberculosis* polypeptide (the "parent" polypeptide) selected from the group consisting of a polypeptide fragment according to the present invention and described in detail above and in the examples, or the amino acid sequence could be derived from any one of the *M. tuberculosis* proteins DnaK, GroEL, urease, glutamine synthetase, the proline rich complex, L-alanine dehydrogenase, phosphate binding protein, Ag 85 complex, HBHA (heparin binding hemagglutinin), MPT51, MPT64, superoxide dismutase, 19 kDa lipoprotein, $\alpha$-crystallin, GroES, MPT59 (when the first amino acid sequence is derived from ESAT-6), and ESAT-6 (when the first amino acid sequence is derived from MPT59). It is preferred that the first and second T-cell epitopes each have a sequence identity of at least 70% with the natively occurring sequence in the proteins from which they are derived and it is even further preferred that the first and/or second amino acid sequence has a sequence identity of at least 70% with the protein from which they are derived. A most preferred embodiment of this fusion polypeptide is one wherein the first amino acid sequence is the amino acid sequence of ESAT-6 or MPT59 and/or the second amino acid sequence is the full-length amino acid sequence of the possible "parent" polypeptides listed above.

In the most preferred embodiment, the fusion polypeptide fragment comprises ESAT-6 fused to MPT59 (advantageously, ESAT-6 is fused to the C-terminus of MPT59) and in one special embodiment, there are no linkers introduced between the two amino acid sequences constituting the two parent polypeptide fragments.

Another part of the invention pertains to a nucleic acid fragment in isolated form which

1) comprises a nucleic acid sequence which encodes a polypeptide or fusion polypeptide as defined above, or comprises a nucleic acid sequence complementary thereto, and/or

2) has a length of at least 10 nucleotides and hybridizes readily under stringent hybridization conditions (as defined in the art, *i.e.* 5-10°C under the melting point $T_m$, cf. Sambrook et al, 1989, pages 11.45-11.49) with a nucleic acid fragment which has a nucleotide sequence selected from

SEQ ID NO: 1 or a sequence complementary thereto,
SEQ ID NO: 3 or a sequence complementary thereto,
SEQ ID NO: 5 or a sequence complementary thereto,
SEQ ID NO: 7 or a sequence complementary thereto,
SEQ ID NO: 9 or a sequence complementary thereto,
SEQ ID NO: 11 or a sequence complementary thereto,
SEQ ID NO: 13 or a sequence complementary thereto,
SEQ ID NO: 15 or a sequence complementary thereto,
SEQ ID NO: 41 or a sequence complementary thereto,
SEQ ID NO: 47 or a sequence complementary thereto,
SEQ ID NO: 49 or a sequence complementary thereto,
SEQ ID NO: 51 or a sequence complementary thereto,
SEQ ID NO: 53 or a sequence complementary thereto,
SEQ ID NO: 55 or a sequence complementary thereto,
SEQ ID NO: 57 or a sequence complementary thereto,
SEQ ID NO: 59 or a sequence complementary thereto,
SEQ ID NO: 61 or a sequence complementary thereto,
SEQ ID NO: 63 or a sequence complementary thereto,
SEQ ID NO: 65 or a sequence complementary thereto,
SEQ ID NO: 67 or a sequence complementary thereto,
SEQ ID NO: 69 or a sequence complementary thereto,
SEQ ID NO: 71 or a sequence complementary thereto,
SEQ ID NO: 87 or a sequence complementary thereto,
SEQ ID NO: 89 or a sequence complementary thereto,
SEQ ID NO: 91 or a sequence complementary thereto,
SEQ ID NO: 93 or a sequence complementary thereto,
SEQ ID NO: 140 or a sequence complementary thereto,
SEQ ID NO: 142 or a sequence complementary thereto,
SEQ ID NO: 144 or a sequence complementary thereto,
SEQ ID NO: 146 or a sequence complementary thereto,
SEQ ID NO: 148 or a sequence complementary thereto,
SEQ ID NO: 150 or a sequence complementary thereto,

SEQ ID NO: 152 or a sequence complementary thereto,
SEQ ID NO: 174 or a sequence complementary thereto,
SEQ ID NO: 176 or a sequence complementary thereto,
SEQ ID NO: 178 or a sequence complementary thereto,
SEQ ID NO: 180 or a sequence complementary thereto,
SEQ ID NO: 182 or a sequence complementary thereto, and
SEQ ID NO: 184 or a sequence complementary thereto

with the proviso that when the nucleic acid fragment comprises a subsequence of SEQ ID NO: 41, then the nucleic acid fragment contains an A corresponding to position 781 in SEQ ID NO: 41 and when the nucleic acid fragment comprises a subsequence of a nucleotide sequence exactly complementary to SEQ ID NO: 41, then the nucleic acid fragment comprises a T corresponding to position 781 in SEQ ID NO: 41.

It is preferred that the nucleic acid fragment is a DNA fragment.

To provide certainty of the advantages in accordance with the invention, the preferred nucleic acid sequence when employed for hybridization studies or assays includes sequences that are complementary to at least a 10 to 40, or so, nucleotide stretch of the selected sequence. A size of at least 10 nucleotides in length helps to ensure that the fragment will be of sufficient length to form a duplex molecule that is both stable and selective. Molecules having complementary sequences over stretches greater than 10 bases in length are generally preferred, though, in order to increase stability and selectivity of the hybrid, and thereby improve the quality and degree of specific hybrid molecules obtained.

Hence, the term "subsequence" when used in connection with the nucleic acid fragments of the invention is intended to indicate a continuous stretch of at least 10 nucleotides exhibits the above hybridization pattern. Normally this will require a minimum sequence identity of at least 70% with a subsequence of the hybridization partner having SEQ ID NO: 1, 3, 5, 7, 9, 11, 12, 15, 21, 41, 47, 49, 51, 53, 55, 57, 59, 61, 63, 65, 67, 69, 71, 87, 89, 91, 93, 140, 142, 144, 146, 148, 150, 152, 174, 176, 178, 180, 182, or 184. It is preferred that the nucleic acid fragment is longer than 10 nucleotides, such as at least 15, at least 20, at least 25, at least 30, at least 35, at least 40, at least 45, at least 50, at least 55, at least 60, at least 65, at least 70, and at least 80 nucleotides long, and the sequence identity should preferable also be higher than 70%, such as at least 75%, at least 80%, at least 85%, at least 90%, at least 92%, at least 94%, at least 96%, and at least 98%. It is most preferred that the sequence identity is 100%. Such fragments may be readily prepared by, for example, directly synthesizing the fragment by chemical means, by application of nucleic acid reproduction technology, such as the PCR technology of U.S. Patent 4,603,102, or by introducing selected sequences into recombinant vectors for recombinant production.

It is well known that the same amino acid may be encoded by various codons, the codon usage being related, *inter alia,* to the preference of the organisms in question expressing the nucleotide sequence. Thus, at least one nucleotide or codon of a nucleic acid fragment of the invention may be exchanged by others which, when expressed, result in a polypeptide identical or substantially identical to the polypeptide encoded by the nucleic acid fragment in question. The invention thus allows for variations in the sequence such as substitution, insertion (including introns), addition, deletion and rearrangement of one or more nucleotides, which variations do not have any substantial effect on the polypeptide encoded by the nucleic acid fragment or a subsequence thereof. The term "substitution" is intended to mean the replacement of one or more nucleotides in the full nucleotide sequence with one or more different nucleotides, "addition" is understood to mean the addition of one or more nucleotides at either end of the full nucleotide sequence, "insertion" is intended to mean the introduction of one or more nucleotides within the full nucleotide sequence, "deletion" is intended to indicate that one or more nucleotides have been deleted from the full nucleotide sequence whether at either end of the sequence or at any suitable point within it, and "rearrangement" is intended to mean that two or more nucleotide residues have been exchanged with each other.

The nucleotide sequence to be modified may be of cDNA or genomic origin as discussed above, but may also be of synthetic origin. Furthermore, the sequence may be of mixed cDNA and genomic, mixed cDNA and synthetic or genomic and synthetic origin as discussed above. The sequence may have been modified, e.g. by site-directed mutagenesis, to result in the desired nucleic acid fragment encoding the desired polypeptide. The following discussion focused on modifications of nucleic acid encoding the polypeptide should be understood to encompass also such possibilities, as well as the possibility of building up the nucleic acid by ligation of two or more DNA fragments to obtain the desired nucleic acid fragment, and combinations of the above-mentioned principles.

The nucleotide sequence may be modified using any suitable technique which results in the production of a nucleic acid fragment encoding a polypeptide of the invention.

The modification of the nucleotide sequence encoding the amino acid sequence of the polypeptide of the invention should be one which does not impair the immunological function of the resulting polypeptide.

A preferred method of preparing variants of the antigens disclosed herein is site-directed mutagenesis. This technique is useful in the preparation of individual peptides, or biologically functional equivalent proteins or peptides, derived from the antigen sequences, through specific mutagenesis of the underlying nucleic acid. The technique further provides a ready ability to prepare and test sequence variants, for example, incorporating one or more of the foregoing considerations, by introducing one or more nucleotide sequence changes into the nucleic acid. Site-specific mutagenesis allows the production of mutants through the use of specific oligonucleotide sequences which encode the nucleotide sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to 25 nucleotides in length is preferred, with about 5 to 10 residues on both sides of the junction of the sequence being altered.

In general, the technique of site-specific mutagenesis is well known in the art as exemplified by publications (Adelman et al., 1983). As will be appreciated, the technique typically employs a phage vector which exists in both a single stranded and double stranded form. Typical vectors useful in site-directed mutagenesis include vectors such as the M13 phage (Messing et al., 1981). These phage are readily commercially available and their use is generally well known to those skilled in the art.

In general, site-directed mutagenesis in accordance herewith is performed by first obtaining a single-stranded vector which includes within its sequence a nucleic acid sequence which encodes the polypeptides of the invention. An oligonucleotide primer bearing the desired mutated sequence is prepared, generally synthetically, for example by the method of Crea et al. (1978). This primer is then annealed with the single-stranded vector, and subjected to DNA polymerizing enzymes such as E. coli polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as E. coli cells, and clones are selected which include recombinant vectors bearing the mutated sequence arrangement.

The preparation of sequence variants of the selected nucleic acid fragments of the invention using site-directed mutagenesis is provided as a means of producing potentially useful species of the genes and is not meant to be limiting as there are other ways in which sequence variants of the nucleic acid fragments of the invention may be obtained. For example, recombinant vectors encoding the desired genes may be treated with mutagenic agents to obtain sequence variants (see, e.g., a method described by Eichenlaub, 1979) for the mutagenesis of plasmid DNA using hydroxylamine.

The invention also relates to a replicable expression vector which comprises a nucleic acid fragment defined above, especially a vector which comprises a nucleic acid fragment encoding a polypeptide fragment of the invention.

The vector may be any vector which may conveniently be subjected to recombinant DNA procedures, and the choice of vector will often depend on the host cell into which it is to be introduced. Thus, the vector may be an autonomously replicating vector, *i.e.* a vector which exists as an extrachromosomal entity, the replication of which is independent of chromosomal replication; examples of such a vector are a plasmid, phage, cosmid, mini-chromosome or virus. Alternatively, the vector may be one which, when introduced in a host cell, is integrated in the host cell genome and replicated together with the chromosome(s) into which it has been integrated.

Expression vectors may be constructed to include any of the DNA segments disclosed herein. Such DNA might encode an antigenic protein specific for virulent strains of mycobacteria or even hybridization probes for detecting mycobacteria nucleic acids in samples. Longer or shorter DNA segments could be used, depending on the antigenic protein desired. Epitopic regions of the proteins expressed or encoded by the disclosed DNA could be included as relatively short segments of DNA. A wide variety of expression vectors is possible including, for example, DNA segments encoding reporter gene products useful for identification of heterologous gene products and/or resistance genes such as antibiotic resistance genes which may be useful in identifying transformed cells.

The vector of the invention may be used to transform cells so as to allow propagation of the nucleic acid fragments of the invention or so as to allow expression of the polypeptide fragments of the invention. Hence, the invention also pertains to a transformed cell harbouring at least one such vector according to the invention, said cell being one which does not natively harbour the vector and/or the nucleic acid fragment of the invention contained therein. Such a transformed cell (which is also a part of the invention) may be any suitable bacterial host cell or any other type of cell such as a unicellular eukaryotic organism, a fungus or yeast, or a cell derived from a multicellular organism, e.g. an animal or a plant. It is especially in cases where glycosylation is desired that a mammalian cell is used, although glycosylation of proteins is a rare event in prokaryotes. Normally, however, a prokaryotic cell is preferred such as a bacterium belonging to the genera *Mycobacterium, Salmonella, Pseudomonas, Bacillus* and *Eschericia.* It is preferred that the transformed cell is an *E. coli*, *B. subtilis,* or *M. bovis* BCG cell, and it is especially preferred that the transformed cell expresses a polypeptide according of the invention. The latter opens for the possibility to produce the polypeptide of the invention by simply recovering it from the culture containing the transformed cell. In the most preferred embodiment of this part of the invention the transformed cell is *Mycobacterium bovis* BCG strain: Danish 1331, which is the *Mycobacterium bovis* strain Copenhagen from the Copenhagen BCG Laboratory, Statens Seruminstitut, Denmark.

The nucleic acid fragments of the invention allow for the recombinant production of the polypeptides fragments of the invention. However, also isolation from the natural source is a way of providing the polypeptide fragments as is peptide synthesis.

Therefore, the invention also pertains to a method for the preparation of a polypeptide fragment of the invention, said method comprising inserting a nucleic acid fragment as defined above into a vector which is able to replicate in a host cell, introducing the resulting recombinant vector into the host cell (transformed cells may be selected using various techniques, including screening by differential hybridization, identification of fused reporter gene products, resistance markers, anti-antigen antibodies and the like), culturing the host cell in a culture medium under conditions sufficient to effect expression of the polypeptide (of course the cell may be cultivated under conditions appropriate to the circumstances, and if DNA is desired, replication conditions are used), and recovering the polypeptide from the host cell or culture medium; or

isolating the polypeptide from a short-term culture filtrate as defined in claim 1; or isolating the polypeptide from whole mycobacteria of the tuberculosis complex or from lysates or fractions thereof, e.g. cell wall containing fractions, or

synthesizing the polypeptide by solid or liquid phase peptide synthesis.

The medium used to grow the transformed cells may be any conventional medium suitable for the purpose. A suitable vector may be any of the vectors described above, and an appropriate host cell may be any of the cell types listed above. The methods employed to construct the vector and effect introduction thereof into the host cell may be any methods known for such purposes within the field of recombinant DNA. In the following a more detailed description of the possibilities will be given:

In general, of course, prokaryotes are preferred for the initial cloning of nucleic sequences of the invention and constructing the vectors useful in the invention. For example, in addition to the particular strains mentioned in the more specific disclosure below, one may mention by way of example, strains such as *E. coli* K12 strain 294 (ATCC No. 31446), *E. coli B,* and *E. coli* X 1776 (ATCC No. 31537). These examples are, of course, intended to be illustrative rather than limiting.

Prokaryotes are also preferred for expression. The aforementioned strains, as well as *E. coli* W3110 (F-, lambda-, prototrophic, ATCC No. 273325), bacilli such as Bacillus subtilis, or other enterobacteriaceae such as Salmonella typhimurium or Serratia marcesans, and various Pseudomonas species may be used. Especially interesting are rapid-growing mycobacteria, e.g. M. *smegmatis*, as these bacteria have a high degree of resemblance with mycobacteria of the tuberculosis complex and therefore stand a good chance of reducing the need of performing post-translational modifications of the expression product.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species (see, e.g., Bolivar et al., 1977, Gene 2: 95). The pBR322 plasmid contains genes for ampicillin and tetracycline resistance and thus provides easy means for identifying transformed cells. The pBR plasmid, or other microbial plasmid or phage must also contain, or be modified to contain, promoters which can be used by the microorganism for expression.

Those promoters most commonly used in recombinant DNA construction include the B-lactamase (penicillinase) and lactose promoter systems (Chang et al., 1978; Itakura et al., 1977; Goeddel et al., 1979) and a tryptophan (trp) promoter system (Goeddel et al., 1979; EPO Appl. Publ. No. 0036776). While these are the most commonly used, other microbial promoters have been discovered and utilized, and details concerning their nucleotide sequences have been published, enabling a skilled worker to ligate them functionally with plasmid vectors (Siebwenlist et al., 1980). Certain genes from prokaryotes may be expressed efficiently in *E. coli* from their own promoter sequences, precluding the need for addition of another promoter by artificial means.

After the recombinant preparation of the polypeptide according to the invention, the isolation of the polypeptide may for instance be carried out by affinity chromatography (or other conventional biochemical procedures based on chromatography), using a monoclonal antibody which substantially specifically binds the polypeptide according to the invention. Another possibility is to employ the simultaneous electroelution technique described by Andersen *et al.* in J. Immunol. Methods **161**: 29-39.

According to the invention the post-translational modifications involves lipidation, glycosylation, cleavage, or elongation of the polypeptide.

In certain aspects, the DNA sequence information provided by this invention allows for the preparation of relatively short DNA (or RNA or PNA) sequences having the ability to specifically hybridize to mycobacterial gene sequences. In these aspects, nucleic acid probes of an appropriate length are prepared based on a consideration of the relevant sequence. The ability of such nucleic acid probes to specifically hybridize to the mycobacterial gene sequences lend them particular utility in a variety of embodiments. Most importantly, the probes can be used in a variety of diagnostic assays for detecting the presence of pathogenic organisms in a given sample. However, either uses are envisioned, including the use of the sequence information for the preparation of mutant species primers, or primers for use in preparing other genetic constructs.

Apart from their use as starting points for the synthesis of polypeptides of the invention and for hybridization probes (useful for direct hybridization assays or as primers in e.g. PCR or other molecular amplification methods) the nucleic acid fragments of the invention may be used for effecting *in vivo* expression of antigens, *i.e.* the nucleic acid fragments may be used in so-called DNA vaccines. Recent research have revealed that a DNA fragment cloned in a vector which is non- replicative in eukaryotic cells may be introduced into an animal (including a human being) by e. g. intramuscular injection or percutaneous administration (the so-called "gene gun" approach). The DNA is taken up by e.g. muscle cells and the gene of interest is expressed by a promoter which is functioning in eukaryotes, e.g. a viral promoter, and the gene product thereafter stimulates the immune system. These newly discovered methods are reviewed in Ulmer et al., 1993, which hereby is included by reference.

Hence, the invention also relates to a vaccine comprising a nucleic acid fragment according to the invention, the vaccine effecting *in vivo* expression of antigen by an animal, including a human being, to whom the vaccine has been administered, the amount of expressed antigen being effective to confer substantially increased resistance to infections with mycobacteria of the tuberculosis complex in an animal, including a human being.

The efficacy of such a "DNA vaccine" can possibly be enhanced by administering the gene encoding the expression product together with a DNA fragment encoding a polypeptide which has the capability of modulating an immune response. For instance, a gene encoding lymphokine precursors or lymphokines (e.g. IFN-γ, IL-2, or IL-12) could be administered together with the gene encoding the immunogenic protein, either by administering two separate DNA fragments or by administering both DNA fragments included in the same vector. It also is a possibility to administer DNA fragments comprising a multitude of nucleotide sequences which each encode relevant epitopes of the polypeptides disclosed herein so as to effect a continuous sensitization of the immune system with a broad spectrum of these epitopes.

As explained above, the polypeptide fragments of the invention are excellent candidates for vaccine constituents or for constituents in an immune diagnostic agent due to their extracellular presence in culture media containing metabolizing virulent mycobacteria belonging to the tuberculosis complex, or because of their high homologies with such extracellular antigens, or because of their absence in *M. bovis* BCG.

Thus, another part of the invention pertains to an immunologic composition comprising a polypeptide or fusion polypeptide according to the invention. In order to ensure optimum performance of such an immunologic composition it is preferred that it comprises an immunologically and pharmaceutically acceptable carrier, vehicle or adjuvant.

Suitable carriers are selected from the group consisting of a polymer to which the polypeptide(s) is/are bound by hydrophobic non-covalent interaction, such as a plastic, e.g. polystyrene, or a polymer to which the polypeptide(s) is/are covalently bound, such as a polysaccharide, or a polypeptide, e.g. bovine serum albumin, ovalbumin or keyhole limpet haemocyanin. Suitable vehicles are selected from the group consisting of a diluent and a suspending agent. The adjuvant is preferably selected from the group consisting of dimethyldioctadecylammonium bromide (DDA), Quil A, poly I:C, Freund's incomplete adjuvant, IFN-γ, IL-2, IL-12, monophosphoryl lipid A (MPL), and muramyl dipeptide (MDP).

A preferred immunologic composition according to the present invention comprising at least two different polypeptide fragments, each different polypeptide fragment being a polypeptide or a fusion polypeptide defined above. It is preferred that the immunologic composition comprises between 3-20 different polypeptide fragments or fusion polypeptides.

Such an immunologic composition may preferably be in the form of a vaccine or in the form of a skin test reagent.

In line with the above, the invention therefore also pertain to a method for producing an immunologic composition according to the invention, the method comprising preparing, synthesizing or isolating a polypeptide according to the invention, and solubilizing or dispersing the polypeptide in a medium for a vaccine, and optionally adding other *M. tuberculosis* antigens and/or a carrier, vehicle and/or adjuvant substance.

Preparation of vaccines which contain peptide sequences as active ingredients is generally well understood in the art, as exemplified by U.S. Patents 4,608,251; 4,601,903; 4,599,231; 4,599,230; 4,596,792; and 4,578,770, all incorporated herein by reference. Typically, such vaccines are prepared as injectables either as liquid solutions or suspensions; solid forms suitable for solution in, or suspension in, liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkalene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%.

The proteins may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired. Suitable dosage ranges are of the order of several hundred micrograms active ingredient per vaccination with a preferred range from about 0.1 µg to 1000 µg, such as in the range from about 1 µg to 300 µg, and especially in the range from about 10 µg to 50 µg. Suitable regimens for initial administration and booster shots are also variable but are typified by an initial administration followed by subsequent inoculations or other administrations.

The manner of application may be varied widely. Any of the conventional methods for administration of a vaccine are applicable. These are believed to include oral application on a solid physiologically acceptable base or in a physiologically acceptable dispersion, parenterally, by injection or the like. The dosage of the vaccine will depend on the route of administration and will vary according to the age of the person to be vaccinated and, to a lesser degree, the size of the person to be vaccinated.

Some of the polypeptides of the vaccine are sufficiently immunogenic in a vaccine, but for some of the others the immune response will be enhanced if the vaccine further comprises an adjuvant substance.

Various methods of achieving adjuvant effect for the vaccine include use of agents such as aluminum hydroxide or phosphate (alum), commonly used as 0.05 to 0.1 percent solution in phosphate buffered saline, admixture with synthetic polymers of sugars (Carbopol) used as 0.25 percent solution, aggregation of the protein in the vaccine by heat treatment with temperatures ranging between 70° to 101°C for 30 second to 2 minute periods respectively. Aggregation by reactivating with pepsin treated (Fab) antibodies to albumin, mixture with bacterial cells such as C. parvum or endotoxins or lipopolysaccharide components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. According to the invention DDA (dimethyldioctadecylammonium bromide) is an interesting candidate for an adjuvant, but also Freund's complete and incomplete adjuvants as well as QuilA and RIBI are interesting possibilities. Further possibilities are monophosphoryl lipid A (MPL), and muramyl dipeptide (MDP).

Another highly interesting (and thus, preferred) possibility of achieving adjuvant effect is to employ the technique described in Gosselin *et al.*, 1992 (which is hereby incorporated by reference herein). In brief, the presentation of a relevant antigen such as an antigen of the present invention can be enhanced by conjugating the antigen to antibodies (or antigen binding antibody fragments) against the Fcγ receptors on monocytes/macrophages. Especially conjugates between antigen and anti-FcγRI have been demonstrated to enhance immunogenicity for the purposes of vaccination.

Other possibilities involve the use of immune modulating substances such as lymphokines (e.g. IFN-γ, IL-2 and IL-12) or synthetic IFN-γ inducers such as poly I:C in combination with the above-mentioned adjuvants. As discussed in example 3, it is contemplated that such mixtures of antigen and adjuvant will lead to superior vaccine formulations.

In many instances, it will be necessary to have multiple administrations of the vaccine, usually not exceeding six vaccinations, more usually not exceeding four vaccinations and preferably one or more, usually at least about three vaccinations. The vaccinations will normally be at from two to twelve week intervals, more usually from three to five week intervals. Periodic boosters at intervals of 1-5 years, usually three years, will be desirable to maintain the desired levels of protective immunity. The course of the immunization may be followed by *in vitro* proliferation assays of PBL (peripheral blood lymphocytes) co-cultured with ESAT-6 or ST-CF, and especially by measuring the levels of IFN-γ released form the primed lymphocytes. The assays may be performed using conventional labels, such as radionuclides, enzymes, fluorescers, and the like. These techniques are well known and may be found in a wide variety of patents, such as U.S. Patent Nos. 3,791,932; 4,174,384 and 3,949,064, as illustrative of these types of assays.

Due to genetic variation, different individuals may react with immune responses of varying strength to the same polypeptide. Therefore, the vaccine according to the invention may comprise several different polypeptides in order to increase the immune response. The vaccine may comprise two or more polypeptides, where all of the polypeptides are as defined above, or some but not all of the peptides may be derived from a bacterium belonging to the *M. tuberculosis* complex. In the latter example the polypeptides not necessarily fulfilling the criteria set forth above for polypeptides may either act due to their own immunogenicity or merely act as adjuvants. Examples of such interesting polypeptides are MPB64, MPT64, and MPB59, but any other substance which can be isolated from mycobacteria are possible candidates.

The vaccine may comprise 3-20 different polypeptides, such as 3-10 different polypeptides.

One reason for admixing the polypeptides of the invention with an adjuvant is to effectively activate a cellular immune response. However, this effect can also be achieved in other ways, for instance by expressing the effective antigen in a vaccine in a non-pathogenic microorganism. A well-known example of such a microorganism is *Mycobacterium bovis* BCG.

Therefore, another important aspect of the present invention is an improvement of the living BCG vaccine presently available, which is a vaccine for immunizing an animal, including a human being, against TB caused by mycobacteria belonging to the tuberculosis-complex, comprising as the effective component a microorganism, wherein one or more copies of a DNA sequence encoding a polypeptide as defined above has been incorporated into the genome of the microorganism in a manner allowing the microorganism to express and secrete the polypeptide.

In the present context the term "genome" refers to the chromosome of the microorganisms as well as extrachromosomally DNA or RNA, such as plasmids. It is, however, preferred that the DNA sequence of the present invention has been introduced into the chromosome of the non-pathogenic microorganism, since this will prevent loss of the genetic material introduced.

It is preferred that the non-pathogenic microorganism is a bacterium, e.g. selected from the group consisting of the genera *Mycobacterium, Salmonella, Pseudomonas* and *Eschericia.* It is especially preferred that the non-pathogenic microorganism is *Mycobacterium bovis* BCG, such as *Mycobacterium bovis* BCG strain: Danish 1331.

The incorporation of one or more copies of a nucleotide sequence encoding the polypeptide according to the invention in a mycobacterium from a *M. bovis* BCG strain will enhance the immunogenic effect of the BCG strain. The incorporation of more than one copy of a nucleotide sequence of the invention is contemplated to enhance the immune response even more, and consequently an aspect of the invention is a vaccine wherein at least 2 copies of a DNA sequence encoding a polypeptide is incorporated in the genome of the microorganism, such as at least 5 copies. The copies of DNA sequences may either be identical encoding identical polypeptides or be variants of the same DNA sequence encoding identical or homologues of a polypeptide, or in another embodiment be different DNA sequences encoding different polypeptides where at least one of the polypeptides is according to the present invention.

The living vaccine of the invention can be prepared by cultivating a transformed non-pathogenic cell according to the invention, and transferring these cells to a medium for a vaccine, and optionally adding a carrier, vehicle and/or adjuvant substance.

The invention also relates to a method of diagnosing TB caused by *Mycobacterium tuberculosis*, *Mycobacterium africanum or Mycobacterium bovis* in an animal, including a human being, comprising intradermally injecting, in the animal, a polypeptide according to the invention or a skin test reagent described above, a positive skin response at the location of injection being indicative of the animal having TB, and a negative skin response at the location of injection being indicative of the animal not having TB. A positive response is a skin reaction having a diameter of at least 5 mm, but larger reactions are preferred, such as at least 1 cm, 1.5 cm, and at least 2 cm in diameter. The composition used as the skin test reagent can be prepared in the same manner as described for the vaccines above.

In line with the disclosure above pertaining to vaccine preparation and use, the invention also pertains to a method for immunising an animal, including a human being, against TB caused by mycobacteria belonging to the tuberculosis complex, comprising administering to the animal the polypeptide of the invention, or a vaccine composition of the invention as described above, or a living vaccine described above. Preferred routes of administration are the parenteral (such as intravenous and intraarterially), intraperitoneal, intramuscular, subcutaneous, intradermal, oral, buccal, sublingual, nasal, rectal or transdermal route.

The protein ESAT-6 which is present in short-term culture filtrates from mycobacteria as well as the *esat*-6 gene in the mycobacterial genome has been demonstrated to have a very limited distribution in other mycobacterial strains that *M. tuberculosis,* e.g. *esat-6* is absent in both BCG and the majority of mycobacterial species isolated from the environment, such as *M. avium* and *M. terrae.* It is believed that this is also the case for at least one of the antigens of the present invention and their genes and therefore, the diagnostic embodiments of the invention are especially well-suited for performing the diagnosis of on-going or previous infection with *virulent* mycobacterial strains of the tuberculosis complex, and it is contemplated that it will be possible to distinguish between 1) subjects (animal or human) which have been previously vaccinated with e.g. BCG vaccines or subjected to antigens from non-virulent mycobacteria and 2) subjects which have or have had active infection with virulent mycobacteria.

A number of possible diagnostic assays and methods can be envisaged:

When diagnosis of previous or ongoing infection with virulent mycobacteria is the aim, a blood sample comprising mononuclear cells (*i.a.* T-lymphocytes) from a patient could be contacted with a sample of one or more polypeptides of the invention. This contacting can be performed *in vitro* and a positive reaction could e.g. be proliferation of the T-cells or release cytokines such as γ-interferon into the extracellular phase (e.g. into a culture supernatant); a suitable *in vivo* test would be a skin test as described above. It is also conceivable to contact a serum sample from a subject to contact with a polypeptide of the invention, the demonstration of a binding between antibodies in the serum sample and the polypeptide being indicative of previous or ongoing infection.

The invention therefore also relates to an *in vitro* method for diagnosing ongoing or previous sensitization in an animal or a human being with bacteria belonging to the tuberculosis complex, the method comprising providing a blood sample from the animal or human being, and contacting the sample from the animal with the polypeptide of the invention, a significant release into the extracellular phase of at least one cytokine by mononuclear cells in the blood sample being indicative of the animal being sensitized. By the term "significant release" is herein meant that the release of the cytokine is significantly higher than the cytokine release from a blood sample derived from a non-tuberculous subject (e.g. a subject which does not react in a traditional skin test for TB). Normally, a significant release is at least two times the release observed from such a sample.

Alternatively, a sample of a possibly infected organ may be contacted with an antibody raised against a polypeptide of the invention. The demonstration of the reaction by means of methods well-known in the art between the sample and the antibody will be indicative of ongoing infection. It is of course also a possibility to demonstrate the presence of anti-mycobacterial antibodies in serum by contacting a serum sample from a subject with at least one of the polypeptide fragments of the invention and using well-known methods for visualizing the reaction between the antibody and antigen.

Also a method of determining the presence of mycobacterial nucleic acids in an animal, including a human being, or in a sample, comprising administering a nucleic acid fragment of the invention to the animal or incubating the sample with the nucleic acid fragment of the invention or a nucleic acid fragment complementary thereto, and detecting the presence of hybridized nucleic acids resulting from the incubation (by using the hybridization assays which are well-known in the art), is also included in the invention. Such a method of diagnosing TB might involve the use of a composition comprising at least a part of a nucleotide sequence as defined above and detecting the presence of nucleotide sequences in a sample from the animal or human being to be tested which hybridize with the nucleic acid fragment (or a complementary fragment) by the use of PCR technique.

The fact that certain of the disclosed antigens are not present in *M. bovis* BCG but are present in virulent mycobacteria point them out as interesting drug targets; the antigens may constitute receptor molecules or toxins which facilitate the infection by the mycobacterium, and if such functionalities are blocked the infectivity of the mycobacterium will be diminshed.

To determine particularly suitable drug targets among the antigens of the invention, the gene encoding at least one of the polypeptides of the invention and the necessary control sequences can be introduced into avirulent strains of mycobacteria (e.g. BCG) so as to determine which of the polypeptides are critical for virulence. Once particular proteins are identified as critical for/contributory to virulence, anti-mycobacterial agents can be designed rationally to inhibit expression of the critical genes or to attack the critical gene products. For instance, antibodies or fragments thereof (such as Fab and (Fab')$_2$ fragments can be prepared against such critical polypeptides by methods known in the art and thereafter used as prophylactic or therapeutic agents. Alternatively, small molecules can be screened for their ability to selectively inhibit expression of the critical gene products, e.g. using recombinant expression systems which include the gene's endogenous promoter, or for their ability to directly interfere with the action of the target. These small molecules are then used as therapeutics or as prophylactic agents to inhibit mycobacterial virulence.

Alternatively, anti-mycobacterial agents which render a virulent mycobacterium avirulent can be operably linked to expression control sequences and used to transform a virulent mycobacterium. Such anti-mycobacterial agents inhibit the replication of a specified mycobacterium upon transcription or translation of the agent in the mycobacterium. Such a "newly avirulent" mycobacterium would constitute a superb alternative to the above described modified BCG for vaccine purposes since it would be immunologically very similar to a virulent mycobacterium compared to e.g. BCG.

Finally, a monoclonal or polyclonal antibody, which is specifically reacting with a polypeptide of the invention in an immuno assay, or a specific binding fragment of said antibody, is also a part of the invention. The production of such polyclonal antibodies requires that a suitable animal be immunized with the polypeptide and that these antibodies are subsequently isolated, suitably by immune affinity chromatography. The production of monoclonals can be effected by methods well-known in the art, since the present invention provides for adequate amounts of antigen for both immunization and screening of positive hybridomas.

LEGENDS TO THE FIGURES

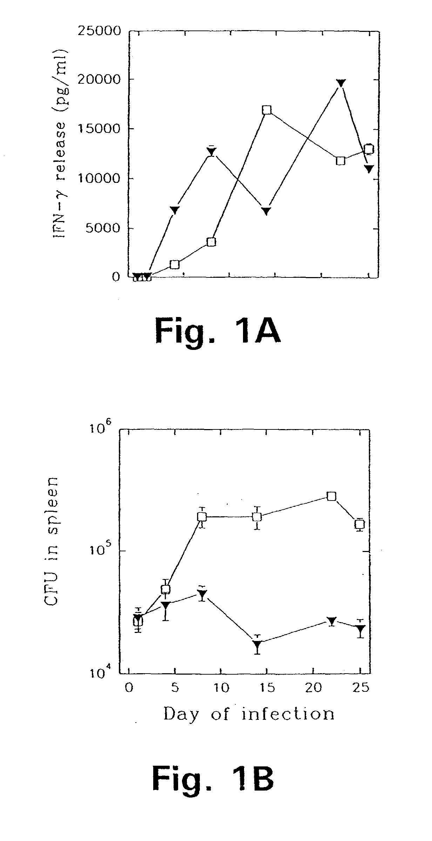

Fig. 1: Long term memory immune mice are very efficiently protected towards an infection with *M. tuberculosis.*

Mice were given a challenge of *M. tuberculosis* and spleens were isolated at different time points. Spleen lymphocytes were stimulated *in vitro* with ST-CF and the release of IFN-$\gamma$ investigated (panel A). The counts of CFU in the spleens of the two groups of mice are indicated in panel B. The memory immune mice control infection within the first week and produce large quantities of IFN-$\gamma$ in response to antigens in ST-CF.

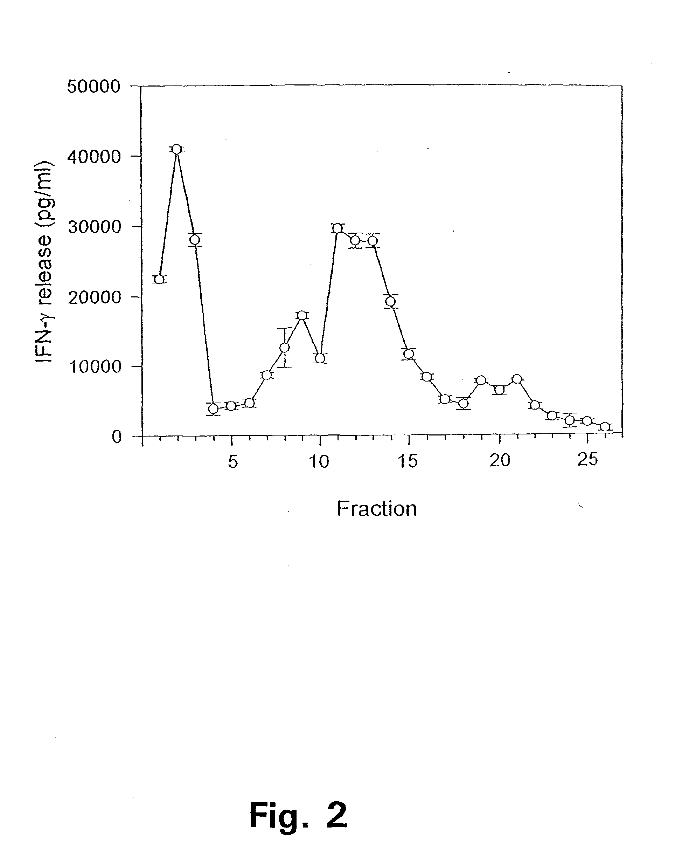

Fig. 2: T cells involved in protective immunity are predominantly directed to molecules from 6-12 and 17-38 kDa. Splenic T cells were isolated four days after the challenge with *M. tuberculosis* and stimulated *in vitro* with narrow molecular mass fractions of ST-CF. The release of IFN-$\gamma$ was investigated

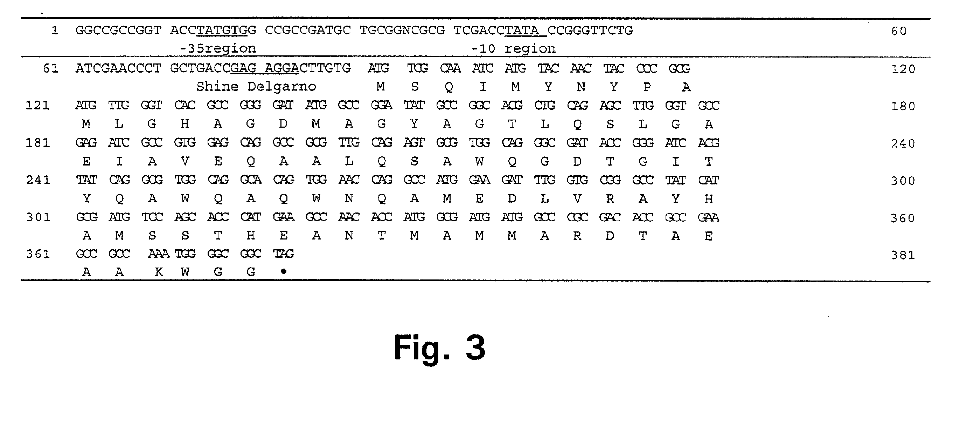

Fig. 3: Nucleotide sequence (SEQ ID NO: 1) of *cfp7*. The deduced amino acid sequence (SEQ ID NO: 2) of CFP7 is given in conventional one-letter code below the nucleotide sequence. The putative ribosome-binding site is written in underlined italics as are the putative -10 and -35 regions. Nucleotides written in bold are those encoding CFP7.

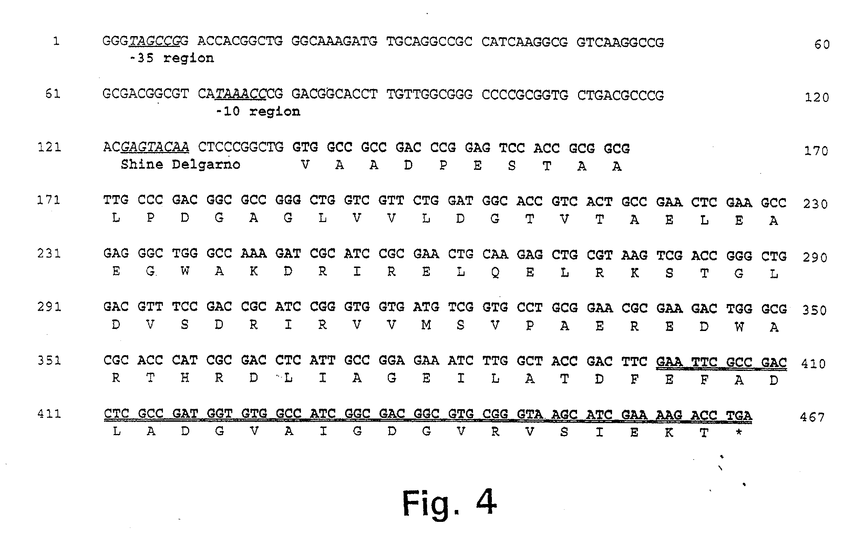

Fig. 4. Nucleotide sequence (SEQ ID NO: 3) of *cfp9.* The deduced amino acid sequence (SEQ ID NO: 4) of CFP9 is given in conventional one-letter code below the nucleotide sequence. The putative ribosome-binding site Shine Delgarno sequence is written in underlined italics as are the putative -10 and -35 regions. Nucleotides in bold writing are those encoding CFP9. The nucleotide sequence obtained from the lambda 226 phage is double underlined.

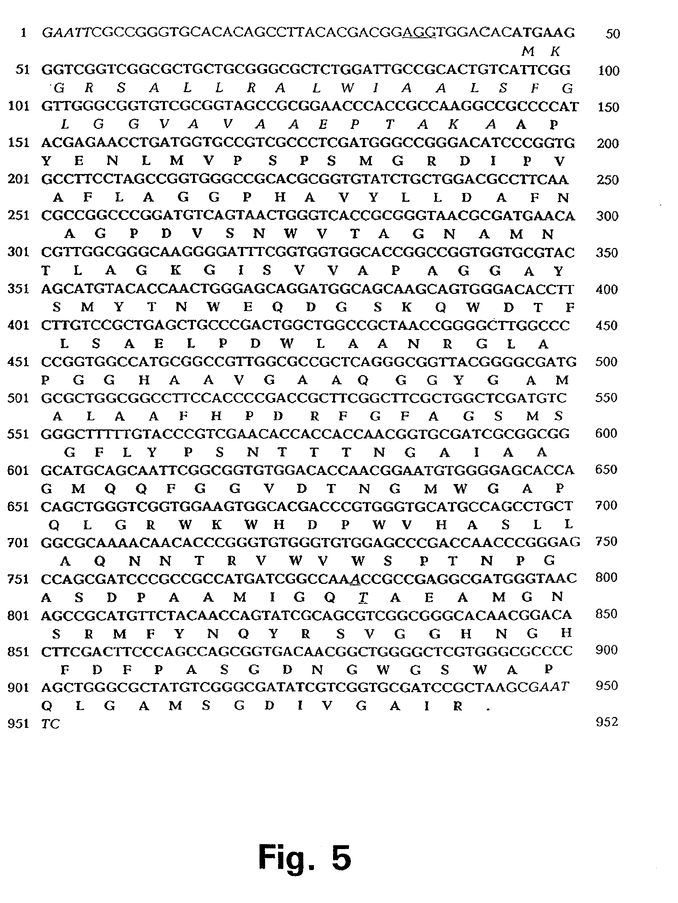

Fig. 5: Nucleotide sequence of *mpt51*. The deduced amino acid sequence of MPT51 is given in a one-letter code below the nucleotide sequence. The signal is indicated in italics. The putative potential ribosome-binding site is underlined. The nucleotide difference and amino acid difference compared to the nucleotide sequence of MPB51 (Ohara *et al*., 1995) are underlined at position 780. The nucleotides given in italics are not present in *M. tuberculosis* H37Rv.

Fig. 6: the position of the purified antigens in the 2DE system have been determined and mapped in a reference gel. The newly purified antigens are encircled and the position of well-known proteins are also indicated.

EXAMPLE 1

*Identification of single culture filtrate antigens involved in protective immunity*

A group of efficiently protected mice was generated by infecting 8-12 weeks old female C57Bl/6j mice with 5 x 10$^4$ *M. tuberculosis* i.v. After 30 days of infection the mice were subjected to 60 days of antibiotic treatment with isoniazid and were then left for 200-240 days to ensure the establishment of resting long-term memory immunity. Such memory immune mice are very efficiently protected against a secondary infection (Fig. 1). Long lasting immunity in this model is mediated by a population of highly reactive CD4 cells recruited to the site of infection and triggered to produce large amounts of IFN-$\gamma$ in response to ST-CF (Fig. 1) (Andersen *et al*. 1995).

We have used this model to identify single antigens recognized by protective T cells. Memory immune mice were reinfected with 1 x 10$^6$ *M. tuberculosis* i.v. and splenic lymphocytes were harvested at day 4-6 of reinfection, a time point where this population is highly reactive to ST-CF. The antigens recognized by these T cells were mapped by the multi-elution technique (Andersen and Heron, 1993). This technique divides complex protein mixtures separated in SDS-PAGE into narrow fractions in a physiological buffer. These fractions were used to stimulate spleen lymphocytes *in vitro* and the release of IFN-$\gamma$ was monitored (Fig. 2). Long-term memory immune mice did not recognize these fractions before TB infection, but splenic lymphocytes obtained during the recall of protective immunity recognized a range of culture filtrate antigens and peak production of IFN-$\gamma$ was found in response to proteins of apparent molecular weight 6-12 and 17-30 kDa (Fig. 2). It is therefore concluded that culture filtrate antigens within these regions are the major targets recognized by memory effector T-cells triggered to release IFN-$\gamma$ during the first phase of a protective immune response.

EXAMPLE 2

*Cloning of genes expressing low mass culture filtrate antigens*

In example 1 it was demonstrated that antigens in the low molecular mass fraction are recognized strongly by cells isolated from memory immune mice. Monoclonal antibodies (mAbs) to these antigens were therefore generated by immunizing with the low mass fraction in RIBI adjuvant (first and second immunization) followed by two injections with the fractions in aluminium hydroxide. Fusion and cloning of the reactive cell lines were done according to standard procedures (Kohler and Milstein 1975). The procedure resulted in the provision of two mAbs: ST-3 directed to a 9 kDa culture filtrate antigen (CFP9) and PV-2 directed to a 7 kDa antigen (CFP7), when the molecular weight is estimated from migration of the antigens in an SDS-PAGE.

In order to identify the antigens binding to the Mab's, the following experiments were carried out:

The recombinant $\lambda$gt11 *M. tuberculosis* DNA library constructed by R. Young (Young, R.A. *et al*. 1985) and obtained through the World Health Organization IMMTUB programme (WHO.0032.wibr) was screened for phages expressing gene products which would bind the monoclonal antibodies ST-3 and PV-2.

Approximately 1 x 10$^5$ pfu of the gene library (containing approximately 25% recombinant phages) were plated on *Eschericia coli* Y 1090 (DlacU 169, proA$^+$, Dlon, araD139, supF, trpC22::*tn10* [pMC9] ATCC#37197) in soft agar and incubated for 2,5 hours at 42°C.

The plates were overlaid with sheets of nitrocellulose saturated with isopropyl-β-D-thiogalactopyranoside and incubation was continued for 2,5 hours at 37°C. The nitrocellulose was removed and incubated with samples of the monoclonal antibodies in PBS with Tween 20 added to a final concentration of 0.05%. Bound monoclonal antibodies were visualized by horseradish peroxidase-conjugated rabbit anti-mouse immunoglobulins (P260, Dako, Glostrup, DK) and a staining reaction involving 5,5',3,3'-tetramethylbenzidine and $H_2O_2$.

Positive plaques were recloned and the phages originating from a single plaque were used to lysogenize *E. coli* Y 1089 (DlacU169, proA$^+$, Dlon, araD139, strA, hfl150 [chr::*tn10*] [pMC9] ATCC nr. 37196). The resultant lysogenic strains were used to propagate phage particles for DNA extraction. These lysogenic *E. coli* strains have been named:

AA226 (expressing ST-3 reactive polypeptide CFP9) which has been deposited 28 June 1993 with the collection of Deutsche Sammlung von Mikroorganismen und Zell-kulturen GmbH (DSM) under the accession number DSM 8377 and in accordance with the provisions of the Budapest Treaty, and

AA242 (expressing PV-2 reactive polypeptide CFP7) which has been deposited 28 June 1993 with the collection of Deutsche Sammlung von Mikroorganismen und Zell-kulturen GmbH (DSM) under the accession number DSM 8379 and in accordance with the provisions of the Budapest Treaty.

These two lysogenic *E. coli* strains are disclosed in WO 95/01441 as are the mycobacterial polypeptide products expressed thereby. However, no information concerning the amino acid sequences of these polypeptides or their genetic origin are given, and therefore only the direct expression products of AA226 and AA242 are made available to the public.

The st-3 binding protein is expressed as a protein fused to β-galactosidase, whereas the pv-2 binding protein appears to be expressed in an unfused version.

Sequencing of the nucleotide sequence encoding the PV-2 and ST-3 binding protein

In order to obtain the nucleotide sequence of the gene encoding the pv-2 binding protein, the approximately 3 kb *M. tuberculosis* derived *EcoR*I - *EcoR*I fragment from AA242 was subcloned in the *EcoR*I site in the pBluescriptSK + (Stratagene) and used to transform *E. coli* XL-1 Blue (Stratagene).

Similarly, to obtain the nucleotide sequence of the gene encoding the st-3 binding protein, the approximately 5 kb *M. tuberculosis* derived *EcoR*I - *EcoR*I fragment from AA226 was subcloned in the *EcoR*I site in the pBluescriptSK + (Stratagene) and used to transform *E. coli* XL-1 Blue (Stratagene).

The complete DNA sequence of both genes were obtained by the dideoxy chain termination method adapted for supercoiled DNA by use of the Sequenase DNA sequencing kit version 1.0 (United States Biochemical Corp., Cleveland, OH) and by cycle sequencing using the Dye Terminator system in combination with an automated gel reader (model 373A; Applied Biosystems) according to the instructions provided. The sequences DNA are shown in SEQ ID NO: 1 (CFP7) and in SEQ ID NO: 3 (CFP9) as well as in Figs. 3 and 4, respectively. Both strands of the DNA were sequenced.

CFP7

An open reading frame (ORF) encoding a sequence of 96 amino acid residues was identified from an ATG start codon at position 91-93 extending to a TAG stop codon at position 379-381. The deduced amino acid sequence is shown in SEQ ID NO: 2 (and in Fig. 3 where conventional one-letter amino acid codes are used).

CFP7 appear to be expressed in *E. coli* as an unfused version. The nucleotide sequence at position 78-84 is expected to be the Shine Delgarno sequence and the sequences from position 47-50 and 14-19 are expected to be the -10 and -35 regions, respectively:

CFP9

The protein recognised by ST-3 was produced as a β-galactosidase fusion protein, when expressed from the AA226 lambda phage. The fusion protein had an approx. size of 116 - 117kDa (Mw for β-galactosidase 116.25 kDa) which may suggest that only part of the CFP9 gene was included in the lambda clone (AA226).

Based on the 90 bp nucleotide sequence obtained on the insert from lambda phage AA226, a search of homology to the nucleotide sequence of the *M. tuberculosis* genome was performed in the Sanger database (Sanger *Mycobacterium tuberculosis* database):

http://www.sanger.ac.uk/pathogens/TB-blast-server.html;

Williams, 1996). 100% identity to the cloned sequence was found on the MTCY48 cosmid. An open reading frame (ORF) encoding a sequence of 109 amino acid residues was identified from a GTG start codon at position 141 - 143 extending to a TGA stop codon at position 465 - 467. The deduced amino acid sequence is shown in Fig. 4 using conventional one letter code.

The nucleotide sequence at position 123 - 130 is expected to be the Shine Delgarno sequence and the sequences from position 73 - 78 and 4 - 9 are expected to be the - 10 and -35 region respectively (Fig. 4). The ORF overlapping with the 5'-end of the sequence of AA229 is shown in Fig. 4 by double underlining.

Subcloning CFP7 and CFP9 in expression vectors

The two ORFs encoding CFP7 and CFP9 were PCR cloned into the pMST24 (Theisen *et al*., 1995) expression vector pRVN01 or the pQE-32 (QIAGEN) expression vector pRVN02, respectively.

The PCR amplification was carried out in a thermal reactor (Rapid cycler, Idaho Technology, Idaho) by mixing 10 ng plasmid DNA with the mastermix (0.5 µM of each oligonucleotide primer, 0.25 µM BSA (Stratagene), low salt buffer (20 mM Tris-HCl, pH 8.8, 10 mM KCl, 10 mM $(NH_4)_2SO_4$, 2 mM $MgSO_4$ and 0,1 % Triton X-100) (Stratagene), 0.25 mM of each deoxynucleoside triphosphate and 0.5 U Taq Plus Long DNA polymerase (Stratagene)). Final volume was 10 µl (all concentrations given are concentrations in the final volume). Predenaturation was carried out at 94°C for 30 s. 30 cycles of the following was performed; Denaturation at 94°C for 30 s, annealing at 55°C for 30 s and elongation at 72°C for 1 min.

The oligonucleotide primers were synthesised automatically on a DNA synthesizer (Applied Biosystems, Forster City, Ca, ABI-391, PCR-mode), deblocked, and purified by ethanol precipitation.

The *cfp7* oligonucleotides (TABLE 1) were synthesised on the basis of the nucleotide sequence from the CFP7 sequence (Fig. 3). The oligonucleotides were engineered to include an *Sma*I restriction enzyme site at the 5' end and a *Bam*HI restriction enzyme site at the 3' end for directed subcloning.

The *cfp9* oligonucleotides (TABLE 1) were synthesized partly on the basis of the nucleotide sequence from the sequence of the AA229 clone and partly from the identical sequence found in the Sanger database cosmid MTCY48 (Fig. 4). The oligonucleotides were engineered to include a *Sma*I restriction enzyme site at the 5' end and a *Hind*III restriction enzyme site at the 3' end for directed subcloning.

CFP7

By the use of PCR a *Sma*I site was engineered immediately 5' of the first codon of the ORF of 291 bp, encoding the *cfp7* gene, so that only the coding region would be expressed, and a *Bam*HI site was incorporated right after the stop codon at the 3' end. The 291 bp PCR fragment was cleaved by *Sma*I and *Bam*HI, purified from an agarose gel and subcloned into the *Sma*I - *Bam*HI sites of the pMST24 expression vector. Vector DNA containing the gene fusion was used to transform the *E. coli* XL1-Blue (pRVN01).

CFP9

By the use of PCR a *Sma*I site was engineered immediately 5' of the first codon of an ORF of 327 bp, encoding the *cfp9* gene, so that only the coding region would be expressed, and a *Hind*III site was incorporated after the stop codon at the 3' end. The 327 bp PCR fragment was cleaved by *Sma*I and *Hind*III, purified from an agarose gel, and subcloned into the *Sma*I - *Hind*III sites of the pQE-32 (QIAGEN) expression vector.

Vector DNA containing the gene fusion was used to transform the *E. coli* XL1-Blue (pRVN02).

Purification of recombinant CFP7 and CFP9

The ORFs were fused N-terminally to the $(His)_6$-tag (cf. EP-A-0 282 242). Recombinant antigen was prepared as follows: Briefly, a single colony of *E. coli* harbouring either the pRVN01 or the pRVN02 plasmid, was inoculated into Luria-Bertani broth containing 100 µg/ml ampicillin and 12.5 µg/ml tetracycline and grown at 37°C to $OD_{600nm}$ = 0.5. IPTG (isopropyl-β-D-thiogalactoside) was then added to a final concentration of 2 mM (expression was regulated either by the strong IPTG inducible $P_{tac}$ or the T5 promoter) and growth was continued for further 2 hours. The cells were harvested by centrifugation at 4,200 x g at 4°C for 8 min. The pelleted bacteria were stored overnight at -20°C. The pellet was resuspended in BC 40/100 buffer (20 mM Tris-HCl pH 7.9, 20% glycerol, 100 mM KCl, 40 mM Imidazole) and cells were broken by sonication (5 times for 30 s with intervals of 30 s) at 4°C. followed by centrifugation at 12,000 x g for 30 min at 4°C, the supernatant (crude extract) was used for purification of the recombinant antigens.

The two Histidine fusion proteins (His-rCFP7 and His-rCFP9) were purified from the crude extract by affinity chromatography on a Ni$^{2+}$-NTA column from QIAGEN with a volume of 100 ml. His-rCFP7 and His-rCFP9 binds to Ni$^{2+}$. After extensive washes of the column in BC 40/100 buffer, the fusion protein was eluted with a BC 1000/100 buffer containing 100 mM imidazole, 20 mM Tris pH 7.9, 20% glycerol and 1 M KCl. subsequently, the purified products were dialysed extensively against 10 mM Tris pH 8.0. His-rCFP7 and His-rCFP9 were then separated from contaminants by fast protein liquid chromatography (FPLC) over an anion-exchange column (Mono Q, Pharmacia, Sweden). in 10 mM Tris pH 8.0 with a linear gradient of NaCl from 0 to 1 M. Aliquots of the fractions were analyzed by 10%-20% gradient sodium dodecyl sulphate polyacrylamide gel electrophoresis (SDS-PAGE). Fractions containing purified either purified His-rCFP7 or His-rCFP9 were pooled.

TABLE 1. Sequence of the *cfp7* and *cfp9* oligonucleotides[a].

| Orientation and oligonucleotide | Sequences (5' → 3') | Position[b] (nucleotide) |
|---|---|---|
| Sense | | |
| pvR3 | GCAACACCCGGGATGTCGCAAATCATG (SEQ ID NO: 43) | 91-105 (SEQ ID NO: 1) |
| stR2 | GTAACACCCGGGGTGGCCGCCGACCCG (SEQ ID NO: 44) | 141-155 (SEQ ID NO: 3) |
| Antisense | | |
| pvF4 | CTACTAAGCTTGGATCCCTAGCCG- CCCCATTTGGCGG (SEQ ID NO: 45) | 381-362 (SEQ ID NO: 1) |
| stF2 | CTACTAAGCTTCCATGGTCAGGTC- TTTTCGATGCTTAC (SEQ ID NO: 46) | 467 - 447 (SEQ ID NO: 3) |

[a] The *cfp7* oligonucleotides were based on the nucleotide sequence shown in Fig. 3 (SEQ ID NO: 1). The *cfp9* oligonucleotides were based on the nucleotide sequence shown in Fig. 4 (SEQ ID NO: 3).

Nucleotides underlined are not contained in the nucleotide sequence of *cfp7* and *cfp9*.

[b] The positions referred to are of the non-underlined part of the primers and correspond to the nucleotide sequence shown in Fig. 3 and Fig. 4, respectively.

EXAMPLE 2A

*Identification of antigens which are not expressed in BCG strains.*

In an effort to control the treat of TB, attenuated bacillus Calmette-Guerin (BCG) has been used as a live attenuated vaccine. BCG is an attenuated derivative of a virulent *Mycobacterium bovis.* The original BCG from the Pasteur Institute in Paris, France was developed from 1908 to 1921 by 231 passages in liquid culture and has never been shown to revert to virulence in animals, indicating that the attenuating mutation(s) in BCG are stable deletions and/or multiple mutations which do not readily revert. While physiological differences between BCG and *M. tuberculosis* and *M. bovis* has been noted, the attenuating mutations which arose during serial passage of the original BCG strain has been unknown until recently. The first mutations described are the loss of the gene encoding MPB64 in some BCG strains (Li et al., 1993, Oettinger and Andersen, 1994) and the gene encoding ESAT-6 in all BCG strain tested (Harboe et al., 1996), later 3 large deletions in BCG have been identified (Mahairas et al., 1996). The region named RD1 includes the gene encoding ESAT-6 and an other (RD2) the gene encoding MPT64. Both antigens have been shown to have diagnostic potential and ESAT-6 has been shown to have properties as a vaccine candidate (cf. PCT/DK94/00273 and PCTIDK/00270). In order to find new *M. tuberculosis* specific diagnostic antigens as well as antigens for a new vaccine against TB, the RD1 region (17.499 bp) of *M. tuberculosis* H37Rv has been analyzed for Open Reading Frames (ORF). ORFs with a minimum length of 96 bp have been predicted using the algorithm described by Borodovsky and McIninch (1993), in total 27 ORFs have been predicted, 20 of these have possible diagnostic and/or vaccine potential, as they are deleted from all known BCG strains. The predicted ORFs include ESAT-6 (RD1-ORF7) and CFP10 (RD1-ORF6) described previously (Sørensen et al., 1995), as a positive control for the ability of the algorithm. In the present is described the potential of 7 of the predicted antigens for diagnosis of TB as well as potential as candidates for a new vaccine against TB.

Seven open reading frames (ORF) from the 17,499kb RD1 region (Accession no. U34848) with possible diagnostic and vaccine potential have been identified and cloned.

Identification of the ORF's *rd1-orf2*, *rd1-orf3*, *rd1-orf4*, *rd1-orf5*, *rd1-orf8*, *rd1-orf9a*, and *rd1-orf9b*.

The nucleotide sequence of *rd1-orf2* from *M. tuberculosis* H37Rv is set forth in SEQ ID NO: 71. The deduced amino acid sequence of RD1-ORF2 is set forth in SEQ ID NO: 72.

The nucleotide sequence of *rd1-orf3* from *M. tuberculosis* H37Rv is set forth in SEQ ID NO: 87. The deduced amino acid sequence of RD1-ORF2 is set forth in SEQ ID NO: 88.

The nucleotide sequence of *rd1-orf4* from *M. tuberculosis* H37Rv is set forth in SEQ ID NO: 89. The deduced amino acid sequence of RD1-ORF2 is set forth in SEQ ID NO: 90.

The nucleotide sequence of *rd1-orf5* from *M. tuberculosis* H37Rv is set forth in SEQ ID NO: 91. The deduced amino acid sequence of RD1-ORF2 is set forth in SEQ ID NO: 92.

The nucleotide sequence of *rd1-orf8* from *M. tuberculosis* H37Rv is set forth in SEQ ID NO: 67. The deduced amino acid sequence of RD1-ORF2 is set forth in SEQ ID NO: 68.

The nucleotide sequence of *rd1-orf9a* from *M. tuberculosis* H37Rv is set forth in SEQ ID NO: 93. The deduced amino acid sequence of RD1-ORF2 is set forth in SEQ ID NO: 94.

The nucleotide sequence of *rd1-orf9b* from *M. tuberculosis* H37Rv is set forth in SEQ ID NO: 69. The deduced amino acid sequence of RD1-ORF2 is set forth in SEQ ID NO: 70.

The DNA sequence *rd1-orf2* (SEQ ID NO: 71) contained an open reading frame starting with an ATG codon at position 889 - 891 and ending with a termination codon (TAA) at position 2662 - 2664 (position numbers referring to the location in RD1). The deduced amino acid sequence (SEQ ID NO: 72) contains 591 residues corresponding to a molecular weight of 64,525.

The DNA sequence *rd1-orf3* (SEQ ID NO: 87) contained an open reading frame starting with an ATG codon at position 2807 - 2809 and ending with a termination codon (TAA) at position 3101 - 3103 (position numbers referring to the location in RD1). The deduced amino acid sequence (SEQ ID NO: 88) contains 98 residues corresponding to a molecular weight of 9,799.

The DNA sequence *rd1-orf4* (SEQ ID NO: 89) contained an open reading frame starting with a GTG codon at position 4014 - 4012 and ending with a termination codon (TAG) at position 3597 - 3595 (position numbers referring to the location in RD1). The deduced amino acid sequence (SEQ ID NO: 90) contains 139 residues corresponding to a molecular weight of 14,210.

The DNA sequence *rd1-orf5* (SEQ ID NO: 91) contained an open reading frame starting with a GTG codon at position 3128 - 3130 and ending with a termination codon (TGA) at position 4241 - 4243 (position numbers referring to the location in RD1). The deduced amino acid sequence (SEQ ID NO: 92) contains 371 residues corresponding to a molecular weight of 37,647.

The DNA sequence *rd1-orf8* (SEQ ID NO: 67) contained an open reading frame starting with a GTG codon at position 5502 - 5500 and ending with a termination codon (TAG) at position 5084 - 5082 (position numbers referring to the location in RD1), and the deduced amino acid sequence (SEQ ID NO: 68) contains 139 residues with a molecular weight of 11,737.

The DNA sequence *rd1-orf9a* (SEQ ID NO: 93) contained an open reading frame starting with a GTG codon at position 6146 - 6148 and ending with a termination codon (TAA) at position 7070 - 7072 (position numbers referring to the location in RD1). The deduced amino acid sequence (SEQ ID NO: 94) contains 308 residues corresponding to a molecular weight of 33,453.

The DNA sequence *rd1-orf9b* (SEQ ID NO: 69) contained an open reading frame starting with an ATG codon at position 5072 - 5074 and ending with a termination codon (TAA) at position 7070 - 7072 (position numbers referring to the location in RD1). The deduced amino acid sequence (SEQ ID NO: 70) contains 666 residues corresponding to a molecular weight of 70,650.

Cloning of the ORF's *rd1-orf2*, *rd1-orf3*, *rd1-orf4*, *rd1-orf5*, *rd1-orf8*, *rd1-orf9a,* and *rd 1-orf9b*.

The ORF's *rd1-orf2*, *rd1-orf3*, *rd1-orf4*, *rd1-orf5*, *rd1-orf8*, *rd1-orf9a* and *rd1-orf9b* were PCR cloned in the pMST24 (Theisen et al., 1995) (*rd1-orf3*) or the pQE32 (QIAGEN) (*rd1-orf2*, *rd1-orf4*, *rd1-orf5*, *rd1-orf8*, *rd1-orf9a* and *rd1-orf9b*) expression vector. Preparation of oligonucleotides and PCR amplification of the *rd1-orf* encoding genes, was carried out as described in example 2. Chromosomal DNA from *M. tuberculosis* H37Rv was used as template in the PCR reactions. Oligonucleotides were synthesized on the basis of the nucleotide sequence from the RD1 region (Accession no. U34848). The oligonucleotide primers were engineered to include an restriction enzyme site at the 5' end and at the 3' end by which a later subcloning was possible. Primers are listed in TABLE 2.

*rd1-orf2*. A *Bam*HI site was engineered immediately 5' of the first codon of *rd1-orf2*, and a *Hind*III site was incorporated right after the stop codon at the 3' end. The gene *rd1-orf2* was subcloned in pQE32, giving pTO96.

*rd1-orf3*. A *Sma*I site was engineered immediately 5' of the first codon of *rd1-orf3*, and a *Nco*I site was incorporated right after the stop codon at the 3' end. The gene *rd1-orf3* was subcloned in pMST24, giving pTO87.

*rd1-orf4*. A *Bam*HI site was engineered immediately 5' of the first codon of *rd1-orf4*, and a *Hind*III site was incorporated right after the stop codon at the 3' end. The gene *rd1-orf4* was subcloned in pQE32, giving pTO89.

*rd1-orf5*. A *Bam*HI site was engineered immediately 5' of the first codon of *rd1-orf5*, and a *Hind*III site was incorporated right after the stop codon at the 3' end. The gene *rd1-orf5* was subcloned in pQE32, giving pTO88.

*rd1-orf8*. A *Bam*HI site was engineered immediately 5' of the first codon of *rd1-orf8*, and a *Nco*I site was incorporated right after the stop codon at the 3' end. The gene *rd1-orf8* was subcloned in pMST24, giving pTO98.

*rd1-orf9a*. A *Bam*HI site was engineered immediately 5' of the first codon of *rd1-orf9a*, and a *Hind*III site was incorporated right after the stop codon at the 3' end. The gene *rd1-orf9a* was subcloned in pQE32, giving pTO91.

*rd1-orf9b*. A Scal site was engineered immediately 5' of the first codon of *rd1-orf9b*, and a *Hind* III site was incorporated right after the stop codon at the 3' end. The gene *rd1-orf9b* was subcloned in pQE32, giving pTO90.

The PCR fragments were digested with the suitable restriction enzymes, purified from an agarose gel and cloned into either pMST24 or pQE-32. The seven constructs were used to transform the *E. coli* XL1-Blue. Endpoints of the gene fusions were determined by the dideoxy chain termination method. Both strands of the DNA were sequenced.

Purification of recombinant RD1-ORF2, RD1-ORF3, RD1-ORF4, RD1-ORF5, RD1-ORF8, RD1-ORF9a and RD1-ORF9b.

The rRD1-ORFs were fused N-terminally to the $(His)_6$-tag. Recombinant antigen was prepared as described in example 2 (with the exception that pTO91 was expressed at 30°C and not at 37°C), using a single colony of *E. coli* harbouring either the pTO87, pTO88, pTO89, pTO90, pTO91, pTO96 or pTO98 for inoculation. Purification of recombinant antigen by $Ni^{2+}$ affinity chromatography was also carried out as described in example 2. Fractions containing purified His-rRD1-ORF2, His-rRD1-ORF3 His-rRD1-ORF4, His-rRD1-ORF5, His-rRD1-ORF8, His-rRD1-ORF9a or His-rRD1-ORF9b were pooled. The His-rRD1-ORF's were extensively dialysed against 10 mM Tris/HCl, pH 8.5, 3 M urea followed by an additional purification step performed on an anion exchange column (Mono Q) using fast protein liquid chromatography (FPLC) (Pharmacia, Uppsala, Sweden). The purification was carried out in 10 mM Tris/HCl, pH 8.5, 3 M urea and protein was eluted by a linear gradient of NaCl from 0 to 1 M. Fractions containing the His-rRD1-ORF's were pooled and subsequently dialysed extensively against 25 mM Hepes, pH 8.0 before use.

**Table 2.** Sequence of the *rd1-orf's* oligonucleotides[a].

| Orientation and oligo-nucleotide | Sequences (5'→ 3') | Position (nt) |
|---|---|---|
| Sense | | |
| RD1-ORF2f | CTGGGGATCCGCATGACTGCTGAACCG | 886 - 903 |
| RD1-ORF3f | CTTCCCGGGATGGAAAAAATGTCAC | 2807 - 2822 |
| RD1-ORF4f | GTAGGATCCTAGGAGACATCAGCGGC | 4028 - 4015 |
| RD1-ORF5f | CTGGGGATCCGCGTGATCACCAT-GCTGTGG | 3028 - 3045 |
| RD1-ORF8f | CTCGGATCCTGTGGGTGCAGGTCCGGCGATGGGC | 5502 - 5479 |
| RD1-ORF9af | GTGATGTGAGCTCAGGTGAAGAA-GGTGAAG | 6144 - 6160 |
| RD1-ORF9bf | GTGATGTGAGCTCCTATGGCGGCCGAC-TACGAC | 5072 - 5089 |
| Antisense | | |
| RD1-ORF2r | TGCAAGCTTTTAACCGGCGCTTGGGGGTGC | 2664 - 2644 |
| RD1-ORF3r | GATGCCATGGTTAGGCGAAGACGC-CGGC | 3103 - 3086 |
| RD1-ORF4r | CGATCTAAGCTTGGCAATGGAGGTCTA | 3582 - 3597 |
| RD1-ORF5r | TGCAAGCTTTCACCAGTCGTCCT-CTTCGTC | 4243 - 4223 |
| RD1-ORF8r | CTCCCATGGCTACGACAAGCTCTTC-CGGCCGC | 5083 - 5105 |
| RD1-ORF9a/br | CGATCTAAGCTTTCAACGACGTCCAGCC | 7073 - 7056 |

[a] The oligonucleotides were constructed from the Accession number U34484 nucleotide sequence (Mahairas et al., 1996). Nucleotides (nt) underlined are not contained in the nucleotide sequence of RD1-ORF's. The positions correspond to the nucleotide sequence of Accession number U34484.

The nucleotide sequences of *rd1-orf2*, *rd1-orf3*, *rd1-orf4*, *rd1-orf5*, *rd1-orf8*, *rd1-orf9a*, and *rd1-orf9b* from *M.*

*tuberculosis* H37Rv are set forth in SEQ ID NO: 71, 87, 89, 91, 67, 93, and 69, respectively. The deduced amino acid sequences of *rd1-orf2*, *rd1-orf3*, *rd1-orf4 rd1-orf5*, *rd1-orf8*, *rd1-orf9a*, and *rd1-orf9b* are set forth in SEQ ID NO: 72, 88, 90, 92, 68, 94, and 70, respectively.

EXAMPLE 3

*Cloning of the genes expressing 17-30 kDa antigens from ST-CF*

Isolation of CFP17, CFP20, CFP21, CFP22, CFP25, and CFP28

ST-CF was precipitated with ammonium sulphate at 80% saturation. The precipitated proteins were removed by centrifugation and after resuspension washed with 8 M urea. CHAPS and glycerol were added to a final concentration of 0.5% (w/v) and 5% (v/v) respectively and the protein solution was applied to a Rotofor isoelectrical Cell (BioRad). The Rotofor Cell had been equilibrated with an 8 M urea buffer containing 0.5% (w/v) CHAPS, 5% (v/v) glycerol, 3% (v/v) Biolyt 3/5 and 1 % (v/v) Biolyt 4/6 (BioRad). Isoelectric focusing was performed in a pH gradient from 3-6. The fractions were analyzed on silver-stained 10-20% SDS-PAGE. Fractions with similar band patterns were pooled and washed three times with PBS on a Centriprep concentrator (Amicon) with a 3 kDa cut off membrane to a final volume of 1-3 ml. An equal volume of SDS containing sample buffer was added and the protein solution boiled for 5 min before further separation on a Prep Cell (BioRad) in a matrix of 16% polyacrylamide under an electrical gradient. Fractions containing pure proteins with an molecular mass from 17-30 kDa were collected.

Isolation of CFP29

Anti-CFP29, reacting with CFP29 was generated by immunization of BALB/c mice with crushed gel pieces in RIBI adjuvant (first and second immunization) or aluminium hydroxide (third immunization and boosting) with two week intervals. SDS-PAGE gel pieces containing 2-5 μg of CFP29 were used for each immunization. Mice were boosted with antigen 3 days before removal of the spleen. Generation of a monoclonal cell line producing antibodies against CFP29 was obtained essentially as described by Köhler and Milstein (1975). Screening of supernatants from growing clones was carried out by immunoblotting of nitrocellulose strips containing ST-CF separated by SDS-PAGE. Each strip contained approximately 50 μg of ST-CF. The antibody class of anti-CFP29 was identified as IgM by the mouse monoclonal antibody isotyping kit, RPN29 (Amersham) according to the manufacturer's instructions.
CFP29 was purified by the following method: ST-CF was concentrated 10 fold by ultrafiltration, and ammonium sulphate precipitation in the 45 to 55% saturation range was performed. The pellet was redissolved in 50 mM sodium phosphate, 1.5 M ammonium sulphate, pH 8.5, and subjected to thiophilic adsorption chromatography (Porath *et al*., 1985) on an Affi-T gel column (Kem-En-Tec). Protein was eluted by a linear 1.5 to 0 M gradient of ammonium sulphate and fractions collected in the range 0.44 to 0.31 M ammonium sulphate were identified as CFP29 containing fractions in Western blot experiments with mAb Anti-CFP29. These fractions were pooled and anion exchange chromatography was performed on a Mono Q HR 5/5 column connected to an FPLC system (Pharmacia). The column was equilibrated with 10 mM Tris-HCl, pH 8.5 and the elution was performed with a linear gradient from 0 to 500 mM NaCl. From 400 to 500 mM sodium chloride, rather pure CFP29 was eluted. As a final purification step the Mono Q fractions containing CFP29 were loaded on a 12.5% SDS-PAGE gel and pure CFP29 was obtained by the multi-elution technique (Andersen and Heron, 1993).

N-terminal sequencing and amino acid analysis

CFP17, CFP20, CFP21, CFP22, CFP25, and CFP28 were washed with water on a Centricon concentrator (Amicon) with cutoff at 10 kDa and then applied to a ProSpin concentrator (Applied Biosystems) where the proteins were collected on a PVDF membrane. The membrane was washed 5 times with 20% methanol before sequencing on a Procise sequencer (Applied Biosystems).
CFP29 containing fractions were blotted to PVDF membrane after tricine SDS-PAGE (Ploug *et al*., 1989). The relevant bands were excised and subjected to amino acid analysis (Barkholt and Jensen, 1989) and N-terminal sequence analysis on a Procise sequencer (Applied Biosystems).
The following N-terminal sequences were obtained:

For CFP17: A/S E L D A P A Q A G T E X A V        (SEQ ID NO: 17)

For CFP20: A Q I T L R G N A I N T V G E      (SEQ ID NO: 18)

For CFP21: D P X S D I A V V F A R G T H      (SEQ ID NO: 19)

For CFP22: T N S P L A T A T A T L H T N      (SEQ ID NO: 20)

For CFP25: A X P D A E V V F A R G R F E      (SEQ ID NO: 21)

For CFP28: X I/V Q K S L E L I V/T V/F T A D/Q E      (SEQ ID NO: 22)

For CFP29: M N N L Y R D L A P V T E A A W A E I      (SEQ ID NO: 23)

"X" denotes an amino acid which could not be determined by the sequencing method used, whereas a "/" between two amino acids denotes that the sequencing method could not determine which of the two amino acids is the one actually present.

Cloning the gene encoding CFP29

The N-terminal sequence of CFP29 was used for a homology search in the EMBL database using the TFASTA program of the Genetics Computer Group sequence analysis software package. The search identified a protein, Linocin M 18, from *Brevibacterium linens* that shares 74% identity with the 19 N-terminal amino acids of CFP29.

Based on this identity between the N-terminal sequence of CFP29 and the sequence of the Linocin M18 protein from *Brevibacterium linens,* a set of degenerated primers were constructed for PCR cloning of the *M. tuberculosis* gene encoding CFP29. PCR reactions were containing 10 ng of *M. tuberculosis* chromosomal DNA in 1 x low salt Taq + buffer from Stratagene supplemented with 250 µM of each of the four nucleotides (Boehringer Mannheim), 0,5 mg/ml BSA (IgG technology), 1 % DMSO (Merck), 5 pmoles of each primer and 0.5 unit Tag+ DNA polymerase (Stratagene) in 10 µl reaction volume. Reactions were initially heated to 94°C for 25 sec. and run for 30 cycles of the program; 94°C for 15 sec., 55°C for 15 sec. and 72°C for 90 sec, using thermocycler equipment from Idaho Technology.

An approx. 300 bp fragment was obtained using primers with the sequences:

1: 5'-CCCGGCTCGAGAACCTSTACCGCGACCTSGCSCC      (SEQ ID NO: 24)

2: 5'-GGGCCGGATCCGASGCSGCGTCCTTSACSGGYTGCCA

(SEQ ID NO: 25)

- where S = G/C and Y = T/C

The fragment was excised from a 1 % agarose gel, purified by Spin-X spinn columns (Costar), cloned into pBluescript SK II + - T vector (Stratagene) and finally sequenced with the Sequenase kit from United States Biochemical.

The first 150 bp of this sequence was used for a homology search using the Blast program of the Sanger *Mycobacterium tuberculosis* database:
(http//www.sanger.ac.uk/projects/M-tuberculosis/blast_server).

This program identified a *Mycobacterium tuberculosis* sequence on cosmid cy444 in the database that is nearly 100% identical to the 150 bp sequence of the CFP29 protein. The sequence is contained within a 795 bp open reading frame of which the 5' end translates into a sequence that is 100% identical to the N-terminally sequenced 19 amino acids of the purified CFP29 protein.

Finally, the 795 bp open reading frame was PCR cloned under the same PCR conditions as described above using the primers:

3:    5'-GGAAGCCCCATATGAACAATCTCTACCG         (SEQ ID NO: 26)

4:    5'-CGCGCTCAGCCCTTAGTGACTGAGCGCGACCG     (SEQ ID NO: 27)

The resulting DNA fragments were purified from agarose gels as described above sequenced with primer 3 and 4 in addition to the following primers:

5: 5'-GGACGTTCAAGCGACACATCGCCG-3'         (SEQ ID NO: 115)

6: 5'-CAGCACGAACGCGCCGTCGATGGC-3'         (SEQ ID NO: 116)

Three independent cloned were sequenced. All three clones were in 100% agreement with the sequence on cosmid cy444.
All other DNA manipulations were done according to Maniatis *et al.* (1989).
All enzymes other than Taq polymerase were from New England Biolabs.

Homology searches in the Sanger database

For CFP17, CFP20, CFP21, CFP22, CFP25, and CFP28 the N-terminal amino acid sequence from each of the proteins were used for a homology search using the blast program of the Sanger *Mycobacterium tuberculosis* database:
http://www.sanger.ac.uk/pathogens/TB-blast-server.html.
For CFP29 the first 150 bp of the DNA sequence was used for the search. Furthermore, the EMBL database was searched for proteins with homology to CFP29.

Thereby, the following information were obtained:

CFP17

Of the 14 determined amino acids in CFP17 a 93% identical sequence was found with MTCY1A11.16c. The difference between the two sequences is in the first amino acid: It is an A or an S in the N-terminal determined sequenced and a S in MTCY1A11. From the N-terminal sequencing it was not possible to determine amino acid number 13.
Within the open reading frame the translated protein is 162 amino acids long. The N-terminal of the protein purified from culture filtrate starts at amino acid 31 in agreement with the presence of a signal sequence that has been cleaved off. This gives a length of the mature protein of 132 amino acids, which corresponds to a theoretical molecular mass of 13833 Da and a theoretical pl of 4.4. The observed mass in SDS-PAGE is 17 kDa.

CFP20

A sequence 100% identical to the 15 determined amino acids of CFP20 was found on the translated cosmid cscy09F9. A stop codon is found at amino acid 166 from the amino acid M at position 1. This gives a predicted length of 165 amino acids, which corresponds to a theoretical molecular mass of 16897 Da and a pl of 4.2. The observed molecular weight in a SDS-PAGE is 20 kDa.
Searching the GenEMBL database using the TFASTA algorithm (Pearson and Lipman, 1988) revealed a number of proteins with homology to the predicted 164 amino acids long translated protein.
The highest homology, 51.5% identity in a 163 amino acid overlap, was found to a Haemophilus influenza Rd toxR reg. (HIHI0751).

CFP21

A sequence 100% identical to the 14 determined amino acids of CFP21 was found at MTCY39. From the N-terminal sequencing it was not possible to determine amino acid number 3; this amino acid is a C in MTCY39. The amino acid C can not be detected on a Sequencer which is probably the explanation of this difference.

Within the open reading frame the translated protein is 217 amino acids long. The N-terminally determined sequence from the protein purified from culture filtrate starts at amino acid 33 in agreement with the presence of a signal sequence that has been cleaved off. This gives a length of the mature protein of 185 amino acids, which corresponds to a theoretical molecular weigh at 18657 Da, and a theoretical pl at 4,6. The observed weight in a SDS-PAGE is 21 kDa.

In a 193 amino acids overlap the protein has 32,6% identity to a cutinase precursor with a length of 209 amino acids (CUTI_ALTBR P41744).

A comparison of the 14 N-terminal determined amino acids with the translated region (RD2) deleted in *M. bovis* BCG revealed a 100% identical sequence (mb3484) (Mahairas *et al.* (1996)).

CFP22

A sequence 100% identical to the 15 determined amino acids of CFP22 was found at MTCY10H4. Within the open reading frame the translated protein is 182 amino acids long. The N-terminal sequence of the protein purified from culture filtrate starts at amino acid 8 and therefore the length of the protein occurring in *M. tuberculosis* culture filtrate is 175 amino acids. This gives a theoretical molecular weigh at 18517 Da and a pl at 6.8. The observed weight in a SDS-PAGE is 22 kDa.

In an 182 amino acids overlap the translated protein has 90,1 % identity with E235739; a peptidyl-prolyl cis-trans isomerase.

CFP25

A sequence 93% identical to the 15 determined amino acids was found on the cosmid MTCY339.08c. The one amino acid that differs between the two sequences is a C in MTCY339.08c and a X from the N-terminal sequence data. On a Sequencer a C can not be detected which is a probable explanation for this difference.

The N-terminally determined sequence from the protein purified from culture filtrate begins at amino acid 33 in agreement with the presence of a signal sequence that has been cleaved off. This gives a length of the mature protein of 187 amino acids, which corresponds to a theoretical molecular weigh at 19665 Da, and a theoretical pl at 4.9. The observed weight in a SDS-PAGE is 25 kDa.

In a 217 amino acids overlap the protein has 42.9% identity to CFP21 (MTCY39.35).

CFP28

No homology was found when using the 10 determined amino acid residues 2-8, 11, 12, and 14 of SEQ ID NO: 22 in the database search.

CFP29

Sanger database searching: A sequence nearly 100% identical to the 150 bp sequence of the CFP29 protein was found on cosmid cy444. The sequence is contained within a 795 bp open reading frame of which the 5' end translates into a sequence that is 100% identical to the N-terminally sequenced 19 amino acids of the purified CFP29 protein. The open reading frame encodes a 265 amino acid protein.

The amino acid analysis performed on the purified protein further confirmed the identity of CFP29 with the protein encoded in open reading frame on cosmid 444.

EMBL database searching: The open reading frame encodes a 265 amino acid protein that is 58% identical and 74% similar to the Linocin M18 protein (61 % identity on DNA level). This is a 28.6 kDa protein with bacteriocin activity (Valdés-Stauber and Scherer, 1994; Valdés-Stauber and Scherer, 1996). The two proteins have the same length (except for 1 amino acid) and share the same theoretical physicochemical properties. We therefore suggest that CFP29 is a mycobacterial homolog to the *Brevibacterium linens* Linocin M18 protein.

The amino acid sequences of the purified antigens as picked from the Sanger database are shown in the following list. The amino acids determined by N-terminal sequencing are marked with bold.

CFP17 (SEQ ID NO: 6):

1 MTDMNPDIEK DQTSDEVTVE TTSVFRADFL **SELDAPAQAG TESAV**SGVEG
51 LPPGSALLVV KRGPNAGSRF LLDQAITSAG RHPDSDIFLD DVTVSRRHAE
101 FRLENNEFNV VDVGSLNGTY VNREPVDSAV LANGDEVQIG KFRLVFLTGP
151 KQGEDDGSTG GP

CFP20 (SEQ ID NO: 8):

1 **MAQITLRGNA INTVG**ELPAV GSPAPAFTLT GGDLGVISSD QFRGKSVLLN
51 IFPSVDTPVC ATSVRTFDER AAASGATVLC VSKDLPFAQK RFCGAEGTEN
101 VMPASAFRDS FGEDYGVTIA DGPMAGLLAR AIVVIGADGN VAYTELVPEI
151 AQEPNYEAAL AALGA

CFP21 (SEQ ID NO: 10):

1 MTPRSLVRIV GVVVATTLAL VSAPAGGRAA HA**DPCSDIAV**
41 **VFARGTH**QAS GLGDVGEAFV DSLTSQVGGR SIGVYAVNYP ASDDYRASAS
91 NGSDDASAHI QRTVASCPNT RIVLGGYSQG ATVIDLSTSA MPPAVADHVA
141 AVALFGEPSS GFSSMLWGGG SLPTIGPLYS SKTINLCAPD DPICTGGGNI
191 MAHVSYVQSG MTSQAATFAA NRLDHAG

CFP22 (SEQ ID NO: 12):

1 MADCDSV**TNS PLATATATLH** TNRGDIKIAL FGNHAPKTVA NFVGLAQGTK
51 DYSTQNASGG PSGPFYDGAV FHRVIQGFMI QGGDPTGTGR GGPGYKFADE
101 FHPELQFDKP YLLAMANAGP GTNGSQFFIT VGKTPHLNRR HTIFGEVIDA
151 ESQRVVEAIS KTATDGNDRP TDPVVIESIT IS

CFP25 (SEQ ID NO: 14):

```
  1 MGAAAAMLAA VLLLTPITVP AGYPGAVAPA TAACPDAEVV FARGRFEPPG
 51 IGTVGNAFVS ALRSKVNKNV GVYAVKYPAD NQIDVGANDM SAHIQSMANS
101 CPNTRLVPGG YSLGAAVTDV VLAVPTQMWG FTNPLPPGSD EHIAAVALFG
151 NGSQWVGPIT NFSPAYNDRT IELCHGDDPV CHPADPNTWE ANWPQHLAGA
201 YVSSGMVNQA ADFVAGKLQ
```

CFP29 (SEQ ID NO: 16):

```
  1 MNNLYRDLAP VTEAAWAEIE LEAARTFKRH IAGRRVVDVS DPGGPVTAAV
 51 STGRLIDVKA PTNGVIAHLR ASKPLVRLRV PFTLSRNEID DVERGSKDSD
101 WEPVKEAAKK LAFVEDRTIF EGYSAASIEG IRSASSNPAL TLPEDPREIP
151 DVISQALSEL RLAGVDGPYS VLLSADVYTK VSETSDHGYP IREHLNRLVD
201 GDIIWAPAID GAFVLTTRGG DFDLQLGTDV AIGYASHDTD TVRLYLQETL
251 TFLCYTAEAS VALSH
```

For all six proteins the molecular weights predicted from the sequences are in agreement with the molecular weights observed on SDS-PAGE.

Cloning of the genes encoding CFP17, CFP20, CFP21, CFP22 and CFP25.

The genes encoding CFP17, CFP20, CFP21, CFP22 and CFP25 were all cloned into the expression vector pMCT6, by PCR amplification with gene specific primers, for recombinant expression in *E. coli* of the proteins.

PCR reactions contained 10 ng of *M. tuberculosis* chromosomal DNA in 1 x low salt Taq+ buffer from Stratagene supplemented with 250 mM of each of the four nucleotides (Boehringer Mannheim), 0,5 mg/ml BSA (IgG technology), 1 % DMSO (Merck), 5 pmoles of each primer and 0.5 unit Tag+ DNA polymerase (Stratagene) in 10 µl reaction volume. Reactions were initially heated to 94°C for 25 sec. and run for 30 cycles according to the following program; 94°C for 10 sec., 55°C for 10 sec. and 72°C for 90 sec, using thermocycler equipment from Idaho Technology.

The DNA fragments were subsequently run on 1 % agarose gels, the bands were excised and purified by Spin-X spin columns (Costar) and cloned into pBluescript SK II + - T vector (Stratagene). Plasmid DNA was thereafter prepared from clones harbouring the desired fragments, digested with suitable restriction enzymes and subcloned into the expression vector pMCT6 in frame with 8 histidine residues which are added to the N-terminal of the expressed proteins. The resulting clones were hereafter sequenced by use of the dideoxy chain termination method adapted for supercoiled DNA using the Sequenase DNA sequencing kit version 1.0 (United States Biochemical Corp., USA) and by cycle sequencing using the Dye Terminator system in combination with an automated gel reader (model 373A; Applied Biosystems) according to the instructions provided. Both strands of the DNA were sequenced.

For cloning of the individual antigens, the following gene specific primers were used:

CFP17: Primers used for cloning of cfp17:

    OPBR-51:     ACAGATCTGTGACGGACATGAACCCG   (SEQ ID NO: 117)

    OPBR-52:     TTTTCCATGGTCACGGGCCCCCGGTACT (SEQ ID NO: 118)

OPBR-51 and OPBR-52 create BglII and NcoI sites, respectively, used for the cloning in pMCT6.

CFP20: Primers used for cloning of cfp20:

    OPBR-53:     ACAGATCTGTGCCCATGGCACAGATA   (SEQ ID NO: 119)

    OPBR-54:     TTTAAGCTTCTAGGCGCCCAGCGCGGC  (SEQ ID NO: 120)

OPBR-53 and OPBR-54 create BglII and HinDIII sites, respectively, used for the cloning in pMCT6.

CFP21: Primers used for cloning of cfp21:

    OPBR-55:     ACAGATCTGCGCATGCGGATCCGTGT   (SEQ ID NO: 121)

    OPBR-56:     TTTTCCATGGTCATCCGGCGTGATCGAG (SEQ ID NO: 122)

OPBR-55 and OPBR-56 create BglII and NcoI sites, respectively, used for the cloning in pMCT6.

CFP22: Primers used for cloning of cfp22:

    OPBR-57:     ACAGATCTGTAATGGCAGACTGTGAT   (SEQ ID NO: 123)

    OPBR-58:     TTTTCCATGGTCAGGAGATGGTGATCGA (SEQ ID NO: 124)

OPBR-57 and OPBR-58 create BglII and NcoI sites, respectively, used for the cloning in pMCT6.

CFP25: Primers used for cloning of cfp25:

OPBR-59:    ACAGATCTGCCGGCTACCCCGGTGCC    (SEQ ID NO: 125)

OPBR-60:    TTTTCCATGGCTATTGCAGCTTTCCGGC  (SEQ ID NO: 126)

OPBR-59 and OPBR-60 create BglII and NcoI sites, respectively, used for the cloning in pMCT6.

Expression/purification of recombinant CFP17, CFP20, CFP21, CFP22 and CFP25 proteins.

Expression and metal affinity purification of recombinant proteins was undertaken essentially as described by the manufacturers. For each protein, 1 l LB-media containing 100 µg/ml ampicillin, was inoculated with 10 ml of an overnight culture of XL1-Blue cells harbouring recombinant pMCT6 plasmids. Cultures were shaken at 37 °C until they reached a density of $OD_{600}$ = 0.4 - 0.6. IPTG was hereafter added to a final concentration of 1 mM and the cultures were further incubated 4 - 16 hours. Cells were harvested, resuspended in 1X sonication buffer + 8 M urea and sonicated 5 X 30 sec. with 30 sec. pausing between the pulses.
After centrifugation, the lysate was applied to a column containing 25 ml of resuspended Talon resin (Clontech, Palo Alto, USA). The column was washed and eluted as described by the manufacturers.
After elution, all fractions (1.5 ml each) were subjected to analysis by SDS-PAGE using the Mighty Small (Hoefer Scientific Instruments, USA) system and the protein concentrations were estimated at 280 nm. Fractions containing recombinant protein were pooled and dialysed against 3 M urea in 10 mM Tris-HCl, pH 8.5. The dialysed protein was further purified by FPLC (Pharmacia, Sweden) using a 6 ml Resource-Q column, eluted with a linear 0-1 M gradient of NaCl. Fractions were analyzed by SDS-PAGE and protein concentrations were estimated at $OD_{280}$. Fractions containing protein were pooled and dialysed against 25 mM Hepes buffer, pH 8.5.
Finally the protein concentration and the LPS content were determined by the BCA (Pierce, Holland) and LAL (Endosafe, Charleston, USA) tests, respectively.

EXAMPLE 3A

*Identification of CFP7A, CFP8A, CFP8B, CFP16, CFP19, CFP19B, CFP22A, CFP23A, CFP23B, CFP25A, CFP27, CFP30A, CWP32 and CFP50.*

Identification of CFP16 and CFP19B.

ST-CF was precipitated with ammonium sulphate at 80% saturation. The precipitated proteins were removed by centrifugation and after resuspension washed with 8 M urea. CHAPS and glycerol were added to a final concentration of 0.5 % (w/v) and 5 % (v/v) respectively and the protein solution was applied to a Rotofor isoelectrical Cell (BioRad). The Rotofor Cell had been equilibrated with a 8M urea buffer containing 0.5 % (w/v) CHAPS, 5% (v/v) glycerol, 3% (v/v) Biolyt 3/5 and 1 % (v/v) Biolyt 4/6 (BioRad). Isoelectric focusing was performed in a pH gradient from 3-6. The fractions were analyzed on silver-stained 10-20% SDS-PAGE. Fractions with similar band patterns were pooled and washed three times with PBS on a Centriprep concentrator (Amicon) with a 3 kDa cut off membrane to a final volume of 1-3 ml. An equal volume of SDS containing sample buffer was added and the protein solution boiled for 5 min before further separation on a Prep Cell (BioRad) in a matrix of 16% polyacrylamide under an electrical gradient. Fractions containing well separated bands in SDS-PAGE were selected for N-terminal sequencing after transfer to PVDF membrane.

Isolation of CFP8A, CFP8B, CFP19, CFP23A, and CFP23B.

ST-CF was precipitated with ammonium sulphate at 80% saturation and redissolved in PBS, pH 7.4, and dialysed 3 times against 25mM Piperazin-HCl, pH 5.5, and subjected to chromatofocusing on a matrix of PBE 94 (Pharmacia) in a column connected to an FPLC system (Pharmacia). The column was equilibrated with 25 mM Piperazin-HCl, pH 5.5, and the elution was performed with 10% PB74-HCl, pH 4.0 (Pharmacia). Fractions with similar band patterns were pooled and washed three times with PBS on a Centriprep concentrator (Amicon) with a 3 kDa cut off membrane to a final volume of 1-3 ml and separated on a Prepcell as described above.

Identification of CFP22A

ST-CF was concentrated approximately 10 fold by ultrafiltration and proteins were precipitated at 80 % saturation, redissolved in PBS, pH 7.4, and dialysed 3 times against PBS, pH 7.4. 5.1 ml of the dialysed ST-CF was treated with RNase (0.2 mg/ml, QUIAGEN) and DNase (0.2 mg/ml, Boehringer Mannheim) for 6 h and placed on top of 6.4 ml of 48 % (w/v) sucrose in PBS, pH 7.4, in Sorvall tubes (Ultracrimp 03987, DuPont Medical Products) and ultracentrifuged for 20 h at $257,300 \times g_{max}$, 10°C. The pellet was redissolved in 200 µl of 25 mM Tris-192 mM glycine, 0.1 % SDS, pH 8.3.

Identification of CFP7A, CFP25A, CFP27, CFP30A and CFP50

For CFP27, CFP30A and CFP50 ST-CF was concentrated approximately 10 fold by ultrafiltration and ammonium sulphate precipitation in the 45 to 55 % saturation range was performed. Proteins were redissolved in 50 mM sodium phosphate, 1.5 M ammonium sulphate, pH 8.5, and subjected to thiophilic adsorption chromatography on an Affi-T gel column (Kem-En-Tec). Proteins were eluted by a 1.5 to 0 M decreasing gradient of ammonium sulphate. Fractions with similar band patterns in SDS-PAGE were pooled and anion exchange chromatography was performed on a Mono Q HR 5/5 column connected to an FPLC system (Pharmacia). The column was equilibrated with 10 mM Tris-HCI, pH 8.5, and the elution was performed with a gradient of NaCl from 0 to 1 M. Fractions containing well separated bands in SDS-PAGE were selected.
CFP7A and CFP25A were obtained as described above except for the following modification: ST-CF was concentrated approximately 10 fold by ultrafiltration and proteins were precipitated at 80 % saturation, redissolved in PBS, pH 7.4, and dialysed 3 times against PBS, pH 7.4. Ammonium sulphate was added to a concentration of 1.5 M, and ST-CF proteins were loaded on an Affi T-gel column. Elution from the Affi T-gel column and anion exchange were performed as described above.

Isolation of CWP32

Heat treated H37Rv was subfractionated into subcellular fractions as described in Sørensen et al 1995. The Cell wall fraction was resuspended in 8 M urea, 0.2 % (w/v) N-octyl $\beta$-$_D$ glucopyranoside (Sigma) and 5 % (v/v) glycerol and the protein solution was applied to a Rotofor isoelectrical Cell (BioRad) which was equilibrated with the same buffer. Isoelectric focusing was performed in a pH gradient from 3-6. The fractions were analyzed by SDS-PAGE and fractions containing well separated bands were polled and subjected to N-terminal sequencing after transfer to PVDF membrane.

N-terminal sequencing

Fractions containing CFP7A, CFP8A, CFP8B, CFP16, CFP19, CFP19B, CFP22A, CFP23A, CFP23B, CFP27, CFP30A, CWP32, and CFP50A were blotted to PVDF membrane after Tricine SDS-PAGE (Ploug et al, 1989). The relevant bands were excised and subjected to N-terminal amino acid sequence analysis on a Procise 494 sequencer (Applied Biosystems). The fraction containing CFP25A was blotted to PVDF membrane after 2-DE PAGE (isoelectric focusing in the first dimension and Tricin SDS-PAGE in the second dimension). The relevant spot was excised and sequenced as described above.
The following N-terminal sequences were obtained:

| | | |
|---|---|---|
| CFP7A: | AEDVRAEIVA SVLEVVVNEG DQIDKGDVVV LLESMYMEIP | |
| | VLAEAAGTVS | (SEQ ID NO: 81) |
| CFP8A: | DPVDDAFIAKLNTAG | (SEQ ID NO: 73) |
| CFP8B: | DPVDAIINLDNYGX | (SEQ ID NO: 74) |
| CFP16: | AKLSTDELLDAFKEM | (SEQ ID NO: 79) |
| CFP19: | TTSPDPYAALPKLPS | (SEQ ID NO: 82) |
| CFP19B: | DPAXAPDVPTAAQLT | (SEQ ID NO: 80) |
| CFP22A: | TEYEGPKTKF HALMQ | (SEQ ID NO: 83) |
| CFP23A: | VIQ/AGMVT/GHIHXVAG | (SEQ ID NO: 76) |
| CFP23B: | AEMKXFKNAIVQEID | (SEQ ID NO: 75) |
| CFP25A: | AIEVSVLRVF TDSDG | (SEQ ID NO: 78) |

| | | |
|---|---|---|
| CWP32: | TNIVVLIKQVPDTWS | (SEQ ID NO: 77) |
| CFP27: | TTIVALKYPG GVVMA | (SEQ ID NO: 84) |
| CFP30A: | SFPYFISPEX AMRE | (SEQ ID NO: 85) |
| CFP50: | THYDVVVLGA GPGGY | (SEQ ID NO: 86) |

<u>N-terminal homology searching in the Sanger database and identification of the corresponding genes.</u>

The N-terminal amino acid sequence from each of the proteins was used for a homology search using the blast program of the Sanger *Mycobacterium tuberculosis* database:
http://www.sanger.ac.uk/projects/m-tuberculosis/TB-blast-server.

For CFP23B, CFP23A, and CFP19B no similarities were found in the Sanger database. This could be due to the fact that only approximately 70% of the *M. tuberculosis* genome had been sequenced when the searches were performed. The genes encoding these proteins could be contained in the remaining 30% of the genome for which no sequence data is yet available.

For CFP7A, CFP8A, CFP8B, CFP16, CFP19, CFP19B , CFP22A, CFP25A, CFP27, CFP30A, CWP32, and CFP50, the following information was obtained:

<u>CFP7A:</u> Of the 50 determined amino acids in CFP7A a 98% identical sequence was found in cosmid csCY07D1 (contig 256):
Score = 226 (100.4 bits), Expect = 1.4e-24, P = 1.4e-24
Identities = 49/50 (98%), Positives = 49/50 (98%), Frame = -1
Query: 1 AEDVRAEIVASVLEVVVNEGDQIDKGDVVVLLESMYMEIPVLAEAAGTVS 50

AEDVRAEIVASVLEVVVNEGDQIDKGDVVVLLESM MEIPVLAEAAGTVS

Sbjct: 257679 AEDVRAEIVASVLEVVVNEGDQIDKGDVVVLLESMKMEIPVLAEAAGTVS 257530

(SEQ ID NOs: 127, 128, and 129)

33

The identity is found within an open reading frame of 71 amino acids length corresponding to a theoretical MW of CFP7A of 7305.9 Da and a pl of 3.762. The observed molecular weight in an SDS-PAGE gel is 7 kDa.

CFP8A: A sequence 80% identical to the 15 N-terminal amino acids was found on contig TB_1884. The N-terminally determined sequence from the protein purified from culture filtrate starts at amino acid 32. This gives a length of the mature protein of 98 amino acids corresponding to a theoretical MW of 9700 Da and a pl of 3.72 This is in good agreement with the observed MW on SDS-PAGE at approximately 8 kDa. The full length protein has a theoretical MW of 12989 Da and a pl of 4.38.

CFP8B: A sequence 71 % identical to the 14 N-terminal amino acids was found on contig TB_653. However, careful re-evaluation of the original N-terminal sequence data confirmed the identification of the protein. The N-terminally determined sequence from the protein purified from culture filtrate starts at amino acid 29. This gives a length of the mature protein of 82 amino acids corresponding to a theoretical MW of 8337 Da and a pl of 4.23. This is in good agreement with the observed MW on SDS-PAGE at approximately 8 kDa. Analysis of the amino acid sequence predicts the presence of a signal peptide which has been cleaved of the mature protein found in culture filtrate.

CFP16: The 15 aa N-terminal sequence was found to be 100% identical to a sequence found on cosmid MTCY20H1.

The identity is found within an open reading frame of 130 amino acids length corresponding to a theoretical MW of CFP16 of 13440.4 Da and a pl of 4.59. The observed molecular weight in an SDS-PAGE gel is 16 kDa.

CFP19: The 15 aa N-terminal sequence was found to be 100% identical to a sequence found on cosmid MTCY270.

The identity is found within an open reading frame of 176 amino acids length corresponding to a theoretical MW of CFP19 of 18633.9 Da and a pl of 5.41. The observed molecular weight in an SDS-PAGE gel is 19 kDa.

CFP22A: The 15 aa N-terminal sequence was found to be 100% identical to a sequence found on cosmid MTCY1A6.

The identity is found within an open reading frame of 181 amino acids length corresponding to a theoretical MW of CFP22A of 20441.9 Da and a pl of 4.73. The observed molecular weight in an SDS-PAGE gel is 22 kDa.

CFP25A: The 15 aa N-terminal sequence was found to be 100% identical to a sequence found on contig 255.

The identity is found within an open reading frame of 228 amino acids length corresponding to a theoretical MW of CFP25A of 24574.3 Da and a pl of 4.95. The observed molecular weight in an SDS-PAGE gel is 25 kDa.

CFP27: The 15 aa N-terminal sequence was found to be 100% identical to a sequence found on cosmid MTCY261.

The identity is found within an open reading frame of 291 amino acids length. The N-terminally determined sequence from the protein purified from culture filtrate starts at amino acid 58. This gives a length of the mature protein of 233 amino acids, which corresponds to a theoretical molecular weigh at 24422.4 Da, and a theoretical pl at 4.64. The observed weight in an SDS-PAGE gel is 27 kDa.

CFP30A: Of the 13 determined amino acids in CFP30A, a 100% identical sequence was found on cosmid MTCY261.

The identity is found within an open reading frame of 248 amino acids length corresponding to a theoretical MW of CFP30A of 26881.0 Da and a pl of 5.41. The observed molecular weight in an SDS-PAGE gel is 30 kDa.

CWP32: The 15 amino acid N-terminal sequence was found to be 100% identical to a sequence found on contig 281. The identity was found within an open reading frame of 266 amino acids length, corresponding to a theoretical MW of CWP32 of 28083 Da and a pl of 4.563. The observed molecular weight in an SDS-PAGE gel is 32 kDa.

CFP50: The 15 aa N-terminal sequence was found to be 100% identical to a sequence found in MTVO38.06. The identity is found within an open reading frame of 464 amino acids length corresponding to a theoretical MW of CFP50 of 49244 Da and a pl of 5.66. The observed molecular weight in an SDS-PAGE gel is 50 kDa.

Use of homology searching in the EMBL database for identification of CFP19A and CFP23.

Homology searching in the EMBL database (using the GCG package of the Biobase, Århus-DK) with the amino acid sequences of two earlier identified highly immunoreactive ST-CF proteins, using the TFASTA algorithm, revealed that these proteins (CFP21 and CFP25, EXAMPLE 3) belong to a family of fungal cutinase homologs. Among the most homologous sequences were also two *Mycobacterium tuberculosis* sequences found on cosmid MTCY13E12. The first, MTCY13E12.04 has 46% and 50% identity to CFP25 and CFP21 respectively. The second, MTCY13E12.05, has also 46% and 50% identity to CFP25 and CFP21. The two proteins share 62.5% aa identity in a 184 residues overlap. On the basis of the high homology to the strong T-cell antigens CFP21 and CFP25, respectively, it is believed that CFP19A and CFP23 are possible new T-cell antigens.

The first reading frame encodes a 254 amino acid protein of which the first 26 aa constitute a putative leader peptide that strongly indicates an extracellular location of the protein. The mature protein is thus 228 aa in length corresponding to a theoretical MW of 23149.0 Da and a Pi of 5.80. The protein is named CFP23.

The second reading frame encodes an 231 aa protein of which the first 44 aa constitute a putative leader peptide that strongly indicates an extracellular location of the protein. The mature protein is thus 187 aa in length corresponding to a theoretical MW of 19020.3 Da and a Pi of 7.03. The protein is named CFP19A.

The presence of putative leader peptides in both proteins (and thereby their presence in the ST-CF) is confirmed by theoretical sequence analysis using the signalP program at the Expasy molecular Biology server (http://expasy.hcuge.ch/wwwltoots.html).

Searching for homologies to CFP7A, CFP16, CFP19, CFP19A, CFP19B, CFP22A, CFP23, CFP25A, CFP27, CFP30A, CWP32 and CFP50 in the EMBL database.

The amino acid sequences derived from the translated genes of the individual antigens were used for homology searching in the EMBL and Genbank databases using the TFASTA algorithm, in order to find homologous proteins and to address eventual functional roles of the antigens.

CFP7A: CFP7A has 44% identity and 70% similarity to hypothetical *Methanococcus jannaschii* protein (*M. jannaschii* from base 1162199-1175341), as well as 43% and 38% identity and 68 and 64% similarity to the C-terminal part of *B. stearothermophilus* pyruvate carboxylase *and Streptococcus mutans* biotin carboxyl carrier protein.

CFP7A contains a consensus sequence EAMKM for a biotin binding site motif which in this case was slightly modified (ESMKM in amino acid residues 34 to 38). By incubation with alkaline phosphatase conjugated streptavidin after SDS-PAGE and transfer to nitrocellulose it was demonstrated that native CFP7A was biotinylated.

CFP16: RpIL gene, 130 aa. Identical to the *M. bovis* 50s ribosomal protein L7/L12 (acc. No P37381).

CFP19: CFP19 has 47% identity and 55% similarity to *E.coli* pectinesterase homolog (ybhC gene) in a 150 aa overlap.

CFP19A: CFP19A has between 38% and 45% identity to several cutinases from different fungal sp.

In addition CFP19A has 46% identity and 61 % similarity to CFP25 as well as 50% identity and 64% similarity to CFP21 (both proteins are earlier isolated from the ST-CF).

CFP19B: No apparent homology

CFP22A: No apparent homology

CFP23: CFP23 has between 38% and 46% identity to several cutinases from different fungal sp.

In addition CFP23 has 46% identity and 61 % similarity to CFP25 as well as 50% identity and 63% similarity to CFP21 (both proteins are earlier isolated from the ST-CF).

CFP25A: CFP25A has 95% identity in a 241 aa overlap to a putative *M. tuberculosis* thymidylate synthase (450 aa accession No p28176).

CFP27: CFP27 has 81 % identity to a hypothetical *M. leprae* protein and 64% identity and 78% similarity to *Rhodococcus* sp. proteasome beta-type subunit 2 (prcB(2) gene).

CFP30A: CFP30A has 67% identity to *Rhodococcus proteasome* alfa-type 1 subunit.

CWP32: The CWP32 N-terminal sequence is 100% identical to the *Mycobacterium leprae* sequence MLCB637.03.

CFP50: The CFP50 N-terminal sequence is 100% identical to a putative lipoamide dehydrogenase from *M. leprae* (Accession 415183)

Cloning of the genes encoding CFP7A, CFP8A, CFP8B, CFP16, CFP19, CFP19A, CFP22A, CFP23, CFP25A, CFP27, CFP30A, CWP32, and CFP50.

The genes encoding CFP7A, CFP8A, CFP8B, CFP16, CFP19, CFP19A, CFP22A, CFP23, CFP25A, CFP27, CFP30A, CWP32 and CFP50 were all cloned into the expression vector pMCT6, by PCR amplification with gene specific primers, for recombinant expression in *E. coli* of the proteins.

PCR reactions contained 10 ng of *M. tuberculosis* chromosomal DNA in 1 X low salt Taq+ buffer from Stratagene supplemented with 250 mM of each of the four nucleotides (Boehringer Mannheim), 0,5 mg/ml BSA (IgG technology), 1 % DMSO (Merck), 5 pmoles of each primer and 0.5 unit Tag + DNA polymerase (Stratagene) in 10 ml reaction volume. Reactions were initially heated to 94°C for 25 sec. and run for 30 cycles of the program; 94°C for 10 sec., 55°C for 10 sec. and 72°C for 90 sec, using thermocycler equipment from Idaho Technology.

The DNA fragments were subsequently run on 1 % agarose gels, the bands were excised and purified by Spin-X spin columns (Costar) and cloned into pBluescript SK II+ - T vector (Stratagene). Plasmid DNA was hereafter prepared from clones harbouring the desired fragments, digested with suitable restriction enzymes and subcloned into the expression vector pMCT6 in frame with 8 histidines which are added to the N-terminal of the expressed proteins.

The resulting clones were hereafter sequenced by use of the dideoxy chain termination method adapted for supercoiled DNA using the Sequenase DNA sequencing kit version 1.0 (United States Biochemical Corp., USA) and by cycle sequencing using the Dye Terminator system in combination with an automated gel reader (model 373A; Applied Biosystems) according to the instructions provided. Both strands of the DNA were sequenced.

For cloning of the individual antigens, the following gene specific primers were used:

CFP7A: Primers used for cloning of *cfp*7A:

OPBR-79:    AAGAGTAGATCTATGATGGCCGAGGATGTTCGCG    (SEQ ID NO: 95)

OPBR-80:    CGGCGACGACGGATCCTACCGCGTCGG (SEQ ID NO: 96)

OPBR-79 and OPBR-80 create *Bgl*II and *Bam*HI sites, respectively, used for the cloning in pMCT6.

CFP8A: Primers used for cloning of *cfp*8A:

CFP8A-F:    CTGAGATCTATGAACCTACGGCGCC    (SEQ ID NO: 154)

CFP8A-R:    CTCCCATGGTACCCTAGGACCCGGGCAGCCCCGGC    (SEQ ID NO: 155)

CFP8A-F and CFP8A-R create *Bgl*II and Ncol sites, respectively, used for the cloning in pMCT6.

CFP8B: Primers used for cloning of *cfp*8B:

CFP8B-F:    CTGAGATCTATGAGGCTGTCGTTGACCGC    (SEQ ID NO: 156)

CFP8B-R:    CTCCCCGGGCTTAATAGTTGTTGCAGGAGC    (SEQ ID NO: 157)

CFP8B-F and CFP8B-R create *Bgl*II and *Sma*I sites, respectively, used for the cloning in pMCT6.

CFP16: Primers used for cloning of *cfp*16:

OPBR-104:    CCGGGAGATCTATGGCAAAGCTCTCCACCGACG    (SEQ ID NOs: 111 and 130)

OPBR-105:    CGCTGGGCAGAGCTACTTGACGGTGACGGTGG    (SEQ ID NOs: 112 and 131)

OPBR-104 and OPBR-105 create *Bgl*II and *Nco*I sites, respectively, used for the cloning in pMCT6.

CFP19: Primers used for cloning of *cfp*19:

OPBR-96:     GAGGAAGATCTATGACAACTTCACCCGACCCG

(SEQ ID NO: 107)

OPBR-97.     CATGAAGCCATGGCCCGCAGGCTGCATG

(SEQ ID NO: 108)

OPBR-96 and OPBR-97 create *Bgl*II and *Nco*I sites, respectively, used for the cloning in pMCT6.
CFP19A: Primers used for cloning of *cfp*19A:

OPBR-88:     CCCCCCAGATCTGCACCACCGGCATCGGCGGGC

(SEQ ID NO: 99)

OPBR-89.     GCGGCGGATCCGTTGCTTAGCCGG     (SEQ ID NO: 100)

OPBR-88 and OPBR-89 create *Bgl*II and *Bam*HI sites, respectively, used for the cloning in pMCT6.
CFP22A: Primers used for cloning of *cfp*22A:

OPBR-90:     CCGGCTGAGATCTATGACAGAATACGAAGGGC

(SEQ ID NO: 101)

OPBR-91:     CCCCGCCAGGGAACTAGAGGCGGC     (SEQ ID NO: 102)

OPBR-90 and OPBR-91 create *Bgl*II and *Nco*I sites, respectively, used for the cloning in pMCT6.
CFP23: Primers used for cloning of *cfp*23:

OPBR-86:     CCTTGGGAGATCTTTGGACCCCGGTTGC

(SEQ ID NO: 97)

OPBR-87:     GACGAGATCTTATGGGCTTACTGAC     (SEQ ID NO: 98)

OPBR-86 and OPBR-87 both create a *Bgl*II site used for the cloning in pMCT6.
CFP25A: Primers used for cloning of *cfp*25A:

OPBR-106:   GGCCCAGATCTATGGCCATTGAGGTTTCGGTGTTGC

(SEQ ID NO: 113)

OPBR-107:   CGCCGTGTTGCATGGCAGCGCTGAGC   (SEQ ID NO: 114)

OPBR-106 and OPBR-107 create *Bgl*II and *Nco*I sites, respectively, used for the cloning in pMCT6.
CFP27: Primers used for cloning of *cfp*27:

OPBR-92:       CTGCCGAGATCTACCACCATTGTCGCGCTGAAATACCC

(SEQ ID NO: 103)

OPBR-93:       CGCCATGGCCTTACGCGCCAACTCG       (SEQ ID NO: 104)

OPBR-92 and OPBR-93 create *Bgl*II and *Nco*I sites, respectively, used for the cloning in pMCT6.
CFP30A: Primers used for cloning of *cfp*30A:

OPBR-94:       GGCGGAGATCTGTGAGTTTTCCGTATTTCATC

(SEQ ID NO: 105)

OPBR-95:       CGCGTCGAGCCATGGTTAGGCGCAG       (SEQ ID NO: 106)

OPBR-94 and OPBR-95 create *Bgl*II and *Nco*I sites, respectively, used for the cloning in pMCT6.
CWP32: Primers used for cloning of *cwp*32:

CWP32-F:   GCTTAGATCTATGATTTTCTGGGCAACCAGGTA

(SEQ ID NO: 158)

CWP32-R:   GCTTCCATGGGCGAGGCACAGGCGTGGGAA   (SEQ ID NO: 159)

CWP32-F and CWP32-R create *Bgl*II and *Nco*I sites, respectively, used for the cloning in pMCT6.
CFP50: Primers used for cloning of *cfp*50:

OPBR-100:   GGCCGAGATCTGTGACCCACTATGACGTCGTCG

(SEQ ID NO: 109)

OPBR-101: GGCGCCCATGGTCAGAAATTGATCATGTGGCCAA

(SEQ ID NO: 110)

OPBR-100 and OPBR-101 create *Bgl*II and *Nco*I sites, respectively, used for the cloning in pMCT6.

Expression/purification of recombinant CFP7A, CFP8A, CFP8B, CFP16, CFP19, CFP19A, CFP22A, CFP23, CFP25A, CFP27, CFP30A, CWP32, and CFP50 proteins.

Expression and metal affinity purification of recombinant proteins was undertaken essentially as described by the manufacturers. For each protein, 1 l LB-media containing 100 $\mu$g/ml ampicillin, was inoculated with 10 ml of an overnight culture of XL1-Blue cells harbouring recombinant pMCT6 plasmids. Cultures were shaken at 37°C until they reached a density of $OD_{600}$ = 0.4 - 0.6. IPTG was hereafter added to a final concentration of 1 mM and the cultures were further incubated 4-16 hours. Cells were harvested, resuspended in 1X sonication buffer + 8 M urea and sonicated 5 X 30 sec. with 30 sec. pausing between the pulses.

After centrifugation, the lysate was applied to a column containing 25 ml of resuspended Talon resin (Clontech, Palo Alto, USA). The column was washed and eluted as described by the manufacturers.

After elution, all fractions (1.5 ml each) were subjected to analysis by SDS-PAGE using the Mighty Small (Hoefer Scientific Instruments, USA) system and the protein concentrations were estimated at 280 nm. Fractions containing recombinant protein were pooled and dialysed against 3 M urea in 10 mM Tris-HCl, pH 8.5. The dialysed protein was further purified by FPLC (Pharmacia, Sweden) using a 6 ml Resource-Q column, eluted with a linear 0-1 M gradient of NaCl. Fractions were analyzed by SDS-PAGE and protein concentrations were estimated at $OD_{280}$. Fractions containing protein were pooled and dialysed against 25 mM Hepes buffer, pH 8.5.

Finally the protein concentration and the LPS content were determined by the BCA (Pierce, Holland) and LAL (Endosafe, Charleston, USA) tests, respectively.

EXAMPLE 3B

*Identification of CFP7B, CFP10A, CFP11 and CFP30B.*

Isolation of CFP7B

ST-CF was precipitated with ammonium sulphate at 80% saturation and redissolved in PBS, pH 7.4, and dialyzed 3 times against 25 mM Piperazin-HCl, pH 5.5, and subjected to cromatofocusing on a matrix of PBE 94 (Pharmacia) in a column connected to an FPLC system (Pharmacia). The column was equilibrated with 25 mM Piperazin-HCl, pH 5.5, and the elution was performed with 10% PB74-HCl, pH 4.0 (Pharmacia). Fractions with similar band patterns were pooled and washed three times with PBS on a Centriprep concentrator (Amicon) with a 3 kDa cut off membrane to a final volume of 1-3 ml. An equal volume of SDS containing sample buffer was added and the protein solution boiled for 5 min before further separation on a MultiEluter (BioRad) in a matrix of 10-20 % polyacrylamid (Andersen,P. & Heron,I., 1993). The fraction containing a well separated band below 10 kDa was selected for N-terminal sequencing after transfer to a PVDF membrane.

Isolation of CFP11

ST-CF was precipitated with ammonium sulphate at 80% saturation. The precipitated proteins were removed by centrifugation and after resuspension washed with 8 M urea. CHAPS and glycerol were added to a final concentration of 0.5 % (w/v) and 5% (v/v) respectively and the protein solution was applied to a Rotofor isoelectrical Cell (BioRad). The Rotofor Cell had been equilibrated with an 8M urea buffer containing 0.5 % (w/v) CHAPS, 5% (v/v) glycerol, 3% (v/v) Biolyt 3/5 and 1% (v/v) Biolyt 4/6 (BioRad). Isoelectric focusing was performed in a pH gradient from 3-6. The fractions were analyzed on silver-stained 10-20% SDS-PAGE. The fractions in the pH gradient 5.5 to 6 were pooled and washed three times with PBS on a Centriprep concentrator (Amicon) with a 3 kDa cut off membrane to a final volume of 1 ml. 300 mg of the protein preparation was separated on a 10-20% Tricine SDS-PAGE (Ploug et al 1989) and transferred to a PVDF membrane and Coomassie stained. The lowest band occurring on the membrane was excised and submitted for N-terminal sequencing.

Isolation of CFP10A and CFP30B

ST-CF was concentrated approximately 10-fold by ultrafiltration and ammonium sulphate precipitation at 80 % saturation. Proteins were redissolved in 50 mM sodium phosphate, 1.5 M ammonium sulphate, pH 8.5, and subjected to thiophilic adsorption chromatography on an Affi-T gel column (Kem-En-Tec). Proteins were eluted by a 1.5 to 0 M decreasing gradient of ammonium sulphate. Fractions with similar band patterns in SDS-PAGE were pooled and anion exchange chromatography was performed on a Mono Q HR 5/5 column connected to an FPLC system (Pharmacia). The column was equilibrated with 10 mM Tris-HCl, pH 8.5, and the elution was performed with a gradient of NaCl from 0 to 1 M. Fractions containing well separated bands in SDS-PAGE were selected.

Fractions containing CFP10A and CFP30B were blotted to PVDF membrane after 2-DE PAGE (Ploug et al, 1989). The relevant spots were excised and subjected to N-terminal amino acid sequence analysis.

N-terminal sequencing

N-terminal amino acid sequence analysis was performed on a Procise 494 sequencer (applied Biosystems).
The following N-terminal sequences were obtained:

| | | |
|---|---|---|
| CFP7B: | PQGTVKWFNAEKGFG | (SEQ ID NO: 168) |
| CFP10A: | NVTVSIPTILRPXXX | (SEQ ID NO: 169) |
| CFP11: | TRFMTDPHAMRDMAG | (SEQ ID NO: 170) |
| CFP30B: | PKRSEYRQGTPNWVD | (SEQ ID NO: 171) |

"X" denotes an amino acid which could not be determined by the sequencing method used.

N-terminal homology searching in the Sanger database and identification of the corresponding genes.

The N-terminal amino acid sequence from each of the proteins was used for a homology search using the blast program of the Sanger *Mycobacterium tuberculosis* genome database:
http//www.sanger.ac.uk/projects/m-tuberculosis/TB-blast-server.
For CFP11 a sequence 100% identical to 15 N-terminal amino acids was found on contig TB_1314. The identity was found within an open reading frame of 98 amino acids length corresponding to a theoretical MW of 10977 Da and a pI of 5.14.
Amino acid number one can also be an Ala (insted of a Thr) as this sequence was also obtained (results not shown), and a 100% identical sequence to this N-terminal is found on contig TB_671 and on locus MTCI364.09.
For CFP7B a sequence 100% identical to 15 N-terminal amino acids was found on contig TB_2044 and on locus MTY15C10.04 with EMBL accession number: z95436. The identity was found within an open reading frame of 67 amino acids length corresponding to a theoretical MW of 7240 Da and a pI of 5.18.
For CFP10A a sequence 100% identical to 12 N-terminal amino acids was found on contig TB_752 and on locus CY130.20 with EMBL accession number: Q10646 and Z73902. The identity was found within an open reading frame of 93 amino acids length corresponding to a theoretical MW of 9557 Da and a pI of 4.78.
For CFP30B a sequence 100% identical to 15 N-terminal amino acids was found on contig TB_335. The identity was found within an open reading frame of 261 amino acids length corresponding to a theoretical MW of 27345 Da and a pI of 4.24.
The amino acid sequences of the purified antigens as picked from the Sanger database are shown in the following list.

CFP7B (SEQ ID NO: 147)

1    MPQGTVKWFN AEKGFGFIAP EDGSADVFVH YTEIQGTGFR TLEENQKVEF

51    EIGHSPKGPQ ATGVRSL

CFP10A (SEQ ID NO: 141)

1      MNVTVSIPTI LRPHTGGQKS VSASGDTLGA VISDLEANYS GISERLMDPS

51     SPGKLHRFVN IYVNDEDVRF SGGLATAIAD GDSVTILPAV AGG

CFP11 protein sequence (SEQ ID NO: 143)

1      MATRFMTDPH AMRDMAGRFE VHAQTVEDEA RRMWASAQNI SGAGWSGMAE

51     ATSLDTMAQM NQAFRNIVNM LHGVRDGLVR DANNYEQQEQ ASQQILSS

CFP30B (SEQ ID NO: 145)

1      MPKRSEYRQG TPNWVDLQTT DQSAAKKFYT SLFGWGYDDN PVPGGGGVYS

51     MATLNGEAVA AIAPMPPGAP EGMPPIWNTY IAVDDVDAVV DKVVPGGGQV

101    MMPAFDIGDA GRMSFITDPT GAAVGLWQAN RHIGATLVNE TGTLIWNELL

151    TDKPDLALAF YEAVVGLTHS SMEIAAGQNY RVLKAGDAEV GGCMEPPMPG

201    VPNHWHVYFA VDDADATAAK AAAAGGQVIA EPADIPSVGR FAVLSDPQGA

251    IFSVLKPAPQ Q

Cloning of the genes encoding CFP7B, CFP10A, CFP11, and CFP30B.

PCR reactions contained 10 ng of *M. tuberculosis* chromosomal DNA in 1 X low salt Taq+ buffer from Stratagene supplemented with 250 mM of each of the four nucleotides (Boehringer Mannheim), 0,5 mg/ml BSA (IgG technology), 1 % DMSO (Merck), 5 pmoles of each primer and 0.5 unit Tag+ DNA polymerase (Stratagene) in 10 ml reaction volume. Reactions were initially heated to 94°C for 25 sec. and run for 30 cycles of the program; 94°C for 10 sec., 55°C for 10 sec. and 72°C for 90 sec., using thermocycler equipment from Idaho Technology.

The DNA fragments were subsequently run on 1 % agarose gels, the bands were excised and purified by Spin-X spin columns (Costar) and cloned into pBluscript SK II + - T vector (Stratagene). Plasmid DNA was hereafter prepared from clones harbouring the desired fragments, digested with suitable restriction enzymes and subcloned into the expression vector pMCT6 in frame with 8 histidines which are added to the N-terminal of the expressed proteins. The resulting clones were hereafter sequenced by use of the dideoxy chain termination method adapted for supercoiled DNA using the Sequenase DNA sequencing kit version 1.0 (United States Biochemical Corp., USA) and by cycle sequencing using the Dye Terminator system in combination with an automated gel reader (model 373A; Applied Biosystems) according to the instructions provided. Both strands of the DNA were sequenced.

For cloning of the individual antigens, the following gene specific primers were used:

CFP7B: Primers used for cloning of *cfp*7B:

CFP7B-F: CTGAGATCTAGAATGCCACAGGGAACTGTG      (SEQ ID NO: 160)

CFP7B-R: TCTCCCGGGGGTAACTCAGAGCGAGCGGAC        (SEQ ID NO: 161)

CFP7B-F and CFP7B-R create *Bgl*II and *Sma*I sites, respectively, used for the cloning in pMCT6.
CFP10A: Primers used for cloning of *cfp*10A:

CFP10A-F:    CTGAGATCTATGAACGTCACCGTATCC  (SEQ ID NO: 162)

CFP10A-R:    TCTCCCGGGGCTCACCCACCGGCCACG  (SEQ ID NO: 163)

CFP10A -F and CFP10A -R create *Bgl*II and *Sma*I sites, respectively, used for the cloning in pMCT6.
CFP11: Primers used for cloning of *cfp*11:

CFP11-F: CTGAGATCTATGGCAACACGTTTTATGACG        (SEQ ID NO: 164)

CFP11-R: CTCCCCGGGTTAGCTGCTGAGGATCTGCTH        (SEQ ID NO: 165)

CFP11-F and CFP11-R create *Bgl*II and *Sma*I sites, respectively, used for the cloning in pMCT6.
CFP30B: Primers used for cloning of *cfp*30B:

CFP30B-F:    CTGAAGATCTATGCCCAAGAGAAGCGAATAC  (SEQ ID NO: 166)

CFP30B -R:    CGGCAGCTGCTAGCATTCTCCGAATCTGCCG    (SEQ ID NO: 167)

CFP30B-F and CFP30B-R create *Bgl*II and *Pvu*II sites, respectively, used for the cloning in pMCT6.

Expression/purification of recombinant CFP7B, CFP10A, CFP11 and CFP30B protein.

Expression and metal affinity purification of recombinant protein was undertaken essentially as described by the manufacturers. 1 l LB-media containing 100 µg/ml ampicillin, was inoculated with 10 ml of an overnight culture of XL1-Blue cells harbouring recombinant pMCT6 plasmid. The culture was shaken at 37 °C until it reached a density of $OD_{600}$ = 0.5. IPTG was hereafter added to a final concentration of 1 mM and the culture was further incubated 4 hours. Cells were harvested, resuspended in 1 X sonication buffer + 8 M urea and sonicated 5 X 30 sec. with 30 sec. pausing between the pulses.
After centrifugation, the lysate was applied to a column containing 25 ml of resuspended Talon resin (Clontech, Palo Alto, USA). The column was washed and eluted as described by the manufacturers.
After elution, all fractions (1.5 ml each) were subjected to analysis by SDS-PAGE using the Mighty Small (Hoefer Scientific Instruments, USA) system and the protein concentrations were estimated at 280 nm. Fractions containing recombinant protein were pooled and dialysed against 3 M urea in 10 mM Tris-HCl, pH 8.5. The dialysed protein was further purified by FPLC (Pharmacia, Sweden) using a 6 ml Resource-Q column, eluted with a linear 0-1 M gradient of NaCl. Fractions were analysed by SDS-PAGE and protein concentrations were estimated at $OD_{280}$. Fractions containing protein were pooled and dialysed against 25 mM Hepes buffer, pH 8.5.
Finally the protein concentration and the LPS content was determined by the BCA (Pierce, Holland) and LAL (Endosafe, Charleston, USA) tests, respectively.

EXAMPLE 3C

*Using homology searching for identification of ORF11-1, ORF11-2, ORF11-3 and ORF11-4.*

A search of the *Mycobacterium tuberculosis* Sanger sequence database with the amino acid sequences of CFP11, a previously identified ST-CF protein, identified 4 new very homologous proteins. All 4 proteins were at least 96% homologous to CFP11.

On the basis of the strong homology to CFP11, it is belived that ORF11-1, ORF11-2, ORF11-3 and ORF11-4 are potential new T-cell antigens.

The first open reading frame, MTCY10G2.11, homologous to CFP11, encodes a protein of 98 amino acids corresponding to a theoretical molecular mass of 10994Da and a pl of 5.14. The protein was named ORF11-1.

The second open reading frame, MTCI364.09, homologous to CFP11, encodes a protein of 98 amino acids corresponding to a theoretical molecular mass of 10964Da and a pl of 5.14. The protein was named ORF11-2.

The third open reading frame, MTV049.14, has an in frame stop codon. Because of the very conserved DNA sequence in this position amongst the 4 open reading frames it is however suggested that this is due to a sequence mistake.

The "T" in position 175 of the DNA sequence is therefor suggested to be a "C" as in the four other ORF's. The Q in position 59 in the amino acid sequence would have been a "stop" if the T in position 175 in the DNA sequence had not been substituted. The open reading frame encodes a protein of 98 amino acids corresponding to a theoretical molecular mass of 10994Da and a pl of 5.14. The protein was named ORF11-3.

The fourth open reading frame, MTCY15C10.32, homologous to CFP11, encodes a protein of 98 amino acids corresponding to a theoretical molecular mass of 11024Da and a pl of 5.14. The protein was named ORF11-4.

Using homology searching for identification of ORF7-1 and ORF7-2.

A search of the *Mycobacterium tuberculosis* Sanger sequence database with the amino acid sequences of a previously identified immunoreactive ST-CF protein, CFP7, identified 2 new very homologous proteins. The protein ORF7-1 (MTV012.33) was 84% identical to CFP7, with a primary structure of the same size as CFP7, and the protein ORF7-2 (MTV012.31) was 68% identical to CFP7 in a 69 amino acid overlap. On the basis of the strong homology to the potent human T-cell antigen CFP7, ORF7-1 and ORF7-2 are belived to be potential new T-cell antigens.

The first open reading frame homologous to CFP7, encodes a protein of 96 amino acids corresponding to a theoretical molecular mass of 10313Da and a pl of 4.186. The protein was named ORF7-1.

The second open reading frame homologous to CFP7, encodes a protein of 1 20 amino acids corresponding to a theoretical molecular mass of 12923.00 Da and a pl of 7.889. The protein was named ORF7-2.

Cloning of the homologous *orf7-1* and *orf7-2*.

Since *ORF7-1* and *ORF7-2* are nearly identical to CFP7 it was nessesary to use the flanking DNA regions in the cloning procedure, to ensure the cloning of the correct ORF. Two PCR reactions were carried out with two different primer sets. PCR reaction 1 was carried out using *M. tuberculosis* chromosomal DNA and a primerset corresponding to the flanking DNA. PCR reaction 2 was carried out directly on the first PCR product using ORF specific primers which introduced restriction sites for use in the later cloning procedure.

The sequences of the primers used are given below;

*Orf7-1*:

Primers used for the initial PCR reaction (1) using *M. tuberculosis* chromosomal DNA as template;

Sence:MTV012.33-R1: 5'- GGAATGAAAAGGGGTTTGTG - 3' (SEQ ID NO: 186)

Antisence:MTV012.33-F1: 5'- GACCACGCCCGCGCCGTGTG - 3'(SEQ ID NO:187)

Primers used for the second round of PCR (2) using PCR product 1 as template;

Sence: MTV012.33-R2:  5' - **_GCAACACCCGGG_**ATGTCGCAGATTATG - 3'

(SEQ ID NO: 188)

(introduces a *Sma*I upstream of the *orf7-1* start codon)


Antisence:MTV012.33-F2:  5' - **_CTAAGCTTGGATCC_**CTAGCCGCCCCACTTG - 3'

((SEQ ID NO: 189)

(introduces a *Bam*HI downstream of the *orf7-1* stop codon).


*Orf7-2:*

Primers used for the initial PCR reaction (1) using *M. tuberculosis* chromosomal DNA as template;


Sence: MTV012.31-R1: 5'- GAATATTTGAAAGGGATTCGTG - 3'   (SEQ ID NO: 190)


Antisence:MTV012.31-F1: 5'- **_CTACTAAGCTTGGATCC_**TTAGTCTCCGGCG - 3'

(SEQ ID NO: 191)

(introduces a *Bam*HI downstream of the *orf7-2* stop codon)


Primers used for the second round of PCR (2) using PCR product 1 as template;


Sence: MTV012.31-R2:  5' -**_GCAACACCCGGG_**GTGTCGCAGAGTATG- 3'

(SEQ ID NO: 192)

(introduces a *Sma*I upstream of the *orf7-2* start codon)


Antisence:MTV012.31-F1: 5'- **_CTACTAAGCTTGGATCC_**TTAGTCTCCGGCG - 3'

(SEQ ID NO: 193)

(introduces a BamHI downstream of the orf7-2 stop codon)


The genes encoding ORF7-1 and ORF7-2 were cloned into the expression vector pMST24, by PCR amplification with gene specific primers, for recombinant expression in *E. coli* of the proteins.
The first PCR reactions contained either 10 ng of *M. tuberculosis* chromosomal DNA (PCR reaction 1) or 10ng PCR product 1 (PCR reaction 2) in 1 x low salt Taq + buffer from Stratagene supplemented with 250 mM of each of the four nucleotides (Boehringer Mannheim), 0,5 mg/ml BSA (IgG technology), 1 % DMSO (Merck), 5 pmoles of each primer and 0.5 unit Tag + DNA polymerase (Stratagene) in 10 ml reaction volume. Reactions were initially heated to 94°C for 25 sec. and run for 30 cycles of the program; 94°C for 10 sec., 55°C for 10 sec. and 72°C for 90 sec, using thermocycler equipment from Idaho Technology.
The DNA fragments were subsequently run on 1 % agarose gels, the bands were excised and purified by Spin-X spin columns (Costar) and cloned into pBluscript SK II + - T vector (Stratagene). Plasmid DNA was hereafter prepared from clones harbouring the desired fragments, digested with suitable restriction enzymes and subcloned into the expression vector pMST24 in frame with 6 histidines which are added to the N-terminal of the expressed proteins. The resulting clones were hereafter sequenced by use of the dideoxy chain termination method adapted for supercoiled DNA using the Sequenase DNA sequencing kit version 1.0 (United States Biochemical Corp., USA) and by cycle sequencing using the Dye Terminator system in combination with an automated gel reader (model 373A; Applied Biosystems) according to the instructions provided. Both strands of the DNA were sequenced.

Expression/purification of recombinant ORF7-1 and ORF7-2 protein.

Expression and metal affinity purification of recombinant protein was undertaken essentially as described by the manufacturers. 1 I LB-media containing 100 µg/ml ampicillin, was inoculated with 10 ml of an overnight culture of XL1-Blue cells harbouring recombinant pMCT6 plasmid. The culture was shaken at 37 °C until it reached a density of OD600 = 0.5. IPTG was hereafter added to a final concentration of 1 mM and the culture was further incubated 2 hours. Cells were harvested, resuspended in 1 X sonication buffer + 8 M urea and sonicated 5 X 30 sec. with 30 sec. pausing between the pulses.

After centrifugation, the lysate was applied to a column containing 25 ml of resuspended Talon resin (Clontech, Palo Alto, USA). The column was washed and eluted as described by the manufacturers.

After elution, all fractions (1.5 ml each) were subjected to analysis by SDS-PAGE using the Mighty Small (Hoefer Scientific Instruments, USA) system. Fractions containing recombinant protein were pooled and dialysed against 3 M urea in 10 mM Tris-HCl, pH 8.5. The dialysed protein was further purified by FPLC (Pharmacia, Sweden) using a 6 ml Resource-Q column, eluted with a linear 0-1 M gradient of NaCl. Fractions were analysed by SDS-PAGE. Fractions containing protein were pooled and dialysed against 25 mM Hepes buffer, pH 8.5.

Finally the protein concentration and the LPS content was determined by the BCA (Pierce, Holland) and LAL (Endosafe, Charleston, USA) tests, respectively.

EXAMPLE 4

*Cloning of the gene expressing CFP26 (MPT51)*

Synthesis and design of probes

Oligonucleotide primers were synthesized automatically on a DNA synthesizer (Applied Biosystems, Forster City, Ca, ABI-391, PCR-mode) deblocked and purified by ethanol precipitation.

Three oligonucleotides were synthesized (TABLE 3) on the basis of the nucleotide sequence from *mpb51* described by Ohara *et al*. (1995). The oligonucleotides were engineered to include an *Eco*RI restriction enzyme site at the 5' end and at the 3' end by which a later subcloning was possible.

Additional four oligonucleotides were synthesized on the basis of the nucleotide sequence from MPT51 (Fig. 5 and SEQ ID NO: 41). The four combinations of the primers were used for the PCR studies.

DNA cloning and PCR technology

Standard procedures were used for the preparation and handling of DNA (Sambrook *et al*., 1989). The gene *mpt51* was cloned from *M. tuberculosis* H37Rv chromosomal DNA by the use of the polymerase chain reactions (PCR) technology as described previously (Oettinger and Andersen, 1994). The PCR product was cloned in the pBluescriptSK + (Stratagene).

Cloning of *mpt51*

The gene, the signal sequence and the Shine Delgarno region of MPT51 was cloned by use of the PCR technology as two fragments of 952 bp and 815 bp in pBluescript SK +, designated pTO52 and pTO53.

DNA Sequencing

The nucleotide sequence of the cloned 952 bp *M. tuberculosis* H37Rv PCR fragment, pTO52, containing the Shine Dalgarno sequence, the signal peptide sequence and the structural gene of MPT51, and the nucleotide sequence of the cloned 815 bp PCR fragment containing the structural gene of MPT51, pTO53, were determined by the dideoxy chain termination method adapted for supercoiled DNA by use of the Sequenase DNA sequencing kit version 1.0 (United States Biochemical Corp., Cleveland, OH) and by cycle sequencing using the Dye Terminator system in combination with an automated gel reader (model 373A; Applied Biosystems) according to the instructions provided. Both strands of the DNA were sequenced.

The nucleotide sequences of pTO52 and pTO53 and the deduced amino acid sequence are shown in Figure 5. The DNA sequence contained an open reading frame starting with a ATG codon at position 45 - 47 and ending with a termination codon (TAA) at position 942 - 944. The nucleotide sequence of the first 33 codons was expected to encode the signal sequence. On the basis of the known N-terminal amino acid sequence (Ala - Pro - Tyr - Glu - Asn) of the purified MPT51 (Nagai *et al.*, 1991) and the features of the signal peptide, it is presumed that the signal peptidase recognition sequence (Ala-X-Ala) (von Heijne, 1984) is located in front of the N-terminal region of the mature protein at position 144. Therefore, a structural gene encoding MPT51, *mpt51*, derived from *M. tuberculosis* H37Rv was found to be located at position 144 - 945 of the sequence shown in Fig. 5. The nucleotide sequence of *mpt51* differed with one nucleotide compared to the nucleotide sequence of MPB51 described by Ohara *et al.* (1995) (Fig. 5). In *mpt51* at position 780 was found a substitution of a guanine to an adenine. From the deduced amino acid sequence this change occurs at a first position of the codon giving a amino acid change from alanine to threonine. Thus it is concluded, that *mpt51* consists of 801 bp and that the deduced amino acid sequence contains 266 residues with a molecular weight of 27,842, and MPT51 show 99,8% identity to MPB51.

Subcloning of *mpt51*

An *Eco*RI site was engineered immediately 5' of the first codon of *mpt51* so that only the coding region of the gene encoding MPT51 would be expressed, and an *Eco*RI site was incorporated right after the stop codon at the 3' end.
DNA of the recombinant plasmid pTO53 was cleaved at the *Eco*RI sites. The 815 bp fragment was purified from an agarose gel and subcloned into the *Eco*RI site of the pMAL-*cR1* expression vector (New England Biolabs), pTO54. Vector DNA containing the gene fusion was used to transform the *E. coli* XL1-Blue by the standard procedures for DNA manipulation.
The endpoints of the gene fusion were determined by the dideoxy chain termination method as described under section DNA sequencing. Both strands of the DNA were sequenced.

Preparation and purification of rMPT51

Recombinant antigen was prepared in accordance with instructions provided by New England Biolabs. Briefly, single colonies of *E. coli* harbouring the pTO54 plasmid were inoculated into Luria-Bertani broth containing 50 µg/ml ampicillin and 12.5 µg/ml tetracycline and grown at 37°C to $2 \times 10^8$ cells/ml. Isopropyl-β-D-thiogalactoside (IPTG) was then added to a final concentration of 0.3 mM and growth was continued for further 2 hours. The pelleted bacteria were stored overnight at -20°C in new column buffer (20 mM Tris/HCl, pH 7.4, 200 mM NaCl, 1 mM EDTA, 1 mM dithiothreitol (DTT))and thawed at 4°C followed by incubation with 1 mg/ml lysozyme on ice for 30 min and sonication (20 times for 10 sec with intervals of 20 sec). After centrifugation at 9,000 x g for 30 min at 4°C, the maltose binding protein -MPT51 fusion protein (MBP-rMPT51 ) was purified from the crude extract by affinity chromatography on amylose resin column. MBP-rMPT51 binds to amylose. After extensive washes of the column, the fusion protein was eluted with 10 mM maltose. Aliquots of the fractions were analyzed on 10% SDS-PAGE. Fractions containing the fusion protein of interest were pooled and was dialysed extensively against physiological saline.
Protein concentration was determined by the BCA method supplied by Pierce (Pierce Chemical Company, Rockford, IL).

TABLE 3.

Sequence of the *mpt51* oligonucleotides[a].

| Orientation and oli- gonucleotide[a] | Sequences (5'→ 3') | Position[b] (nucleotide) |
|---|---|---|
| Sense | | |
| MPT51-1 | CTCGAATTCGCCGGGTGCACACAG (SEQ ID NO: 28) | 6 - 21 (SEQ ID NO: 41) |
| MPT51-3 | CTCGAATTCGCCCCATACGAGAAC (SEQ ID NO: 29) | 143 - 158 (SEQ ID NO: 41) |
| MPT51-5 | GTGTATCTGCTGGAC (SEQ ID NO: 30) | 228 - 242 (SEQ ID NO: 41) |
| MPT51-7 | CCGACTGGCTGGCCG (SEQ ID NO: 31) | 418 - 432 (SEQ ID NO: 41) |
| Antisense | | |
| MPT51-2 | GAGGAATTCGCTTAGCGGATCGCA (SEQ ID NO: 32) | 946 - 932 (SEQ ID NO: 41) |
| MPT51-4 | CCCACATTCCGTTGG (SEQ ID NO: 33) | 642 - 628 (SEQ ID NO: 41) |
| MPT51-6 | GTCCAGCAGATACAC (SEQ ID NO: 34) | 242 - 228 (SEQ ID NO: 41) |

[a] The oligonucleotides MPT51-1 and MPT51-2 were constructed from the MPB51 nucleotide sequence (Ohara *et al.*, 1995). The other oligonucleotides constructions were based on the nucleotide sequence obtained from *mpt51* reported in this work. Nucleotides (nt) underlined are not contained in the nucleotide sequence of MPB/T51.

[b] The positions referred to are of the non-underlined parts of the primers and correspond to the nucleotide sequence shown in SEQ ID NO: 41.

Cloning of *mpt51* in the expression vector pMST24.

A PCR fragment was produced from pTO52 using the primer combination MPT51-F and MPT51-R (TABLE 4). A *Bam*HI site was engineered immediately 5' of the first codon of *mpt51* so that only the coding region of the gene encoding MPT51 would be expressed, and an *Nco*I site was incorporated right after the stop codon at the 3' end.
The PCR product was cleaved at the *Bam*HI and the *Nco*I site. The 811 bp fragment was purified from an agarose gel and subcloned into the *Bam*HI and the *Nco*I site of the pMST24 expression vector, pTO86. Vector DNA containing the gene fusion was used to transform the *E. coli* XL1-Blue by the standard procedures for DNA manipulation.
The nucleotide sequence of complete gene fusion was determined by the dideoxy chain termination method as described under section DNA sequencing. Both strands of the DNA were sequenced.

Preparation and purification of rMPT51.

Recombinant antigen was prepared from single colonies of *E. coli* harbouring the pTO86 plasmid inoculated into Luria-Bertani broth containing 50 µg/ml ampicillin and 12.5 µg/ml tetracycline and grown at 37°C to $2 \times 10^8$ cells/ml. Isopropyl-β-D-thiogalactoside (IPTG) was then added to a final concentration of 1 mM and growth was continued for further 2 hours. The pelleted bacteria were resuspended in BC 100/20 buffer (100 mM KCl, 20 mM Imidazole, 20 mM Tris/HCl, pH 7.9, 20 % glycerol). Cells were broken by sonication (20 times for 10 sec with intervals of 20 sec). After centrifugation at 9,000 x g for 30 min. at 4°C the insoluble matter was resuspended in BC 100/20 buffer with 8 M urea followed by sonication and centrifugation as above. The 6 x His tag-MPT51 fusion protein (His-rMPT51) was purified by affinity chromatography on Ni-NTA resin column (Qiagen, Hilden, Germany). His-rMPT51 binds to Ni-NTA. After extensive washes of the column, the fusion protein was eluted with BC 100/40 buffer (100 mM KCl, 40 mM Imidazole, 20 mM Tris/HCl, pH 7.9, 20 % glycerol) with 8 M urea and BC 1000/40 buffer (1000 mM KCl, 40 mM Imidazole, 20 mM Tris/HCl, pH 7.9, 20 % glycerol) with 8 M urea. His-rMPT51 was extensive dialysed against 10 mM Tris/HCl, pH 8.5, 3 M urea followed by purification using fast protein liquid chromatography (FPLC) (Pharmacia, Uppsala, Sweden), over an anion exchange column (Mono Q) using 10 mM Tris/HCl, pH 8.5, 3 M urea with a 0 - 1 M NaCl linear gradient. Fractions containing rMPT51 were pooled and subsequently dialysed extensively against 25 mM Hepes, pH 8.0 before use.
Protein concentration was determined by the BCA method supplied by Pierce (Pierce Chemical Company, Rockford, IL).
The lipopolysaccharide (LPS) content was determined by the limulus amoebocyte lysate test (LAL) to be less than 0.004 ng/µg rMPT51, and this concentration had no influence on cellular activity.

**TABLE 4.** Sequence of the *mpt51* oligonucleotides.

| Orientation and oligonucleotide | Sequences (5' → 3') | Position (nt) |
|---|---|---|
| Sense | | |
| MPT51-F | <u>CTCGGATCCT</u>GCCCCATACGAGAACCTG | 139 - 156 |
| Antisense | | |
| MPT51-R | <u>CTCCCATGG</u>TTAGCGGATCGCACCG | 939 - 924 |

EXAMPLE 4A

*Cloning of the ESAT6-MPT59 and the MPT59-ESA T6 hybrides.*

Background for ESAT-MPT59 and MPT59-ESAT6 fusion

Several studies have demonstrated that ESAT-6 is a an immunogen which is relatively difficult to adjuvate in order to obtain consistent results when immunizing therewith. To detect an *in vitro* recognition of ESAT-6 after immunization with the antigen is very difficult compared to the strong recognition of the antigen that has been found during the recall of memory immunity to *M. tuberculosis.* ESAT-6 has been found in ST-CF in a truncated version were amino acids 1-15 have been deleted. The deletion includes the main T-cell epitopes recognized by C57BL/6j mice (Brandt et al., 1996). This result indicates that ESAT-6 either is N-terminally processed or proteolytically degraded in STCF. In order to optimize ESAT-6 as an immunogen, a gene fusion between ESAT-6 and another major T cell antigen MPT59 has been constructed. Two different construct have been made: MPT59-ESAT-6 (SEQ ID NO: 172) and ESAT-6-MPT59 (SEQ ID NO: 173). In the first hybrid ESAT-6 is N-terminally protected by MPT59 and in the latter it is expected that the fusion of two dominant T-cell antigens can have a synergistic effect.

The genes encoding the ESAT6-MPT59 and the MPT59-ESAT6 hybrides were cloned into the expression vector pMCT6, by PCR amplification with gene specific primers, for recombinant expression in *E. coli* of the hybrid proteins.

Construction of the hybrid MPT59-ESAT6.

The cloning was carried out in three steps. First the genes encoding the two components of the hybrid, ESAT6 and MPT59, were PCR amplified using the following primer constructions:

ESAT6:

OPBR-4:       GGCGCCGGCAAGCTTGCCATGACAGAGCAGCAGTGG

(SEQ ID NO: 132)

OPBR-28:      CGAACTCGCCGGATCCCGTGTTTCGC     (SEQ ID NO: 133)

OPBR-4 and OPBR-28 create HinDIII and BamHI sites, respectively.

MPT59:

OPBR-48:      GGCAACCGCGAGATCTTTCTCCCGGCCGGGGC (SEQ ID NO: 134)

OPBR-3:       GGCAAGCTTGCCGGCGCCTAACGAACT  (SEQ ID NO: 135)

OPBR-48 and OPBR-3 create BglII and HinDIII, respectively. Additionally OPBR-3 deletes the stop codon of MPT59.

PCR reactions contained 10 ng of *M. tuberculosis* chromosomal DNA in 1 x low salt Taq+ buffer from Stratagene supplemented with 250 mM of each of the four nucleotides (Boehringer Mannheim), 0,5 mg/ml BSA (IgG technology), 1 % DMSO (Merck), 5 pmoles of each primer and 0.5 unit Tag + DNA polymerase (Stratagene) in 10 $\mu$l reaction volume. Reactions were initially heated to 94°C for 25 sec. and run for 30 cycles of the program; 94°C for 10 sec., 55°C for 10 sec. and 72°C for 90 sec, using thermocycler equipment from Idaho Technology.

The DNA fragments were subsequently run on 1 % agarose gels, the bands were excised and purified by Spin-X spin columns (Costar). The two PCR fragments were digested with HinDIII and ligated. A PCR amplification of the ligated PCR fragments encoding MPT59-ESAT6 was carried out using the primers OPBR-48 and OPBR-28. PCR reaction was initially heated to 94°C for 25 sec. and run for 30 cycles of the program; 94°C for 30 sec., 55°C for 30 sec. and 72°C for 90 sec. The resulting PCR fragment was digested with BglII and BamHI and cloned into the expression vector pMCT6 in frame with 8 histidines which are added to the N-terminal of the expressed protein hybrid. The resulting clones were hereafter sequenced by use of the dideoxy chain termination method adapted for supercoiled DNA using the Sequenase DNA sequencing kit version 1.0 (United States Biochemical Corp., USA) and by cycle sequencing using the Dye Terminator system in combination with an automated gel reader (model 373A; Applied Biosystems) according to the instructions provided. Both strands of the DNA were sequenced.

Construction of the hybrid ESAT6-MPT59.

Construction of the hybrid ESAT6-MPT59 was carried out as described for the hybrid MPT59-ESAT6. The primers used for the construction and cloning were:

ESAT6:

OPBR-75:    GGACCCAGATCTATGACAGAGCAGCAGTGG    (SEQ ID NO: 136)

OPBR-76:    CCGGCAGCCCCGGCCGGGAGAAAAGCTTTGCGAACATCCCAGTGACG
(SEQ ID NO: 137)

OPBR-75 and OPBR-76 create BglII and HinDIII sites, respectively. Additionally OPBR-76 deletes the stop codon of ESAT6.

MPT59:

OPBR-77:    GTTCGCAAAGCTTTTCTCCCGGCCGGGGCTGCCGGTCGAGTACC
(SEQ ID NO: 138)

OPBR-18:    CCTTCGGTGGATCCCGTCAG    (SEQ ID NO: 139)

OPBR-77 and OPBR-18 create HinDIII and BamHI sites, respectively.

Expression/purification of MPT59-ESAT6 and ESAT6-MPT59 hybrid proteins.

Expression and metal affinity purification of recombinant proteins was undertaken essentially as described by the manufacturers. For each protein, 1l LB-media containing 100 µg/ml ampicillin, was inoculated with 10 ml of an overnight culture of XL1-Blue cells harbouring recombinant pMCT6 plasmids. Cultures were shaken at 37 °C until they reached a density of $OD_{600}$ = 0.4 - 0.6. IPTG was hereafter added to a final concentration of 1 mM and the cultures were further incubated 4 - 16 hours. Cells were harvested, resuspended in 1X sonication buffer + 8 M urea and sonicated 5 X 30 sec. with 30 sec. pausing between the pulses.
After centrifugation, the lysate was applied to a column containing 25 ml of resuspended Talon resin (Clontech, Palo Alto, USA). The column was washed and eluted as described by the manufacturers.
After elution, all fractions (1.5 ml each) were subjected to analysis by SDS-PAGE using the Mighty Small (Hoefer Scientific Instruments, USA) system and the protein concentrations were estimated at 280 nm. Fractions containing recombinant protein were pooled and dialysed against 3 M urea in 10 mM Tris-HCl, pH 8.5. The dialysed protein was further purified by FPLC (Pharmacia, Sweden) using a 6 ml Resource-Q column, eluted with a linear 0-1 M gradient of NaCl. Fractions were analyzed by SDS-PAGE and protein concentrations were estimated at $OD_{280}$. Fractions containing protein were pooled and dialysed against 25 mM Hepes buffer, pH 8.5.
Finally the protein concentration and the LPS content were determined by the BCA (Pierce, Holland) and LAL (Endosafe, Charleston, USA) tests, respectively.
The biological activity of the MPT59-ESAT6 fusion protein is described in Example 6A.

EXAMPLE 5

*Mapping of the purified antigens in a 2DE system.*

In order to characterize the purified antigens they were mapped in a 2-dimensional electrophoresis (2DE) reference system. This consists of a silver stained gel containing ST-CF proteins separated by isoelectrical focusing followed by a separation according to size in a polyacrylamide gel electrophoresis. The 2DE was performed according to Hochstrasser *et al*. (1988). 85 μg of ST-CF was applied to the isoelectrical focusing tubes where BioRad ampholytes BioLyt 4-6 (2 parts) and BioLyt 5-7 (3 parts) were included. The first dimension was performed in acrylamide/piperazin diacrylamide tube gels in the presence of urea, the detergent CHAPS and the reducing agent DTT at 400 V for 18 hours and 800 V for 2 hours. The second dimension 10-20% SDS-PAGE was performed at 100 V for 18 hours and silver stained. The identification of CFP7, CFP7A, CFP7B, CFP8A, CFP8B, CFP9, CFP11, CFP16, CFP17, CFP19, CFP20, CFP21, CFP22, CFP25, CFP27, CFP28, CFP29, CFP30A, CFP50, and MPT51 in the 2DE reference gel were done by comparing the spot pattern of the purified antigen with ST-CF with and without the purified antigen. By the assistance of an analytical 2DE software system (Phoretix International, UK) the spots have been identified in Fig. 6. The position of MPT51 and CFP29 were confirmed by a Western blot of the 2DE gel using the Mab's anti-CFP29 and HBT 4.

EXAMPLE 6

*Biological activity of the purified antigens.*

IFN-γ induction in the mouse model of TB infection

The recognition of the purified antigens in the mouse model of memory immunity to TB (described in example 1) was investigated. The results shown in TABLE 5 are representative for three experiments.
A very high IFN-γ response was induced by two of the antigens CFP17 and CFP21 at almost the same high level as ST-CF.

TABLE 5

| IFN-γ release from splenic memory effector cells from C57BL/6J mice isolated after reinfection with *M. tuberculosis* after stimulation with native antigens. | |
| --- | --- |
| Antigen[a] | IFN-γ (pg/ml)[b] |
| ST-CF | 12564 |
| CFP7 | ND[d] |
| CFP9 | ND |
| CFP17 | 9251 |
| CFP20 | 2388 |
| CFP21 | 10732 |
| CFP22 + CFP25[c] | 5342 |
| CFP26 (MPT51) | ND |
| CFP28 | 2818 |
| CFP29 | 3700 |

The data is derived from a representative experiment out of three.
[a] ST-CF was tested in a concentration of 5 μg/ml and the individual antigens in a concentration of 2 μg/ml.
[b] Four days after rechallenge a pool of cells from three mice were tested. The results are expressed as mean of duplicate values and the difference between duplicate cultures are < 15% of mean. The IFN-γ release of cultures incubated without antigen was 390 pg/ml.
[c] A pool of CFP22 and CFP25 was tested.
[d] ND, not determined.

Skin test reaction in TB infected guinea pigs

The skin test activity of the purified proteins was tested in *M. tuberculosis* infected guinea pigs.
1 group of guinea pigs was infected via an ear vein with $1 \times 10^4$ CFU of *M. tuberculosis* H37Rv in 0,2 ml PBS. After 4 weeks skin tests were performed and 24 hours after injection erythema diameter was measured.
As seen in TABLES 6 and 6a all of the antigens induced a significant Delayed Type Hypersensitivity (DTH) reaction.

TABLE 6

| DTH erythema diameter in guinea pigs infected with 1 x 10⁴ CFU of *M. tuberculosis,* after stimulation with native antigens. | |
|---|---|
| Antigen[a] | Skin reaction (mm)[b] |
| Control | 2.00 |
| PPD[c] | 15.40 (0.53) |
| CFP7 | ND[e] |
| CFP9 | ND |
| CFP17 | 11.25 (0.84) |
| CFP20 | 8.88 (0.13) |
| CFP21 | 12.44 (0.79) |
| CFP22 + CFP25[d] | 9.19 (3.10) |
| CFP26 (MPT51) | ND |
| CFP28 | 2.90 (1.28) |
| CFP29 | 6.63 (0.88) |

The values presented are the mean of erythema diameter of four animals and the SEM's are indicated in the brackets. For PPD and CFP29 the values are mean of erythema diameter of ten animals.
[a] The antigens were tested in a concentration of 0,1 µg except for CFP29 which was tested in a concentration of 0,8 µg.
[b] The skin reactions are measured in mm erythema 24 h after intradermal injection.
[c] 10 TU of PPD was used.
[d] A pool of CFP22 and CFP25 was tested.
[e] ND, not determined.

Together these analyses indicate that most of the antigens identified were highly biologically active and recognized during TB infection in different animal models.

TABLE 6a

| DTH erythema diameter of recombinant antigens in outbred guinea pigs infected with $1 \times 10^4$ CFU of *M. Tuberculosis.* | |
|---|---|
| Antigen[a] | Skin reaction (mm)[b] |
| Control | 2.9 (0.3) |
| PPD[c] | 14.5 (1.0) |
| CFP 7a | 13.6 (1.4) |
| CFP 17 | 6.8 (1.9) |
| CFP 20 | 6.4 (1.4) |
| CFP 21 | 5.3 (0.7) |
| CFP 25 | 10.8 (0.8) |
| CFP 29 | 7.4 (2.2) |
| MPT 51 | 4.9 (1.1) |

The values presented are the mean of erythema diameter of four animals and the SEM's are indicated in the brackets. For Control, PPD, and CFP 20 the values are mean of erythema diameter of eight animals.
[a] The antigens were tested in a concentration of 1,0 µg.
[b] The skin test reactions are measured in mm erythema 24 h after intradermal infection.
[c] 10 TU of PPD was used.

Table 6B.

| DTH erythema diameter in guinea pigs i.v. infected with 1 x 10⁴ CFU *M. tuberculo sis,* after stimulation with 10 g antigen. | | |
|---|---|---|
| Antigen | Mean (mm) | SEM |
| PBS | 3,25 | 0,48 |

Table 6B.   (continued)

| DTH erythema diameter in guinea pigs i.v. infected with 1 x $10^4$ CFU *M. tuberculo sis,* after stimulation with 10 g antigen. | | |
|---|---|---|
| Antigen | Mean (mm) | SEM |
| PPD (2TU) | 10,88 | 1 |
| nCFP7B | 7,0 | 0,46 |
| nCFP19 | 6,5 | 0,74 |
| MPT59-ESAT6 | 14,75 | 1,5 |

The values presented are the mean of erythema diameter of four animals.

The results in Table 6B indicates biological activity of nCFP7B, nCFP19 and MPT59-ESAT-6. MPT59-ESAT-6 resulting in a DTH response at the level of PPD.

*Biological activity of the purified recombinant antigens.*

Interferon-$\gamma$ induction in the mouse model of TB infection.

**Primary infections.** 8 to 12 weeks old female C57BL/6j(H-$2^b$), CBA/J(H-$2^k$), DBA.2(H-$2^d$) and A.SW(H-$2^s$) mice (Bomholtegaard, Ry) were given intravenous infections via the lateral tail vein with an inoculum of 5 x $10^4$ *M. tuberculosis* suspended in PBS in a vol. of 0.1 ml. 14 days postinfection the animals were sacrificed and spleen cells were isolated and tested for the recognition of recombinant antigen.

As seen in TABLE 7 the recombinant antigens rCFP7A, rCFP17, rCFP21, rCFP25, and rCFP29 were all recognized in at least two strains of mice at a level comparable to ST-CF. rMPT51 and rCFP7 were only recognized in one or two strains respectively, at a level corresponding to no more than 1/3 of the response detected after ST-CF stimulation. Neither of the antigens rCFP20 and rCFP22 were recognized by any of the four mouse strains.

As shown in TABLE 7A, the recombinant antigens rCFP27, RD1-ORF2, MPT59-ESAT6, rCFP10A, rCFP19, and rCFP25A were all recognized in at least two strains of mice at a level comparable to ST-CF, whereas ESAT6-MPT59, rCFP23, and rCFP30B only were recognized in one strain at this level. rCFP30A , RD1-ORF5, rCFP16 gave rise to an IFN-$\gamma$ release in two mice strains at a level corresponding to 2/3 of the response after stimulation with ST-CF. RD1-ORF3 was recognized in two strains at a level of 1/3 of ST-CF.

The native CFP7B was recognized in two strains at a level of 1/3 of the response seen after stimulation with ST-CF.

**Memory responses.** 8-12 weeks old female C57BL/6j(H-$2^b$) mice (Bomholtegaard, Ry) were given intravenous infections via the lateral tail vein with an inoculum of 5 x $10^4$ *M. tuberculosis* suspended in PBS in a vol. of 0.1 ml. After 1 month of infection the mice were treated with isoniazid (Merck and Co., Rahway, NJ) and rifabutin (Farmatalia Carlo Erba, Milano, Italy) in the drinking water, for two months. The mice were rested for 4-6 months before being used in experiments. For the study of the recall of memory immunity, animals were infected with an inoculum of 1 x $10^6$ bacteria i.v. and sacrificed at day 4 postinfection. Spleen cells were isolated and tested for the recognition of recombinant antigen.

As seen from TABLE 8, IFN-$\gamma$ release after stimulation with rCFP17, rCFP21 and rCFP25 was at the same level as seen from spleen cells stimulated with ST-CF. Stimulation with rCFP7, rCFP7A and rCFP29 all resulted in an IFN-$\gamma$ no higher than 1/3 of the response seen with ST-CF. rCFP22 was not recognized by IFN-$\gamma$ producing cells. None of the antigens stimulated IFN-$\gamma$ release in naive mice. Additionally non of the antigens were toxic to the cell cultures.

As shown in TABLE 8A, IFN-$\gamma$ release after stimulation with RD1-ORF2, MPT59-ESAT6, ESAT6-MPT59, and rCFP19 was at the same level as seen from spleen cells stimulated with ST-CF. Stimulation with rCFP10A and rCFP30A gave rise to an IFN-$\gamma$ release of 2/3 of the response after stimulation with ST-CF, whereas rCFP27, RD1-ORF5, rCFP23, rCFP25A and rCFP30B all resulted in an IFN-$\gamma$ release no higher than 1/3 of the response seen with ST-CF. RD1-ORF3 and rCFP16 were not recognized by IFN-$\gamma$ producing memory cells.

TABLE 7.

| T cell responses in primary TB infection. | | | | |
|---|---|---|---|---|
| Name | c57BL/6J(H$2^b$) | DBA.2(H$2^d$) | CBA/J(H$2^k$) | A.SW(H$2^s$) |
| rCFP7 | + | + | - | - |
| rCFP7A | +++ | +++ | +++ | + |

TABLE 7.   (continued)

| T cell responses in primary TB infection. | | | | |
|---|---|---|---|---|
| Name | c57BL/6J(H2b) | DBA.2(H2d) | CBA/J(H2k) | A.SW(H2s) |
| rCFP17 | +++ | + | +++ | + |
| rCFP20 | - | - | - | - |
| rCFP21 | +++ | +++ | +++ | + |
| rCFP22 | - | - | - | - |
| rCFP25 | +++ | ++ | +++ | + |
| rCFP29 | +++ | +++ | +++ | ++ |
| rMPT51 | + | - | - | - |
| Mouse IFN-γ release 14 days after primary infection with *M. tuberculosis.* - :no response; +: 1/3 of ST-CF; + +: 2/3 of ST-CF; + + + : level of ST-CF. | | | | |

TABLE 7A.

| T cell responses in primary TB infection. | | | | |
|---|---|---|---|---|
| Name | C57B1/6j (H2b) | DBA.2 (H2d) | CBA/J (H2k) | A. SW (H2s) |
| rCFP27 | ++ | ++ | +++ | +++ |
| rCFP30A | - | + | ++ | ++ |
| RD1-ORF2 | +++ | +++ | +++ | ++ |
| RD1-ORF3 | - | - | + | + |
| RD1-ORF5 | + | + | ++ | ++ |
| MPT59-ESAT6 | +++ | +++ | +++ | ++ |
| ESAT6-MPT59 | +++ | - | + | - |
| rCFP10A | +++ | n.d. | +++ | n.d. |
| rCFP16 | ++ | n.d. | ++ | n.d. |
| rCFP19 | +++ | n.d. | +++ | n.d. |
| rCFP23 | ++ | n.d. | +++ | n.d. |
| rCFP25A | +++ | n.d. | +++ | n.d. |
| rCFP30B | + | n.d. | +++ | n.d. |
| CFP7B(native) | + | n.d. | + | n.d. |
| Mouse IFN-γ release 14 days after primary infection with *M. tuberculosis.* -: no response; +: 1 /3 of ST-CF; + +: 2/3 of ST-CF; +++: level of ST-CF.  n.d. = not determined. | | | | |

TABLE 8.

| T cell responses in memory immune animals. | |
|---|---|
| Name | Memory response |
| rCFP7 | + |
| rCFP7A | ++ |
| rCFP17 | +++ |
| rCFP21 | +++ |
| rCFP22 | - |
| rCFP29 | + |
| rCFP25 | +++ |
| rMPT51 | + |
| Mouse IFN-γ release during recall of memory immunity to *M. tuberculosis.* - :no response; +: 1/3 of ST-CF; + +: 2/3 of ST-CF; + + +: level of ST-CF. | |

TABLE 8A.

| T cell responses in memory immune animals. | |
|---|---|
| Name | Memory response |
| rCFP27 | + |
| rCFP30A | ++ |
| RD1-ORF2 | +++ |
| RD1-ORF3 | - |
| RD1-ORF5 | + |
| MPT59-ESAT6 | +++ |
| ESAT6-MPT59 | +++ |
| rCFP10A | ++ |
| rCFP16 | - |
| rCFP19 | +++ |
| rCFP23 | + |
| rCFP25A | + |
| rCFP30B | + |
| Mouse IFN-γ release during recall of memory immunity to *M. tuberculosis*. -: no response; + : 1/3 of ST-CF; + + : 2/3 of ST-CF; + + + : level of ST-CF. | |

Interferon-γ induction in human TB patients and BCG vaccinated people.

**Human donors:** PBMC were obtained from healthy BCG vaccinated donors with no known exposure to patients with TB and from patients with culture or microscopy proven infection with *Mycobacterium tuberculosis.* Blood samples were drawn from the TB patients 1-4 months after diagnosis.

**Lymphocyte preparations and cell culture:** PBMC were freshly isolated by gradient centrifugation of heparinized blood on Lymphoprep (Nycomed, Oslo, Norway). The cells were resuspended in complete medium: RPMI 1640 (Gibco, Grand Island, N.Y.) supplemented with 40 μg/ml streptomycin, 40 U/ml penicillin, and 0.04 mM/ml glutamine, (all from Gibco Laboratories, Paisley, Scotland) and 10% normal human ABO serum (NHS) from the local blood bank. The number and the viability of the cells were determined by trypan blue staining. Cultures were established with 2,5 x 10$^5$ PBMC in 200 μl in microtitre plates (Nunc, Roskilde, Denmark) and stimulated with no antigen, ST-CF, PPD (2.5μg/ml); rCFP7, rCFP7A, rCFP17, rCFP20, rCFP21, rCFP22, rCFP25, rCFP26, rCFP29, in a final concentration of 5 μg/ml. Phytohaemagglutinin, 1 μg/ml (PHA, Difco laboratories, Detroit, MI. was used as a positive control. Supernatants for the detection of cytokines were harvested after 5 days of culture, pooled and stored at -80°C until use.

**Cytokine analysis:** Interferon-γ (IFN-γ ) was measured with a standard ELISA technique using a commercially available pair of mAb's from Endogen and used according to the instructions for use. Recombinant IFN-γ (Gibco laboratories) was used as a standard. The detection level for the assay was 50 pg/ml. The variation between the duplicate wells did not exceed 10 % of the mean. Responses of 9 individual donors are shown in TABLE 9.

A seen in TABLE 9 high levels of IFN-γ release are obtained after stimulation with several of the recombinant antigens. rCFP7a and rCFP17 gives rise to responses comparable to STCF in almost all donors. rCFP7 seems to be most strongly recognized by BCG vaccinated healthy donors. rCFP21, rCFP25, rCFP26, and rCFP29 gives rise to a mixed picture with intermediate responses in each group, whereas low responses are obtained by rCFP20 and rCFP22.

As is seen from Table 9A RD1-ORF2 and RD1-ORF5 give rise to IFN-γ responses close to the level of ST-CF. Between 60% and 90% of the donors show high IFN-γ responses (> 1000 pg/ml). rCFP30A gives rise to a mixed response with 40-50% high responders, whilst low responses are obtained with RD1-ORF3.

As seen from Table 9B MPT59-ESAT6 and ESAT6-MPT59 both give rise to IFN-γ responses at the level of ST-CF and 67-89 % show high responses (> 1000 pg/ml).

TABLE 9. Results from the stimulation of human blood cells from 5 healthy BCG vaccinated and 4 Tb patients with recombinant antigen. ST-CF, PPD and PHA are shown for comparison. Results are given in pg IFN-γ/ml.

Controls, Healthy, BCG vaccinated, no known TB exposure

| donor: | no ag | PHA | PPD | STCF | CFP7 | CFP17 | CFP7A | CFP20 | CFP21 | CFP22 | CFP25 | CFP26 | CFP29 |
|---|---|---|---|---|---|---|---|---|---|---|---|---|---|
| 1 | 6 | 9564 | 6774 | 3966 | 7034 | 69 | 1799 | 58 | 152 | 73 | 182 | 946 | 86 |
| 2 | 48 | 12486 | 6603 | 8067 | 3146 | 10044 | 5267 | 29 | 6149 | 51 | 1937 | 526 | 2065 |
| 3 | 190 | 11929 | 10000 | 8299 | 8015 | 11563 | 8641 | 437 | 3194 | 669 | 2531 | 8076 | 6098 |
| 4 | 10 | 21029 | 4106 | 3537 | 1323 | 1939 | 5211 | 1 | 284 | 1 | 1344 | 20 | 125 |
| 5 | 1 | 18750 | 14209 | 13027 | 17725 | 8038 | 19002 | 1 | 3008 | 1 | 2103 | 974 | 8181 |

TB patients, 1-4 month after diagnosis

| | no ag | PHA | PPD | STCF | CFP7 | CFP17 | CFP7A | CFP20 | CFP21 | CFP22 | CFP25 | CFP26 | CFP29 |
|---|---|---|---|---|---|---|---|---|---|---|---|---|---|
| 6 | 9 | 8973 | 5096 | 6145 | 852 | 4250 | 4019 | 284 | 1131 | 48 | 2400 | 1078 | 4584 |
| 7 | 1 | 12413 | 6281 | 3393 | 168 | 6375 | 4505 | 11 | 4335 | 16 | 3082 | 1370 | 5115 |
| 8 | 4 | 11915 | 7671 | 7375 | 104 | 2753 | 3356 | 119 | 407 | 437 | 2069 | 712 | 5284 |
| 9 | 32 | 22130 | 16417 | 17213 | 8450 | 9783 | 16319 | 91 | 5957 | 67 | 10043 | 13313 | 9953 |

EP 1 484 405 A1

Table 9A.

| Results from the stimulation of human blood cells from 10 healthy BCG vaccinated or non vaccinated ST-CF responsive healthy donors and 10 Tb patients with recombinant antigen are shown. ST-CF, PPD and PHA are included for comparison. Results are given in pg IFN-γ/ml and negative values below 300 pg/ml are shown as "<". nd = not done. | | | | | | | | |
|---|---|---|---|---|---|---|---|---|
| Controls, Healthy BCG vaccinated, or non vaccinated ST-CF positive | | | | | | | | |
| Donor | no ag | PHA | PPD | STCF | RD1-ORF2 | RD1-ORF3 | RD1-ORF5 | rCFP30A |
| 10 | < | nd | 3500 | 4200 | 1250 | < | 690 | nd |
| 11 | < | nd | 5890 | 4040 | 5650 | 880 | 9030 | nd |
| 12 | < | nd | 6480 | 3330 | 2310 | < | 3320 | nd |
| 13 | < | nd | 7440 | 4570 | 920 | < | 1230 | nd |
| 14 | < | 8310 | nd | 2990 | 1870 | < | 4880 | < |
| 15 | < | 10820 | nd | 4160 | 5690 | < | 810 | 3380 |
| 16 | < | 8710 | nd | 5690 | 1630 | < | 5600 | < |
| 17 | < | 7020 | 4480 | 5340 | 2030 | nd | 670 | < |
| 18 | < | 8370 | 6250 | 4780 | 3850 | nd | 370 | 1730 |
| 19 | < | 8520 | 1600 | 310 | 5110 | nd | 2330 | 1800 |
| Tb patients, 1-4 month after diagnosis | | | | | | | | |
| Donor | no ag | PHA | PPD | STCF | RD1-ORF2 | RD1-ORF3 | RD1-ORF5 | rCFP30A |
| 20 | < | nd | 10670 | 12680 | 2020 | < | 9670 | nd |
| 21 | < | nd | 3010 | 1420 | 850 | < | 350 | nd |
| 22 | < | nd | 8450 | 7850 | 430 | < | 1950 | nd |
| 23 | < | 10060 | nd | 3730 | < | < | 350 | < |
| 24 | < | 10830 | nd | 6180 | 2090 | < | 320 | 730 |
| 25 | < | 9000 | nd | 3200 | 4760 | < | 4960 | 2820 |
| 26 | < | 10740 | nd | 7650 | 4710 | < | 1170 | 2280 |
| 27 | < | 7550 | 6430 | 6220 | 2030 | nd | 3390 | 3069 |
| 28 | < | 8090 | 5790 | 4850 | 1100 | nd | 2095 | 550 |
| 29 | < | 7790 | 4800 | 4260 | 2800 | nd | 1210 | 420 |

Table 9B.

| Results from the stimulation of human blood cells from 9 Healthy BCG vaccinated, or non vaccinated ST-CF positive and 8 Tb patients with recombinant MPT59-ESAT6 and ESAT6-MPT59 are shown. ST-CF, PPD and PHA are included for comparison. Results are given in pg IFN-γ/ml and negative values below 300 pg/ml are shown as " < ". nd = not done. | | | | | | |
|---|---|---|---|---|---|---|
| Controls, Healthy BCG vaccinated, or non vaccinated ST-CF positive. | | | | | | |
| Donor | no ag | PHA | PPD | STCF | MPT59-ESAT6 | ESAT6-MPT59 |
| 1 | < | 9560 | 6770 | 3970 | 2030 | < |
| 2 | < | 12490 | 6600 | 8070 | 5660 | 5800 |
| 4 | < | 21030 | 4100 | 3540 | < | < |
| 5 | < | 18750 | 14200 | 13030 | 8540 | < |
| 11 | < | nd | 5890 | 4040 | 4930 | 8870 |
| 12 | < | nd | 6470 | 3330 | 2070 | 6450 |
| 14 | < | 8310 | nd | 2990 | 10270 | 11030 |
| 15 | < | 10830 | nd | 4160 | 3880 | 4540 |
| 16 | < | 8710 | nd | 5690 | 2240 | 5820 |

Table 9B.   (continued)

| Results from the stimulation of human blood cells from 9 Healthy BCG vaccinated, or non vaccinated ST-CF positive and 8 Tb patients with recombinant MPT59-ESAT6 and ESAT6-MPT59 are shown. ST-CF, PPD and PHA are included for comparison. Results are given in pg IFN-γ/ml and negative values below 300 pg/ml are shown as " < ". nd = not done. | | | | | | |
|---|---|---|---|---|---|---|
| Tb patients, 1-4 month after diagnosis | | | | | | |
| Donor | no ag | PHA | PPD | STCF | MPT59-ESAT6 | ESAT6-MPT59 |
| 6 | < | 8970 | 5100 | 6150 | 4150 | 4120 |
| 7 | < | 12410 | 6280 | 3390 | 5050 | 2040 |
| 8 | < | 11920 | 7670 | 7370 | 800 | 1350 |
| 9 | < | 22130 | 16420 | 17210 | 13660 | 5630 |
| 23 | < | 10070 | nd | 3730 | 1740 | 2390 |
| 24 | < | 10820 | nd | 6180 | 1270 | 1570 |
| 25 | < | 9010 | nd | 3200 | 3680 | 5340 |
| 26 | < | 10740 | nd | 7650 | 2070 | 620 |

EXAMPLE 6A

Four groups of 6-8 weeks old, female C57Bl/6J mice (Bomholtegård, Denmark) were immunized subcutaneously at the base of the tail with vaccines of the following compositions:

Group 1: 10 μg ESAT-6/DDA (250 μg)
Group 2: 10 μg MPT59/DDA (250μg)
Group 3: 10 μg MPT59-ESAT-6 /DDA (250 μg)
Group 4: Adjuvant control group: DDA (250 μg) in NaCl

The animals were injected with a volume of 0.2 ml. Two weeks after the first injection and 3 weeks after the second injection the mice were boosted a little further up the back.
One week after the last immunization the mice were bled and the blood cells were isolated. The immune response induced was monitored by release of IFN-γ into the culture supernatants when stimulated in vitro with relevant antigens (see the following table).

| Immunogen 10 μg/ dose | For restimulation[a]): Ag in vitro | | | |
|---|---|---|---|---|
| | no antigen | ST-CF | ESAT-6 | MPT59 |
| ESAT-6 | 219 ± 219 | 569 ± 569 | 835 ± 633 | - |
| MPT59 | 0 | 802 ± 182 | - | 5647 ± 159 |
| Hybrid: MPT59-ESAT-6 | 127 ± 127 | 7453 ± 581 | 15133 ± 861 | 16363 ± 1002 |

[a]) **Blood cells** were isolated 1 week after the last immunization and the release of IFN-γ (pg/ml) after 72h of antigen stimulation (5 μg/ml) was measured. The values shown are mean of triplicates performed on cells pooled from three mice ± SEM
[b]) - not determined

The experiment demonstrates that immunization with the hybrid stimulates T cells which recognize ESAT-6 and MPT59 stronger than after single antigen immunization. Especially the recognition of ESAT-6 was enhanced by immunization with the MPT59-ESAT-6 hybrid. IFN-γ release in control mice immunized with DDA never exceeded 1000 pg/ml.

EXAMPLE 6B

The recombinant antigens were tested individually as subunit vaccines in mice. Eleven groups of 6-8 weeks old, female C57Bl/6j mice (Bomholtegård, Denmark) were immunized subcutaneously at the base of the tail with vaccines of the following composition:

Group 1: 10 µg CFP7
Group 2: 10 µg CFP17
Group 3: 10 µg CFP21
Group 4: 10 µg CFP22
Group 5: 10 µg CFP25
Group 6: 10 µg CFP29
Group 7: 10 µg MPT51
Group 8: 50 µg ST-CF
Group 9: Adjuvant control group
Group 10: BCG 2,5 x $10^5$/ml, 0,2 ml
Group 11: Control group: Untreated

All the subunit vaccines were given with DDA as adjuvant. The animals were vaccinated with a volume of 0.2 ml. Two weeks after the first injection and three weeks after the second injection group 1-9 were boosted a little further up the back. One week after the last injection the mice were bled and the blood cells were isolated. The immune response induced was monitored by release of IFN-γ into the culture supernatant when stimulated in vitro with the homologous protein.

6 weeks after the last immunization the mice were aerosol challenged with 5 x $10^6$ viable *Mycobacterium tuberculosis*/ml. After 6 weeks of infection the mice were killed and the number of viable bacteria in lung and spleen of infected mice was determined by plating serial 3-fold dilutions of organ homogenates on 7H11 plates. Colonies were counted after 2-3 weeks of incubation. The protective efficacy is expressed as the difference between $1og_{10}$ values of the geometric mean of counts obtained from five mice of the relevant group and the geometric mean of counts obtained from five mouse of the relevant control group.

The results from the experiments are presented in the following table.

Immunogenicity and protective efficacy in mice, of ST-CF and 7 subunit vaccines

| Subunit Vaccine | Immunogenicity | Protective efficacy |
|---|---|---|
| ST-CF | +++ | +++ |
| CFP7 | ++ | - |
| CFP17 | +++ | +++ |
| CFP21 | +++ | ++ |
| CFP22 | - | - |
| CFP25 | +++ | +++ |
| CFP29 | +++ | +++ |
| MPT51 | +++ | ++ |

+ + + Strong immunogen / high protection (level of BCG)

+ + Medium immunogen / medium protection

- No recognition / no protection

In conclusion, we have identified a number of proteins inducing high levels of protection. Three of these CFP17, CFP25 and CFP29 giving rise to similar levels of protection as ST-CF and BCG while two proteins CFP21 and MPT51 induces protections around 2/3 the level of BCG and ST-CF. Two of the proteins CFP7 and CFP22 did not induce protection in the mouse model.

As is described for rCFP7, rCFP17, rCFP21, rCFP22, rCFP25, rCFP29 and rMPT51 the two antigens rCFP7A and rCFP30A were tested individually as subunit vaccines in mice. C57BI/6j mice were immunized as described for rCFP7, rCFP17, rCFP21, rCFP22, rCFP25, rCFP29 and rMPT51 using either 10µg rCFP7A or 10µg rCFP30A. Controls were the same as in the experiment including rCFP7, rCFP17, rCFP21, rCFP22, rCFP25, rCFP29 and rMPT51.

Immunogenicity and protective efficacy in mice, of ST-CF and 2 subunit vaccines.

| Subunit vaccine | Immunogenicity | Protective efficacy |
|---|---|---|
| ST-CF | +++ | +++ |
| rCFP7A | +++ | +++ |

+ + + Strong immunogen/high protection (level of BCG)

(continued)

| Subunit vaccine | Immunogenicity | Protective efficacy |
|:---:|:---:|:---:|
| rCFP30A | +++ | - |

+ + + Strong immunogen/high protection (level of BCG)

+ + Medium immunogen/medium protection

- No recognition/no protection

In conclusion we have identified two strong immunogens of which one, rCFP7A, induces protection at the level of ST-CF.

EXAMPLE 7

*Species distribution of cfp7, cfp9, mpt51, rd1-orf2, rd1-orf3, rd1-orf4, rd1-orf5, rd1-orf8, rd1-orf9a and rd1-orf9b as well as of cfp7a, cfp7b, cfp10a, cfp17, cfp20, cfp21, cfp22, cfp22a, cfp23, cfp25 and cfp25a.*

Presence of *cfp7, cfp9, mpt51, rd1-orf2, rd1-orf3, rd1-orf4, rd1-orf5, rd1-orf8, rd1-orf9a* and *rd1-orf9b* in different mycobacterial species.

In order to determine the distribution of the *cfp7, cfp9,* mpt51, *rd1-orf2, rd1-orf3, rd1-orf4, rd1-orf5, rd1-orf8, rd1-orf9a* and *rd1-orf9b* genes in species belonging to the *M. tuberculosis*-complex and in other mycobacteria PCR and/or Southern blotting was used. The bacterial strains used are listed in TABLE 10. Genomic DNA was prepared from mycobacterial cells as described previously (Andersen et al. 1992).

PCR analyses were used in order to determine the distribution of the *cfp7, cfp9* and *mpt51* gene in species belonging to the tuberculosis-complex and in other mycobacteria. The bacterial strains used are listed in TABLE 10. PCR was performed on genomic DNA prepared from mycobacterial cells as described previously (Andersen *et al*., 1992).

The oligonucleotide primers used were synthesised automatically on a DNA synthesizer (Applied Biosystems, Forster City, Ca, ABI-391, PCR-mode), deblocked, and purified by ethanol precipitation. The primers used for the analyses are shown in TABLE 11.

The PCR amplification was carried out in a thermal reactor (Rapid cycler, Idaho Technology, Idaho) by mixing 20 ng chromosomal with the mastermix (contained 0.5 µM of each oligonucleotide primer, 0.25 µM BSA (Stratagene), low salt buffer (20 mM Tris-HCl, pH8.8 , 10 mM KCl, 10 mM $(NH_4)_2SO_4$, 2 mM $MgSO_4$ and 0,1 % Triton X-100) (Stratagene), 0.25 mM of each deoxynucleoside triphosphate and 0.5 U Taq Plus Long DNA polymerase (Stratagene)). Final volume was 10 µl (all concentrations given are concentrations in the final volume). Predenaturation was carried out at 94°C for 30 s. 30 cycles of the following was performed: Denaturation at 94°C for 30 s, annealing at 55°C for 30 s and elongation at 72°C for 1 min.

The following primer combinations were used (the length of the amplified products are given in parentheses):

*mpt51*: MPT51-3 and MPT51-2 (820 bp), MPT51-3 and MPT51-6 (108 bp), MPT51-5 and MPT51-4 (415 bp), MPT51-7 and MPT51-4 (325 bp).
*cfp7:* pVF1 and PVR1 (274 bp), pVF1 and PVR2 (197 bp), pVF3 and PVR1 (302 bp), pVF3 and PVR2 (125 bp).
*cfp9:* stR3 and stF1 (351 bp).

TABLE 10.

| Mycobacterial strains used in this Example. | | | |
|---|---|---|---|
| Species and strain(s) | | | Source |
| 1. | *M. tuberculosis* | H37Rv (ATCC 27294) | ATCC[a] |
| 2. | | H37Ra (ATCC 25177) | ATCC |
| 3. | | Erdman | Obtained from A. Lazlo, Ottawa, Canada |
| 4. | *M. bovis* BCG substrain:Danish 1331 | | SSI[b] |

[a] American Type Culture Collection, USA.

[b] Statens Serum Institut, Copenhagen, Denmark.

TABLE 10.   (continued)

| Mycobacterial strains used in this Example. | | | |
|---|---|---|---|
| Species and strain(s) | | | Source |
| 5. | | Chinese | SSI[c] |
| 6. | | Canadian | SSI[c] |
| 7. | | Glaxo | SSI[c] |
| 8. | | Russia | SSI[c] |
| 9. | | Pasteur | SSI[c] |
| 10. | | Japan | WHO[e] |
| 11. | M. bovis MNC 27 | | SSI[c] |
| 12. | *M. africanum* | | Isolated from a Danish patient |
| 13. | M. leprae (armadillo-derived) | | Obtained from J. M. Colston, London, UK |
| 14. | *M. avium* (ATCC 15769) | | ATCC |
| 15. | *M. kansasii* (ATCC 12478) | | ATCC |
| 16. | *M. marinum* (ATCC 927) | | ATCC |
| 17. | M. *scrofulaceum* (ATCC 19275) | | ATCC |
| 18. | *M. intercellulare* (ATCC 15985) | | ATCC |
| 19. | *M. fortuitum* (ATCC 6841) | | ATCC |
| 20. | *M. xenopi* | | Isolated from a Danish patient |
| 21. | *M. flavescens* | | Isolated from a Danish patient |
| 22. | *M. szulgai* | | Isolated from a Danish patient |
| 23. | M. terrae | | SSI[c] |
| 24. | *E. coli* | | SSI[d] |
| 25. | *S.aureus* | | SSI[d] |

[c] Our collection Department of Mycobacteriology, Statens Serum Institut, Copenhagen, Denmark.

[d] Department of Clinical Microbiology, Statens Serum Institut, Denmark.

[e] WHO International Laboratory for Biological Standards, Statens Serum Institut, Copenhagen, Denmark.

TABLE 11.

Sequence of the *mpt51*, *cfp7* and *cfp9* oligonucleotides.

| Orientation and oligonucleotide | Sequences (5'→3')[a] | Position[b] (nucleotides) |
|---|---|---|
| Sense | | |
| MPT51-1 | CTCGAATTCGCCGGGTGCACACAG (SEQ ID NO: 28) | 6 - 21 (SEQ ID NO: 41) |
| MPT51-3 | CTCGAATTCGCCCCATACGAGAAC (SEQ ID NO: 29) | 143 - 158 (SEQ ID NO: 41) |
| MPT51-5 | GTGTATCTGCTGGAC (SEQ ID NO: 30) | 228 - 242 (SEQ ID NO: 41) |
| MPT51-7 | CCGACTGGCTGGCCG (SEQ ID NO: 31) | 418 - 432 (SEQ ID NO: 41) |
| pvR1 | GTACGAGAATTCATGTCGCAAATCATG (SEQ ID NO: 35) | 91 - 105 (SEQ ID NO: 1) |
| pvR2 | GTACGAGAATTCGAGCTTGGGGTGCCG (SEQ ID NO: 36) | 168 - 181 (SEQ ID NO: 1) |
| stR3 | CGATTCCAAGCTTGTGGCCGCCGACCCG (SEQ ID NO: 37) | 141 - 155 (SEQ ID NO: 3) |
| | | |
| Antisense | | |
| MPT51-2 | GAGGAATTCGCTTAGCGGATCGCA (SEQ ID NO: 32) | 946 - 932 (SEQ ID NO: 41) |
| MPT51-4 | CCCACATTCCGTTGG (SEQ ID NO: 33) | 642 - 628 (SEQ ID NO: 41) |
| MPT51-6 | GTCCAGCAGATACAC (SEQ ID NO: 34) | 242 - 228 (SEQ ID NO: 41) |
| pvF1 | CGTTAGGGATCCTCATCGCCATGGTGTTGG (SEQ ID NO: 38) | 340 - 323 (SEQ ID NO: 1) |
| pvF3 | CGTTAGGGATCCGGTTCCACTGTGCC (SEQ ID NO: 39) | 268 - 255 (SEQ ID NO: 1) |
| stF1 | CGTTAGGGATCCTCAGGTCTTTTCGATG (SEQ ID NO: 40) | 467 - 452 (SEQ ID NO: 3) |

[a] Nucleotides underlined are not contained in the nucleotide sequences of *mpt*51, *cfp*7, and *cfp*9.

[b] The positions referred to are of the non-underlined parts of the primers and correspond to the nucleotide sequence shown in SEQ ID NOs: 41, 1, and 3 for *mpt*51, *cfp*7, and *cfp*9, respectively.

The Southern blotting was carried out as described previously (Oettinger and Andersen, 1994) with the following modifications: 2 µg of genomic DNA was digested with *Pvu*II, electrophoresed in an 0.8% agarose gel, and transferred onto a nylon membrane (Hybond N-plus; Amersham International plc, Little Chalfont, United Kingdom) with a vacuum transfer device (Milliblot, TM-v; Millipore Corp., Bedford, MA). The *cfp7*, *cfp9*, mpt51, *rd1-orf2*, *rd1-orf3*, *rd7-orf4*, *rd1-orf5*, *rd1-orf8*, *rd1-orf9a* and *rd1-orf9b* gene fragments were amplified by PCR from the plasmids pRVN01, pRVN02, pTO52, pTO87, pTO88, pT089, pT090, pT091, pT096 or pTO98 by using the primers shown in TABLE 11 and TABLE 2 (in Example 2a). The probes were labelled non-radioactively with an enhanced chemiluminescence kit (ECL; Amersham International plc, Little Chalfont, United Kingdom). Hybridization and detection was performed according to the instructions provided by the manufacturer. The results are summarized in TABLES 12 and 13.

**TABLE 12.** Interspecies analysis of the *cfp7, cfp9* and *mpt51* genes by PCR and/or Southern blotting and of MPT51 protein by Western blotting.

| Species and strain | PCR | | | Southern blot | | | Western blot |
|---|---|---|---|---|---|---|---|
| | cfp7 | cfp9 | mpt51 | cfp7 | cfp9 | mpt51 | MPT51 |
| 1. M. tub. H37Rv | + | + | + | + | + | + | + |
| 2. M. tub. H37Ra | + | + | + | N.D. | N.D. | + | + |
| 3. M. tub. Erdmann | + | + | + | + | + | + | + |
| 4. M. bovis | + | + | + | | | + | + |
| 5. M. bovis BCG Danish 1331 | + | + | + | + | + | + | + |
| 6. M. bovis BCG Japan | + | + | N.D. | + | + | + | N.D. |
| 7. M. bovis BCG Chinese | + | + | N.D. | + | + | N.D. | N.D. |
| 8. M. bovis BCG Canadian | + | + | N.D. | + | + | N.D. | N.D. |
| 9. M. bovis BCG Glaxo | + | + | N.D. | + | + | N.D. | N.D. |
| 10. M. bovis BCG Russia | + | + | N.D. | + | + | N.D. | N.D. |
| 11. M. bovis BCG Pasteur | + | + | N.D. | + | + | N.D. | N.D. |
| 12. M. africanum | + | + | + | + | + | + | + |
| 13. M. leprae | – | – | – | – | – | – | – |
| 14. M. avium | + | + | – | + | + | + | – |
| 15. M. kansasii | + | – | – | + | + | + | – |
| 16. M. marinum | – | (+) | – | + | + | + | – |
| 17. M. scrofulaceum | – | – | – | – | – | – | – |
| 18. M. intercellul-are | + | (+) | – | + | + | + | – |
| 19. M. fortuitum | – | – | – | – | – | – | – |
| 20. M. flavescens | + | (+) | – | + | + | + | N.D. |
| 21. M. xenopi | – | – | – | N.D. | N.D. | + | – |
| 22. M. szulgai | (+) | (+) | – | – | + | – | – |
| 23. M. terrae | – | – | N.D. | N.D. | N.D. | N.D. | N.D. |

+, positive reaction; -, no reaction, N.D. not determined.

*cfp7, cfp9* and *mpt51* were found in the *M. tuberculosis* complex including BCG and the environmental mycobacteria; *M. avium, M. kansasii, M. marinum, M. intracellular* and *M. flavescens. cfp9* was additionally found in *M. szulgai* and *mpt51* in *M. xenopi.*

Furthermore the presence of native MPT51 in culture filtrates from different mycobacterial strains was investigated with western blots developed with Mab HBT4.

There is a strong band at around 26 kDa in *M. tuberculosis* H37Rv, Ra, Erdman, M. *bovis* AN5, *M. bovis* BCG substrain Danish 1331 and *M. africanum.* No band was seen in the region in any other tested mycobacterial strains.

TABLE 13a.

| Interspecies analysis of the *rd1-orf2*, *rd1-orf3*, *rd1-orf4*, *rd1-orf5*, *rd1- orf8*, *rd1-orf9a* and *rd1-orf9b* genes by Southern blotting. | | | | | | | |
|---|---|---|---|---|---|---|---|
| Species and strain | *rd1- orf2* | *rd1- orf3* | *rd1- orf4* | *rd1- orf5* | *rd1- orf8* | *rd1- orf9a* | *rd1- orf9b* |
| *1.* M. tub. H37Rv | + | + | + | + | + | + | + |
| *2. M. bovis* | + | + | + | + | N.D. | + | + |
| *3. M. bovis* BCG Danish 1331 | + | - | - | - | N.D. | - | - |
| 4. *M. bovis* BCG Japan | + | - | - | - | N.D. | - | - |
| 5. *M. avium* | - | - | - | - | N.D. | - | - |
| 6. M. *kansasii* | - | - | - | - | N.D. | - | - |
| 7. M. marinum | + | - | + | - | N.D. | - | - |
| 8. *M. scrofu- laceum* | + | - | - | - | N.D. | - | - |
| 9. *M. intercellu- lare* | - | - | - | - | N.D. | - | - |
| 10. M. *fortuitum* | - | - | - | - | N.D. | - | - |
| 11. *M. xenopi* | - | - | - | - | N.D. | - | - |
| 12. *M. szulgai* | + | - | - | - | N.D. | - | - |
| +, positive reaction; -, no reaction, N.D. not determined. | | | | | | | |

Positive results for *rd1-orf2*, *rd1-orf3*, *rd1-orf4*, *rd1-orf5*, *rd1-orf8*, *rd1-orf9a* and *rd1-orf9b* were only obtained when using genomic DNA from *M. tuberculosis* and *M. bovis,* and not from *M. bovis* BCG or other mycobacteria analyzed except *rd1-orf4* which also was found in *M. marinum*.

Presence of *cfp7a*, *cfp7b*, *cfp10a*, *cfp17*, *cfp20*, *cfp21*, *cfp22*, *cfp22a*, *cfp23*, *cfp25* and *cfp25a* in different mycobacterial species.

Southern blotting was carried out as described for *rd1-orf2*, *rd1-orf3*, *rd1-orf4*, *rd1-orf5*, *rd1-orf8*, *rd1-orf9a* and *rd1-orf9b*. The *cfp7a, cfp7b, cfp10a, cfp17, cfp20, cfp21, cfp22, cfp22a, cfp23, cfp25* and *cfp25a* gene fragments were amplified by PCR from the recombinant pMCT6 plasmids encoding the individual genes. The primers used (same as the primers used for cloning) are described in example 3, 3A and 3B. The results are summarized in Table 13b.

TABLE 13b.

| Interspecies analysis of the *cfp7a, cfp7b, cfp10a, cfp17, cfp20, cfp21, cfp22, cfp22a, cfp23, cfp25,* and *cfp25a* genes by Southern blotting. | | | | | | | | | | | |
|---|---|---|---|---|---|---|---|---|---|---|---|
| Species and strain | *cfp7a* | *cfp7b* | *cfp-10a* | *cfp 17* | *cfp 20* | *cfp21* | *cfp22* | *cfp-22a* | *cfp 23* | *cfp 25* | *cfp-25a* |
| *1. M. tub.* H37Rv | + | + | + | + | + | + | + | + | + | + | + |
| *2. M. bo vis* | + | + | + | + | + | + | + | + | + | + | + |
| *3. M. bovis* BCG Danish 1331 | + | + | + | + | + | N.D. | + | + | + | + | + |
| *4. M. bovis* BCG Japan | + | + | + | + | + | + | + | + | + | + | + |

TABLE 13b. (continued)

| Species and strain | cfp7a | cfp7b | cfp-10a | cfp 17 | cfp 20 | cfp21 | cfp22 | cfp-22a | cfp 23 | cfp 25 | cfp-25a |
|---|---|---|---|---|---|---|---|---|---|---|---|
| *Interspecies analysis of the cfp7a, cfp7b, cfp10a, cfp17, cfp20, cfp21, cfp22, cfp22a, cfp23, cfp25, and cfp25a genes by Southern blotting.* | | | | | | | | | | | |
| 5. *M. avium* | + | N.D. | - | + | - | + | + | + | + | + | - |
| 6. *M. kansasii* | - | N.D. | + | - | - | - | + | - | + | - | - |
| 7. *M. marinum* | + | + | - | + | + | + | + | + | + | + | + |
| 8. *M. scrofu-laceum* | - | - | + | - | + | + | - | + | + | + | - |
| 9. *M. intercel-lulare* | + | + | - | + | - | + | + | - | + | + | - |
| 10. *M. fortui-tum* | - | N.D. | - | - | - | - | - | - | + | - | - |
| 11. *M. xenopi* | + | + | + | + | + | + | + | + | + | + | + |
| 12. *M. szulgai* | + | + | - | + | + | + | + | + | + | + | + |
| +, positive reaction; -, no reaction, N.D. not determined. | | | | | | | | | | | |

LIST OF REFERENCES

Andersen, P. and Heron, I, 1993, J. Immunol. Methods 161: 29-39.

Andersen, Å. B. *et al.*, 1992, Infect. Immun. **60**: 2317-2323.

Andersen P., 1994, Infect. Immun. **62**: 2536-44.

Andersen P. *et al.*, 1995, J. Immunol. **154**: 3359-72

Barkholt, V. and Jensen, A. L., 1989, Anal. Biochem. **177**: 318-322.

Borodovsky, M., and J. McIninch. 1993, Computers Chem. **17**: 123-133.

van Dyke M. W. *et al.*, 1992. Gene pp. 99-104.

Gosselin *et al.*, 1992, J. Immunol. **149**: 3477-3481.

Harboe, M. *et al.*, 1996, Infect. Immun. **64**: 16-22.

von Heijne, G., 1984, J. Mol. Biol. **173**: 243-251.

Hochstrasser, D.F. *et al.*, 1988, Anal.Biochem. **173**: 424-435

Köhler, G. and Milstein, C., 1975, Nature **256**: 495-497.

Li, H. *et al.*, 1993, Infect. Immun. **61**: 1730-1734.

Lindblad E.B. *et al.*, 1997, Infect. Immun. **65**: 623-629.

Mahairas, G. G. *et al.*, 1996, J. Bacteriol **178**: 1274-1282.

Maniatis T. *et al.*, 1989, "Molecular cloning: a laboratory manual", 2nd ed., Cold Spring Harbor Laboratory, Cold Spring Harbor, N.Y.

Nagai, S. *et al.*, 1991, Infect. Immun. **59**: 372-382.

Oettinger, T. and Andersen, Å. B., 1994, Infect. Immun. **62:** 2058-2064.

Ohara, N. *et al.*, 1995, Scand. J. immunol. **41**: 233-442.

Pal P. G. and Horwitz M. A., 1992, Infect. Immun. **60**: 4781-92.

Pearson, W. R. and Lipman D. J., 1988. Proc. Natl. Acad. Sci. USA **85**: 2444-2448.

Ploug, M. *et al.*, 1989, Anal. Biochem. **181**: 33-39.

Porath, J. *et al.*, 1985, FEBS Lett. **185**: 306-310.

Roberts, A.D. *et al.*, 1995, Immunol. **85**: 502-508.

Sørensen, A.L. *et al.*, 1995, Infect. Immun. **63**: 1710-1717.

Theisen, M. *et al.,* 1995, Clinical and Diagnostic Laboratory Immunology, **2**: 30-34.

Valdés-Stauber, N. and Scherer, S., 1994, Appl. Environ. Microbiol. **60:** 3809-3814.

Valdés-Stauber, N. and Scherer, S., 1996, Appl. Environ. Microbiol. **62:** 1283-1286.

Williams, N., 1996, Science **272**: 27.

Young, R. *A. et al*., 1985, Proc. Natl. Acad. Sci. USA **82**: 2583-2587.

SEQUENCE LISTING

(1) GENERAL INFORMATION:

    (i) APPLICANT:
        (A) NAME: Statens Seruminstitut
        (B) STREET: Artillerivej 5
        (C) CITY: Copenhagen
        (E) COUNTRY: Denmark
        (F) POSTAL CODE (ZIP): 2300 S

    (ii) TITLE OF INVENTION: Nucleic acid fragments and polypeptide
        fragments derived from M. tuberculosis

   (iii) NUMBER OF SEQUENCES: 193

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)

(2) INFORMATION FOR SEQ ID NO: 1:

    (i) SEQUENCE CHARACTERISTICS:
        (A) LENGTH: 381 base pairs
        (B) TYPE: nucleic acid
        (C) STRANDEDNESS: double
        (D) TOPOLOGY: circular

    (ii) MOLECULE TYPE: DNA (genomic)

    (vi) ORIGINAL SOURCE:
        (A) ORGANISM: Mycobacterium tuberculosis
        (B) STRAIN: H37Rv

    (ix) FEATURE:
        (A) NAME/KEY: CDS
        (B) LOCATION: 91..381

    (ix) FEATURE:
        (A) NAME/KEY: -35_signal
        (B) LOCATION: 14..19

    (ix) FEATURE:
        (A) NAME/KEY: -10_signal
        (B) LOCATION: 47..50

    (ix) FEATURE:
        (A) NAME/KEY: RBS
        (B) LOCATION: 78..84

    (ix) FEATURE:
        (A) NAME/KEY: mat_peptide
        (B) LOCATION: 91..381

    (xi) SEQUENCE DESCRIPTION: SEQ ID NO: 1:

```
GGCCGCCGGT ACCTATGTGG CCGCCGATGC TGCGGACGCG TCGACCTATA CCGGGTTCTG          60

ATCGAACCCT GCTGACCGAG AGGACTTGTG ATG TCG CAA ATC ATG TAC AAC TAC          114
                                 Met Ser Gln Ile Met Tyr Asn Tyr
                                  1                   5

CCC GCG ATG TTG GGT CAC GCC GGG GAT ATG GCC GGA TAT GCC GGC ACG          162
Pro Ala Met Leu Gly His Ala Gly Asp Met Ala Gly Tyr Ala Gly Thr
     10                  15                  20

CTG CAG AGC TTG GGT GCC GAG ATC GCC GTG GAG CAG GCC GCG TTG CAG          210
Leu Gln Ser Leu Gly Ala Glu Ile Ala Val Glu Gln Ala Ala Leu Gln
 25                  30                  35                  40

AGT GCG TGG CAG GGC GAT ACC GGG ATC ACG TAT CAG GCG TGG CAG GCA          258
Ser Ala Trp Gln Gly Asp Thr Gly Ile Thr Tyr Gln Ala Trp Gln Ala
                 45                  50                  55

CAG TGG AAC CAG GCC ATG GAA GAT TTG GTG CGG GCC TAT CAT GCG ATG          306
Gln Trp Asn Gln Ala Met Glu Asp Leu Val Arg Ala Tyr His Ala Met
                 60                  65                  70

TCC AGC ACC CAT GAA GCC AAC ACC ATG GCG ATG ATG GCC CGC GAC ACC          354
Ser Ser Thr His Glu Ala Asn Thr Met Ala Met Met Ala Arg Asp Thr
             75                  80                  85

GCC GAA GCC GCC AAA TGG GGC GGC TAG                                      381
Ala Glu Ala Ala Lys Trp Gly Gly
         90                  95
```

(2) INFORMATION FOR SEQ ID NO: 2:

    (i) SEQUENCE CHARACTERISTICS:
        (A) LENGTH: 96 amino acids
        (B) TYPE: amino acid
        (D) TOPOLOGY: linear

    (ii) MOLECULE TYPE: protein
(xi) SEQUENCE DESCRIPTION: SEQ ID NO: 2:

```
Met Ser Gln Ile Met Tyr Asn Tyr Pro Ala Met Leu Gly His Ala Gly
 1                   5                  10                  15

Asp Met Ala Gly Tyr Ala Gly Thr Leu Gln Ser Leu Gly Ala Glu Ile
                 20                  25                  30

Ala Val Glu Gln Ala Ala Leu Gln Ser Ala Trp Gln Gly Asp Thr Gly
                 35                  40                  45

Ile Thr Tyr Gln Ala Trp Gln Ala Gln Trp Asn Gln Ala Met Glu Asp
         50                  55                  60

Leu Val Arg Ala Tyr His Ala Met Ser Ser Thr His Glu Ala Asn Thr
 65                  70                  75                  80

Met Ala Met Met Ala Arg Asp Thr Ala Glu Ala Ala Lys Trp Gly Gly
                 85                  90                  95
```

(2) INFORMATION FOR SEQ ID NO: 3:

(i) SEQUENCE CHARACTERISTICS:
    (A) LENGTH: 467 base pairs
    (B) TYPE: nucleic acid
    (C) STRANDEDNESS: double
    (D) TOPOLOGY: circular

(ii) MOLECULE TYPE: DNA (genomic)

(vi) ORIGINAL SOURCE:
    (A) ORGANISM: Mycobacterium tuberculosis
    (B) STRAIN: H37Rv

(ix) FEATURE:
    (A) NAME/KEY: CDS
    (B) LOCATION: 141..467

(ix) FEATURE:
    (A) NAME/KEY: -10_signal
    (B) LOCATION: 73..78

(ix) FEATURE:
    (A) NAME/KEY: -35_signal
    (B) LOCATION: 4..9

(ix) FEATURE:
    (A) NAME/KEY: RBS
    (B) LOCATION: 123..130

(ix) FEATURE:
    (A) NAME/KEY: mat_peptide
    (B) LOCATION: 141..467

(xi) SEQUENCE DESCRIPTION: SEQ ID NO: 3:

```
GGGTAGCCGG ACCACGGCTG GGCAAAGATG TGCAGGCCGC CATCAAGGCG GTCAAGGCCG          60

GCGACGGCGT CATAAACCCG GACGGCACCT TGTTGGCGGG CCCCGCGGTG CTGACGCCCG         120

ACGAGTACAA CTCCCGGCTG GTG GCC GCC GAC CCG GAG TCC ACC GCG GCG          170
                        Met Ala Ala Asp Pro Glu Ser Thr Ala Ala
                         1                   5                   10

TTG CCC GAC GGC GCC GGG CTG GTC GTT CTG GAT GGC ACC GTC ACT GCC          218
Leu Pro Asp Gly Ala Gly Leu Val Val Leu Asp Gly Thr Val Thr Ala
              15                  20                  25

GAA CTC GAA GCC GAG GGC TGG GCC AAA GAT CGC ATC CGC GAA CTG CAA          266
Glu Leu Glu Ala Glu Gly Trp Ala Lys Asp Arg Ile Arg Glu Leu Gln
              30                  35                  40

GAG CTG CGT AAG TCG ACC GGG CTG GAC GTT TCC GAC CGC ATC CGG GTG          314
Glu Leu Arg Lys Ser Thr Gly Leu Asp Val Ser Asp Arg Ile Arg Val
              45                  50                  55

GTG ATG TCG GTG CCT GCG GAA CGC GAA GAC TGG GCG CGC ACC CAT CGC          362
Val Met Ser Val Pro Ala Glu Arg Glu Asp Trp Ala Arg Thr His Arg
              60                  65                  70

GAC CTC ATT GCC GGA GAA ATC TTG GCT ACC GAC TTC GAA TTC GCC GAC          410
```

```
Asp Leu Ile Ala Gly Glu Ile Leu Ala Thr Asp Phe Glu Phe Ala Asp
 75              80              85              90

CTC GCC GAT GGT GTG GCC ATC GGC GAC GGC GTG CGG GTA AGC ATC GAA        458
Leu Ala Asp Gly Val Ala Ile Gly Asp Gly Val Arg Val Ser Ile Glu
                95              100             105

AAG ACC TGA                                                           467
Lys Thr
```

(2) INFORMATION FOR SEQ ID NO: 4:

    (i) SEQUENCE CHARACTERISTICS:
        (A) LENGTH: 108 amino acids
        (B) TYPE: amino acid
        (D) TOPOLOGY: linear

    (ii) MOLECULE TYPE: protein
    (xi) SEQUENCE DESCRIPTION: SEQ ID NO: 4:

```
Met Ala Ala Asp Pro Glu Ser Thr Ala Ala Leu Pro Asp Gly Ala Gly
 1               5               10              15

Leu Val Val Leu Asp Gly Thr Val Thr Ala Glu Leu Glu Ala Glu Gly
                20              25              30

Trp Ala Lys Asp Arg Ile Arg Glu Leu Gln Glu Leu Arg Lys Ser Thr
            35              40              45

Gly Leu Asp Val Ser Asp Arg Ile Arg Val Val Met Ser Val Pro Ala
        50              55              60

Glu Arg Glu Asp Trp Ala Arg Thr His Arg Asp Leu Ile Ala Gly Glu
 65              70              75              80

Ile Leu Ala Thr Asp Phe Glu Phe Ala Asp Leu Ala Asp Gly Val Ala
                85              90              95

Ile Gly Asp Gly Val Arg Val Ser Ile Glu Lys Thr
            100             105
```

(2) INFORMATION FOR SEQ ID NO: 5:

    (i) SEQUENCE CHARACTERISTICS:
        (A) LENGTH: 889 base pairs
        (B) TYPE: nucleic acid
        (C) STRANDEDNESS: double
        (D) TOPOLOGY: circular

    (ii) MOLECULE TYPE: DNA (genomic)

    (vi) ORIGINAL SOURCE:
        (A) ORGANISM: Mycobacterium tuberculosis
        (B) STRAIN: H37Rv

    (ix) FEATURE:
        (A) NAME/KEY: CDS
        (B) LOCATION: 201..689

(ix) FEATURE:
    (A) NAME/KEY: sig_peptide
    (B) LOCATION: 201..290

(ix) FEATURE:
    (A) NAME/KEY: mat_peptide
    (B) LOCATION: 291..689

(xi) SEQUENCE DESCRIPTION: SEQ ID NO: 5:

```
CGGGTCTGCA CGGATCCGGG CCGGGCAGGG CAATCGAGCC TGGGATCCGC TGGGGTGCGC        60

ACATCGCGGA CCCGTGCGCG GTACGGTCGA GACAGCGGCA CGAGAAAGTA GTAAGGGCGA       120

TAATAGGCGG TAAAGAGTAG CGGGAAGCCG GCCGAACGAC TCGGTCAGAC AACGCCACAG       180

CGGCCAGTGA GGAGCAGCGG GTG ACG GAC ATG AAC CCG GAT ATT GAG AAG          230
                        Met Thr Asp Met Asn Pro Asp Ile Glu Lys
                        -30             Met         -25

GAC CAG ACC TCC GAT GAA GTC ACG GTA GAG ACG ACC TCC GTC TTC CGC       278
Asp Gln Thr Ser Asp Glu Val Thr Val Glu Thr Thr Ser Val Phe Arg
-20             -15             -10                         -5

GCA GAC TTC CTC AGC GAG CTG GAC GCT CCT GCG CAA GCG GGT ACG GAG       326
Ala Asp Phe Leu Ser Glu Leu Asp Ala Pro Ala Gln Ala Gly Thr Glu
                1               5               10

AGC GCG GTC TCC GGG GTG GAA GGG CTC CCG CCG GGC TCG GCG TTG CTG       374
Ser Ala Val Ser Gly Val Glu Gly Leu Pro Pro Gly Ser Ala Leu Leu
        15              20              25

GTA GTC AAA CGA GGC CCC AAC GCC GGG TCC CGG TTC CTA CTC GAC CAA       422
Val Val Lys Arg Gly Pro Asn Ala Gly Ser Arg Phe Leu Leu Asp Gln
    30              35              40

GCC ATC ACG TCG GCT GGT CGG CAT CCC GAC AGC GAC ATA TTT CTC GAC       470
Ala Ile Thr Ser Ala Gly Arg His Pro Asp Ser Asp Ile Phe Leu Asp
45              50              55                      60

GAC GTG ACC GTG AGC CGT CGC CAT GCT GAA TTC CGG TTG GAA AAC AAC       518
Asp Val Thr Val Ser Arg Arg His Ala Glu Phe Arg Leu Glu Asn Asn
            65              70                      75

GAA TTC AAT GTC GTC GAT GTC GGG AGT CTC AAC GGC ACC TAC GTC AAC       566
Glu Phe Asn Val Val Asp Val Gly Ser Leu Asn Gly Thr Tyr Val Asn
            80              85              90

CGC GAG CCC GTG GAT TCG GCG GTG CTG GCG AAC GGC GAC GAG GTC CAG       614
Arg Glu Pro Val Asp Ser Ala Val Leu Ala Asn Gly Asp Glu Val Gln
        95              100             105

ATC GGC AAG TTC CGG TTG GTG TTC TTG ACC GGA CCC AAG CAA GGC GAG       662
Ile Gly Lys Phe Arg Leu Val Phe Leu Thr Gly Pro Lys Gln Gly Glu
    110             115             120

GAT GAC GGG AGT ACC GGG GGC CCG TGA GCGCACCCGA TAGCCCCGCG             709
Asp Asp Gly Ser Thr Gly Gly Pro
125             130
```

CTGGCCGGGA TGTCGATCGG GGCGGTCCTC GACCTGCTAC GACCGGATTT TCCTGATGTC    769

ACCATCTCCA AGATTCGATT CTTGGAGGCT GAGGGTCTGG TGACGCCCCG GCGGGCCTCA    829

TCGGGGTATC GGCGGTTCAC CGCATACGAC TGCGCACGGC TGCGATTCAT TCTCACTGCC    889


(2) INFORMATION FOR SEQ ID NO: 6:

    (i) SEQUENCE CHARACTERISTICS:
        (A) LENGTH: 162 amino acids
        (B) TYPE: amino acid
        (D) TOPOLOGY: linear

    (ii) MOLECULE TYPE: protein
    (xi) SEQUENCE DESCRIPTION: SEQ ID NO: 6:

```
Met Thr Asp Met Asn Pro Asp Ile Glu Lys Asp Gln Thr Ser Asp Glu
-30              -25              -20              -15

Val Thr Val Glu Thr Thr Ser Val Phe Arg Ala Asp Phe Leu Ser Glu
             -10              -5                   1

Leu Asp Ala Pro Ala Gln Ala Gly Thr Glu Ser Ala Val Ser Gly Val
         5                10               15

Glu Gly Leu Pro Pro Gly Ser Ala Leu Leu Val Val Lys Arg Gly Pro
     20              25              30

Asn Ala Gly Ser Arg Phe Leu Leu Asp Gln Ala Ile Thr Ser Ala Gly
    35              40               45                   50

Arg His Pro Asp Ser Asp Ile Phe Leu Asp Asp Val Thr Val Ser Arg
             55                  60                   65

Arg His Ala Glu Phe Arg Leu Glu Asn Asn Glu Phe Asn Val Val Asp
             70                  75                   80

Val Gly Ser Leu Asn Gly Thr Tyr Val Asn Arg Glu Pro Val Asp Ser
         85                  90                   95

Ala Val Leu Ala Asn Gly Asp Glu Val Gln Ile Gly Lys Phe Arg Leu
    100             105              110

Val Phe Leu Thr Gly Pro Lys Gln Gly Glu Asp Asp Gly Ser Thr Gly
115              120              125                  130

Gly Pro
```


(2) INFORMATION FOR SEQ ID NO: 7:

    (i) SEQUENCE CHARACTERISTICS:
        (A) LENGTH: 898 base pairs
        (B) TYPE: nucleic acid
        (C) STRANDEDNESS: double
        (D) TOPOLOGY: circular

    (ii) MOLECULE TYPE: DNA (genomic)

(vi) ORIGINAL SOURCE:
    (A) ORGANISM: Mycobacterium tuberculosis
    (B) STRAIN: H37Rv

(ix) FEATURE:
    (A) NAME/KEY: CDS
    (B) LOCATION: 201..698

(ix) FEATURE:
    (A) NAME/KEY: mat_peptide
    (B) LOCATION: 201..698


(xi) SEQUENCE DESCRIPTION: SEQ ID NO: 7:

```
TCGACTCCGG CGCCACCGGG CAGGATCACG GTGTCGACGG GGTCGCCGGG GAATCCCACG          60

ATAACCACTC TTCGCGCCAT GAATGCCAGT GTTGGCCAGG CGCTGGCCTG GCGTCCACGC         120

CACACACCGC ACAGATTAGG ACACGCCGGC GGCGCAGCCC TGCCCGAAAG ACCGTGCACC         180

GGTCTTGGCA GACTGTGCCC ATG GCA CAG ATA ACC CTG CGA GGA AAC GCG           230
                       Met Ala Gln Ile Thr Leu Arg Gly Asn Ala
                        1               5                  10

ATC AAT ACC GTC GGT GAG CTA CCT GCT GTC GGA TCC CCG GCC CCG GCC         278
Ile Asn Thr Val Gly Glu Leu Pro Ala Val Gly Ser Pro Ala Pro Ala
                15                  20                  25

TTC ACC CTG ACC GGG GGC GAT CTG GGG GTG ATC AGC AGC GAC CAG TTC         326
Phe Thr Leu Thr Gly Gly Asp Leu Gly Val Ile Ser Ser Asp Gln Phe
            30                  35                  40

CGG GGT AAG TCC GTG TTG CTG AAC ATC TTT CCA TCC GTG GAC ACA CCG         374
Arg Gly Lys Ser Val Leu Leu Asn Ile Phe Pro Ser Val Asp Thr Pro
            45                  50                  55


GTG TGC GCG ACG AGT GTG CGA ACC TTC GAC GAG CGT GCG GCG GCA AGT         422
Val Cys Ala Thr Ser Val Arg Thr Phe Asp Glu Arg Ala Ala Ala Ser
        60                  65                  70

GGC GCT ACC GTG CTG TGT GTC TCG AAG GAT CTG CCG TTC GCC CAG AAG         470
Gly Ala Thr Val Leu Cys Val Ser Lys Asp Leu Pro Phe Ala Gln Lys
    75                  80                  85                  90

CGC TTC TGC GGC GCC GAG GGC ACC GAA AAC GTC ATG CCC GCG TCG GCA         518
Arg Phe Cys Gly Ala Glu Gly Thr Glu Asn Val Met Pro Ala Ser Ala
                95                  100                 105

TTC CGG GAC AGC TTC GGC GAG GAT TAC GGC GTG ACC ATC GCC GAC GGG         566
Phe Arg Asp Ser Phe Gly Glu Asp Tyr Gly Val Thr Ile Ala Asp Gly
            110                 115                 120

CCG ATG GCC GGG CTG CTC GCC CGC GCA ATC GTG GTG ATC GGC GCG GAC         614
Pro Met Ala Gly Leu Leu Ala Arg Ala Ile Val Val Ile Gly Ala Asp
            125                 130                 135

GGC AAC GTC GCC TAC ACG GAA TTG GTG CCG GAA ATC GCG CAA GAA CCC         662
Gly Asn Val Ala Tyr Thr Glu Leu Val Pro Glu Ile Ala Gln Glu Pro
```

```
Gly Asn Val Ala Tyr Thr Glu Leu Val Pro Glu Ile Ala Gln Glu Pro
    140                 145                 150

AAC TAC GAA GCG GCG CTG GCC GCG CTG GGC GCC TAG GCTTTCACAA              708
Asn Tyr Glu Ala Ala Leu Ala Ala Leu Gly Ala
155                 160                 165

GCCCCGCGCG TTCGGCGAGC AGCGCACGAT TTCGAGCGCT GCTCCCGAAA AGCGCCTCGG      768

TGGTCTTGGC CCGGCGGTAA TACAGGTGCA GGTCGTGCTC CCACGTGAAG GCGATGGCAC      828

CGTGGATCTG AAGAGCGGAG CCGGCGCATA ACACAAAGGT TTCCGCGGTC TGCGCCTTCG      888

CCAGCGGCGC                                                            898
```

(2) INFORMATION FOR SEQ ID NO: 8:

    (i) SEQUENCE CHARACTERISTICS:
      (A) LENGTH: 165 amino acids
      (B) TYPE: amino acid
      (D) TOPOLOGY: linear

    (ii) MOLECULE TYPE: protein
    (xi) SEQUENCE DESCRIPTION: SEQ ID NO: 8:

```
Met Ala Gln Ile Thr Leu Arg Gly Asn Ala Ile Asn Thr Val Gly Glu
 1               5                10                15

Leu Pro Ala Val Gly Ser Pro Ala Pro Ala Phe Thr Leu Thr Gly Gly
                20                25                30

Asp Leu Gly Val Ile Ser Ser Asp Gln Phe Arg Gly Lys Ser Val Leu
            35                40                45

Leu Asn Ile Phe Pro Ser Val Asp Thr Pro Val Cys Ala Thr Ser Val
        50                55                60

Arg Thr Phe Asp Glu Arg Ala Ala Ala Ser Gly Ala Thr Val Leu Cys
 65                 70                75                80

Val Ser Lys Asp Leu Pro Phe Ala Gln Lys Arg Phe Cys Gly Ala Glu
                85                90                95

Gly Thr Glu Asn Val Met Pro Ala Ser Ala Phe Arg Asp Ser Phe Gly
            100               105               110

Glu Asp Tyr Gly Val Thr Ile Ala Asp Gly Pro Met Ala Gly Leu Leu
            115               120               125

Ala Arg Ala Ile Val Val Ile Gly Ala Asp Gly Asn Val Ala Tyr Thr
            130               135               140

Glu Leu Val Pro Glu Ile Ala Gln Glu Pro Asn Tyr Glu Ala Ala Leu
145               150               155               160

Ala Ala Leu Gly Ala
            165
```

(2) INFORMATION FOR SEQ ID NO: 9:

```
(i)  SEQUENCE CHARACTERISTICS:
     (A)  LENGTH: 1054 base pairs
     (B)  TYPE: nucleic acid
     (C)  STRANDEDNESS: double
     (D)  TOPOLOGY: circular

(ii) MOLECULE TYPE: DNA (genomic)

(vi) ORIGINAL SOURCE:
     (A)  ORGANISM: Mycobacterium tuberculosis
     (B)  STRAIN: H37Rv

(ix) FEATURE:
     (A)  NAME/KEY: CDS
     (B)  LOCATION: 201..854

(ix) FEATURE:
     (A)  NAME/KEY: sig_peptide
     (B)  LOCATION: 201..296

(ix) FEATURE:
     (A)  NAME/KEY: mat_peptide
     (B)  LOCATION: 297..854


(xi) SEQUENCE DESCRIPTION: SEQ ID NO: 9:

ATAATCAGCT CACCGTTGGG ACCGACCTCG ACCAGGGGTC CTTTGTGACT GCCGGGCTTG      60

ACGCGGACGA CCACAGAGTC GGTCATCGCC TAAGGCTACC GTTCTGACCT GGGGCTGCGT     120

GGGCGCCGAC GACGTGAGGC ACGTCATGTC TCAGCGGCCC ACCGCCACCT CGGTCGCCGG     180


CAGTATGTCA GCATGTGCAG ATG ACT CCA CGC AGC CTT GTT CGC ATC GTT         230
                      Met Thr Pro Arg Ser Leu Val Arg Ile Val
                      -32      -30                 -25

GGT GTC GTG GTT GCG ACG ACC TTG GCG CTG GTG AGC GCA CCC GCC GGC       278
Gly Val Val Val Ala Thr Thr Leu Ala Leu Val Ser Ala Pro Ala Gly
        -20                 -15                 -10

GGT CGT GCC GCG CAT GCG GAT CCG TGT TCG GAC ATC GCG GTC GTT TTC       326
Gly Arg Ala Ala His Ala Asp Pro Cys Ser Asp Ile Ala Val Val Phe
    -5                  1                 5                   10

GCT CGC GGC ACG CAT CAG GCT TCT GGT CTT GGC GAC GTC GGT GAG GCG       374
Ala Arg Gly Thr His Gln Ala Ser Gly Leu Gly Asp Val Gly Glu Ala
                15                  20                  25

TTC GTC GAC TCG CTT ACC TCG CAA GTT GGC GGG CGG TCG ATT GGG GTC       422
Phe Val Asp Ser Leu Thr Ser Gln Val Gly Gly Arg Ser Ile Gly Val
                30                  35                  40

TAC GCG GTG AAC TAC CCA GCA AGC GAC GAC TAC CGC GCG AGC GCG TCA       470
Tyr Ala Val Asn Tyr Pro Ala Ser Asp Asp Tyr Arg Ala Ser Ala Ser
            45                  50                  55

AAC GGT TCC GAT GAT GCG AGC GCC CAC ATC CAG CGC ACC GTC GCC AGC       518
Asn Gly Ser Asp Asp Ala Ser Ala His Ile Gln Arg Thr Val Ala Ser
```

```
            60                    65                    70

TGC CCG AAC ACC AGG ATT GTG CTT GGT GGC TAT TCG CAG GGT GCG ACG    566
Cys Pro Asn Thr Arg Ile Val Leu Gly Gly Tyr Ser Gln Gly Ala Thr
    75                  80                  85                  90

GTC ATC GAT TTG TCC ACC TCG GCG ATG CCG CCC GCG GTG GCA GAT CAT    614
Val Ile Asp Leu Ser Thr Ser Ala Met Pro Pro Ala Val Ala Asp His
                95                  100                 105

GTC GCC GCT GTC GCC CTT TTC GGC GAG CCA TCC AGT GGT TTC TCC AGC    662
Val Ala Ala Val Ala Leu Phe Gly Glu Pro Ser Ser Gly Phe Ser Ser
            110                 115                 120

ATG TTG TGG GGC GGC GGG TCG TTG CCG ACA ATC GGT CCG CTG TAT AGC    710
Met Leu Trp Gly Gly Gly Ser Leu Pro Thr Ile Gly Pro Leu Tyr Ser
            125                 130                 135

TCT AAG ACC ATA AAC TTG TGT GCT CCC GAC GAT CCA ATA TGC ACC GGA    758
Ser Lys Thr Ile Asn Leu Cys Ala Pro Asp Asp Pro Ile Cys Thr Gly
            140                 145                 150

GGC GGC AAT ATT ATG GCG CAT GTT TCG TAT GTT CAG TCG GGG ATG ACA    806
Gly Gly Asn Ile Met Ala His Val Ser Tyr Val Gln Ser Gly Met Thr
155                 160                 165                 170

AGC CAG GCG GCG ACA TTC GCG GCG AAC AGG CTC GAT CAC GCC GGA TGA    854
Ser Gln Ala Ala Thr Phe Ala Ala Asn Arg Leu Asp His Ala Gly
            175                 180                 185

TCAAAGACTG TTGTCCCTAT ACCGCTGGGG CTGTAGTCGA TGTACACCGG CTGGAATCTG    914

AAGGGCAAGA ACCCGGTATT CATCAGGCCG GATGAAATGA CGGTCGGGCG GTAATCGTTT    974

GTGTTGAACG CGTAGAGCCG ATCACCGCCG GGGCTGGTGT AGACCTCAAT GTTTGTGTTC    1034

GCCGGCAGGG TTCCGGATCC                                                 1054
```

(2) INFORMATION FOR SEQ ID NO: 10:

    (i) SEQUENCE CHARACTERISTICS:
        (A) LENGTH: 217 amino acids
        (B) TYPE: amino acid
        (D) TOPOLOGY: linear

    (ii) MOLECULE TYPE: protein
    (xi) SEQUENCE DESCRIPTION: SEQ ID NO: 10:

```
Met Thr Pro Arg Ser Leu Val Arg Ile Val Gly Val Val Val Ala Thr
-32     -30                 -25                 -20

Thr Leu Ala Leu Val Ser Ala Pro Ala Gly Gly Arg Ala Ala His Ala
    -15                 -10                 -5

Asp Pro Cys Ser Asp Ile Ala Val Val Phe Ala Arg Gly Thr His Gln
 1               5                   10                  15

Ala Ser Gly Leu Gly Asp Val Gly Glu Ala Phe Val Asp Ser Leu Thr
            20                  25                  30
```

```
Ser Gln Val Gly Gly Arg Ser Ile Gly Val Tyr Ala Val Asn Tyr Pro
        35              40              45

Ala Ser Asp Asp Tyr Arg Ala Ser Ala Ser Asn Gly Ser Asp Asp Ala
        50              55              60

Ser Ala His Ile Gln Arg Thr Val Ala Ser Cys Pro Asn Thr Arg Ile
65              70              75              80

Val Leu Gly Gly Tyr Ser Gln Gly Ala Thr Val Ile Asp Leu Ser Thr
        85              90              95

Ser Ala Met Pro Pro Ala Val Ala Asp His Val Ala Ala Val Ala Leu
        100             105             110

Phe Gly Glu Pro Ser Ser Gly Phe Ser Ser Met Leu Trp Gly Gly Gly
        115             120             125

Ser Leu Pro Thr Ile Gly Pro Leu Tyr Ser Ser Lys Thr Ile Asn Leu
        130             135             140

Cys Ala Pro Asp Asp Pro Ile Cys Thr Gly Gly Gly Asn Ile Met Ala
145             150             155             160

His Val Ser Tyr Val Gln Ser Gly Met Thr Ser Gln Ala Ala Thr Phe
            165             170             175

Ala Ala Asn Arg Leu Asp His Ala Gly
        180             185
```

(2) INFORMATION FOR SEQ ID NO: 11:

    (i) SEQUENCE CHARACTERISTICS:
        (A) LENGTH: 949 base pairs
        (B) TYPE: nucleic acid
        (C) STRANDEDNESS: double
        (D) TOPOLOGY: circular

    (ii) MOLECULE TYPE: DNA (genomic)

    (vi) ORIGINAL SOURCE:
        (A) ORGANISM: Mycobacterium tuberculosis
        (B) STRAIN: H37Rv

    (ix) FEATURE:
        (A) NAME/KEY: CDS
        (B) LOCATION: 201..749

    (ix) FEATURE:
        (A) NAME/KEY: mat_peptide
        (B) LOCATION: 224..749

    (xi) SEQUENCE DESCRIPTION: SEQ ID NO: 11:

```
AGCCGCTCGC GTGGGGTCAA CCGGGTTTCC ACCTGCTCAC TCATTTTGCC GCCTTTCTGT      60

GTCCGGGCCG AGGCTTGCGC TCAATAACTC GGTCAAGTTC CTTCACAGAC TGCCATCACT     120

GGCCCGTCGG CGGGCTCGTT GCGGGTGCGC CGCGTGCGGG TTTGTGTTCC GGGCACCGGG     180
```

```
TGGGGGCCCG CCCGGGCGTA ATG GCA GAC TGT GAT TCC GTG ACT AAC AGC        230
                      Met Ala Asp Cys Asp Ser Val Thr Asn Ser
                       -7       -5                    1

CCC CTT GCG ACC GCT ACC GCC ACG CTG CAC ACT AAC CGC GGC GAC ATC      278
Pro Leu Ala Thr Ala Thr Ala Thr Leu His Thr Asn Arg Gly Asp Ile
     5                    10                  15

AAG ATC GCC CTG TTC GGA AAC CAT GCG CCC AAG ACC GTC GCC AAT TTT      326
Lys Ile Ala Leu Phe Gly Asn His Ala Pro Lys Thr Val Ala Asn Phe
 20                  25                  30                  35

GTG GGC CTT GCG CAG GGC ACC AAG GAC TAT TCG ACC CAA AAC GCA TCA      374
Val Gly Leu Ala Gln Gly Thr Lys Asp Tyr Ser Thr Gln Asn Ala Ser
                 40                  45                  50

GGT GGC CCG TCC GGC CCG TTC TAC GAC GGC GCG GTC TTT CAC CGG GTG      422
Gly Gly Pro Ser Gly Pro Phe Tyr Asp Gly Ala Val Phe His Arg Val
             55                  60                  65

ATC CAG GGC TTC ATG ATC CAG GGT GGC GAT CCA ACC GGG ACG GGT CGC      470
Ile Gln Gly Phe Met Ile Gln Gly Gly Asp Pro Thr Gly Thr Gly Arg
             70                  75                  80

GGC GGA CCC GGC TAC AAG TTC GCC GAC GAG TTC CAC CCC GAG CTG CAA      518
Gly Gly Pro Gly Tyr Lys Phe Ala Asp Glu Phe His Pro Glu Leu Gln
     85                  90                  95

TTC GAC AAG CCC TAT CTG CTC GCG ATG GCC AAC GCC GGT CCG GGC ACC      566
Phe Asp Lys Pro Tyr Leu Leu Ala Met Ala Asn Ala Gly Pro Gly Thr
100                 105                 110                 115

AAC GGC TCA CAG TTT TTC ATC ACC GTC GGC AAG ACT CCG CAC CTG AAC      614
Asn Gly Ser Gln Phe Phe Ile Thr Val Gly Lys Thr Pro His Leu Asn
                 120                 125                 130

CGG CGC CAC ACC ATT TTC GGT GAA GTG ATC GAC GCG GAG TCA CAG CGG      662
Arg Arg His Thr Ile Phe Gly Glu Val Ile Asp Ala Glu Ser Gln Arg
                 135                 140                 145

GTT GTG GAG GCG ATC TCC AAG ACG GCC ACC GAC GGC AAC GAT CGG CCG      710
Val Val Glu Ala Ile Ser Lys Thr Ala Thr Asp Gly Asn Asp Arg Pro
             150                 155                 160

ACG GAC CCG GTG GTG ATC GAG TCG ATC ACC ATC TCC TGA CCCGAAGCTA      759
Thr Asp Pro Val Val Ile Glu Ser Ile Thr Ile Ser
         165                 170                 175

CGTCGGCTCG TCGCTCGAAT ACACCTTGTG GACCCGCCAG GGCACGTGGC GGTACACCGA    819

CACGCCGTTG GGGCCGTTCA ACCGGACGCC CTCACGCCAA GTCCGCTCAC CTTTGGCCGC    879

GACCGGCGTA ACCGGCAGCG GTAAGCGCAT CGAGCACCTC CACTGGGTCG GTGCCGAGAT    939

CCCAGCGGGA                                                          949
```

(2) INFORMATION FOR SEQ ID NO: 12:

    (i) SEQUENCE CHARACTERISTICS:
        (A) LENGTH: 182 amino acids

```
        (B)  TYPE: amino acid
        (D)  TOPOLOGY: linear

     (ii)  MOLECULE TYPE: protein
     (xi)  SEQUENCE DESCRIPTION: SEQ ID NO: 12:

Met Ala Asp Cys Asp Ser Val Thr Asn Ser Pro Leu Ala Thr Ala Thr
 -7      -5                   1                   5

Ala Thr Leu His Thr Asn Arg Gly Asp Ile Lys Ile Ala Leu Phe Gly
 10              15                  20                      25

Asn His Ala Pro Lys Thr Val Ala Asn Phe Val Gly Leu Ala Gln Gly
             30                  35                  40

Thr Lys Asp Tyr Ser Thr Gln Asn Ala Ser Gly Gly Pro Ser Gly Pro
             45                  50                  55

Phe Tyr Asp Gly Ala Val Phe His Arg Val Ile Gln Gly Phe Met Ile
         60                  65                  70

Gln Gly Gly Asp Pro Thr Gly Thr Gly Arg Gly Gly Pro Gly Tyr Lys
     75                  80                  85


Phe Ala Asp Glu Phe His Pro Glu Leu Gln Phe Asp Lys Pro Tyr Leu
 90                  95                  100                 105

Leu Ala Met Ala Asn Ala Gly Pro Gly Thr Asn Gly Ser Gln Phe Phe
             110                 115                 120

Ile Thr Val Gly Lys Thr Pro His Leu Asn Arg Arg His Thr Ile Phe
             125                 130                 135

Gly Glu Val Ile Asp Ala Glu Ser Gln Arg Val Val Glu Ala Ile Ser
         140                 145                 150

Lys Thr Ala Thr Asp Gly Asn Asp Arg Pro Thr Asp Pro Val Val Ile
     155                 160                 165

Glu Ser Ile Thr Ile Ser
170                 175

(2)  INFORMATION FOR SEQ ID NO: 13:

     (i)  SEQUENCE CHARACTERISTICS:
          (A)  LENGTH: 1060 base pairs
          (B)  TYPE: nucleic acid
          (C)  STRANDEDNESS: double
          (D)  TOPOLOGY: circular

     (ii)  MOLECULE TYPE: DNA (genomic)

     (vi)  ORIGINAL SOURCE:
          (A)  ORGANISM: Mycobacterium tuberculosis
          (B)  STRAIN: H37Rv

     (ix)  FEATURE:
          (A)  NAME/KEY: CDS
          (B)  LOCATION: 201..860
```

(ix) FEATURE:
    (A) NAME/KEY: sig_peptide
    (B) LOCATION: 201..296

(ix) FEATURE:
    (A) NAME/KEY: mat_peptide
    (B) LOCATION: 297..860

(xi) SEQUENCE DESCRIPTION: SEQ ID NO: 13:

```
TGGACCTTCA CCGGCGGTCC CTTCGCTTCG GGGGCGACAC CTAACATACT GGTCGTCAAC        60

CTACCGCGAC ACCGCTGGGA CTTTGTGCCA TTGCCGGCCA CTCGGGGCCG CTGCGGCCTG       120

GAAAAATTGG TCGGGCACGG GCGGCCGCGG GTCGCTACCA TCCCACTGTG AATGATTTAC       180

TGACCCGCCG ACTGCTCACC ATG GGC GCG GCC GCC GCA ATG CTG GCC GCG          230
                      Met Gly Ala Ala Ala Ala Met Leu Ala Ala
                      -32     -30                 -25


GTG CTT CTG CTT ACT CCC ATC ACC GTT CCC GCC GGC TAC CCC GGT GCC        278
Val Leu Leu Leu Thr Pro Ile Thr Val Pro Ala Gly Tyr Pro Gly Ala
        -20                 -15                 -10


GTT GCA CCG GCC ACT GCA GCC TGC CCC GAC GCC GAA GTG GTG TTC GCC        326
Val Ala Pro Ala Thr Ala Ala Cys Pro Asp Ala Glu Val Val Phe Ala
        -5                  1               5                   10


CGC GGC CGC TTC GAA CCG CCC GGG ATT GGC ACG GTC GGC AAC GCA TTC        374
Arg Gly Arg Phe Glu Pro Pro Gly Ile Gly Thr Val Gly Asn Ala Phe
                15                  20                  25


GTC AGC GCG CTG CGC TCG AAG GTC AAC AAG AAT GTC GGG GTC TAC GCG        422
Val Ser Ala Leu Arg Ser Lys Val Asn Lys Asn Val Gly Val Tyr Ala
            30                  35                  40


GTG AAA TAC CCC GCC GAC AAT CAG ATC GAT GTG GGC GCC AAC GAC ATG        470
Val Lys Tyr Pro Ala Asp Asn Gln Ile Asp Val Gly Ala Asn Asp Met
            45                  50                  55


AGC GCC CAC ATT CAG AGC ATG GCC AAC AGC TGT CCG AAT ACC CGC CTG        518
Ser Ala His Ile Gln Ser Met Ala Asn Ser Cys Pro Asn Thr Arg Leu
        60                  65                  70


GTG CCC GGC GGT TAC TCG CTG GGC GCG GCC GTC ACC GAC GTG GTA CTC        566
Val Pro Gly Gly Tyr Ser Leu Gly Ala Ala Val Thr Asp Val Val Leu
75                  80                  85                  90


GCG GTG CCC ACC CAG ATG TGG GGC TTC ACC AAT CCC CTG CCT CCC GGC        614
Ala Val Pro Thr Gln Met Trp Gly Phe Thr Asn Pro Leu Pro Pro Gly
                95                  100                 105


AGT GAT GAG CAC ATC GCC GCG GTC GCG CTG TTC GGC AAT GGC AGT CAG        662
Ser Asp Glu His Ile Ala Ala Val Ala Leu Phe Gly Asn Gly Ser Gln
                110                 115                 120


TGG GTC GGC CCC ATC ACC AAC TTC AGC CCC GCC TAC AAC GAT CGG ACC        710
Trp Val Gly Pro Ile Thr Asn Phe Ser Pro Ala Tyr Asn Asp Arg Thr
        125                 130                 135
```

```
ATC GAG TTG TGT CAC GGC GAC GAC CCC GTC TGC CAC CCT GCC GAC CCC        758
Ile Glu Leu Cys His Gly Asp Asp Pro Val Cys His Pro Ala Asp Pro
    140                 145                 150

AAC ACC TGG GAG GCC AAC TGG CCC CAG CAC CTC GCC GGG GCC TAT GTC        806
Asn Thr Trp Glu Ala Asn Trp Pro Gln His Leu Ala Gly Ala Tyr Val
155                 160                 165                 170

TCG TCG GGC ATG GTC AAC CAG GCG GCT GAC TTC GTT GCC GGA AAG CTG        854
Ser Ser Gly Met Val Asn Gln Ala Ala Asp Phe Val Ala Gly Lys Leu
                175                 180                 185

CAA TAG CCACCTAGCC CGTGCGCGAG TCTTTGCTTC ACGCTTTCGC TAACCGACCA         910
Gln


ACGCGCGCAC GATGGAGGGG TCCGTGGTCA TATCAAGACA AGAAGGGAGT AGGCGATGCA       970

CGCAAAAGTC GGCGACTACC TCGTGGTGAA GGGCACAACC ACGGAACGGC ATGATCAACA      1030

TGCTGAGATC ATCGAGGTGC GCTCCGCAGA                                      1060
```

(2) INFORMATION FOR SEQ ID NO: 14:

    (i) SEQUENCE CHARACTERISTICS:
        (A) LENGTH: 219 amino acids
        (B) TYPE: amino acid
        (D) TOPOLOGY: linear

    (ii) MOLECULE TYPE: protein
(xi) SEQUENCE DESCRIPTION: SEQ ID NO: 14:

```
Met Gly Ala Ala Ala Ala Met Leu Ala Ala Val Leu Leu Leu Thr Pro
-32     -30             -25             -20

Ile Thr Val Pro Ala Gly Tyr Pro Gly Ala Val Ala Pro Ala Thr Ala
    -15             -10              -5

Ala Cys Pro Asp Ala Glu Val Val Phe Ala Arg Gly Arg Phe Glu Pro
 1           5               10                  15

Pro Gly Ile Gly Thr Val Gly Asn Ala Phe Val Ser Ala Leu Arg Ser
            20                  25                  30

Lys Val Asn Lys Asn Val Gly Val Tyr Ala Val Lys Tyr Pro Ala Asp
        35                  40                  45

Asn Gln Ile Asp Val Gly Ala Asn Asp Met Ser Ala His Ile Gln Ser
        50                  55                  60

Met Ala Asn Ser Cys Pro Asn Thr Arg Leu Val Pro Gly Gly Tyr Ser
65                  70                  75                  80

Leu Gly Ala Ala Val Thr Asp Val Val Leu Ala Val Pro Thr Gln Met
                85                  90                  95

Trp Gly Phe Thr Asn Pro Leu Pro Pro Gly Ser Asp Glu His Ile Ala
                100                 105                 110
```

81

```
Ala Val Ala Leu Phe Gly Asn Gly Ser Gln Trp Val Gly Pro Ile Thr
        115                 120                 125

Asn Phe Ser Pro Ala Tyr Asn Asp Arg Thr Ile Glu Leu Cys His Gly
        130                 135                 140

Asp Asp Pro Val Cys His Pro Ala Asp Pro Asn Thr Trp Glu Ala Asn
145                 150                 155                 160

Trp Pro Gln His Leu Ala Gly Ala Tyr Val Ser Ser Gly Met Val Asn
                165                 170                 175

Gln Ala Ala Asp Phe Val Ala Gly Lys Leu Gln
            180                 185
```

(2) INFORMATION FOR SEQ ID NO: 15:

    (i) SEQUENCE CHARACTERISTICS:
        (A) LENGTH: 1198 base pairs
        (B) TYPE: nucleic acid
        (C) STRANDEDNESS: double
        (D) TOPOLOGY: circular

    (ii) MOLECULE TYPE: DNA (genomic)

    (vi) ORIGINAL SOURCE:
        (A) ORGANISM: Mycobacterium tuberculosis
        (B) STRAIN: H37Rv

    (ix) FEATURE:
        (A) NAME/KEY: CDS
        (B) LOCATION: 201..998

    (ix) FEATURE:
        (A) NAME/KEY: mat_peptide
        (B) LOCATION: 201..998

    (xi) SEQUENCE DESCRIPTION: SEQ ID NO: 15:

```
CAGATGCTGC GCAACATGTT TCTCGGCGAT CCGGCAGGCA ACACCGATCG AGTGCTTGAC      60

TTTTCCACCG CGGTGACCGG CGGACTGTTC TTCTCACCCA CCATCGACTT TCTCGACCAT     120

CCACCGCCCC TACCGCAGGC GGCGACGCCA ACTCTGGCAG CCGGGTCGCT ATCGATCGGC     180

AGCTTGAAAG GAAGCCCCCG ATG AAC AAT CTC TAC CGC GAT TTG GCA CCG        230
                        Met Asn Asn Leu Tyr Arg Asp Leu Ala Pro
                          1               5                   10

GTC ACC GAA GCC GCT TGG GCG GAA ATC GAA TTG GAG GCG GCG CGG ACG     278
Val Thr Glu Ala Ala Trp Ala Glu Ile Glu Leu Glu Ala Ala Arg Thr
            15                  20                  25

TTC AAG CGA CAC ATC GCC GGG CGC CGG GTG GTC GAT GTC AGT GAT CCC     326
Phe Lys Arg His Ile Ala Gly Arg Arg Val Val Asp Val Ser Asp Pro
        30                  35                  40

GGG GGG CCC GTC ACC GCG GCG GTC AGC ACC GGC CGG CTG ATC GAT GTT     374
Gly Gly Pro Val Thr Ala Ala Val Ser Thr Gly Arg Leu Ile Asp Val
        45                  50                  55
```

```
AAG GCA CCA ACC AAC GGC GTG ATC GCC CAC CTG CGG GCC AGC AAA CCC      422
Lys Ala Pro Thr Asn Gly Val Ile Ala His Leu Arg Ala Ser Lys Pro
    60                  65                  70

CTT GTC CGG CTA CGG GTT CCG TTT ACC CTG TCG CGC AAC GAG ATC GAC      470
Leu Val Arg Leu Arg Val Pro Phe Thr Leu Ser Arg Asn Glu Ile Asp
    75                  80                  85                  90

GAC GTG GAA CGT GGC TCT AAG GAC TCC GAT TGG GAA CCG GTA AAG GAG      518
Asp Val Glu Arg Gly Ser Lys Asp Ser Asp Trp Glu Pro Val Lys Glu
                    95                  100                 105


GCG GCC AAG AAG CTG GCC TTC GTC GAG GAC CGC ACA ATA TTC GAA GGC      566
Ala Ala Lys Lys Leu Ala Phe Val Glu Asp Arg Thr Ile Phe Glu Gly
                110                 115                 120

TAC AGC GCC GCA TCA ATC GAA GGG ATC CGC AGC GCG AGT TCG AAC CCG      614
Tyr Ser Ala Ala Ser Ile Glu Gly Ile Arg Ser Ala Ser Ser Asn Pro
        125                 130                 135

GCG CTG ACG TTG CCC GAG GAT CCC CGT GAA ATC CCT GAT GTC ATC TCC      662
Ala Leu Thr Leu Pro Glu Asp Pro Arg Glu Ile Pro Asp Val Ile Ser
    140                 145                 150

CAG GCA TTG TCC GAA CTG CGG TTG GCC GGT GTG GAC GGA CCG TAT TCG      710
Gln Ala Leu Ser Glu Leu Arg Leu Ala Gly Val Asp Gly Pro Tyr Ser
155                 160                 165                 170

GTG TTG CTC TCT GCT GAC GTC TAC ACC AAG GTT AGC GAG ACT TCC GAT      758
Val Leu Leu Ser Ala Asp Val Tyr Thr Lys Val Ser Glu Thr Ser Asp
                175                 180                 185

CAC GGC TAT CCC ATC CGT GAG CAT CTG AAC CGG CTG GTG GAC GGG GAC      806
His Gly Tyr Pro Ile Arg Glu His Leu Asn Arg Leu Val Asp Gly Asp
                190                 195                 200

ATC ATT TGG GCC CCG GCC ATC GAC GGC GCG TTC GTG CTG ACC ACT CGA      854
Ile Ile Trp Ala Pro Ala Ile Asp Gly Ala Phe Val Leu Thr Thr Arg
            205                 210                 215

GGC GGC GAC TTC GAC CTA CAG CTG GGC ACC GAC GTT GCA ATC GGG TAC      902
Gly Gly Asp Phe Asp Leu Gln Leu Gly Thr Asp Val Ala Ile Gly Tyr
    220                 225                 230

GCC AGC CAC GAC ACG GAC ACC GAG CGC CTC TAC CTG CAG GAG ACG CTG      950
Ala Ser His Asp Thr Asp Thr Glu Arg Leu Tyr Leu Gln Glu Thr Leu
235                 240                 245                 250

ACG TTC CTT TGC TAC ACC GCC GAG GCG TCG GTC GCG CTC AGC CAC TAA      998
Thr Phe Leu Cys Tyr Thr Ala Glu Ala Ser Val Ala Leu Ser His
                255                 260                 265

GGCACGAGCG CGAGCAATAG CTCCTATGGC AAGCGGCCGC GGGTTGGGTG TGTTCGGAGC     1058

TGGGCTGGTG GACGGTGCGC AGGGCCTGGA AGACGGTGCG GGCTAGGCGG CGTTTGAGGC     1118

AGCGTAGTGC TGCGCGTTTG GTTTTCCCGG CGTCTTGCAG CCTTTGGTAG TAGGCCTGGC     1178

CCCGGCTGTC GGTCATCCGG     1198
```

(2) INFORMATION FOR SEQ ID NO: 16:

    (i) SEQUENCE CHARACTERISTICS:
        (A) LENGTH: 265 amino acids
        (B) TYPE: amino acid
        (D) TOPOLOGY: linear

    (ii) MOLECULE TYPE: protein
    (xi) SEQUENCE DESCRIPTION: SEQ ID NO: 16:

```
Met Asn Asn Leu Tyr Arg Asp Leu Ala Pro Val Thr Glu Ala Ala Trp
 1               5                  10                  15

Ala Glu Ile Glu Leu Glu Ala Ala Arg Thr Phe Lys Arg His Ile Ala
            20                  25                  30

Gly Arg Arg Val Val Asp Val Ser Asp Pro Gly Gly Pro Val Thr Ala
            35                  40                  45

Ala Val Ser Thr Gly Arg Leu Ile Asp Val Lys Ala Pro Thr Asn Gly
        50                  55                  60

Val Ile Ala His Leu Arg Ala Ser Lys Pro Leu Val Arg Leu Arg Val
 65                  70                  75                  80

Pro Phe Thr Leu Ser Arg Asn Glu Ile Asp Asp Val Glu Arg Gly Ser
                85                  90                  95

Lys Asp Ser Asp Trp Glu Pro Val Lys Glu Ala Ala Lys Lys Leu Ala
            100                 105                 110

Phe Val Glu Asp Arg Thr Ile Phe Glu Gly Tyr Ser Ala Ala Ser Ile
            115                 120                 125

Glu Gly Ile Arg Ser Ala Ser Ser Asn Pro Ala Leu Thr Leu Pro Glu
            130                 135                 140

Asp Pro Arg Glu Ile Pro Asp Val Ile Ser Gln Ala Leu Ser Glu Leu
145                 150                 155                 160

Arg Leu Ala Gly Val Asp Gly Pro Tyr Ser Val Leu Leu Ser Ala Asp
                165                 170                 175

Val Tyr Thr Lys Val Ser Glu Thr Ser Asp His Gly Tyr Pro Ile Arg
            180                 185                 190

Glu His Leu Asn Arg Leu Val Asp Gly Asp Ile Ile Trp Ala Pro Ala
            195                 200                 205

Ile Asp Gly Ala Phe Val Leu Thr Thr Arg Gly Gly Asp Phe Asp Leu
            210                 215                 220

Gln Leu Gly Thr Asp Val Ala Ile Gly Tyr Ala Ser His Asp Thr Asp
225                 230                 235                 240

Thr Glu Arg Leu Tyr Leu Gln Glu Thr Leu Thr Phe Leu Cys Tyr Thr
                245                 250                 255

Ala Glu Ala Ser Val Ala Leu Ser His
```

260                265

(2) INFORMATION FOR SEQ ID NO: 17:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide

    (v) FRAGMENT TYPE: N-terminal

    (vi) ORIGINAL SOURCE:
        (A) ORGANISM: Mycobacterium tuberculosis
        (B) STRAIN: H37Rv

    (ix) FEATURE:
        (A) NAME/KEY: Duplication
        (B) LOCATION: 1
        (D) OTHER INFORMATION: Ala is Ala or Ser

    (ix) FEATURE:
        (A) NAME/KEY: Duplication
        (B) LOCATION: 13
        (D) OTHER INFORMATION: Xaa is unknown

    (xi) SEQUENCE DESCRIPTION: SEQ ID NO: 17:

Ala Glu Leu Asp Ala Pro Ala Gln Ala Gly Thr Glu Xaa Ala Val
1               5                   10                  15

(2) INFORMATION FOR SEQ ID NO: 18:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide

    (v) FRAGMENT TYPE: N-terminal

    (vi) ORIGINAL SOURCE:
        (A) ORGANISM: Mycobacterium tuberculosis
        (B) STRAIN: H37Rv

    (xi) SEQUENCE DESCRIPTION: SEQ ID NO: 18:

Ala Gln Ile Thr Leu Arg Gly Asn Ala Ile Asn Thr Val Gly Glu
1               5                   10                  15

(2) INFORMATION FOR SEQ ID NO: 19:

    (i) SEQUENCE CHARACTERISTICS:

            (A) LENGTH: 15 amino acids
            (B) TYPE: amino acid
            (C) STRANDEDNESS: single
            (D) TOPOLOGY: linear

     (ii) MOLECULE TYPE: peptide

      (v) FRAGMENT TYPE: N-terminal

     (vi) ORIGINAL SOURCE:
            (A) ORGANISM: Mycobacterium tuberculosis
            (B) STRAIN: H37Rv

     (ix) Feature:
            (A) NAME/KEY: Other
            (B) LOCATION: 3
            (C) OTHER INFORMATION: Xaa is unknown


     (xi) SEQUENCE DESCRIPTION: SEQ ID NO: 19:

     Asp Pro Xaa Ser Asp Ile Ala Val Val Phe Ala Arg Gly Thr His
     1               5                   10                  15

(2) INFORMATION FOR SEQ ID NO: 20:

      (i) SEQUENCE CHARACTERISTICS:
            (A) LENGTH: 15 amino acids
            (B) TYPE: amino acid
            (C) STRANDEDNESS: single
            (D) TOPOLOGY: linear

     (ii) MOLECULE TYPE: peptide

      (v) FRAGMENT TYPE: N-terminal

     (vi) ORIGINAL SOURCE:
            (A) ORGANISM: Mycobacterium tuberculosis
            (B) STRAIN: H37Rv


     (xi) SEQUENCE DESCRIPTION: SEQ ID NO: 20:

     Thr Asn Ser Pro Leu Ala Thr Ala Thr Ala Thr Leu His Thr Asn
     1               5                   10                  15

(2) INFORMATION FOR SEQ ID NO: 21:

      (i) SEQUENCE CHARACTERISTICS:
            (A) LENGTH: 15 amino acids
            (B) TYPE: amino acid
            (C) STRANDEDNESS: single
            (D) TOPOLOGY: linear

     (ii) MOLECULE TYPE: peptide

      (v) FRAGMENT TYPE: N-terminal

     (vi) ORIGINAL SOURCE:
            (A) ORGANISM: Mycobacterium tuberculosis
            (B) STRAIN: H37Rv

```
(ix) Feature:
     (A) NAME/KEY: Other
     (B) LOCATION: 2
     (C) OTHER INFORMATION: Xaa is unknown


(xi) SEQUENCE DESCRIPTION: SEQ ID NO: 21:

Ala Xaa Pro Asp Ala Glu Val Val Phe Ala Arg Gly Arg Phe Glu
1               5                   10                  15

(2) INFORMATION FOR SEQ ID NO: 22:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide

    (v) FRAGMENT TYPE: N-terminal

    (vi) ORIGINAL SOURCE:
         (A) ORGANISM: Mycobacterium tuberculosis
         (B) STRAIN: H37Rv

    (ix) Feature:
         (A) NAME/KEY: Other
         (B) LOCATION: 1
         (C) OTHER INFORMATION: Xaa is unknown

    (ix) FEATURE:
         (A) NAME/KEY: Duplication
         (B) LOCATION: 2
         (D) OTHER INFORMATION: Ile is Ile or Val

    (ix) FEATURE:
         (A) NAME/KEY: Duplication
         (B) LOCATION: 10
         (D) OTHER INFORMATION: Val is Val or Thr

    (ix) FEATURE:
         (A) NAME/KEY: Duplication
         (B) LOCATION: 11
         (D) OTHER INFORMATION: Val is Val or Phe

    (ix) FEATURE:
         (A) NAME/KEY: Duplication
         (B) LOCATION: 14
         (D) OTHER INFORMATION: Asp is Asp or Gln


(xi) SEQUENCE DESCRIPTION: SEQ ID NO: 22:

Xaa Ile Gln Lys Ser Leu Glu Leu Ile Val Val Thr Ala Asp Glu
1               5                   10                  15

(2) INFORMATION FOR SEQ ID NO: 23:
```

(i) SEQUENCE CHARACTERISTICS:
    (A) LENGTH: 19 amino acids
    (B) TYPE: amino acid
    (C) STRANDEDNESS: single
    (D) TOPOLOGY: linear

(ii) MOLECULE TYPE: peptide

(v) FRAGMENT TYPE: N-terminal

(vi) ORIGINAL SOURCE:
    (A) ORGANISM: Mycobacterium tuberculosis
    (B) STRAIN: H37Rv

(xi) SEQUENCE DESCRIPTION: SEQ ID NO: 23:

Met Asn Asn Leu Tyr Arg Asp Leu Ala Pro Val Thr Glu Ala Ala Trp
1               5                   10              15

Ala Glu Ile

(2) INFORMATION FOR SEQ ID NO: 24:

    (i) SEQUENCE CHARACTERISTICS:
        (A) LENGTH: 34 base pairs
        (B) TYPE: nucleic acid
        (C) STRANDEDNESS: single
        (D) TOPOLOGY: linear

    (ii) MOLECULE TYPE: DNA (synthetic)

    (xi) SEQUENCE DESCRIPTION: SEQ ID NO: 24:

CCCGGCTCGA GAACCTSTAC CGCGACCTSG CSCC                                    34

(2) INFORMATION FOR SEQ ID NO: 25:

    (i) SEQUENCE CHARACTERISTICS:
        (A) LENGTH: 37 base pairs
        (B) TYPE: nucleic acid
        (C) STRANDEDNESS: single
        (D) TOPOLOGY: linear

    (ii) MOLECULE TYPE: DNA (synthetic)

    (xi) SEQUENCE DESCRIPTION: SEQ ID NO: 25:

GGGCCGGATC CGASGCSGCG TCCTTSACSG GYTGCCA                                 37

(2) INFORMATION FOR SEQ ID NO: 26:

    (i) SEQUENCE CHARACTERISTICS:

          (A) LENGTH: 28 base pairs
          (B) TYPE: nucleic acid
          (C) STRANDEDNESS: single
          (D) TOPOLOGY: linear

    (ii) MOLECULE TYPE: DNA (synthetic)

    (xi) SEQUENCE DESCRIPTION: SEQ ID NO: 26:

GGAAGCCCCA TATGAACAAT CTCTACCG                     28

(2) INFORMATION FOR SEQ ID NO: 27:

    (i) SEQUENCE CHARACTERISTICS:
          (A) LENGTH: 32 base pairs
          (B) TYPE: nucleic acid
          (C) STRANDEDNESS: single
          (D) TOPOLOGY: linear

    (ii) MOLECULE TYPE: DNA (synthetic)

    (xi) SEQUENCE DESCRIPTION: SEQ ID NO: 27:

CGCGCTCAGC CCTTAGTGAC TGAGCGCGAC CG               32

(2) INFORMATION FOR SEQ ID NO: 28:

    (i) SEQUENCE CHARACTERISTICS:
          (A) LENGTH: 24 base pairs
          (B) TYPE: nucleic acid
          (C) STRANDEDNESS: single
          (D) TOPOLOGY: linear

    (ii) MOLECULE TYPE: DNA (synthetic)

    (iv) ANTI-SENSE: NO

    (xi) SEQUENCE DESCRIPTION: SEQ ID NO: 28:

CTCGAATTCG CCGGGTGCAC ACAG                     24

(2) INFORMATION FOR SEQ ID NO: 29:

    (i) SEQUENCE CHARACTERISTICS:
          (A) LENGTH: 25 base pairs
          (B) TYPE: nucleic acid
          (C) STRANDEDNESS: single
          (D) TOPOLOGY: linear

    (ii) MOLECULE TYPE: DNA (synthetic)

    (iv) ANTI-SENSE: NO

(xi) SEQUENCE DESCRIPTION: SEQ ID NO: 29:

CTCGAATTCG CCCCCATACG AGAAC                                             25

(2) INFORMATION FOR SEQ ID NO: 30:

    (i) SEQUENCE CHARACTERISTICS:
        (A) LENGTH: 15 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

(xi) SEQUENCE DESCRIPTION: SEQ ID NO: 30:

GTGTATCTGC TGGAC                                                       15

(2) INFORMATION FOR SEQ ID NO: 31:

    (i) SEQUENCE CHARACTERISTICS:
        (A) LENGTH: 15 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

(xi) SEQUENCE DESCRIPTION: SEQ ID NO: 31:

CCGACTGGCT GGCCG                                                       15

(2) INFORMATION FOR SEQ ID NO: 32:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: YES

(xi) SEQUENCE DESCRIPTION: SEQ ID NO: 32:

GAGGAATTCG CTTAGCGGAT CGCA                                    24

(2) INFORMATION FOR SEQ ID NO: 33:

    (i) SEQUENCE CHARACTERISTICS:
        (A) LENGTH: 15 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: YES

(xi) SEQUENCE DESCRIPTION: SEQ ID NO: 33:

CCCACATTCC GTTGG                                              15

(2) INFORMATION FOR SEQ ID NO: 34:

    (i) SEQUENCE CHARACTERISTICS:
        (A) LENGTH: 15 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: YES

(xi) SEQUENCE DESCRIPTION: SEQ ID NO: 34:

GTCCAGCAGA TACAC                                              15

(2) INFORMATION FOR SEQ ID NO: 35:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

(xi) SEQUENCE DESCRIPTION: SEQ ID NO: 35:

GTACGAGAAT TCATGTCGCA AATCATG                                 27

(2) INFORMATION FOR SEQ ID NO: 36:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

    (xi) SEQUENCE DESCRIPTION: SEQ ID NO: 36:

GTACGAGAAT TCGAGCTTGG GGTGCCG               27

(2) INFORMATION FOR SEQ ID NO: 37:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

    (xi) SEQUENCE DESCRIPTION: SEQ ID NO: 37:

CGATTCCAAG CTTGTGGCCG CCGACCCG            28

(2) INFORMATION FOR SEQ ID NO: 38:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: YES

    (xi) SEQUENCE DESCRIPTION: SEQ ID NO: 38:

CGTTAGGGAT CCTCATCGCC ATGGTGTTGG           30

(2) INFORMATION FOR SEQ ID NO: 39:

    (i) SEQUENCE CHARACTERISTICS:
        (A) LENGTH: 26 base pairs

             (B) TYPE: nucleic acid
             (C) STRANDEDNESS: single
             (D) TOPOLOGY: linear

    (ii) MOLECULE TYPE: DNA (synthetic)

    (iv) ANTI-SENSE: YES


    (xi) SEQUENCE DESCRIPTION: SEQ ID NO: 39:

CGTTAGGGAT CCGGTTCCAC TGTGCC                                    26

(2) INFORMATION FOR SEQ ID NO: 40:

    (i) SEQUENCE CHARACTERISTICS:
             (A) LENGTH: 28 base pairs
             (B) TYPE: nucleic acid
             (C) STRANDEDNESS: single
             (D) TOPOLOGY: linear

    (ii) MOLECULE TYPE: DNA (synthetic)

    (iv) ANTI-SENSE: YES


    (xi) SEQUENCE DESCRIPTION: SEQ ID NO: 40:

CGTTAGGGAT CCTCAGGTCT TTTCGATG                                  28

(2) INFORMATION FOR SEQ ID NO: 41:

    (i) SEQUENCE CHARACTERISTICS:
             (A) LENGTH: 952 base pairs
             (B) TYPE: nucleic acid
             (C) STRANDEDNESS: double
             (D) TOPOLOGY: circular

    (ii) MOLECULE TYPE: DNA (genomic)

    (vi) ORIGINAL SOURCE:
             (A) ORGANISM: Mycobacterium tuberculosis
             (B) STRAIN: H37Rv

    (ix) FEATURE:
             (A) NAME/KEY: CDS
             (B) LOCATION: 45..944

    (ix) FEATURE:
             (A) NAME/KEY: sig_peptide
             (B) LOCATION: 45..143

    (ix) FEATURE:
             (A) NAME/KEY: mat_peptide
             (B) LOCATION: 144..941

(xi) SEQUENCE DESCRIPTION: SEQ ID NO: 41:

```
GAATTCGCCG GGTGCACACA GCCTTACACG ACGGAGGTGG ACAC ATG AAG GGT CGG          56
                                                    Met Lys Gly Arg
                                                    -33          -30

TCG GCG CTG CTG CGG GCG CTC TGG ATT GCC GCA CTG TCA TTC GGG TTG          104
Ser Ala Leu Leu Arg Ala Leu Trp Ile Ala Ala Leu Ser Phe Gly Leu
            -25             -20             -15

GGC GGT GTC GCG GTA GCC GCG GAA CCC ACC GCC AAG GCC GCC CCA TAC          152
Gly Gly Val Ala Val Ala Ala Glu Pro Thr Ala Lys Ala Ala Pro Tyr
            -10             -5              1

GAG AAC CTG ATG GTG CCG TCG CCC TCG ATG GGC CGG GAC ATC CCG GTG          200
Glu Asn Leu Met Val Pro Ser Pro Ser Met Gly Arg Asp Ile Pro Val
        5               10              15

GCC TTC CTA GCC GGT GGG CCG CAC GCG GTG TAT CTG CTG GAC GCC TTC          248
Ala Phe Leu Ala Gly Gly Pro His Ala Val Tyr Leu Leu Asp Ala Phe
    20              25              30              35

AAC GCC GGC CCG GAT GTC AGT AAC TGG GTC ACC GCG GGT AAC GCG ATG          296
Asn Ala Gly Pro Asp Val Ser Asn Trp Val Thr Ala Gly Asn Ala Met
                40              45              50

AAC ACG TTG GCG GGC AAG GGG ATT TCG GTG GTG GCA CCG GCC GGT GGT          344
Asn Thr Leu Ala Gly Lys Gly Ile Ser Val Val Ala Pro Ala Gly Gly
            55              60              65

GCG TAC AGC ATG TAC ACC AAC TGG GAG CAG GAT GGC AGC AAG CAG TGG          392
Ala Tyr Ser Met Tyr Thr Asn Trp Glu Gln Asp Gly Ser Lys Gln Trp
            70              75              80

GAC ACC TTC TTG TCC GCT GAG CTG CCC GAC TGG CTG GCC GCT AAC CGG          440
Asp Thr Phe Leu Ser Ala Glu Leu Pro Asp Trp Leu Ala Ala Asn Arg
    85              90              95

GGC TTG GCC CCC GGT GGC CAT GCG GCC GTT GGC GCC GCT CAG GGC GGT          488
Gly Leu Ala Pro Gly Gly His Ala Ala Val Gly Ala Ala Gln Gly Gly
    100             105             110             115

TAC GGG GCG ATG GCG CTG GCG GCC TTC CAC CCC GAC CGC TTC GGC TTC          536
Tyr Gly Ala Met Ala Leu Ala Ala Phe His Pro Asp Arg Phe Gly Phe
            120             125             130

GCT GGC TCG ATG TCG GGC TTT TTG TAC CCG TCG AAC ACC ACC ACC AAC          584
Ala Gly Ser Met Ser Gly Phe Leu Tyr Pro Ser Asn Thr Thr Thr Asn
            135             140             145

GGT GCG ATC GCG GCG GGC ATG CAG CAA TTC GGC GGT GTG GAC ACC AAC          632
Gly Ala Ile Ala Ala Gly Met Gln Gln Phe Gly Gly Val Asp Thr Asn
            150             155             160

GGA ATG TGG GGA GCA CCA CAG CTG GGT CGG TGG AAG TGG CAC GAC CCG          680
Gly Met Trp Gly Ala Pro Gln Leu Gly Arg Trp Lys Trp His Asp Pro
    165             170             175

TGG GTG CAT GCC AGC CTG CTG GCG CAA AAC AAC ACC CGG GTG TGG GTG          728
Trp Val His Ala Ser Leu Leu Ala Gln Asn Asn Thr Arg Val Trp Val
180             185             190             195
```

```
TGG AGC CCG ACC AAC CCG GGA GCC AGC GAT CCC GCC GCC ATG ATC GGC      776
Trp Ser Pro Thr Asn Pro Gly Ala Ser Asp Pro Ala Ala Met Ile Gly
            200             205             210

CAA ACC GCC GAG GCG ATG GGT AAC AGC CGC ATG TTC TAC AAC CAG TAT      824
Gln Thr Ala Glu Ala Met Gly Asn Ser Arg Met Phe Tyr Asn Gln Tyr
            215             220             225

CGC AGC GTC GGC GGG CAC AAC GGA CAC TTC GAC TTC CCA GCC AGC GGT      872
Arg Ser Val Gly Gly His Asn Gly His Phe Asp Phe Pro Ala Ser Gly
            230             235             240

GAC AAC GGC TGG GGC TCG TGG GCG CCC CAG CTG GGC GCT ATG TCG GGC      920
Asp Asn Gly Trp Gly Ser Trp Ala Pro Gln Leu Gly Ala Met Ser Gly
        245             250             255

GAT ATC GTC GGT GCG ATC CGC TAA GCGAATTC                             952
Asp Ile Val Gly Ala Ile Arg
260             265
```

(2) INFORMATION FOR SEQ ID NO: 42:

    (i) SEQUENCE CHARACTERISTICS:
       (A) LENGTH: 299 amino acids
       (B) TYPE: amino acid
       (D) TOPOLOGY: linear

    (ii) MOLECULE TYPE: protein
    (xi) SEQUENCE DESCRIPTION: SEQ ID NO: 42:

```
Met Lys Gly Arg Ser Ala Leu Leu Arg Ala Leu Trp Ile Ala Ala Leu
-33         -30             -25             -20

Ser Phe Gly Leu Gly Gly Val Ala Val Ala Ala Glu Pro Thr Ala Lys
        -15             -10             -5

Ala Ala Pro Tyr Glu Asn Leu Met Val Pro Ser Pro Ser Met Gly Arg
    1               5               10              15

Asp Ile Pro Val Ala Phe Leu Ala Gly Gly Pro His Ala Val Tyr Leu
            20              25              30

Leu Asp Ala Phe Asn Ala Gly Pro Asp Val Ser Asn Trp Val Thr Ala
            35              40              45

Gly Asn Ala Met Asn Thr Leu Ala Gly Lys Gly Ile Ser Val Val Ala
        50              55              60

Pro Ala Gly Gly Ala Tyr Ser Met Tyr Thr Asn Trp Glu Gln Asp Gly
        65              70              75

Ser Lys Gln Trp Asp Thr Phe Leu Ser Ala Glu Leu Pro Asp Trp Leu
80              85              90              95

Ala Ala Asn Arg Gly Leu Ala Pro Gly Gly His Ala Ala Val Gly Ala
            100             105             110

Ala Gln Gly Gly Tyr Gly Ala Met Ala Leu Ala Ala Phe His Pro Asp
            115             120             125
```

95

```
Arg Phe Gly Phe Ala Gly Ser Met Ser Gly Phe Leu Tyr Pro Ser Asn
        130                 135                 140

Thr Thr Thr Asn Gly Ala Ile Ala Ala Gly Met Gln Gln Phe Gly Gly
        145                 150                 155

Val Asp Thr Asn Gly Met Trp Gly Ala Pro Gln Leu Gly Arg Trp Lys
160                 165                 170                 175

Trp His Asp Pro Trp Val His Ala Ser Leu Leu Ala Gln Asn Asn Thr
                180                 185                 190

Arg Val Trp Val Trp Ser Pro Thr Asn Pro Gly Ala Ser Asp Pro Ala
                195                 200                 205

Ala Met Ile Gly Gln Thr Ala Glu Ala Met Gly Asn Ser Arg Met Phe
        210                 215                 220

Tyr Asn Gln Tyr Arg Ser Val Gly Gly His Asn Gly His Phe Asp Phe
        225                 230                 235

Pro Ala Ser Gly Asp Asn Gly Trp Gly Ser Trp Ala Pro Gln Leu Gly
240                 245                 250                 255

Ala Met Ser Gly Asp Ile Val Gly Ala Ile Arg
                260                 265
```

(2) INFORMATION FOR SEQ ID NO: 43:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

    (xi) SEQUENCE DESCRIPTION: SEQ ID NO: 43:

GCAACACCCG GGATGTCGCA AATCATG                        27

(2) INFORMATION FOR SEQ ID NO: 44:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: NO

(xi) SEQUENCE DESCRIPTION: SEQ ID NO: 44:

GTAACACCCG GGGTGGCCGC CGACCCG                                27

(2) INFORMATION FOR SEQ ID NO: 45:

    (i) SEQUENCE CHARACTERISTICS:
        (A) LENGTH: 37 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: YES

    (xi) SEQUENCE DESCRIPTION: SEQ ID NO: 45:

CTACTAAGCT TGGATCCCTA GCCGCCCCAT TTGGCGG                     37

(2) INFORMATION FOR SEQ ID NO: 46:

    (i) SEQUENCE CHARACTERISTICS:
        (A) LENGTH: 38 base pairs
        (B) TYPE: nucleic acid
        (C) STRANDEDNESS: single
        (D) TOPOLOGY: linear

    (ii) MOLECULE TYPE: DNA (synthetic)

    (iv) ANTI-SENSE: YES

    (xi) SEQUENCE DESCRIPTION: SEQ ID NO: 46:

CTACTAAGCT TCCATGGTCA GGTCTTTTCG ATGCTTAC                    38

(2) INFORMATION FOR SEQ ID NO: 47:

    (i) SEQUENCE CHARACTERISTICS:
        (A) LENGTH: 450 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 105...320

    (xi) SEQUENCE DESCRIPTION: SEQ ID NO: 47:

```
GTGCCGCGCT CCCCAGGGTT CTTATGGTTC GATATACCTG AGTTTGATGG AAGTCCGATG        60

ACCAGCAGTC AGCATACGGC ATGGCCGAAA AGAGTGGGGT GATG ATG GCC GAG GAT       116
                                                   Met Ala Glu Asp
                                                    1

GTT CGC GCC GAG ATC GTG GCC AGC GTT CTC GAA GTC GTT GTC AAC GAA        164
Val Arg Ala Glu Ile Val Ala Ser Val Leu Glu Val Val Val Asn Glu
 5               10                  15                  20

GGC GAT CAG ATC GAC AAG GGC GAC GTC GTG GTG CTG CTG GAG TCG ATG        212
Gly Asp Gln Ile Asp Lys Gly Asp Val Val Val Leu Leu Glu Ser Met
             25                  30                  35

AAG ATG GAG ATC CCC GTC CTG GCC GAA GCT GCC GGA ACG GTC AGC AAG        260
Lys Met Glu Ile Pro Val Leu Ala Glu Ala Ala Gly Thr Val Ser Lys
             40                  45                  50

GTG GCG GTA TCG GTG GGC GAT GTC ATT CAG GCC GGC GAC CTT ATC GCG        308
Val Ala Val Ser Val Gly Asp Val Ile Gln Ala Gly Asp Leu Ile Ala
         55                  60                  65

GTG ATC AGC TAGTCGTTGA TAGTCACTCA TGTCCACACT CGGTGATCTG CTCGCCGAA      366
Val Ile Ser
         70

CACACGGTGC TGCCGGGCAG CGCGGTGGAC CACCTGCATG CGGTGGTCGG GGAGTGGCAG      426

CTCCTTGCCG ACTTGTCGTT TGCC                                             450
```

(2) INFORMATION FOR SEQ ID NO: 48:

    (i) SEQUENCE CHARACTERISTICS:
        (A) LENGTH: 71 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 48:

```
Met Ala Glu Asp Val Arg Ala Glu Ile Val Ala Ser Val Leu Glu Val
 1               5                  10                  15

Val Val Asn Glu Gly Asp Gln Ile Asp Lys Gly Asp Val Val Val Leu
             20                  25                  30

Leu Glu Ser Met Lys Met Glu Ile Pro Val Leu Ala Glu Ala Ala Gly
         35                  40                  45

Thr Val Ser Lys Val Ala Val Ser Val Gly Asp Val Ile Gln Ala Gly
         50                  55                  60

Asp Leu Ile Ala Val Ile Ser
 65                  70
```

(2) INFORMATION FOR SEQ ID NO: 49:

```
(i)  SEQUENCE CHARACTERISTICS:
     (A)  LENGTH: 750 base pairs
     (B)  TYPE: nucleic acid
     (C)  STRANDEDNESS: single
     (D)  TOPOLOGY: linear

(ix) FEATURE:

     (A)  NAME/KEY: Coding Sequence
     (B)  LOCATION: 113...640
     (D)  OTHER INFORMATION:

  (xi) SEQUENCE DESCRIPTION: SEQ ID NO: 49:

GGGTACCCAT CGATGGGTTG CGGTTCGGCA CCGAGGTGCT AACGCACTTG CTGACACACT      60

GCTAGTCGAA AACGAGGCTA GTCGCAACGT CGATCACACG AGAGGACTGA CC ATG ACA     118
                                                           Met Thr
                                                            1

ACT TCA CCC GAC CCG TAT GCC GCG CTG CCC AAG CTG CCG TCC TTC AGC      166
Thr Ser Pro Asp Pro Tyr Ala Ala Leu Pro Lys Leu Pro Ser Phe Ser
          5                   10                  15

CTG ACG TCA ACC TCG ATC ACC GAT GGG CAG CCG CTG GCT ACA CCC CAG      214
Leu Thr Ser Thr Ser Ile Thr Asp Gly Gln Pro Leu Ala Thr Pro Gln
         20                  25                  30

GTC AGC GGG ATC ATG GGT GCG GGC GGG GCG GAT GCC AGT CCG CAG CTG      262
Val Ser Gly Ile Met Gly Ala Gly Gly Ala Asp Ala Ser Pro Gln Leu
 35                  40                  45                  50

AGG TGG TCG GGA TTT CCC AGC GAG ACC CGC AGC TTC GCG GTA ACC GTC      310
Arg Trp Ser Gly Phe Pro Ser Glu Thr Arg Ser Phe Ala Val Thr Val
             55                  60                  65

TAC GAC CCT GAT GCC CCC ACC CTG TCC GGG TTC TGG CAC TGG GCG GTG      358
Tyr Asp Pro Asp Ala Pro Thr Leu Ser Gly Phe Trp His Trp Ala Val
             70                  75                  80

GCC AAC CTG CCT GCC AAC GTC ACC GAG TTG CCC GAG GGT GTC GGC GAT      406
Ala Asn Leu Pro Ala Asn Val Thr Glu Leu Pro Glu Gly Val Gly Asp
             85                  90                  95

GGC CGC GAA CTG CCG GGC GGG GCA CTG ACA TTG GTC AAC GAC GCC GGT      454
Gly Arg Glu Leu Pro Gly Gly Ala Leu Thr Leu Val Asn Asp Ala Gly
         100                 105                 110

ATG CGC CGG TAT GTG GGT GCG GCG CCG CCT CCC GGT CAT GGG GTG CAT      502
Met Arg Arg Tyr Val Gly Ala Ala Pro Pro Pro Gly His Gly Val His
115                 120                 125                 130

CGC TAC TAC GTC GCG GTA CAC GCG GTG AAG GTC GAA AAG CTC GAC CTC      550
Arg Tyr Tyr Val Ala Val His Ala Val Lys Val Glu Lys Leu Asp Leu
             135                 140                 145

CCC GAG GAC GCG AGT CCT GCA TAT CTG GGA TTC AAC CTG TTC CAG CAC      598
Pro Glu Asp Ala Ser Pro Ala Tyr Leu Gly Phe Asn Leu Phe Gln His
             150                 155                 160
```

```
GCG ATT GCA CGA GCG GTC ATC TTC GGC ACC TAC GAG CAG CGT TAGCGCTTT      649
Ala Ile Ala Arg Ala Val Ile Phe Gly Thr Tyr Glu Gln Arg
        165                 170                 175

AGCTGGGTTG CCGACGTCTT GCCGAGCCGA CCGCTTCGTG CAGCGAGCCG AACCCGCCGT      709

CATGCAGCCT GCGGGCAATG CCTTCATGGA TGTCCTTGGC C                          750
```

(2) INFORMATION FOR SEQ ID NO: 50:

    (i) SEQUENCE CHARACTERISTICS:
        (A) LENGTH: 176 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 50:

```
Met Thr Thr Ser Pro Asp Pro Tyr Ala Ala Leu Pro Lys Leu Pro Ser
 1               5                10              15

Phe Ser Leu Thr Ser Thr Ser Ile Thr Asp Gly Gln Pro Leu Ala Thr
            20              25              30

Pro Gln Val Ser Gly Ile Met Gly Ala Gly Gly Ala Asp Ala Ser Pro
        35              40              45

Gln Leu Arg Trp Ser Gly Phe Pro Ser Glu Thr Arg Ser Phe Ala Val
    50              55              60

Thr Val Tyr Asp Pro Asp Ala Pro Thr Leu Ser Gly Phe Trp His Trp
 65             70              75              80

Ala Val Ala Asn Leu Pro Ala Asn Val Thr Glu Leu Pro Glu Gly Val
            85              90              95

Gly Asp Gly Arg Glu Leu Pro Gly Gly Ala Leu Thr Leu Val Asn Asp
            100             105             110

Ala Gly Met Arg Arg Tyr Val Gly Ala Ala Pro Pro Pro Gly His Gly
            115             120             125

Val His Arg Tyr Tyr Val Ala Val His Ala Val Lys Val Glu Lys Leu
    130             135             140

Asp Leu Pro Glu Asp Ala Ser Pro Ala Tyr Leu Gly Phe Asn Leu Phe
145             150             155             160

Gln His Ala Ile Ala Arg Ala Val Ile Phe Gly Thr Tyr Glu Gln Arg
            165             170             175
```

(2) INFORMATION FOR SEQ ID NO: 51:

    (i) SEQUENCE CHARACTERISTICS:
        (A) LENGTH: 800 base pairs
        (B) TYPE: nucleic acid

(C) STRANDEDNESS: single
(D) TOPOLOGY: linear

(ix) FEATURE:

(A) NAME/KEY: Coding Sequence
(B) LOCATION: 18...695
(D) OTHER INFORMATION:

(A) NAME/KEY: Signal Sequence
(B) LOCATION: 18...134
(D) OTHER INFORMATION:

(xi) SEQUENCE DESCRIPTION: SEQ ID NO: 51:

```
TCATGAGGTT CATCGGG GTG ATC CCA CGC CCG CAG CCG CAT TCG GGC CGC         50
                    Met Ile Pro Arg Pro Gln Pro His Ser Gly Arg
                        -35                     -30

TGG CGA GCC GGT GCC GCA CGC CGC CTC ACC AGC CTG GTG GCC GCC GCC         98
Trp Arg Ala Gly Ala Ala Arg Arg Leu Thr Ser Leu Val Ala Ala Ala
        -25             -20                 -15

TTT GCG GCG GCC ACA CTG TTG CTT ACC CCC GCG CTG GCA CCA CCG GCA         146
Phe Ala Ala Ala Thr Leu Leu Leu Thr Pro Ala Leu Ala Pro Pro Ala
        -10             -5                  1                   5

TCG GCG GGC TGC CCG GAT GCC GAG GTG GTG TTC GCC CGC GGA ACC GGC         194
Ser Ala Gly Cys Pro Asp Ala Glu Val Val Phe Ala Arg Gly Thr Gly
                10                  15                  20

GAA CCA CCT GGC CTC GGT CGG GTA GGC CAA GCT TTC GTC AGT TCA TTG         242
Glu Pro Pro Gly Leu Gly Arg Val Gly Gln Ala Phe Val Ser Ser Leu
            25                  30                  35

CGC CAG CAG ACC AAC AAG AGC ATC GGG ACA TAC GGA GTC AAC TAC CCG         290
Arg Gln Gln Thr Asn Lys Ser Ile Gly Thr Tyr Gly Val Asn Tyr Pro
            40                  45                  50

GCC AAC GGT GAT TTC TTG GCC GCC GCT GAC GGC GCG AAC GAC GCC AGC         338
Ala Asn Gly Asp Phe Leu Ala Ala Ala Asp Gly Ala Asn Asp Ala Ser
        55                  60                  65

GAC CAC ATT CAG CAG ATG GCC AGC GCG TGC CGG GCC ACG AGG TTG GTG         386
Asp His Ile Gln Gln Met Ala Ser Ala Cys Arg Ala Thr Arg Leu Val
70                  75                  80                      85

CTC GGC GGC TAC TCC CAG GGT GCG GCC GTG ATC GAC ATC GTC ACC GCC         434
Leu Gly Gly Tyr Ser Gln Gly Ala Ala Val Ile Asp Ile Val Thr Ala
                90                  95                  100

GCA CCA CTG CCC GGC CTC GGG TTC ACG CAG CCG TTG CCG CCC GCA GCG         482
Ala Pro Leu Pro Gly Leu Gly Phe Thr Gln Pro Leu Pro Pro Ala Ala
            105                 110                 115

GAC GAT CAC ATC GCC GCG ATC GCC CTG TTC GGG AAT CCC TCG GGC CGC         530
Asp Asp His Ile Ala Ala Ile Ala Leu Phe Gly Asn Pro Ser Gly Arg
            120                 125                 130

GCT GGC GGG CTG ATG AGC GCC CTG ACC CCT CAA TTC GGG TCC AAG ACC         578
Ala Gly Gly Leu Met Ser Ala Leu Thr Pro Gln Phe Gly Ser Lys Thr
```

```
            135              140              145
ATC AAC CTC TGC AAC AAC GGC GAC CCG ATT TGT TCG GAC GGC AAC CGG    626
Ile Asn Leu Cys Asn Asn Gly Asp Pro Ile Cys Ser Asp Gly Asn Arg
150              155              160              165

TGG CGA GCG CAC CTA GGC TAC GTG CCC GGG ATG ACC AAC CAG GCG GCG    674
Trp Arg Ala His Leu Gly Tyr Val Pro Gly Met Thr Asn Gln Ala Ala
                170              175              180

CGT TTC GTC GCG AGC AGG ATC TAACGCGAGC CGCCCCATAG ATTCCGGCTA AGCA    729
Arg Phe Val Ala Ser Arg Ile
            185

ACGGCTGCGC CGCCGCCCGG CCACGAGTGA CCGCCGCCGA CTGGCACACC GCTTACCACG    789

GCCTTATGCT G                                                         800
```

(2) INFORMATION FOR SEQ ID NO: 52:

    (i) SEQUENCE CHARACTERISTICS:
        (A) LENGTH: 226 amino acids
        (B) TYPE: amino acid
        (C) STRANDEDNESS: single
        (D) TOPOLOGY: linear

    (ii) MOLECULE TYPE: protein
    (v) FRAGMENT TYPE: internal
    (ix) FEATURE:

        (A) NAME/KEY: Signal Sequence
        (B) LOCATION: 1...38
        (D) OTHER INFORMATION:

    (xi) SEQUENCE DESCRIPTION: SEQ ID NO: 52:

```
Met Ile Pro Arg Pro Gln Pro His Ser Gly Arg Trp Arg Ala Gly Ala
        -35              -30              -25

Ala Arg Arg Leu Thr Ser Leu Val Ala Ala Ala Phe Ala Ala Ala Thr
        -20              -15              -10

Leu Leu Leu Thr Pro Ala Leu Ala Pro Pro Ala Ser Ala Gly Cys Pro
        -5               1                5                        10

Asp Ala Glu Val Val Phe Ala Arg Gly Thr Gly Glu Pro Pro Gly Leu
                15              20              25

Gly Arg Val Gly Gln Ala Phe Val Ser Ser Leu Arg Gln Gln Thr Asn
                30              35              40

Lys Ser Ile Gly Thr Tyr Gly Val Asn Tyr Pro Ala Asn Gly Asp Phe
            45              50              55

Leu Ala Ala Ala Asp Gly Ala Asn Asp Ala Ser Asp His Ile Gln Gln
            60              65              70

Met Ala Ser Ala Cys Arg Ala Thr Arg Leu Val Leu Gly Gly Tyr Ser
    75              80              85              90
```

```
Gln Gly Ala Ala Val Ile Asp Ile Val Thr Ala Ala Pro Leu Pro Gly
            95                  100                 105

Leu Gly Phe Thr Gln Pro Leu Pro Pro Ala Ala Asp Asp His Ile Ala
            110             115                 120

Ala Ile Ala Leu Phe Gly Asn Pro Ser Gly Arg Ala Gly Gly Leu Met
        125             130             135

Ser Ala Leu Thr Pro Gln Phe Gly Ser Lys Thr Ile Asn Leu Cys Asn
    140             145             150

Asn Gly Asp Pro Ile Cys Ser Asp Gly Asn Arg Trp Arg Ala His Leu
155             160             165             170

Gly Tyr Val Pro Gly Met Thr Asn Gln Ala Ala Arg Phe Val Ala Ser
            175             180             185

Arg Ile
```

```
(2) INFORMATION FOR SEQ ID NO: 53:

    (i) SEQUENCE CHARACTERISTICS:
        (A) LENGTH: 700 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 73...615
        (D) OTHER INFORMATION:

    (xi) SEQUENCE DESCRIPTION: SEQ ID NO: 53:
```

```
CTAGGAAAGC CTTTCCTGAG TAAGTATTGC CTTCGTTGCA TACCGCCCTT TACCTGCGTT          60

AATCTGCATT TT ATG ACA GAA TAC GAA GGG CCT AAG ACA AAA TTC CAC GCG         111
              Met Thr Glu Tyr Glu Gly Pro Lys Thr Lys Phe His Ala
                1               5                   10

TTA ATG CAG GAA CAG ATT CAT AAC GAA TTC ACA GCG GCA CAA CAA TAT          159
Leu Met Gln Glu Gln Ile His Asn Glu Phe Thr Ala Ala Gln Gln Tyr
    15              20              25

GTC GCG ATC GCG GTT TAT TTC GAC AGC GAA GAC CTG CCG CAG TTG GCG          207
Val Ala Ile Ala Val Tyr Phe Asp Ser Glu Asp Leu Pro Gln Leu Ala
    30              35              40              45

AAG CAT TTT TAC AGC CAA GCG GTC GAG GAA CGA AAC CAT GCA ATG ATG          255
Lys His Phe Tyr Ser Gln Ala Val Glu Glu Arg Asn His Ala Met Met
                50              55              60

CTC GTG CAA CAC CTG CTC GAC CGC GAC CTT CGT GTC GAA ATT CCC GGC          303
Leu Val Gln His Leu Leu Asp Arg Asp Leu Arg Val Glu Ile Pro Gly
                65              70              75

GTA GAC ACG GTG CGA AAC CAG TTC GAC AGA CCC CGC GAG GCA CTG GCG          351
Val Asp Thr Val Arg Asn Gln Phe Asp Arg Pro Arg Glu Ala Leu Ala
```

```
              80                    85                    90
CTG GCG CTC GAT CAG GAA CGC ACA GTC ACC GAC CAG GTC GGT CGG CTG       399
Leu Ala Leu Asp Gln Glu Arg Thr Val Thr Asp Gln Val Gly Arg Leu
     95                  100                  105

ACA GCG GTG GCC CGC GAC GAG GGC GAT TTC CTC GGC GAG CAG TTC ATG       447
Thr Ala Val Ala Arg Asp Glu Gly Asp Phe Leu Gly Glu Gln Phe Met
110                  115                  120                  125

CAG TGG TTC TTG CAG GAA CAG ATC GAA GAG GTG GCC TTG ATG GCA ACC       495
Gln Trp Phe Leu Gln Glu Gln Ile Glu Glu Val Ala Leu Met Ala Thr
             130                  135                  140

CTG GTG CGG GTT GCC GAT CGG GCC GGG GCC AAC CTG TTC GAG CTA GAG       543
Leu Val Arg Val Ala Asp Arg Ala Gly Ala Asn Leu Phe Glu Leu Glu
                 145                  150                  155

AAC TTC GTC GCA CGT GAA GTG GAT GTG GCG CCG GCC GCA TCA GGC GCC       591
Asn Phe Val Ala Arg Glu Val Asp Val Ala Pro Ala Ala Ser Gly Ala
         160                  165                  170

CCG CAC GCT GCC GGG GGC CGC CTC TAGATCCCTG GCGGGGATCA GCGAGTGGTC      645
Pro His Ala Ala Gly Gly Arg Leu
     175                  180

CCGTTCGCCC GCCCGTCTTC CAGCCAGGCC TTGGTGCGGC CGGGGTGGTG AGTAC         700
```

(2) INFORMATION FOR SEQ ID NO: 54:

    (i) SEQUENCE CHARACTERISTICS:
        (A) LENGTH: 181 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 54:

```
Met Thr Glu Tyr Glu Gly Pro Lys Thr Lys Phe His Ala Leu Met Gln
  1               5                  10                  15

Glu Gln Ile His Asn Glu Phe Thr Ala Ala Gln Gln Tyr Val Ala Ile
             20                  25                  30

Ala Val Tyr Phe Asp Ser Glu Asp Leu Pro Gln Leu Ala Lys His Phe
         35                  40                  45

Tyr Ser Gln Ala Val Glu Glu Arg Asn His Ala Met Met Leu Val Gln
     50                  55                  60

His Leu Leu Asp Arg Asp Leu Arg Val Glu Ile Pro Gly Val Asp Thr
 65                  70                  75                  80

Val Arg Asn Gln Phe Asp Arg Pro Arg Glu Ala Leu Ala Leu Ala Leu
                 85                  90                  95

Asp Gln Glu Arg Thr Val Thr Asp Gln Val Gly Arg Leu Thr Ala Val
```

```
                 100                    105                     110
       Ala Arg Asp Glu Gly Asp Phe Leu Gly Glu Gln Phe Met Gln Trp Phe
               115                   120                   125

       Leu Gln Glu Gln Ile Glu Glu Val Ala Leu Met Ala Thr Leu Val Arg
           130                   135                   140

       Val Ala Asp Arg Ala Gly Ala Asn Leu Phe Glu Leu Glu Asn Phe Val
       145                   150                   155                   160

       Ala Arg Glu Val Asp Val Ala Pro Ala Ala Ser Gly Ala Pro His Ala
                       165                   170                   175

       Ala Gly Gly Arg Leu
                   180
```

(2)  INFORMATION FOR SEQ ID NO: 55:

       (i)  SEQUENCE CHARACTERISTICS:
               (A) LENGTH: 950 base pairs
               (B) TYPE: nucleic acid
               (C) STRANDEDNESS: single
               (D) TOPOLOGY: linear

       (ix)  FEATURE:

               (A) NAME/KEY: Coding Sequence
               (B) LOCATION: 133...918
               (D) OTHER INFORMATION:

               (A) NAME/KEY: Signal Sequence
               (B) LOCATION: 133...233
               (D) OTHER INFORMATION:

       (xi)  SEQUENCE DESCRIPTION: SEQ ID NO: 55:

```
       TGGGCTCGGC ACTGGCTCTC CCACGGTGGC GCGCTGATTT CTCCCCACGG TAGGCGTTGC        60

       GACGCATGTT CTTCACCGTC TATCCACAGC TACCGACATT TGCTCCGGCT GGATCGCGGG       120

       TAAAATTCCG TC GTG AAC AAT CGA CCC ATC CGC CTG CTG ACA TCC GGC AGG       171
                  Met Asn Asn Arg Pro Ile Arg Leu Leu Thr Ser Gly Arg
                            -30                   -25

       GCT GGT TTG GGT GCG GGC GCA TTG ATC ACC GCC GTC GTC CTG CTC ATC       219
       Ala Gly Leu Gly Ala Gly Ala Leu Ile Thr Ala Val Val Leu Leu Ile
       -20                   -15                   -10                   -5

       GCC TTG GGC GCT GTT TGG ACC CCG GTT GCC TTC GCC GAT GGA TGC CCG       267
       Ala Leu Gly Ala Val Trp Thr Pro Val Ala Phe Ala Asp Gly Cys Pro
                         1                   5                   10

       GAC GCC GAA GTC ACG TTC GCC CGC GGC ACC GGC GAG CCG CCC GGA ATC       315
       Asp Ala Glu Val Thr Phe Ala Arg Gly Thr Gly Glu Pro Pro Gly Ile
                15                   20                   25

       GGG CGC GTT GGC CAG GCG TTC GTC GAC TCG CTG CGC CAG CAG ACT GGC       363
       Gly Arg Val Gly Gln Ala Phe Val Asp Ser Leu Arg Gln Gln Thr Gly
           30                   35                   40
```

```
ATG GAG ATC GGA GTA TAC CCG GTG AAT TAC GCC GCC AGC CGC CTA CAG      411
Met Glu Ile Gly Val Tyr Pro Val Asn Tyr Ala Ala Ser Arg Leu Gln
    45              50              55              60

CTG CAC GGG GGA GAC GGC GCC AAC GAC GCC ATA TCG CAC ATT AAG TCC      459
Leu His Gly Gly Asp Gly Ala Asn Asp Ala Ile Ser His Ile Lys Ser
                65              70              75

ATG GCC TCG TCA TGC CCG AAC ACC AAG CTG GTC TTG GGC GGC TAT TCG      507
Met Ala Ser Ser Cys Pro Asn Thr Lys Leu Val Leu Gly Gly Tyr Ser
            80              85              90

CAG GGC GCA ACC GTG ATC GAT ATC GTG GCC GGG GTT CCG TTG GGC AGC      555
Gln Gly Ala Thr Val Ile Asp Ile Val Ala Gly Val Pro Leu Gly Ser
            95              100             105

ATC AGC TTT GGC AGT CCG CTA CCT GCG GCA TAC GCA GAC AAC GTC GCA      603
Ile Ser Phe Gly Ser Pro Leu Pro Ala Ala Tyr Ala Asp Asn Val Ala
    110             115             120

GCG GTC GCG GTC TTC GGC AAT CCG TCC AAC CGC GCC GGC GGA TCG CTG      651
Ala Val Ala Val Phe Gly Asn Pro Ser Asn Arg Ala Gly Gly Ser Leu
125             130             135             140

TCG AGC CTG AGC CCG CTA TTC GGT TCC AAG GCG ATT GAC CTG TGC AAT      699
Ser Ser Leu Ser Pro Leu Phe Gly Ser Lys Ala Ile Asp Leu Cys Asn
            145             150             155

CCC ACC GAT CCG ATC TGC CAT GTG GGC CCC GGC AAC GAA TTC AGC GGA      747
Pro Thr Asp Pro Ile Cys His Val Gly Pro Gly Asn Glu Phe Ser Gly
            160             165             170

CAC ATC GAC GGC TAC ATA CCC ACC TAC ACC ACC CAG GCG GCT AGT TTC      795
His Ile Asp Gly Tyr Ile Pro Thr Tyr Thr Thr Gln Ala Ala Ser Phe
            175             180             185

GTC GTG CAG AGG CTC CGC GCC GGG TCG GTG CCA CAT CTG CCT GGA TCC      843
Val Val Gln Arg Leu Arg Ala Gly Ser Val Pro His Leu Pro Gly Ser
    190             195             200

GTC CCG CAG CTG CCC GGG TCT GTC CTT CAG ATG CCC GGC ACT GCC GCA      891
Val Pro Gln Leu Pro Gly Ser Val Leu Gln Met Pro Gly Thr Ala Ala
205             210             215             220

CCG GCT CCC GAA TCG CTG CAC GGT CGC TGACGCTTTG TCAGTAAGCC CATAAAA    945
Pro Ala Pro Glu Ser Leu His Gly Arg
            225

TCGCG                                                               950
```

(2) INFORMATION FOR SEQ ID NO: 56:

    (i) SEQUENCE CHARACTERISTICS:
        (A) LENGTH: 262 amino acids
        (B) TYPE: amino acid
        (C) STRANDEDNESS: single
        (D) TOPOLOGY: linear

    (ii) MOLECULE TYPE: protein

(v) FRAGMENT TYPE: internal
(ix) FEATURE:

    (A) NAME/KEY: Signal Sequence
    (B) LOCATION: 1...33
    (D) OTHER INFORMATION:

(xi) SEQUENCE DESCRIPTION: SEQ ID NO: 57:

```
Met Asn Asn Arg Pro Ile Arg Leu Leu Thr Ser Gly Arg Ala Gly Leu
            -30             -25             -20

Gly Ala Gly Ala Leu Ile Thr Ala Val Val Leu Leu Ile Ala Leu Gly
        -15             -10             -5

Ala Val Trp Thr Pro Val Ala Phe Ala Asp Gly Cys Pro Asp Ala Glu
     1               5               10                  15

Val Thr Phe Ala Arg Gly Thr Gly Glu Pro Pro Gly Ile Gly Arg Val
            20              25                  30

Gly Gln Ala Phe Val Asp Ser Leu Arg Gln Gln Thr Gly Met Glu Ile
            35              40                  45

Gly Val Tyr Pro Val Asn Tyr Ala Ala Ser Arg Leu Gln Leu His Gly
        50              55                  60

Gly Asp Gly Ala Asn Asp Ala Ile Ser His Ile Lys Ser Met Ala Ser
    65              70                  75

Ser Cys Pro Asn Thr Lys Leu Val Leu Gly Gly Tyr Ser Gln Gly Ala
80                  85                  90                      95

Thr Val Ile Asp Ile Val Ala Gly Val Pro Leu Gly Ser Ile Ser Phe
                100             105                 110

Gly Ser Pro Leu Pro Ala Ala Tyr Ala Asp Asn Val Ala Ala Val Ala
            115             120             125

Val Phe Gly Asn Pro Ser Asn Arg Ala Gly Gly Ser Leu Ser Ser Leu
        130             135             140

Ser Pro Leu Phe Gly Ser Lys Ala Ile Asp Leu Cys Asn Pro Thr Asp
    145             150             155

Pro Ile Cys His Val Gly Pro Gly Asn Glu Phe Ser Gly His Ile Asp
160                 165             170                 175

Gly Tyr Ile Pro Thr Tyr Thr Thr Gln Ala Ala Ser Phe Val Val Gln
            180             185             190

Arg Leu Arg Ala Gly Ser Val Pro His Leu Pro Gly Ser Val Pro Gln
        195             200             205

Leu Pro Gly Ser Val Leu Gln Met Pro Gly Thr Ala Ala Pro Ala Pro
        210             215             220

Glu Ser Leu His Gly Arg
    225
```

(2) INFORMATION FOR SEQ ID NO: 57:

    (i) SEQUENCE CHARACTERISTICS:
        (A) LENGTH: 1000 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 94...966
        (D) OTHER INFORMATION:

        (A) NAME/KEY: Signal Sequence
        (B) LOCATION: 94...264
        (D) OTHER INFORMATION:

    (xi) SEQUENCE DESCRIPTION: SEQ ID NO: 57:

```
CGAGGAGACC GACGATCTGC TCGACGAAAT CGACGACGTC CTCGAGGAGA ACGCCGAGGA        60

CTTCGTCCGC GCATACGTCC AAAAGGGCGG ACA GTG ACC TGG CCG TTG CCC GAT       114
                                  Met Thr Trp Pro Leu Pro Asp
                                  -55                      -50

CGC CTG TCC ATT AAT TCA CTC TCT GGA ACA CCC GCT GTA GAC CTA TCT       162
Arg Leu Ser Ile Asn Ser Leu Ser Gly Thr Pro Ala Val Asp Leu Ser
            -45              -40                  -35

TCT TTC ACT GAC TTC CTG CGC CGC CAG GCG CCG GAG TTG CTG CCG GCA       210
Ser Phe Thr Asp Phe Leu Arg Arg Gln Ala Pro Glu Leu Leu Pro Ala
        -30              -25                  -20

AGC ATC AGC GGC GGT GCG CCA CTC GCA GGC GGC GAT GCG CAA CTG CCG       258
Ser Ile Ser Gly Gly Ala Pro Leu Ala Gly Gly Asp Ala Gln Leu Pro
        -15              -10                  -5

CAC GGC ACC ACC ATT GTC GCG CTG AAA TAC CCC GGC GGT GTT GTC ATG       306
His Gly Thr Thr Ile Val Ala Leu Lys Tyr Pro Gly Gly Val Val Met
     1               5               10                      15

GCG GGT GAC CGG CGT TCG ACG CAG GGC AAC ATG ATT TCT GGG CGT GAT       354
Ala Gly Asp Arg Arg Ser Thr Gln Gly Asn Met Ile Ser Gly Arg Asp
             20                  25                      30

GTG CGC AAG GTG TAT ATC ACC GAT GAC TAC ACC GCT ACC GGC ATC GCT       402
Val Arg Lys Val Tyr Ile Thr Asp Asp Tyr Thr Ala Thr Gly Ile Ala
         35                  40                  45

GGC ACG GCT GCG GTC GCG GTT GAG TTT GCC CGG CTG TAT GCC GTG GAA       450
Gly Thr Ala Ala Val Ala Val Glu Phe Ala Arg Leu Tyr Ala Val Glu
         50                  55                  60

CTT GAG CAC TAC GAG AAG CTC GAG GGT GTG CCG CTG ACG TTT GCC GGC       498
Leu Glu His Tyr Glu Lys Leu Glu Gly Val Pro Leu Thr Phe Ala Gly
     65                  70                  75

AAA ATC AAC CGG CTG GCG ATT ATG GTG CGT GGC AAT CTG GCG GCC GCG       546
Lys Ile Asn Arg Leu Ala Ile Met Val Arg Gly Asn Leu Ala Ala Ala
 80                  85                  90                      95
```

```
ATG CAG GGT CTG CTG GCG TTG CCG TTG CTG GCG GGC TAC GAC ATT CAT      594
Met Gln Gly Leu Leu Ala Leu Pro Leu Leu Ala Gly Tyr Asp Ile His
            100                 105                 110

GCG TCT GAC CCG CAG AGC GCG GGT CGT ATC GTT TCG TTC GAC GCC GCC      642
Ala Ser Asp Pro Gln Ser Ala Gly Arg Ile Val Ser Phe Asp Ala Ala
            115                 120                 125

GGC GGT TGG AAC ATC GAG GAA GAG GGC TAT CAG GCG GTG GGC TCG GGT      690
Gly Gly Trp Asn Ile Glu Glu Glu Gly Tyr Gln Ala Val Gly Ser Gly
            130                 135                 140

TCG CTG TTC GCG AAG TCG TCG ATG AAG AAG TTG TAT TCG CAG GTT ACC      738
Ser Leu Phe Ala Lys Ser Ser Met Lys Lys Leu Tyr Ser Gln Val Thr
        145                 150                 155

GAC GGT GAT TCG GGG CTG CGG GTG GCG GTC GAG GCG CTC TAC GAC GCC      786
Asp Gly Asp Ser Gly Leu Arg Val Ala Val Glu Ala Leu Tyr Asp Ala
160                 165                 170                 175

GCC GAC GAC GAC TCC GCC ACC GGC GGT CCG GAC CTG GTG CGG GGC ATC      834
Ala Asp Asp Asp Ser Ala Thr Gly Gly Pro Asp Leu Val Arg Gly Ile
            180                 185                 190

TTT CCG ACG GCG GTG ATC ATC GAC GCC GAC GGG GCG GTT GAC GTG CCG      882
Phe Pro Thr Ala Val Ile Ile Asp Ala Asp Gly Ala Val Asp Val Pro
            195                 200                 205

GAG AGC CGG ATT GCC GAA TTG GCC CGC GCG ATC ATC GAA AGC CGT TCG      930
Glu Ser Arg Ile Ala Glu Leu Ala Arg Ala Ile Ile Glu Ser Arg Ser
            210                 215                 220

GGT GCG GAT ACT TTC GGC TCC GAT GGC GGT GAG AAG TGAGTTTTCC GTATTT    982
Gly Ala Asp Thr Phe Gly Ser Asp Gly Gly Glu Lys
        225                 230                 235

CATCTCGCCT GAGCAGGC                                                  1000
```

(2) INFORMATION FOR SEQ ID NO: 58:

    (i) SEQUENCE CHARACTERISTICS:
        (A) LENGTH: 291 amino acids
        (B) TYPE: amino acid
        (C) STRANDEDNESS: single
        (D) TOPOLOGY: linear

    (ii) MOLECULE TYPE: protein
    (v) FRAGMENT TYPE: internal
    (ix) FEATURE:

        (A) NAME/KEY: Signal Sequence
        (B) LOCATION: 1...56
        (D) OTHER INFORMATION:

    (xi) SEQUENCE DESCRIPTION: SEQ ID NO: 58:

```
Met Thr Trp Pro Leu Pro Asp Arg Leu Ser Ile Asn Ser Leu Ser Gly
    -55                 -50                 -45
```

```
Thr Pro Ala Val Asp Leu Ser Ser Phe Thr Asp Phe Leu Arg Arg Gln
-40               -35               -30               -25

Ala Pro Glu Leu Leu Pro Ala Ser Ile Ser Gly Gly Ala Pro Leu Ala
              -20               -15               -10

Gly Gly Asp Ala Gln Leu Pro His Gly Thr Thr Ile Val Ala Leu Lys
          -5                    1                 5

Tyr Pro Gly Gly Val Val Met Ala Gly Asp Arg Arg Ser Thr Gln Gly
    10              15              20

Asn Met Ile Ser Gly Arg Asp Val Arg Lys Val Tyr Ile Thr Asp Asp
25              30              35                          40

Tyr Thr Ala Thr Gly Ile Ala Gly Thr Ala Ala Val Ala Val Glu Phe
            45              50              55

Ala Arg Leu Tyr Ala Val Glu Leu Glu His Tyr Glu Lys Leu Glu Gly
        60              65              70

Val Pro Leu Thr Phe Ala Gly Lys Ile Asn Arg Leu Ala Ile Met Val
        75              80              85

Arg Gly Asn Leu Ala Ala Ala Met Gln Gly Leu Leu Ala Leu Pro Leu
    90              95              100

Leu Ala Gly Tyr Asp Ile His Ala Ser Asp Pro Gln Ser Ala Gly Arg
105             110             115                         120

Ile Val Ser Phe Asp Ala Ala Gly Gly Trp Asn Ile Glu Glu Glu Gly
            125             130             135

Tyr Gln Ala Val Gly Ser Gly Ser Leu Phe Ala Lys Ser Ser Met Lys
            140             145             150

Lys Leu Tyr Ser Gln Val Thr Asp Gly Asp Ser Gly Leu Arg Val Ala
        155             160             165

Val Glu Ala Leu Tyr Asp Ala Ala Asp Asp Asp Ser Ala Thr Gly Gly
    170             175             180

Pro Asp Leu Val Arg Gly Ile Phe Pro Thr Ala Val Ile Ile Asp Ala
185             190             195                         200

Asp Gly Ala Val Asp Val Pro Glu Ser Arg Ile Ala Glu Leu Ala Arg
            205             210             215

Ala Ile Ile Glu Ser Arg Ser Gly Ala Asp Thr Phe Gly Ser Asp Gly
            220             225             230

Gly Glu Lys
    235
```

(2) INFORMATION FOR SEQ ID NO: 59:

   (i) SEQUENCE CHARACTERISTICS:
       (A) LENGTH: 900 base pairs
       (B) TYPE: nucleic acid
       (C) STRANDEDNESS: single

(D) TOPOLOGY: linear

(ix) FEATURE:

    (A) NAME/KEY: Coding Sequence
    (B) LOCATION: 66...808
    (D) OTHER INFORMATION:

(xi) SEQUENCE DESCRIPTION: SEQ ID NO: 59:

```
TTGGCCCGCG CGATCATCGA AAGCCGTTCG GGTGCGGATA CTTTCGGCTC CGATGGCGGT        60
GAGAA GTG AGT TTT CCG TAT TTC ATC TCG CCT GAG CAG GCG ATG CGC GAG       110
      Met Ser Phe Pro Tyr Phe Ile Ser Pro Glu Gln Ala Met Arg Glu
       1           5                   10                  15

CGC AGC GAG TTG GCG CGT AAG GGC ATT GCG CGG GCC AAA AGC GTG GTG        158
Arg Ser Glu Leu Ala Arg Lys Gly Ile Ala Arg Ala Lys Ser Val Val
                20                  25                  30

GCG CTG GCC TAT GCC GGT GGT GTG CTG TTC GTC GCG GAG AAT CCG TCG        206
Ala Leu Ala Tyr Ala Gly Gly Val Leu Phe Val Ala Glu Asn Pro Ser
                35                  40                  45

CGG TCG CTG CAG AAG ATC AGT GAG CTC TAC GAT CGG GTG GGT TTT GCG        254
Arg Ser Leu Gln Lys Ile Ser Glu Leu Tyr Asp Arg Val Gly Phe Ala
            50                  55                  60

GCT GCG GGC AAG TTC AAC GAG TTC GAC AAT TTG CGC CGC GGC GGG ATC        302
Ala Ala Gly Lys Phe Asn Glu Phe Asp Asn Leu Arg Arg Gly Gly Ile
    65                  70                  75

CAG TTC GCC GAC ACC CGC GGT TAC GCC TAT GAC CGT CGT GAC GTC ACG        350
Gln Phe Ala Asp Thr Arg Gly Tyr Ala Tyr Asp Arg Arg Asp Val Thr
80                  85                  90                  95

GGT CGG CAG TTG GCC AAT GTC TAC GCG CAG ACT CTA GGC ACC ATC TTC        398
Gly Arg Gln Leu Ala Asn Val Tyr Ala Gln Thr Leu Gly Thr Ile Phe
                100                 105                 110

ACC GAA CAG GCC AAG CCC TAC GAG GTT GAG TTG TGT GTG GCC GAG GTG        446
Thr Glu Gln Ala Lys Pro Tyr Glu Val Glu Leu Cys Val Ala Glu Val
                115                 120                 125

GCG CAT TAC GGC GAG ACG AAA CGC CCT GAG TTG TAT CGT ATT ACC TAC        494
Ala His Tyr Gly Glu Thr Lys Arg Pro Glu Leu Tyr Arg Ile Thr Tyr
            130                 135                 140

GAC GGG TCG ATC GCC GAC GAG CCG CAT TTC GTG GTG ATG GGC GGC ACC        542
Asp Gly Ser Ile Ala Asp Glu Pro His Phe Val Val Met Gly Gly Thr
            145                 150                 155

ACG GAG CCG ATC GCC AAC GCG CTC AAA GAG TCG TAT GCC GAG AAC GCC        590
Thr Glu Pro Ile Ala Asn Ala Leu Lys Glu Ser Tyr Ala Glu Asn Ala
160                 165                 170                 175

AGC CTG ACC GAC GCC CTG CGT ATC GCG GTC GCT GCA TTG CGG GCC GGC        638
Ser Leu Thr Asp Ala Leu Arg Ile Ala Val Ala Ala Leu Arg Ala Gly
                180                 185                 190

AGT GCC GAC ACC TCG GGT GGT GAT CAA CCC ACC CTT GGC GTG GCC AGC        686
Ser Ala Asp Thr Ser Gly Gly Asp Gln Pro Thr Leu Gly Val Ala Ser
```

```
                195                      200                      205

        TTA GAG GTG GCC GTT CTC GAT GCC AAC CGG CCA CGG CGC GCG TTC CGG        734
        Leu Glu Val Ala Val Leu Asp Ala Asn Arg Pro Arg Arg Ala Phe Arg
            210                     215                 220

        CGC ATC ACC GGC TCC GCC CTG CAA GCG TTG CTG GTA GAC CAG GAA AGC        782
        Arg Ile Thr Gly Ser Ala Leu Gln Ala Leu Leu Val Asp Gln Glu Ser
            225                     230                 235

        CCG CAG TCT GAC GGC GAA TCG TCG GG CTGAGTCCGA AAGTCCGACG CGTGTCTG      836
        Pro Gln Ser Asp Gly Glu Ser Ser Gly
        240                     245

        GGACCCCGCT GCGACGTTAA CTGCGCCTAA CCCCGGCTCG ACGCGTCGCC GGCCGTCCTG      896

        ACTT                                                                   900
```

(2) INFORMATION FOR SEQ ID NO: 60:

    (i) SEQUENCE CHARACTERISTICS:
        (A) LENGTH: 248 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 60:

```
Met Ser Phe Pro Tyr Phe Ile Ser Pro Glu Gln Ala Met Arg Glu Arg
  1               5                  10                  15

Ser Glu Leu Ala Arg Lys Gly Ile Ala Arg Ala Lys Ser Val Val Ala
             20                  25                  30

Leu Ala Tyr Ala Gly Gly Val Leu Phe Val Ala Glu Asn Pro Ser Arg
             35                  40                  45

Ser Leu Gln Lys Ile Ser Glu Leu Tyr Asp Arg Val Gly Phe Ala Ala
        50                  55                  60

Ala Gly Lys Phe Asn Glu Phe Asp Asn Leu Arg Arg Gly Gly Ile Gln
 65                  70                  75                  80

Phe Ala Asp Thr Arg Gly Tyr Ala Tyr Asp Arg Arg Asp Val Thr Gly
                 85                  90                  95

Arg Gln Leu Ala Asn Val Tyr Ala Gln Thr Leu Gly Thr Ile Phe Thr
            100                 105                 110

Glu Gln Ala Lys Pro Tyr Glu Val Glu Leu Cys Val Ala Glu Val Ala
            115                 120                 125

His Tyr Gly Glu Thr Lys Arg Pro Glu Leu Tyr Arg Ile Thr Tyr Asp
            130                 135                 140

Gly Ser Ile Ala Asp Glu Pro His Phe Val Val Met Gly Gly Thr Thr
145                 150                 155                 160
```

Glu Pro Ile Ala Asn Ala Leu Lys Glu Ser Tyr Ala Glu Asn Ala Ser
165 170 175

Leu Thr Asp Ala Leu Arg Ile Ala Val Ala Ala Leu Arg Ala Gly Ser
180 185 190

Ala Asp Thr Ser Gly Gly Asp Gln Pro Thr Leu Gly Val Ala Ser Leu
195 200 205

Glu Val Ala Val Leu Asp Ala Asn Arg Pro Arg Arg Ala Phe Arg Arg
210 215 220

Ile Thr Gly Ser Ala Leu Gln Ala Leu Leu Val Asp Gln Glu Ser Pro
225 230 235 240

Gln Ser Asp Gly Glu Ser Ser Gly
245


(2) INFORMATION FOR SEQ ID NO:61:

    (i) SEQUENCE CHARACTERISTICS:
      (A) LENGTH: 1560 base pairs
      (B) TYPE: nucleic acid
      (C) STRANDEDNESS: single
      (D) TOPOLOGY: linear

    (ix) FEATURE:

      (A) NAME/KEY: Coding Sequence
      (B) LOCATION: 98...1487
      (D) OTHER INFORMATION:

    (xi) SEQUENCE DESCRIPTION: SEQ ID NO: 61:

```
GAGTCATTGC CTGGTCGGCG TCATTCCGTA CTAGTCGGTT GTCGGACTTG ACCTACTGGG    60
TCAGGCCGAC GAGCACTCGA CCATTAGGGT AGGGGCC GTG ACC CAC TAT GAC GTC   115
                                        Met Thr His Tyr Asp Val
                                         1                   5

GTC GTT CTC GGA GCC GGT CCC GGC GGG TAT GTC GCG GCG ATT CGC GCC   163
Val Val Leu Gly Ala Gly Pro Gly Gly Tyr Val Ala Ala Ile Arg Ala
             10              15              20

GCA CAG CTC GGC CTG AGC ACT GCA ATC GTC GAA CCC AAG TAC TGG GGC   211
Ala Gln Leu Gly Leu Ser Thr Ala Ile Val Glu Pro Lys Tyr Trp Gly
        25              30              35

GGA GTA TGC CTC AAT GTC GGC TGT ATC CCA TCC AAG GCG CTG TTG CGC   259
Gly Val Cys Leu Asn Val Gly Cys Ile Pro Ser Lys Ala Leu Leu Arg
    40              45              50

AAC GCC GAA CTG GTC CAC ATC TTC ACC AAG GAC GCC AAA GCA TTT GGC   307
Asn Ala Glu Leu Val His Ile Phe Thr Lys Asp Ala Lys Ala Phe Gly
55              60              65              70

ATC AGC GGC GAG GTG ACC TTC GAC TAC GGC ATC GCC TAT GAC CGC AGC   355
Ile Ser Gly Glu Val Thr Phe Asp Tyr Gly Ile Ala Tyr Asp Arg Ser
            75              80              85
```

113

```
CGA AAG GTA GCC GAG GGC AGG GTG GCC GGT GTG CAC TTC CTG ATG AAG    403
Arg Lys Val Ala Glu Gly Arg Val Ala Gly Val His Phe Leu Met Lys
         90              95                  100

AAG AAC AAG ATC ACC GAG ATC CAC GGG TAC GGC ACA TTT GCC GAC GCC    451
Lys Asn Lys Ile Thr Glu Ile His Gly Tyr Gly Thr Phe Ala Asp Ala
        105             110                 115

AAC ACG TTG TTG GTT GAT CTC AAC GAC GGC GGT ACA GAA TCG GTC ACG    499
Asn Thr Leu Leu Val Asp Leu Asn Asp Gly Gly Thr Glu Ser Val Thr
        120             125                 130

TTC GAC AAC GCC ATC ATC GCG ACC GGC AGT AGC ACC CGG CTG GTT CCC    547
Phe Asp Asn Ala Ile Ile Ala Thr Gly Ser Ser Thr Arg Leu Val Pro
135             140             145                 150

GGC ACC TCA CTG TCG GCC AAC GTA GTC ACC TAC GAG GAA CAG ATC CTG    595
Gly Thr Ser Leu Ser Ala Asn Val Val Thr Tyr Glu Glu Gln Ile Leu
            155             160                 165

TCC CGA GAG CTG CCG AAA TCG ATC ATT ATT GCC GGA GCT GGT GCC ATT    643
Ser Arg Glu Leu Pro Lys Ser Ile Ile Ile Ala Gly Ala Gly Ala Ile
            170             175                 180

GGC ATG GAG TTC GGC TAC GTG CTG AAG AAC TAC GGC GTT GAC GTG ACC    691
Gly Met Glu Phe Gly Tyr Val Leu Lys Asn Tyr Gly Val Asp Val Thr
            185             190                 195

ATC GTG GAA TTC CTT CCG CGG GCG CTG CCC AAC GAG GAC GCC GAT GTG    739
Ile Val Glu Phe Leu Pro Arg Ala Leu Pro Asn Glu Asp Ala Asp Val
        200             205                 210

TCC AAG GAG ATC GAG AAG CAG TTC AAA AAG CTG GGT GTC ACG ATC CTG    787
Ser Lys Glu Ile Glu Lys Gln Phe Lys Lys Leu Gly Val Thr Ile Leu
215             220             225                 230

ACC GCC ACG AAG GTC GAG TCC ATC GCC GAT GGC GGG TCG CAG GTC ACC    835
Thr Ala Thr Lys Val Glu Ser Ile Ala Asp Gly Gly Ser Gln Val Thr
            235             240                 245

GTG ACC GTC ACC AAG GAC GGC GTG GCG CAA GAG CTT AAG GCG GAA AAG    883
Val Thr Val Thr Lys Asp Gly Val Ala Gln Glu Leu Lys Ala Glu Lys
            250             255                 260

GTG TTG CAG GCC ATC GGA TTT GCG CCC AAC GTC GAA GGG TAC GGG CTG    931
Val Leu Gln Ala Ile Gly Phe Ala Pro Asn Val Glu Gly Tyr Gly Leu
            265             270                 275

GAC AAG GCA GGC GTC GCG CTG ACC GAC CGC AAG GCT ATC GGT GTC GAC    979
Asp Lys Ala Gly Val Ala Leu Thr Asp Arg Lys Ala Ile Gly Val Asp
        280             285                 290

GAC TAC ATG CGT ACC AAC GTG GGC CAC ATC TAC GCT ATC GGC GAT GTC    1027
Asp Tyr Met Arg Thr Asn Val Gly His Ile Tyr Ala Ile Gly Asp Val
295             300             305                 310

AAT GGA TTA CTG CAG CTG GCG CAC GTC GCC GAG GCA CAA GGC GTG GTA    1075
Asn Gly Leu Leu Gln Leu Ala His Val Ala Glu Ala Gln Gly Val Val
            315             320                 325

GCC GCC GAA ACC ATT GCC GGT GCA GAG ACT TTG ACG CTG GGC GAC CAT    1123
```

```
                Ala Ala Glu Thr Ile Ala Gly Ala Glu Thr Leu Thr Leu Gly Asp His
                        330                 335                 340

CGG ATG TTG CCG CGC GCG ACG TTC TGT CAG CCA AAC GTT GCC AGC TTC   1171
Arg Met Leu Pro Arg Ala Thr Phe Cys Gln Pro Asn Val Ala Ser Phe
        345                 350                 355

GGG CTC ACC GAG CAG CAA GCC CGC AAC GAA GGT TAC GAC GTG GTG GTG   1219
Gly Leu Thr Glu Gln Gln Ala Arg Asn Glu Gly Tyr Asp Val Val Val
        360                 365                 370

GCC AAG TTC CCG TTC ACG GCC AAC GCC AAG GCG CAC GGC GTG GGT GAC   1267
Ala Lys Phe Pro Phe Thr Ala Asn Ala Lys Ala His Gly Val Gly Asp
375                 380                 385                 390

CCC AGT GGG TTC GTC AAG CTG GTG GCC GAC GCC AAG CAC GGC GAG CTA   1315
Pro Ser Gly Phe Val Lys Leu Val Ala Asp Ala Lys His Gly Glu Leu
            395                 400                 405

CTG GGT GGG CAC CTG GTC GGC CAC GAC GTG GCC GAG CTG CTG CCG GAG   1363
Leu Gly Gly His Leu Val Gly His Asp Val Ala Glu Leu Leu Pro Glu
            410                 415                 420

CTC ACG CTG GCG CAG AGG TGG GAC CTG ACC GCC AGC GAG CTG GCT CGC   1411
Leu Thr Leu Ala Gln Arg Trp Asp Leu Thr Ala Ser Glu Leu Ala Arg
        425                 430                 435

AAC GTC CAC ACC CAC CCA ACG ATG TCT GAG GCG CTG CAG GAG TGC TTC   1459
Asn Val His Thr His Pro Thr Met Ser Glu Ala Leu Gln Glu Cys Phe
        440                 445                 450

CAC GGC CTG GTT GGC CAC ATG ATC AAT T TCTGAGCGGC TCATGACGAG GCGCG   1512
His Gly Leu Val Gly His Met Ile Asn Phe
455                 460

CGAGCACTGA CACCCCCCAG ATCATCATGG GTGCCATCGG TGGTGTGG             1560
```

                    (2) INFORMATION FOR SEQ ID NO: 62:

                    (i) SEQUENCE CHARACTERISTICS:
                        (A) LENGTH: 464 amino acids
                        (B) TYPE: amino acid
                        (C) STRANDEDNESS: single
                        (D) TOPOLOGY: linear

                    (ii) MOLECULE TYPE: protein
                    (v) FRAGMENT TYPE: internal

                    (xi) SEQUENCE DESCRIPTION: SEQ ID NO: 62:

```
Met Thr His Tyr Asp Val Val Val Leu Gly Ala Gly Pro Gly Gly Tyr
1               5                   10                  15
Val Ala Ala Ile Arg Ala Ala Gln Leu Gly Leu Ser Thr Ala Ile Val
                20                  25                  30
Glu Pro Lys Tyr Trp Gly Gly Val Cys Leu Asn Val Gly Cys Ile Pro
            35                  40                  45
Ser Lys Ala Leu Leu Arg Asn Ala Glu Leu Val His Ile Phe Thr Lys
        50                  55                  60
Asp Ala Lys Ala Phe Gly Ile Ser Gly Glu Val Thr Phe Asp Tyr Gly
65                  70                  75                  80
Ile Ala Tyr Asp Arg Ser Arg Lys Val Ala Glu Gly Arg Val Ala Gly
```

```
                        85          .                    90                              95
        Val His Phe Leu Met Lys Lys Asn Lys Ile Thr Glu Ile His Gly Tyr
                        100                             105                 110
        Gly Thr Phe Ala Asp Ala Asn Thr Leu Leu Val Asp Leu Asn Asp Gly
                        115                             120                 125
        Gly Thr Glu Ser Val Thr Phe Asp Asn Ala Ile Ile Ala Thr Gly Ser
                    130                             135                 140
        Ser Thr Arg Leu Val Pro Gly Thr Ser Leu Ser Ala Asn Val Val Thr
        145                             150                 155                     160
        Tyr Glu Glu Gln Ile Leu Ser Arg Glu Leu Pro Lys Ser Ile Ile Ile
                            165                             170                 175
        Ala Gly Ala Gly Ala Ile Gly Met Glu Phe Gly Tyr Val Leu Lys Asn
                        180                             185                 190
        Tyr Gly Val Asp Val Thr Ile Val Glu Phe Leu Pro Arg Ala Leu Pro
                        195                             200                 205
        Asn Glu Asp Ala Asp Val Ser Lys Glu Ile Glu Lys Gln Phe Lys Lys
                    210                             215                 220
        Leu Gly Val Thr Ile Leu Thr Ala Thr Lys Val Glu Ser Ile Ala Asp
        225                             230                 235                     240
        Gly Gly Ser Gln Val Thr Val Thr Val Thr Lys Asp Gly Val Ala Gln
                        245                             250                 255
        Glu Leu Lys Ala Glu Lys Val Leu Gln Ala Ile Gly Phe Ala Pro Asn
                        260                             265                 270
        Val Glu Gly Tyr Gly Leu Asp Lys Ala Gly Val Ala Leu Thr Asp Arg
                    275                             280                 285
        Lys Ala Ile Gly Val Asp Asp Tyr Met Arg Thr Asn Val Gly His Ile
                    290                             295                 300
        Tyr Ala Ile Gly Asp Val Asn Gly Leu Leu Gln Leu Ala His Val Ala
        305                             310                 315                     320
        Glu Ala Gln Gly Val Val Ala Ala Glu Thr Ile Ala Gly Ala Glu Thr
                        325                             330                 335
        Leu Thr Leu Gly Asp His Arg Met Leu Pro Arg Ala Thr Phe Cys Gln
                        340                             345                 350
        Pro Asn Val Ala Ser Phe Gly Leu Thr Glu Gln Gln Ala Arg Asn Glu
                    355                             360                 365
        Gly Tyr Asp Val Val Val Ala Lys Phe Pro Phe Thr Ala Asn Ala Lys
                    370                             375                 380
        Ala His Gly Val Gly Asp Pro Ser Gly Phe Val Lys Leu Val Ala Asp
        385                             390                 395                     400
        Ala Lys His Gly Glu Leu Leu Gly Gly His Leu Val Gly His Asp Val
                        405                             410                 415
        Ala Glu Leu Leu Pro Glu Leu Thr Leu Ala Gln Arg Trp Asp Leu Thr
                        420                             425                 430
        Ala Ser Glu Leu Ala Arg Asn Val His Thr His Pro Thr Met Ser Glu
                        435                             440                 445
        Ala Leu Gln Glu Cys Phe His Gly Leu Val Gly His Met Ile Asn Phe
            450                             455                 460
```

(2) INFORMATION FOR SEQ ID NO: 63:

    (i) SEQUENCE CHARACTERISTICS:
        (A) LENGTH: 550 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 101...490
        (D) OTHER INFORMATION:

(xi) SEQUENCE DESCRIPTION: SEQ ID NO: 63:

```
GGCCCGGCTC GCGGCCGCCC TGCAGGAAAA GAAGGCCTGC CCAGGCCCAG ACTCAGCCGA          60

GTAGTCACCC AGTACCCCAC ACCAGGAAGG ACCGCCCATC  ATG GCA AAG CTC TCC          115
                                             Met Ala Lys Leu Ser
                                              1                5

ACC GAC GAA CTG CTG GAC GCG TTC AAG GAA ATG ACC CTG TTG GAG CTC          163
Thr Asp Glu Leu Leu Asp Ala Phe Lys Glu Met Thr Leu Leu Glu Leu
                 10                  15                  20

TCC GAC TTC GTC AAG AAG TTC GAG GAG ACC TTC GAG GTC ACC GCC GCC          211
Ser Asp Phe Val Lys Lys Phe Glu Glu Thr Phe Glu Val Thr Ala Ala
             25                  30                  35

GCT CCA GTC GCC GTC GCC GCC GCC GGT GCC GCC CCG GCC GGT GCC GCC          259
Ala Pro Val Ala Val Ala Ala Ala Gly Ala Ala Pro Ala Gly Ala Ala
         40                  45                  50

GTC GAG GCT GCC GAG GAG CAG TCC GAG TTC GAC GTG ATC CTT GAG GCC          307
Val Glu Ala Ala Glu Glu Gln Ser Glu Phe Asp Val Ile Leu Glu Ala
     55                  60                  65

GCC GGC GAC AAG AAG ATC GGC GTC ATC AAG GTG GTC CGG GAG ATC GTT          355
Ala Gly Asp Lys Lys Ile Gly Val Ile Lys Val Val Arg Glu Ile Val
 70                  75                  80                  85

TCC GGC CTG GGC CTC AAG GAG GCC AAG GAC CTG GTC GAC GGC GCG CCC          403
Ser Gly Leu Gly Leu Lys Glu Ala Lys Asp Leu Val Asp Gly Ala Pro
                 90                  95                 100

AAG CCG CTG CTG GAG AAG GTC GCC AAG GAG GCC GCC GAC GAG GCC AAG          451
Lys Pro Leu Leu Glu Lys Val Ala Lys Glu Ala Ala Asp Glu Ala Lys
             105                 110                 115

GCC AAG CTG GAG GCC GCC GGC GCC ACC GTC ACC GTC AAG TAGCTCTGCC CA        502
Ala Lys Leu Glu Ala Ala Gly Ala Thr Val Thr Val Lys
         120                 125                 130

GCGTGTTCTT TTGCGTCTGC TCGGCCCGTA GCGAACACTG CGCCCGCT                     550
```

(2) INFORMATION FOR SEQ ID NO: 64:

      (i) SEQUENCE CHARACTERISTICS:
          (A) LENGTH: 130 amino acids
          (B) TYPE: amino acid
          (C) STRANDEDNESS: single
          (D) TOPOLOGY: linear

      (ii) MOLECULE TYPE: protein
      (v) FRAGMENT TYPE: internal

      (xi) SEQUENCE DESCRIPTION: SEQ ID NO: 64:

```
Met Ala Lys Leu Ser Thr Asp Glu Leu Leu Asp Ala Phe Lys Glu Met
 1               5                  10                  15

Thr Leu Leu Glu Leu Ser Asp Phe Val Lys Lys Phe Glu Glu Thr Phe
```

117

```
                   20                  25                  30
     Glu Val Thr Ala Ala Ala Pro Val Ala Val Ala Ala Ala Gly Ala Ala
             35                  40                  45

     Pro Ala Gly Ala Ala Val Glu Ala Ala Glu Glu Gln Ser Glu Phe Asp
             50                  55                  60

     Val Ile Leu Glu Ala Ala Gly Asp Lys Lys Ile Gly Val Ile Lys Val
     65                  70                  75                  80

     Val Arg Glu Ile Val Ser Gly Leu Gly Leu Lys Glu Ala Lys Asp Leu
                     85                  90                  95

     Val Asp Gly Ala Pro Lys Pro Leu Leu Glu Lys Val Ala Lys Glu Ala
                 100                 105                 110

     Ala Asp Glu Ala Lys Ala Lys Leu Glu Ala Ala Gly Ala Thr Val Thr
                 115                 120                 125

     Val Lys
         130
```

(2) INFORMATION FOR SEQ ID NO: 65:

    (i) SEQUENCE CHARACTERISTICS:
        (A) LENGTH: 900 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 87...770
        (D) OTHER INFORMATION:

    (xi) SEQUENCE DESCRIPTION: SEQ ID NO: 65:

```
TGAACGCCAT CGGGTCCAAC GAACGCAGCG CTACCTGATC ACCACCGGGT CTGTTAGGGC       60

TCTTCCCCAG GTCGTACAGT CGGGCC ATG GCC ATT GAG GTT TCG GTG TTG CGG      113
                             Met Ala Ile Glu Val Ser Val Leu Arg
                              1               5

GTT TTC ACC GAT TCA GAC GGG AAT TTC GGT AAT CCG CTG GGG GTG ATC       161
Val Phe Thr Asp Ser Asp Gly Asn Phe Gly Asn Pro Leu Gly Val Ile
 10              15                  20                  25

AAC GCC AGC AAG GTC GAA CAC CGC GAC AGG CAG CAG CTG GCA GCC CAA       209
Asn Ala Ser Lys Val Glu His Arg Asp Arg Gln Gln Leu Ala Ala Gln
                30                  35                  40

TCG GGC TAC AGC GAA ACC ATA TTC GTC GAT CTT CCC AGC CCC GGC TCA       257
Ser Gly Tyr Ser Glu Thr Ile Phe Val Asp Leu Pro Ser Pro Gly Ser
            45                  50                  55

ACC ACC GCA CAC GCC ACC ATC CAT ACT CCC CGC ACC GAA ATT CCG TTC      305
Thr Thr Ala His Ala Thr Ile His Thr Pro Arg Thr Glu Ile Pro Phe
            60                  65                  70
```

```
GCC GGA CAC CCG ACC GTG GGA GCG TCC TGG TGG CTG CGC GAG AGG GGG        353
Ala Gly His Pro Thr Val Gly Ala Ser Trp Trp Leu Arg Glu Arg Gly
     75                  80                  85

ACG CCA ATT AAC ACG CTG CAG GTG CCG GCC GGC ATC GTC CAG GTG AGC        401
Thr Pro Ile Asn Thr Leu Gln Val Pro Ala Gly Ile Val Gln Val Ser
 90                  95                  100                 105

TAC CAC GGT GAT CTC ACC GCC ATC AGC GCC CGC TCG GAA TGG GCA CCC        449
Tyr His Gly Asp Leu Thr Ala Ile Ser Ala Arg Ser Glu Trp Ala Pro
                110                 115                 120

GAG TTC GCC ATC CAC GAC CTG GAT TCA CTT GAT GCG CTT GCC GCC GCC        497
Glu Phe Ala Ile His Asp Leu Asp Ser Leu Asp Ala Leu Ala Ala Ala
            125                 130                 135

GAC CCC GCC GAC TTT CCG GAC GAC ATC GCG CAC TAC CTC TGG ACC TGG        545
Asp Pro Ala Asp Phe Pro Asp Asp Ile Ala His Tyr Leu Trp Thr Trp
            140                 145                 150

ACC GAC CGC TCC GCT GGC TCG CTG CGC GCC CGC ATG TTT GCC GCC AAC        593
Thr Asp Arg Ser Ala Gly Ser Leu Arg Ala Arg Met Phe Ala Ala Asn
            155                 160                 165

TTG GGC GTC ACC GAA GAC GAA GCG ACC GGT GCC GCG GCC ATC CGG ATT        641
Leu Gly Val Thr Glu Asp Glu Ala Thr Gly Ala Ala Ala Ile Arg Ile
170                 175                 180                 185

ACC GAT TAC CTC AGC CGT GAC CTC ACC ATC ACC CAG GGC AAA GGA TCG        689
Thr Asp Tyr Leu Ser Arg Asp Leu Thr Ile Thr Gln Gly Lys Gly Ser
                190                 195                 200

TTG ATC CAC ACC ACC TGG AGT CCC GAG GGC TGG GTT CGG GTA GCC GGC        737
Leu Ile His Thr Thr Trp Ser Pro Glu Gly Trp Val Arg Val Ala Gly
            205                 210                 215

CGA GTT GTC AGC GAC GGT GTG GCA CAA CTC GAC TGACGTAGAG CTCAGCGCTG       790
Arg Val Val Ser Asp Gly Val Ala Gln Leu Asp
            220                 225

CCGATGCAAC ACGGCGGCAA GGTGATCCTG CAGGGGTTGC CCGACCGCGC GCATCTGCAA       850

CGAGTACGAA AGCTCGTCGC CGTCGATGCG GTAGGAACGG TCAAGGGCGG                  900
```

(2) INFORMATION FOR SEQ ID NO: 66:

    (i) SEQUENCE CHARACTERISTICS:
        (A) LENGTH: 228 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 66:

```
Met Ala Ile Glu Val Ser Val Leu Arg Val Phe Thr Asp Ser Asp Gly
  1               5                   10                  15
```

```
Asn Phe Gly Asn Pro Leu Gly Val Ile Asn Ala Ser Lys Val Glu His
            20                  25                  30

Arg Asp Arg Gln Gln Leu Ala Ala Gln Ser Gly Tyr Ser Glu Thr Ile
            35                  40                  45

Phe Val Asp Leu Pro Ser Pro Gly Ser Thr Thr Ala His Ala Thr Ile
        50                  55                  60

His Thr Pro Arg Thr Glu Ile Pro Phe Ala Gly His Pro Thr Val Gly
    65                  70                  75                  80

Ala Ser Trp Trp Leu Arg Glu Arg Gly Thr Pro Ile Asn Thr Leu Gln
                85                  90                  95

Val Pro Ala Gly Ile Val Gln Val Ser Tyr His Gly Asp Leu Thr Ala
                100                 105                 110

Ile Ser Ala Arg Ser Glu Trp Ala Pro Glu Phe Ala Ile His Asp Leu
            115                 120                 125

Asp Ser Leu Asp Ala Leu Ala Ala Ala Asp Pro Ala Asp Phe Pro Asp
        130                 135                 140

Asp Ile Ala His Tyr Leu Trp Thr Trp Thr Asp Arg Ser Ala Gly Ser
145                 150                 155                 160

Leu Arg Ala Arg Met Phe Ala Ala Asn Leu Gly Val Thr Glu Asp Glu
                165                 170                 175

Ala Thr Gly Ala Ala Ala Ile Arg Ile Thr Asp Tyr Leu Ser Arg Asp
                180                 185                 190

Leu Thr Ile Thr Gln Gly Lys Gly Ser Leu Ile His Thr Thr Trp Ser
            195                 200                 205

Pro Glu Gly Trp Val Arg Val Ala Gly Arg Val Val Ser Asp Gly Val
    210                 215                 220

Ala Gln Leu Asp
225
```

(2) INFORMATION FOR SEQ ID NO:  67:

    (i) SEQUENCE CHARACTERISTICS:
        (A) LENGTH: 500 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 49...465
        (D) OTHER INFORMATION:

    (xi) SEQUENCE DESCRIPTION: SEQ ID NO: 67:

```
GTTTGTGGTG TCGGTGGTCT GGGGGGCGCC AACTGGGATT CGGTTGGG GTG GGT GCA      57
                                                       Met Gly Ala
```

1

```
GGT CCG GCG ATG GGC ATC GGA GGT GTG GGT GGT TTG GGT GGG GCC GGT        105
Gly Pro Ala Met Gly Ile Gly Gly Val Gly Gly Leu Gly Gly Ala Gly
        5                  10                  15

TCG GGT CCG GCG ATG GGC ATG GGG GGT GTG GGT GGT TTG GGT GGG GCC        153
Ser Gly Pro Ala Met Gly Met Gly Gly Val Gly Gly Leu Gly Gly Ala
 20                  25                  30                  35

GGT TCG GGT CCG GCG ATG GGC ATG GGG GGT GTG GGT GGT TTA GAT GCG        201
Gly Ser Gly Pro Ala Met Gly Met Gly Gly Val Gly Gly Leu Asp Ala
                40                  45                  50

GCC GGT TCC GGC GAG GGC GGC TCT CCT GCG GCG ATC GGC ATC GGA GTT        249
Ala Gly Ser Gly Glu Gly Gly Ser Pro Ala Ala Ile Gly Ile Gly Val
                55                  60                  65

GGC GGA GGC GGA GGT GGG GGT GGG GGT GGC GGC GGC GGG GCC GAC ACG        297
Gly Gly Gly Gly Gly Gly Gly Gly Gly Gly Gly Gly Gly Ala Asp Thr
                70                  75                  80

AAC CGC TCC GAC AGG TCG TCG GAC GTC GGG GGC GGA GTC TGG CCG TTG        345
Asn Arg Ser Asp Arg Ser Ser Asp Val Gly Gly Gly Val Trp Pro Leu
        85                  90                  95

GGC TTC GGT AGG TTT GCC GAT GCG GGC GCC GGC GGA AAC GAA GCA CTG        393
Gly Phe Gly Arg Phe Ala Asp Ala Gly Ala Gly Gly Asn Glu Ala Leu
100                 105                 110                 115

GGG TCG AAG AAC GGC TGC GCT GCC ATA TCG TCC GGA GCT TCC ATA CCT        441
Gly Ser Lys Asn Gly Cys Ala Ala Ile Ser Ser Gly Ala Ser Ile Pro
                120                 125                 130

TCG TGC GGC CGG AAG AGC TTG TCG TAGTCGGCCG CCATGACAAC CTCTCAGAGT       495
Ser Cys Gly Arg Lys Ser Leu Ser
                135

GCGCT                                                                  500
```

(2) INFORMATION FOR SEQ ID NO: 68:

    (i) SEQUENCE CHARACTERISTICS:
        (A) LENGTH: 139 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 68:

```
Met Gly Ala Gly Pro Ala Met Gly Ile Gly Gly Val Gly Gly Leu Gly
 1               5                  10                  15

Gly Ala Gly Ser Gly Pro Ala Met Gly Met Gly Gly Val Gly Gly Leu
            20                  25                  30

Gly Gly Ala Gly Ser Gly Pro Ala Met Gly Met Gly Gly Val Gly Gly
```

121

```
                35                    40                        45

        Leu Asp Ala Ala Gly Ser Gly Glu Gly Gly Ser Pro Ala Ala Ile Gly
            50                    55                    60

        Ile Gly Val Gly Gly Gly Gly Gly Gly Gly Gly Gly Gly Gly Gly Gly
        65                    70                    75                    80

        Ala Asp Thr Asn Arg Ser Asp Arg Ser Ser Asp Val Gly Gly Gly Val
                        85                    90                    95

        Trp Pro Leu Gly Phe Gly Arg Phe Ala Asp Ala Gly Ala Gly Gly Asn
                    100                   105                   110

        Glu Ala Leu Gly Ser Lys Asn Gly Cys Ala Ala Ile Ser Ser Gly Ala
                115                   120                   125

        Ser Ile Pro Ser Cys Gly Arg Lys Ser Leu Ser
            130                   135
```

(2) INFORMATION FOR SEQ ID NO: 69:

    (i) SEQUENCE CHARACTERISTICS:
        (A) LENGTH: 2050 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 22...2019
        (D) OTHER INFORMATION:

    (xi) SEQUENCE DESCRIPTION: SEQ ID NO: 69:

```
AGCGCACTCT GAGAGGTTGT C ATG GCG GCC GAC TAC GAC AAG CTC TTC CGG      51
                         Met Ala Ala Asp Tyr Asp Lys Leu Phe Arg
                          1               5                   10

CCG CAC GAA GGT ATG GAA GCT CCG GAC GAT ATG GCA GCG CAG CCG TTC      99
Pro His Glu Gly Met Glu Ala Pro Asp Asp Met Ala Ala Gln Pro Phe
                15                    20                    25

TTC GAC CCC AGT GCT TCG TTT CCG CCG GCG CCC GCA TCG GCA AAC CTA     147
Phe Asp Pro Ser Ala Ser Phe Pro Pro Ala Pro Ala Ser Ala Asn Leu
            30                    35                    40

CCG AAG CCC AAC GGC CAG ACT CCG CCC CCG ACG TCC GAC GAC CTG TCG     195
Pro Lys Pro Asn Gly Gln Thr Pro Pro Pro Thr Ser Asp Asp Leu Ser
            45                    50                    55

GAG CGG TTC GTG TCG GCC CCG CCG CCG CCA CCC CCA CCC CCA CCT CCG     243
Glu Arg Phe Val Ser Ala Pro Pro Pro Pro Pro Pro Pro Pro Pro Pro
        60                    65                    70

CCT CCG CCA ACT CCG ATG CCG ATC GCC GCA GGA GAG CCG CCC TCG CCG     291
Pro Pro Pro Thr Pro Met Pro Ile Ala Ala Gly Glu Pro Pro Ser Pro
75                    80                    85                    90
```

122

```
GAA CCG GCC GCA TCT AAA CCA CCC ACA CCC CCC ATG CCC ATC GCC GGA      339
Glu Pro Ala Ala Ser Lys Pro Pro Thr Pro Pro Met Pro Ile Ala Gly
                 95              100             105

CCC GAA CCG GCC CCA CCC AAA CCA CCC ACA CCC CCC ATG CCC ATC GCC      387
Pro Glu Pro Ala Pro Pro Lys Pro Pro Thr Pro Pro Met Pro Ile Ala
         110             115             120

GGA CCC GAA CCG GCC CCA CCC AAA CCA CCC ACA CCT CCG ATG CCC ATC      435
Gly Pro Glu Pro Ala Pro Pro Lys Pro Pro Thr Pro Pro Met Pro Ile
         125             130             135

GCC GGA CCT GCA CCC ACC CCA ACC GAA TCC CAG TTG GCG CCC CCC AGA      483
Ala Gly Pro Ala Pro Thr Pro Thr Glu Ser Gln Leu Ala Pro Pro Arg
     140             145             150

CCA CCG ACA CCA CAA ACG CCA ACC GGA GCG CCG CAG CAA CCG GAA TCA      531
Pro Pro Thr Pro Gln Thr Pro Thr Gly Ala Pro Gln Gln Pro Glu Ser
155             160             165             170

CCG GCG CCC CAC GTA CCC TCG CAC GGG CCA CAT CAA CCC CGG CGC ACC      579
Pro Ala Pro His Val Pro Ser His Gly Pro His Gln Pro Arg Arg Thr
             175             180             185

GCA CCA GCA CCG CCC TGG GCA AAG ATG CCA ATC GGC GAA CCC CCG CCC      627
Ala Pro Ala Pro Pro Trp Ala Lys Met Pro Ile Gly Glu Pro Pro Pro
         190             195             200

GCT CCG TCC AGA CCG TCT GCG TCC CCG GCC GAA CCA CCG ACC CGG CCT      675
Ala Pro Ser Arg Pro Ser Ala Ser Pro Ala Glu Pro Pro Thr Arg Pro
         205             210             215

GCC CCC CAA CAC TCC CGA CGT GCG CGC CGG GGT CAC CGC TAT CGC ACA      723
Ala Pro Gln His Ser Arg Arg Ala Arg Arg Gly His Arg Tyr Arg Thr
         220             225             230

GAC ACC GAA CGA AAC GTC GGG AAG GTA GCA ACT GGT CCA TCC ATC CAG      771
Asp Thr Glu Arg Asn Val Gly Lys Val Ala Thr Gly Pro Ser Ile Gln
235             240             245             250

GCG CGG CTG CGG GCA GAG GAA GCA TCC GGC GCG CAG CTC GCC CCC GGA      819
Ala Arg Leu Arg Ala Glu Glu Ala Ser Gly Ala Gln Leu Ala Pro Gly
         255             260             265

ACG GAG CCC TCG CCA GCG CCG TTG GGC CAA CCG AGA TCG TAT CTG GCT      867
Thr Glu Pro Ser Pro Ala Pro Leu Gly Gln Pro Arg Ser Tyr Leu Ala
         270             275             280

CCG CCC ACC CGC CCC GCG CCG ACA GAA CCT CCC CCC AGC CCC TCG CCG      915
Pro Pro Thr Arg Pro Ala Pro Thr Glu Pro Pro Pro Ser Pro Ser Pro
         285             290             295

CAG CGC AAC TCC GGT CGG CGT GCC GAG CGA CGC GTC CAC CCC GAT TTA      963
Gln Arg Asn Ser Gly Arg Arg Ala Glu Arg Arg Val His Pro Asp Leu
         300             305             310

GCC GCC CAA CAT GCC GCG GCG CAA CCT GAT TCA ATT ACG GCC GCA ACC      1011
Ala Ala Gln His Ala Ala Ala Gln Pro Asp Ser Ile Thr Ala Ala Thr
315             320             325             330

ACT GGC GGT CGT CGC CGC AAG CGT GCA GCG CCG GAT CTC GAC GCG ACA      1059
```

```
        Thr Gly Gly Arg Arg Arg Lys Arg Ala Ala Pro Asp Leu Asp Ala Thr
                    335                 340                 345

        CAG AAA TCC TTA AGG CCG GCG GCC AAG GGG CCG AAG GTG AAG AAG GTG      1107
        Gln Lys Ser Leu Arg Pro Ala Ala Lys Gly Pro Lys Val Lys Lys Val
                    350                 355                 360

        AAG CCC CAG AAA CCG AAG GCC ACG AAG CCG CCC AAA GTG GTG TCG CAG      1155
        Lys Pro Gln Lys Pro Lys Ala Thr Lys Pro Pro Lys Val Val Ser Gln
                    365                 370                 375

        CGC GGC TGG CGA CAT TGG GTG CAT GCG TTG ACG CGA ATC AAC CTG GGC      1203
        Arg Gly Trp Arg His Trp Val His Ala Leu Thr Arg Ile Asn Leu Gly
                    380                 385                 390

        CTG TCA CCC GAC GAG AAG TAC GAG CTG GAC CTG CAC GCT CGA GTC CGC      1251
        Leu Ser Pro Asp Glu Lys Tyr Glu Leu Asp Leu His Ala Arg Val Arg
        395                 400                 405                 410

        CGC AAT CCC CGC GGG TCG TAT CAG ATC GCC GTC GTC GGT CTC AAA GGT      1299
        Arg Asn Pro Arg Gly Ser Tyr Gln Ile Ala Val Val Gly Leu Lys Gly
                    415                 420                 425

        GGG GCT GGC AAA ACC ACG CTG ACA GCA GCG TTG GGG TCG ACG TTG GCT      1347
        Gly Ala Gly Lys Thr Thr Leu Thr Ala Ala Leu Gly Ser Thr Leu Ala
                    430                 435                 440

        CAG GTG CGG GCC GAC CGG ATC CTG GCT CTA GAC GCG GAT CCA GGC GCC      1395
        Gln Val Arg Ala Asp Arg Ile Leu Ala Leu Asp Ala Asp Pro Gly Ala
                    445                 450                 455

        GGA AAC CTC GCC GAT CGG GTA GGG CGA CAA TCG GGC GCG ACC ATC GCT      1443
        Gly Asn Leu Ala Asp Arg Val Gly Arg Gln Ser Gly Ala Thr Ile Ala
                    460                 465                 470

        GAT GTG CTT GCA GAA AAA GAG CTG TCG CAC TAC AAC GAC ATC CGC GCA      1491
        Asp Val Leu Ala Glu Lys Glu Leu Ser His Tyr Asn Asp Ile Arg Ala
        475                 480                 485                 490

        CAC ACT AGC GTC AAT GCG GTC AAT CTG GAA GTG CTG CCG GCA CCG GAA      1539
        His Thr Ser Val Asn Ala Val Asn Leu Glu Val Leu Pro Ala Pro Glu
                    495                 500                 505

        TAC AGC TCG GCG CAG CGC GCG CTC AGC GAC GCC GAC TGG CAT TTC ATC      1587
        Tyr Ser Ser Ala Gln Arg Ala Leu Ser Asp Ala Asp Trp His Phe Ile
                    510                 515                 520

        GCC GAT CCT GCG TCG AGG TTT TAC AAC CTC GTC TTG GCT GAT TGT GGG      1635
        Ala Asp Pro Ala Ser Arg Phe Tyr Asn Leu Val Leu Ala Asp Cys Gly
                    525                 530                 535

        GCC GGC TTC TTC GAC CCG CTG ACC CGC GGC GTG CTG TCC ACG GTG TCC      1683
        Ala Gly Phe Phe Asp Pro Leu Thr Arg Gly Val Leu Ser Thr Val Ser
                    540                 545                 550

        GGT GTC GTG GTC GTG GCA AGT GTC TCA ATC GAC GGC GCA CAA CAG GCG      1731
        Gly Val Val Val Val Ala Ser Val Ser Ile Asp Gly Ala Gln Gln Ala
        555                 560                 565                 570

        TCG GTC GCG TTG GAC TGG TTG CGC AAC AAC GGT TAC CAA GAT TTG GCG      1779
        Ser Val Ala Leu Asp Trp Leu Arg Asn Asn Gly Tyr Gln Asp Leu Ala
```

```
                575                  580                  585

AGC CGC GCA TGC GTG GTC ATC AAT CAC ATC ATG CCG GGA GAA CCC AAT    1827
Ser Arg Ala Cys Val Val Ile Asn His Ile Met Pro Gly Glu Pro Asn
        590                  595                  600

GTC GCA GTT AAA GAC CTG GTG CGG CAT TTC GAA CAG CAA GTT CAA CCC    1875
Val Ala Val Lys Asp Leu Val Arg His Phe Glu Gln Gln Val Gln Pro
        605                  610                  615

GGC CGG GTC GTG GTC ATG CCG TGG GAC AGG CAC ATT GCG GCC GGA ACC    1923
Gly Arg Val Val Val Met Pro Trp Asp Arg His Ile Ala Ala Gly Thr
        620                  625                  630

GAG ATT TCA CTC GAC TTG CTC GAC CCT ATC TAC AAG CGC AAG GTC CTC    1971
Glu Ile Ser Leu Asp Leu Leu Asp Pro Ile Tyr Lys Arg Lys Val Leu
635                  640                  645                  650

GAA TTG GCC GCA GCG CTA TCC GAC GAT TTC GAG AGG GCT GGA CGT CGT T  2020
Glu Leu Ala Ala Ala Leu Ser Asp Asp Phe Glu Arg Ala Gly Arg Arg
        655                  660                  665

GAGCGCACCT GCTGTTGCTG CTGGTCCTAC                                   2050
```

(2) INFORMATION FOR SEQ ID NO: 70:

    (i) SEQUENCE CHARACTERISTICS:
        (A) LENGTH: 666 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 70:

```
Met Ala Ala Asp Tyr Asp Lys Leu Phe Arg Pro His Glu Gly Met Glu
1               5                   10                  15

Ala Pro Asp Asp Met Ala Ala Gln Pro Phe Phe Asp Pro Ser Ala Ser
            20                  25                  30

Phe Pro Pro Ala Pro Ala Ser Ala Asn Leu Pro Lys Pro Asn Gly Gln
        35                  40                  45

Thr Pro Pro Pro Thr Ser Asp Asp Leu Ser Glu Arg Phe Val Ser Ala
    50                  55                  60

Pro Pro Pro Pro Pro Pro Pro Pro Pro Pro Pro Pro Thr Pro Met
65                  70                  75                  80

Pro Ile Ala Ala Gly Glu Pro Pro Ser Pro Glu Pro Ala Ala Ser Lys
            85                  90                  95

Pro Pro Thr Pro Pro Met Pro Ile Ala Gly Pro Glu Pro Ala Pro Pro
            100                 105                 110

Lys Pro Pro Thr Pro Pro Met Pro Ile Ala Gly Pro Glu Pro Ala Pro
        115                 120                 125
```

```
Pro Lys Pro Pro Thr Pro Pro Met Pro Ile Ala Gly Pro Ala Pro Thr
    130                 135             140

Pro Thr Glu Ser Gln Leu Ala Pro Pro Arg Pro Pro Thr Pro Gln Thr
145             150             155             160

Pro Thr Gly Ala Pro Gln Gln Pro Glu Ser Pro Ala Pro His Val Pro
                165             170             175

Ser His Gly Pro His Gln Pro Arg Arg Thr Ala Pro Ala Pro Pro Trp
                180             185             190

Ala Lys Met Pro Ile Gly Glu Pro Pro Pro Ala Pro Ser Arg Pro Ser
            195             200             205

Ala Ser Pro Ala Glu Pro Pro Thr Arg Pro Ala Pro Gln His Ser Arg
    210             215             220

Arg Ala Arg Arg Gly His Arg Tyr Arg Thr Asp Thr Glu Arg Asn Val
225             230             235             240

Gly Lys Val Ala Thr Gly Pro Ser Ile Gln Ala Arg Leu Arg Ala Glu
            245             250             255

Glu Ala Ser Gly Ala Gln Leu Ala Pro Gly Thr Glu Pro Ser Pro Ala
            260             265             270

Pro Leu Gly Gln Pro Arg Ser Tyr Leu Ala Pro Pro Thr Arg Pro Ala
        275             280             285

Pro Thr Glu Pro Pro Pro Ser Pro Ser Pro Gln Arg Asn Ser Gly Arg
        290             295             300

Arg Ala Glu Arg Arg Val His Pro Asp Leu Ala Ala Gln His Ala Ala
305             310             315             320

Ala Gln Pro Asp Ser Ile Thr Ala Ala Thr Thr Gly Gly Arg Arg Arg
                325             330             335

Lys Arg Ala Ala Pro Asp Leu Asp Ala Thr Gln Lys Ser Leu Arg Pro
            340             345             350

Ala Ala Lys Gly Pro Lys Val Lys Lys Val Lys Pro Gln Lys Pro Lys
        355             360             365

Ala Thr Lys Pro Pro Lys Val Val Ser Gln Arg Gly Trp Arg His Trp
    370             375             380

Val His Ala Leu Thr Arg Ile Asn Leu Gly Leu Ser Pro Asp Glu Lys
385             390             395             400

Tyr Glu Leu Asp Leu His Ala Arg Val Arg Arg Asn Pro Arg Gly Ser
            405             410             415

Tyr Gln Ile Ala Val Val Gly Leu Lys Gly Gly Ala Gly Lys Thr Thr
        420             425             430

Leu Thr Ala Ala Leu Gly Ser Thr Leu Ala Gln Val Arg Ala Asp Arg
        435             440             445
```

```
Ile Leu Ala Leu Asp Ala Asp Pro Gly Ala Gly Asn Leu Ala Asp Arg
    450                 455                 460

Val Gly Arg Gln Ser Gly Ala Thr Ile Ala Asp Val Leu Ala Glu Lys
465                 470                 475                 480

Glu Leu Ser His Tyr Asn Asp Ile Arg Ala His Thr Ser Val Asn Ala
                485                 490                 495

Val Asn Leu Glu Val Leu Pro Ala Pro Glu Tyr Ser Ser Ala Gln Arg
                500                 505                 510

Ala Leu Ser Asp Ala Asp Trp His Phe Ile Ala Asp Pro Ala Ser Arg
            515                 520                 525

Phe Tyr Asn Leu Val Leu Ala Asp Cys Gly Ala Gly Phe Phe Asp Pro
    530                 535                 540

Leu Thr Arg Gly Val Leu Ser Thr Val Ser Gly Val Val Val Val Ala
545                 550                 555                 560

Ser Val Ser Ile Asp Gly Ala Gln Gln Ala Ser Val Ala Leu Asp Trp
                565                 570                 575

Leu Arg Asn Asn Gly Tyr Gln Asp Leu Ala Ser Arg Ala Cys Val Val
            580                 585                 590

Ile Asn His Ile Met Pro Gly Glu Pro Asn Val Ala Val Lys Asp Leu
    595                 600                 605

Val Arg His Phe Glu Gln Gln Val Gln Pro Gly Arg Val Val Val Met
    610                 615                 620

Pro Trp Asp Arg His Ile Ala Ala Gly Thr Glu Ile Ser Leu Asp Leu
625                 630                 635                 640

Leu Asp Pro Ile Tyr Lys Arg Lys Val Leu Glu Leu Ala Ala Ala Leu
                645                 650                 655

Ser Asp Asp Phe Glu Arg Ala Gly Arg Arg
            660                 665
```

(2) INFORMATION FOR SEQ ID NO: 71:

      (i) SEQUENCE CHARACTERISTICS:
          (A) LENGTH: 1890 base pairs
          (B) TYPE: nucleic acid
          (C) STRANDEDNESS: single
          (D) TOPOLOGY: linear

     (ix) FEATURE:

          (A) NAME/KEY: Coding Sequence
          (B) LOCATION: 79...1851
          (D) OTHER INFORMATION:

     (xi) SEQUENCE DESCRIPTION: SEQ ID NO: 71:

```
GCAGCGATGA GGAGGAGCGG CGCCAACGGC CCGCGCCGGC GACGATGCAA AGCGCAGCGA        60
```

```
TGAGGAGGAG CGGCGCGC ATG ACT GCT GAA CCG GAA GTA CGG ACG CTG CGC      111
                    Met Thr Ala Glu Pro Glu Val Arg Thr Leu Arg
                     1               5                  10

GAG GTT GTG CTG GAC CAG CTC GGC ACT GCT GAA TCG CGT GCG TAC AAG      159
Glu Val Val Leu Asp Gln Leu Gly Thr Ala Glu Ser Arg Ala Tyr Lys
         15              20                  25

ATG TGG CTG CCG CCG TTG ACC AAT CCG GTC CCG CTC AAC GAG CTC ATC      207
Met Trp Leu Pro Pro Leu Thr Asn Pro Val Pro Leu Asn Glu Leu Ile
         30              35                  40

GCC CGT GAT CGG CGA CAA CCC CTG CGA TTT GCC CTG GGG ATC ATG GAT      255
Ala Arg Asp Arg Arg Gln Pro Leu Arg Phe Ala Leu Gly Ile Met Asp
         45              50                  55

GAA CCG CGC CGC CAT CTA CAG GAT GTG TGG GGC GTA GAC GTT TCC GGG      303
Glu Pro Arg Arg His Leu Gln Asp Val Trp Gly Val Asp Val Ser Gly
 60              65                  70                  75

GCC GGC GGC AAC ATC GGT ATT GGG GGC GCA CCT CAA ACC GGG AAG TCG      351
Ala Gly Gly Asn Ile Gly Ile Gly Gly Ala Pro Gln Thr Gly Lys Ser
                 80              85                  90

ACG CTA CTG CAG ACG ATG GTG ATG TCG GCC GCC GCC ACA CAC TCA CCG      399
Thr Leu Leu Gln Thr Met Val Met Ser Ala Ala Ala Thr His Ser Pro
             95              100                 105

CGC AAC GTT CAG TTC TAT TGC ATC GAC CTA GGT GGC GGC GGG CTG ATC      447
Arg Asn Val Gln Phe Tyr Cys Ile Asp Leu Gly Gly Gly Gly Leu Ile
             110             115                 120

TAT CTC GAA AAC CTT CCA CAC GTC GGT GGG GTA GCC AAT CGG TCC GAG      495
Tyr Leu Glu Asn Leu Pro His Val Gly Gly Val Ala Asn Arg Ser Glu
         125             130                 135

CCC GAC AAG GTC AAC CGG GTG GTC GCA GAG ATG CAA GCC GTC ATG CGG      543
Pro Asp Lys Val Asn Arg Val Val Ala Glu Met Gln Ala Val Met Arg
140             145                 150                 155

CAA CGG GAA ACC ACC TTC AAG GAA CAC CGA GTG GGC TCG ATC GGG ATG      591
Gln Arg Glu Thr Thr Phe Lys Glu His Arg Val Gly Ser Ile Gly Met
             160             165                 170

TAC CGG CAG CTG CGT GAC GAT CCA AGT CAA CCC GTT GCG TCC GAT CCA      639
Tyr Arg Gln Leu Arg Asp Asp Pro Ser Gln Pro Val Ala Ser Asp Pro
             175             180                 185

TAC GGC GAC GTC TTT CTG ATC ATC GAC GGA TGG CCC GGT TTT GTC GGC      687
Tyr Gly Asp Val Phe Leu Ile Ile Asp Gly Trp Pro Gly Phe Val Gly
         190             195                 200

GAG TTC CCC GAC CTT GAG GGG CAG GTT CAA GAT CTG GCC GCC CAG GGG      735
Glu Phe Pro Asp Leu Glu Gly Gln Val Gln Asp Leu Ala Ala Gln Gly
         205             210                 215

CTG GGG TTC GGC GTC CAC GTC ATC ATC TCC ACG CCA CGC TGG ACA GAG      783
Leu Gly Phe Gly Val His Val Ile Ile Ser Thr Pro Arg Trp Thr Glu
220             225                 230                 235

CTG AAG TCG CGT GTT CGC GAC TAC CTC GGC ACC AAG ATC GAG TTC CGG      831
```

```
Leu Lys Ser Arg Val Arg Asp Tyr Leu Gly Thr Lys Ile Glu Phe Arg
            240             245             250

CTT GGT GAC GTC AAT GAA ACC CAG ATC GAC CGG ATT ACC CGC GAG ATC    879
Leu Gly Asp Val Asn Glu Thr Gln Ile Asp Arg Ile Thr Arg Glu Ile
            255             260             265

CCG GCG AAT CGT CCG GGT CGG GCA GTG TCG ATG GAA AAG CAC CAT CTG    927
Pro Ala Asn Arg Pro Gly Arg Ala Val Ser Met Glu Lys His His Leu
            270             275             280

ATG ATC GGC GTG CCC AGG TTC GAC GGC GTG CAC AGC GCC GAT AAC CTG    975
Met Ile Gly Val Pro Arg Phe Asp Gly Val His Ser Ala Asp Asn Leu
            285             290             295

GTG GAG GCG ATC ACC GCG GGG GTG ACG CAG ATC GCT TCC CAG CAC ACC   1023
Val Glu Ala Ile Thr Ala Gly Val Thr Gln Ile Ala Ser Gln His Thr
300             305             310             315

GAA CAG GCA CCT CCG GTG CGG GTC CTG CCG GAG CGT ATC CAC CTG CAC   1071
Glu Gln Ala Pro Pro Val Arg Val Leu Pro Glu Arg Ile His Leu His
            320             325             330

GAA CTC GAC CCG AAC CCG CCG GGA CCA GAG TCC GAC TAC CGC ACT CGC   1119
Glu Leu Asp Pro Asn Pro Pro Gly Pro Glu Ser Asp Tyr Arg Thr Arg
            335             340             345

TGG GAG ATT CCG ATC GGC TTG CGC GAG ACG GAC CTG ACG CCG GCT CAC   1167
Trp Glu Ile Pro Ile Gly Leu Arg Glu Thr Asp Leu Thr Pro Ala His
            350             355             360

TGC CAC ATG CAC ACG AAC CCG CAC CTA CTG ATC TTC GGT GCG GCC AAA   1215
Cys His Met His Thr Asn Pro His Leu Leu Ile Phe Gly Ala Ala Lys
            365             370             375

TCG GGC AAG ACG ACC ATT GCC CAC GCG ATC GCG CGC GCC ATT TGT GCC   1263
Ser Gly Lys Thr Thr Ile Ala His Ala Ile Ala Arg Ala Ile Cys Ala
380             385             390             395

CGA AAC AGT CCC CAG CAG GTG CGG TTC ATG CTC GCG GAC TAC CGC TCG   1311
Arg Asn Ser Pro Gln Gln Val Arg Phe Met Leu Ala Asp Tyr Arg Ser
            400             405             410

GGC CTG CTG GAC GCG GTG CCG GAC ACC CAT CTG CTG GGC GCC GGC GCG   1359
Gly Leu Leu Asp Ala Val Pro Asp Thr His Leu Leu Gly Ala Gly Ala
            415             420             425

ATC AAC CGC AAC AGC GCG TCG CTA GAC GAG GCC GCT CAA GCA CTG GCG   1407
Ile Asn Arg Asn Ser Ala Ser Leu Asp Glu Ala Ala Gln Ala Leu Ala
            430             435             440

GTC AAC CTG AAG AAG CGG TTG CCG CCG ACC GAC CTG ACG ACG GCG CAG   1455
Val Asn Leu Lys Lys Arg Leu Pro Pro Thr Asp Leu Thr Thr Ala Gln
            445             450             455

CTA CGC TCG CGT TCG TGG TGG AGC GGA TTT GAC GTC GTG CTT CTG GTC   1503
Leu Arg Ser Arg Ser Trp Trp Ser Gly Phe Asp Val Val Leu Leu Val
460             465             470             475

GAC GAT TGG CAC ATG ATC GTG GGT GCC GCC GGG GGG ATG CCG CCG ATG   1551
Asp Asp Trp His Met Ile Val Gly Ala Ala Gly Gly Met Pro Pro Met
```

```
                480                    485                    490
GCA CCG CTG GCC CCG TTA TTG CCG GCG GCG GCA GAT ATC GGG TTG CAC    1599
Ala Pro Leu Ala Pro Leu Leu Pro Ala Ala Ala Asp Ile Gly Leu His
        495                    500                    505

ATC ATT GTC ACC TGT CAG ATG AGC CAG GCT TAC AAG GCA ACC ATG GAC    1647
Ile Ile Val Thr Cys Gln Met Ser Gln Ala Tyr Lys Ala Thr Met Asp
        510                    515                    520

AAG TTC GTC GGC GCC GCA TTC GGG TCG GGC GCT CCG ACA ATG TTC CTT    1695
Lys Phe Val Gly Ala Ala Phe Gly Ser Gly Ala Pro Thr Met Phe Leu
        525                    530                    535

TCG GGC GAG AAG CAG GAA TTC CCA TCC AGT GAG TTC AAG GTC AAG CGG    1743
Ser Gly Glu Lys Gln Glu Phe Pro Ser Ser Glu Phe Lys Val Lys Arg
540                    545                    550                    555

CGC CCC CCT GGC CAG GCA TTT CTC GTC TCG CCA GAC GGC AAA GAG GTC    1791
Arg Pro Pro Gly Gln Ala Phe Leu Val Ser Pro Asp Gly Lys Glu Val
                560                    565                    570

ATC CAG GCC CCC TAC ATC GAG CCT CCA GAA GAA GTG TTC GCA GCA CCC    1839
Ile Gln Ala Pro Tyr Ile Glu Pro Pro Glu Glu Val Phe Ala Ala Pro
                575                    580                    585

CCA AGC GCC GGT TAAGATTATT TCATTGCCGG TGTAGCAGGA CCCGAGCTC         1890
Pro Ser Ala Gly
        590
```

(2) INFORMATION FOR SEQ ID NO: 72:

    (i) SEQUENCE CHARACTERISTICS:
        (A) LENGTH: 591 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 72:

```
Met Thr Ala Glu Pro Glu Val Arg Thr Leu Arg Glu Val Val Leu Asp
 1               5                  10                  15

Gln Leu Gly Thr Ala Glu Ser Arg Ala Tyr Lys Met Trp Leu Pro Pro
                20                  25                  30

Leu Thr Asn Pro Val Pro Leu Asn Glu Leu Ile Ala Arg Asp Arg Arg
            35                  40                  45

Gln Pro Leu Arg Phe Ala Leu Gly Ile Met Asp Glu Pro Arg Arg His
        50                  55                  60

Leu Gln Asp Val Trp Gly Val Asp Val Ser Gly Ala Gly Gly Asn Ile
65                  70                  75                  80

Gly Ile Gly Gly Ala Pro Gln Thr Gly Lys Ser Thr Leu Leu Gln Thr
                85                  90                  95
```

```
Met Val Met Ser Ala Ala Ala Thr His Ser Pro Arg Asn Val Gln Phe
            100                 105                 110

Tyr Cys Ile Asp Leu Gly Gly Gly Leu Ile Tyr Leu Glu Asn Leu
            115                 120                 125

Pro His Val Gly Gly Val Ala Asn Arg Ser Glu Pro Asp Lys Val Asn
            130                 135                 140

Arg Val Val Ala Glu Met Gln Ala Val Met Arg Gln Arg Glu Thr Thr
145                 150                 155                 160

Phe Lys Glu His Arg Val Gly Ser Ile Gly Met Tyr Arg Gln Leu Arg
                165                 170                 175

Asp Asp Pro Ser Gln Pro Val Ala Ser Asp Pro Tyr Gly Asp Val Phe
            180                 185                 190

Leu Ile Ile Asp Gly Trp Pro Gly Phe Val Gly Glu Phe Pro Asp Leu
            195                 200                 205

Glu Gly Gln Val Gln Asp Leu Ala Ala Gln Gly Leu Gly Phe Gly Val
            210                 215                 220

His Val Ile Ile Ser Thr Pro Arg Trp Thr Glu Leu Lys Ser Arg Val
225                 230                 235                 240

Arg Asp Tyr Leu Gly Thr Lys Ile Glu Phe Arg Leu Gly Asp Val Asn
                245                 250                 255

Glu Thr Gln Ile Asp Arg Ile Thr Arg Glu Ile Pro Ala Asn Arg Pro
                260                 265                 270

Gly Arg Ala Val Ser Met Glu Lys His His Leu Met Ile Gly Val Pro
            275                 280                 285

Arg Phe Asp Gly Val His Ser Ala Asp Asn Leu Val Glu Ala Ile Thr
            290                 295                 300

Ala Gly Val Thr Gln Ile Ala Ser Gln His Thr Glu Gln Ala Pro Pro
305                 310                 315                 320

Val Arg Val Leu Pro Glu Arg Ile His Leu His Glu Leu Asp Pro Asn
                325                 330                 335

Pro Pro Gly Pro Glu Ser Asp Tyr Arg Thr Arg Trp Glu Ile Pro Ile
            340                 345                 350

Gly Leu Arg Glu Thr Asp Leu Thr Pro Ala His Cys His Met His Thr
            355                 360                 365

Asn Pro His Leu Leu Ile Phe Gly Ala Ala Lys Ser Gly Lys Thr Thr
            370                 375                 380

Ile Ala His Ala Ile Ala Arg Ala Ile Cys Ala Arg Asn Ser Pro Gln
385                 390                 395                 400

Gln Val Arg Phe Met Leu Ala Asp Tyr Arg Ser Gly Leu Leu Asp Ala
                405                 410                 415
```

```
Val Pro Asp Thr His Leu Leu Gly Ala Gly Ala Ile Asn Arg Asn Ser
            420             425             430

Ala Ser Leu Asp Glu Ala Ala Gln Ala Leu Ala Val Asn Leu Lys Lys
            435             440             445

Arg Leu Pro Pro Thr Asp Leu Thr Thr Ala Gln Leu Arg Ser Arg Ser
            450             455             460

Trp Trp Ser Gly Phe Asp Val Val Leu Leu Val Asp Asp Trp His Met
465             470             475             480

Ile Val Gly Ala Ala Gly Gly Met Pro Pro Met Ala Pro Leu Ala Pro
            485             490             495

Leu Leu Pro Ala Ala Ala Asp Ile Gly Leu His Ile Ile Val Thr Cys
            500             505             510

Gln Met Ser Gln Ala Tyr Lys Ala Thr Met Asp Lys Phe Val Gly Ala
            515             520             525

Ala Phe Gly Ser Gly Ala Pro Thr Met Phe Leu Ser Gly Glu Lys Gln
            530             535             540

Glu Phe Pro Ser Ser Glu Phe Lys Val Lys Arg Arg Pro Pro Gly Gln
545             550             555             560

Ala Phe Leu Val Ser Pro Asp Gly Lys Glu Val Ile Gln Ala Pro Tyr
            565             570             575

Ile Glu Pro Pro Glu Glu Val Phe Ala Ala Pro Pro Ser Ala Gly
            580             585             590
```

(2)  INFORMATION FOR SEQ ID NO: 73:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: None

    (xi) SEQUENCE DESCRIPTION: SEQ ID NO: 73:

```
Asp Pro Val Asp Asp Ala Phe Ile Ala Lys Leu Asn Thr Ala Gly
 1               5               10              15
```

(2)  INFORMATION FOR SEQ ID NO: 74:

    (i) SEQUENCE CHARACTERISTICS:
        (A) LENGTH: 14 amino acids
        (B) TYPE: amino acid
        (C) STRANDEDNESS: single
        (D) TOPOLOGY: linear

    (ii) MOLECULE TYPE: None

    (ix) Feature:
        (A) NAME/KEY: Other

```
          (B) LOCATION: 14
          (C) OTHER INFORMATION: Xaa is unknown


     (xi) SEQUENCE DESCRIPTION: SEQ ID NO: 74:

Asp Pro Val Asp Ala Ile Ile Asn Leu Asp Asn Tyr Gly Xaa
 1               5                   10


(2) INFORMATION FOR SEQ ID NO: 75:

     (i) SEQUENCE CHARACTERISTICS:
          (A) LENGTH: 15 amino acids
          (B) TYPE: amino acid
          (C) STRANDEDNESS: single
          (D) TOPOLOGY: linear

     (ii) MOLECULE TYPE: None

     (ix) Feature:
          (A) NAME/KEY: Other
          (B) LOCATION: 5
          (C) OTHER INFORMATION: Xaa is unknown


     (xi) SEQUENCE DESCRIPTION: SEQ ID NO: 75:

Ala Glu Met Lys Xaa Phe Lys Asn Ala Ile Val Gln Glu Ile Asp
 1               5                   10                  15

(2) INFORMATION FOR SEQ ID NO: 76:

     (i) SEQUENCE CHARACTERISTICS:
          (A) LENGTH: 14 amino acids
          (B) TYPE: amino acid
          (C) STRANDEDNESS: single
          (D) TOPOLOGY: linear

     (ii) MOLECULE TYPE: None
     (ix) FEATURE:

          (A) NAME/KEY: Other
          (B) LOCATION: 3...3
          (D) OTHER INFORMATION: Ala is Ala or Gln

          (A) NAME/KEY: Other
          (B) LOCATION: 7...7
          (D) OTHER INFORMATION: Thr is Gly or Thr
     (ix) Feature:
          (A) NAME/KEY: Other
          (B) LOCATION: 11
          (C) OTHER INFORMATION: Xaa is unknown


     (xi) SEQUENCE DESCRIPTION: SEQ ID NO: 76:

Val Ile Ala Gly Met Val Thr His Ile His Xaa Val Ala Gly
 1               5                   10
```

(2) INFORMATION FOR SEQ ID NO: 77:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide
    (v) FRAGMENT TYPE: N-terminal

    (xi) SEQUENCE DESCRIPTION: SEQ ID NO: 77:

Thr Asn Ile Val Val Leu Ile Lys Gln Val Pro Asp Thr Trp Ser
1            5           10          15

(2) INFORMATION FOR SEQ ID NO: 78:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide
    (v) FRAGMENT TYPE: N-terminal

    (xi) SEQUENCE DESCRIPTION: SEQ ID NO: 78:

Ala Ile Glu Val Ser Val Leu Arg Val Phe Thr Asp Ser Asp Gly
1            5           10          15

(2) INFORMATION FOR SEQ ID NO: 79:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide
    (v) FRAGMENT TYPE: N-terminal

    (xi) SEQUENCE DESCRIPTION: SEQ ID NO: 79:

Ala Lys Leu Ser Thr Asp Glu Leu Leu Asp Ala Phe Lys Glu Met
1            5           10          15

(2) INFORMATION FOR SEQ ID NO: 80:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide
    (v) FRAGMENT TYPE: N-terminal
    (ix) FEATURE:

        (A) NAME/KEY: Other

```
        (B) LOCATION: 4...4
        (D) OTHER INFORMATION: Asp is Asp or Glu

    (xi) SEQUENCE DESCRIPTION: SEQ ID NO: 80:

Asp Pro Ala Asp Ala Pro Asp Val Pro Thr Ala Ala Gln Leu Thr
 1               5                   10                  15


(2) INFORMATION FOR SEQ ID NO: 81:

        (i) SEQUENCE CHARACTERISTICS:
            (A) LENGTH: 50 amino acids
            (B) TYPE: amino acid
            (C) STRANDEDNESS: single
            (D) TOPOLOGY: linear

        (ii) MOLECULE TYPE: peptide
        (v) FRAGMENT TYPE: N-terminal

        (xi) SEQUENCE DESCRIPTION: SEQ ID NO: 81:

Ala Glu Asp Val Arg Ala Glu Ile Val Ala Ser Val Leu Glu Val Val
 1               5                   10                  15

Val Asn Glu Gly Asp Gln Ile Asp Lys Gly Asp Val Val Val Leu Leu
            20                  25                  30

Glu Ser Met Tyr Met Glu Ile Pro Val Leu Ala Glu Ala Ala Gly Thr
        35                  40                  45
Val Ser
    50


(2) INFORMATION FOR SEQ ID NO: 82:

        (i) SEQUENCE CHARACTERISTICS:
            (A) LENGTH: 15 amino acids
            (B) TYPE: amino acid
            (C) STRANDEDNESS: single
            (D) TOPOLOGY: linear

        (ii) MOLECULE TYPE: peptide
        (v) FRAGMENT TYPE: N-terminal

        (xi) SEQUENCE DESCRIPTION: SEQ ID NO: 82:

Thr Thr Ser Pro Asp Pro Tyr Ala Ala Leu Pro Lys Leu Pro Ser
 1               5                   10                  15

(2) INFORMATION FOR SEQ ID NO: 83:

        (i) SEQUENCE CHARACTERISTICS:
            (A) LENGTH: 15 amino acids
            (B) TYPE: amino acid
            (C) STRANDEDNESS: single
            (D) TOPOLOGY: linear

        (ii) MOLECULE TYPE: peptide
        (v) FRAGMENT TYPE: N-terminal

        (xi) SEQUENCE DESCRIPTION: SEQ ID NO: 83:
```

```
Thr Glu Tyr Glu Gly Pro Lys Thr Lys Phe His Ala Leu Met Gln
 1               5                  10                  15
```

(2)  INFORMATION FOR SEQ ID NO: 84:

    (i)  SEQUENCE CHARACTERISTICS:
        (A)  LENGTH: 15 amino acids
        (B)  TYPE: amino acid
        (C)  STRANDEDNESS: single
        (D)  TOPOLOGY: linear

    (ii) MOLECULE TYPE: peptide
    (v)  FRAGMENT TYPE: N-terminal

    (xi) SEQUENCE DESCRIPTION: SEQ ID NO: 84:

```
Thr Thr Ile Val Ala Leu Lys Tyr Pro Gly Gly Val Val Met Ala
 1               5                  10                  15
```

(2)  INFORMATION FOR SEQ ID NO: 85:

    (i)  SEQUENCE CHARACTERISTICS:
        (A)  LENGTH: 15 amino acids
        (B)  TYPE: amino acid
        (C)  STRANDEDNESS: single
        (D)  TOPOLOGY: linear

    (ii) MOLECULE TYPE: peptide
    (v)  FRAGMENT TYPE: N-terminal

    (ix) FEATURE:
        (A)  NAME/KEY: Other
        (B)  LOCATION: 10
        (D)  OTHER INFORMATION: Xaa is unknown

    (ix) FEATURE:
        (A)  NAME/KEY: Other
        (B)  LOCATION: 15
        (D)  OTHER INFORMATION: Xaa is unknown

    (xi) SEQUENCE DESCRIPTION: SEQ ID NO: 85:

```
Ser Phe Pro Tyr Phe Ile Ser Pro Glu Xaa Ala Met Arg Glu Xaa
 1               5                  10                  15
```

(2)  INFORMATION FOR SEQ ID NO: 86:

    (i)  SEQUENCE CHARACTERISTICS:
        (A)  LENGTH: 15 amino acids
        (B)  TYPE: amino acid
        (C)  STRANDEDNESS: single
        (D)  TOPOLOGY: linear

    (ii) MOLECULE TYPE: peptide
    (v)  FRAGMENT TYPE: N-terminal

    (xi) SEQUENCE DESCRIPTION: SEQ ID NO: 86:

```
Thr His Tyr Asp Val Val Val Leu Gly Ala Gly Pro Gly Gly Tyr
 1               5                  10                  15
```

(2) INFORMATION FOR SEQ ID NO: 87:

    (i) SEQUENCE CHARACTERISTICS:
        (A) LENGTH: 450 base pairs
        (B) TYPE: nucleic acid
        (C) STRANDEDNESS: single
        (D) TOPOLOGY: linear

    (ii) MOLECULE TYPE: Other
    (ix) FEATURE:

        (A) NAME/KEY: Coding Sequence
        (B) LOCATION: 107...400
        (D) OTHER INFORMATION:

    (xi) SEQUENCE DESCRIPTION: SEQ ID NO: 87:

```
AGCCCGGTAA TCGAGTTCGG GCAATGCTGA CCATCGGGTT TGTTTCCGGC TATAACCGAA        60

CGGTTTGTGT ACGGGATACA AATACAGGGA GGGAAGAAGT AGGCAA ATG GAA AAA          115
                                                     Met Glu Lys
                                                       1

ATG TCA CAT GAT CCG ATC GCT GCC GAC ATT GGC ACG CAA GTG AGC GAC        163
Met Ser His Asp Pro Ile Ala Ala Asp Ile Gly Thr Gln Val Ser Asp
        5                  10                  15

AAC GCT CTG CAC GGC GTG ACG GCC GGC TCG ACG GCG CTG ACG TCG GTG        211
Asn Ala Leu His Gly Val Thr Ala Gly Ser Thr Ala Leu Thr Ser Val
 20                  25                  30                  35

ACC GGG CTG GTT CCC GCG GGG GCC GAT GAG GTC TCC GCC CAA GCG GCG        259
Thr Gly Leu Val Pro Ala Gly Ala Asp Glu Val Ser Ala Gln Ala Ala
                  40                  45                  50

ACG GCG TTC ACA TCG GAG GGC ATC CAA TTG CTG GCT TCC AAT GCA TCG        307
Thr Ala Phe Thr Ser Glu Gly Ile Gln Leu Leu Ala Ser Asn Ala Ser
              55                  60                  65

GCC CAA GAC CAG CTC CAC CGT GCG GGC GAA GCG GTC CAG GAC GTC GCC        355
Ala Gln Asp Gln Leu His Arg Ala Gly Glu Ala Val Gln Asp Val Ala
              70                  75                  80

CGC ACC TAT TCG CAA ATC GAC GAC GGC GCC GCC GGC GTC TTC GCC TAATA      405
Arg Thr Tyr Ser Gln Ile Asp Asp Gly Ala Ala Gly Val Phe Ala
              85                  90                  95

GGCCCCCAAC ACATCGGAGG GAGTGATCAC CATGCTGTGG CACGC                      450
```

(2) INFORMATION FOR SEQ ID NO: 88:

    (i) SEQUENCE CHARACTERISTICS:
        (A) LENGTH: 98 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

(xi) SEQUENCE DESCRIPTION: SEQ ID NO: 88:

```
Met Glu Lys Met Ser His Asp Pro Ile Ala Ala Asp Ile Gly Thr Gln
  1               5               10              15

Val Ser Asp Asn Ala Leu His Gly Val Thr Ala Gly Ser Thr Ala Leu
         20              25              30

Thr Ser Val Thr Gly Leu Val Pro Ala Gly Ala Asp Glu Val Ser Ala
         35              40              45

Gln Ala Ala Thr Ala Phe Thr Ser Glu Gly Ile Gln Leu Leu Ala Ser
     50              55              60

Asn Ala Ser Ala Gln Asp Gln Leu His Arg Ala Gly Glu Ala Val Gln
 65              70              75              80

Asp Val Ala Arg Thr Tyr Ser Gln Ile Asp Asp Gly Ala Ala Gly Val
             85              90              95

Phe Ala
```

(2) INFORMATION FOR SEQ ID NO: 89:

       (i) SEQUENCE CHARACTERISTICS:
           (A) LENGTH: 460 base pairs
           (B) TYPE: nucleic acid
           (C) STRANDEDNESS: single
           (D) TOPOLOGY: linear

       (ix) FEATURE:

           (A) NAME/KEY: Coding Sequence
           (B) LOCATION: 37...453
           (D) OTHER INFORMATION:

       (xi) SEQUENCE DESCRIPTION: SEQ ID NO: 89:

```
GCAACCGGCT TTTCGATCAG CTGAGACATC AGCGGC GTG CGG GTC AAC GAC CCA        54
                                        Met Arg Val Asn Asp Pro
                                          1               5

CCT GCG CCA GGT AGC GAC TCC GCG CGC AGC AGG CCC GCG CCC GCG CTG       102
Pro Ala Pro Gly Ser Asp Ser Ala Arg Ser Arg Pro Ala Pro Ala Leu
             10              15              20

GGG CCT GAT CCA CCA GCC AGC GGA TGG TTC GAC AGC GGA CTG GTG CCG       150
Gly Pro Asp Pro Pro Ala Ser Gly Trp Phe Asp Ser Gly Leu Val Pro
             25              30              35

AGC AGG CCC ATC TGC GCG GCT TCC TCG TCG GCT GGG TTG CCG CCG CCG       198
Ser Arg Pro Ile Cys Ala Ala Ser Ser Ser Ala Gly Leu Pro Pro Pro
         40              45              50

GTG CCG CCC ACC TGG CTG AAC AAC GAC GTC ACC TGC TGC AGC GGC TGG       246
Val Pro Pro Thr Trp Leu Asn Asn Asp Val Thr Cys Cys Ser Gly Trp
 55              60              65              70

GTC AGC TGC TGC ATC GGG CCG CTC ATC TCA CCC AGT TGG CCG AGG GTC       294
```

```
Val Ser Cys Cys Ile Gly Pro Leu Ile Ser Pro Ser Trp Pro Arg Val
            75                  80              85
```

```
TGG GTA GCC GCC GGC GGC AAC TGG CCA ACC GGT GTT GAG CTG CCA GGG      342
Trp Val Ala Ala Gly Gly Asn Trp Pro Thr Gly Val Glu Leu Pro Gly
            90                  95              100
```

```
GAG GGC ATT CCG AAG ATC GGG TTC GTC GTG CTC TGG CTC GCG CCG GGA      390
Glu Gly Ile Pro Lys Ile Gly Phe Val Val Leu Trp Leu Ala Pro Gly
            105                 110             115
```

```
TCA AGG ATC GAC GCC ATC GGC TCG AGC TTC TCG AAA AGC GTG TTA ACC      438
Ser Arg Ile Asp Ala Ile Gly Ser Ser Phe Ser Lys Ser Val Leu Thr
    120                 125             130
```

```
GCG GTC TCG GCC TGG TAGACCT                                          460
Ala Val Ser Ala Trp
135
```

(2)  INFORMATION FOR SEQ ID NO: 90:

    (i)  SEQUENCE CHARACTERISTICS:
       (A)  LENGTH: 139 amino acids
       (B)  TYPE: amino acid
       (C)  STRANDEDNESS: single
       (D)  TOPOLOGY: linear

    (ii)  MOLECULE TYPE: protein
    (v)  FRAGMENT TYPE: internal

    (xi)  SEQUENCE DESCRIPTION: SEQ ID NO: 90:

```
Met Arg Val Asn Asp Pro Pro Ala Pro Gly Ser Asp Ser Ala Arg Ser
1                   5                   10                  15
```

```
Arg Pro Ala Pro Ala Leu Gly Pro Asp Pro Pro Ala Ser Gly Trp Phe
            20                  25                  30
```

```
Asp Ser Gly Leu Val Pro Ser Arg Pro Ile Cys Ala Ala Ser Ser Ser
            35                  40                  45
```

```
Ala Gly Leu Pro Pro Pro Val Pro Pro Thr Trp Leu Asn Asn Asp Val
    50                  55                  60
```

```
Thr Cys Cys Ser Gly Trp Val Ser Cys Cys Ile Gly Pro Leu Ile Ser
65                  70                  75                  80
```

```
Pro Ser Trp Pro Arg Val Trp Val Ala Ala Gly Gly Asn Trp Pro Thr
            85                  90                  95
```

```
Gly Val Glu Leu Pro Gly Glu Gly Ile Pro Lys Ile Gly Phe Val Val
            100                 105                 110
```

```
Leu Trp Leu Ala Pro Gly Ser Arg Ile Asp Ala Ile Gly Ser Ser Phe
            115                 120                 125
```

```
Ser Lys Ser Val Leu Thr Ala Val Ser Ala Trp
    130                 135
```

(2) INFORMATION FOR SEQ ID NO: 91:

    (i) SEQUENCE CHARACTERISTICS:
        (A) LENGTH: 1200 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 28...1140
        (D) OTHER INFORMATION:

    (xi) SEQUENCE DESCRIPTION: SEQ ID NO: 91:

```
TAATAGGCCC CCAACACATC GGAGGGA GTG ATC ACC ATG CTG TGG CAC GCA ATG      54
                               Met Ile Thr Met Leu Trp His Ala Met
                                 1                   5

CCA CCG GAG CTA AAT ACC GCA CGG CTG ATG GCC GGC GCG GGT CCG GCT       102
Pro Pro Glu Leu Asn Thr Ala Arg Leu Met Ala Gly Ala Gly Pro Ala
 10              15              20                      25

CCA ATG CTT GCG GCG GCC GCG GGA TGG CAG ACG CTT TCG GCG GCT CTG      150
Pro Met Leu Ala Ala Ala Ala Gly Trp Gln Thr Leu Ser Ala Ala Leu
                30              35                      40

GAC GCT CAG GCC GTC GAG TTG ACC GCG CGC CTG AAC TCT CTG GGA GAA      198
Asp Ala Gln Ala Val Glu Leu Thr Ala Arg Leu Asn Ser Leu Gly Glu
            45              50              55

GCC TGG ACT GGA GGT GGC AGC GAC AAG GCG CTT GCG GCT GCA ACG CCG      246
Ala Trp Thr Gly Gly Gly Ser Asp Lys Ala Leu Ala Ala Ala Thr Pro
            60              65              70

ATG GTG GTC TGG CTA CAA ACC GCG TCA ACA CAG GCC AAG ACC CGT GCG      294
Met Val Val Trp Leu Gln Thr Ala Ser Thr Gln Ala Lys Thr Arg Ala
     75              80              85

ATG CAG GCG ACG GCG CAA GCC GCG GCA TAC ACC CAG GCC ATG GCC ACG      342
Met Gln Ala Thr Ala Gln Ala Ala Ala Tyr Thr Gln Ala Met Ala Thr
 90              95              100                     105

ACG CCG TCG CTG CCG GAG ATC GCC GCC AAC CAC ATC ACC CAG GCC GTC      390
Thr Pro Ser Leu Pro Glu Ile Ala Ala Asn His Ile Thr Gln Ala Val
                110             115                     120

CTT ACG GCC ACC AAC TTC TTC GGT ATC AAC ACG ATC CCG ATC GCG TTG      438
Leu Thr Ala Thr Asn Phe Phe Gly Ile Asn Thr Ile Pro Ile Ala Leu
                125             130                     135

ACC GAG ATG GAT TAT TTC ATC CGT ATG TGG AAC CAG GCA GCC CTG GCA      486
Thr Glu Met Asp Tyr Phe Ile Arg Met Trp Asn Gln Ala Ala Leu Ala
            140             145             150

ATG GAG GTC TAC CAG GCC GAG ACC GCG GTT AAC ACG CTT TTC GAG AAG      534
Met Glu Val Tyr Gln Ala Glu Thr Ala Val Asn Thr Leu Phe Glu Lys
            155             160             165

CTC GAG CCG ATG GCG TCG ATC CTT GAT CCC GGC GCG AGC CAG AGC ACG      582
```

```
Leu Glu Pro Met Ala Ser Ile Leu Asp Pro Gly Ala Ser Gln Ser Thr
170             175             180             185

ACG AAC CCG ATC TTC GGA ATG CCC TCC CCT GGC AGC TCA ACA CCG GTT    630
Thr Asn Pro Ile Phe Gly Met Pro Ser Pro Gly Ser Ser Thr Pro Val
                    190             195             200

GGC CAG TTG CCG CCG GCG GCT ACC CAG ACC CTC GGC CAA CTG GGT GAG    678
Gly Gln Leu Pro Pro Ala Ala Thr Gln Thr Leu Gly Gln Leu Gly Glu
                205             210             215

ATG AGC GGC CCG ATG CAG CAG CTG ACC CAG CCG CTG CAG CAG GTG ACG    726
Met Ser Gly Pro Met Gln Gln Leu Thr Gln Pro Leu Gln Gln Val Thr
            220             225             230

TCG TTG TTC AGC CAG GTG GGC GGC ACC GGC GGC GGC AAC CCA GCC GAC    774
Ser Leu Phe Ser Gln Val Gly Gly Thr Gly Gly Gly Asn Pro Ala Asp
            235             240             245

GAG GAA GCC GCG CAG ATG GGC CTG CTC GGC ACC AGT CCG CTG TCG AAC    822
Glu Glu Ala Ala Gln Met Gly Leu Leu Gly Thr Ser Pro Leu Ser Asn
250             255             260             265

CAT CCG CTG GCT GGT GGA TCA GGC CCC AGC GCG GGC GCG GGC CTG CTG    870
His Pro Leu Ala Gly Gly Ser Gly Pro Ser Ala Gly Ala Gly Leu Leu
                270             275             280

CGC GCG GAG TCG CTA CCT GGC GCA GGT GGG TCG TTG ACC CGC ACG CCG    918
Arg Ala Glu Ser Leu Pro Gly Ala Gly Gly Ser Leu Thr Arg Thr Pro
                285             290             295

CTG ATG TCT CAG CTG ATC GAA AAG CCG GTT GCC CCC TCG GTG ATG CCG    966
Leu Met Ser Gln Leu Ile Glu Lys Pro Val Ala Pro Ser Val Met Pro
            300             305             310

GCG GCT GCT GCC GGA TCG TCG GCG ACG GGT GGC GCC GCT CCG GTG GGT    1014
Ala Ala Ala Ala Gly Ser Ser Ala Thr Gly Gly Ala Ala Pro Val Gly
            315             320             325

GCG GGA GCG ATG GGC CAG GGT GCG CAA TCC GGC GGC TCC ACC AGG CCG    1062
Ala Gly Ala Met Gly Gln Gly Ala Gln Ser Gly Gly Ser Thr Arg Pro
330             335             340             345

GGT CTG GTC GCG CCG GCA CCG CTC GCG CAG GAG CGT GAA GAA GAC GAC    1110
Gly Leu Val Ala Pro Ala Pro Leu Ala Gln Glu Arg Glu Glu Asp Asp
                350             355             360

GAG GAC GAC TGG GAC GAA GAG GAC GAC TGG TGAGCTCCCG TAATGACAAC AGA  1163
Glu Asp Asp Trp Asp Glu Glu Asp Asp Trp
                365             370

CTTCCCGGCC ACCCGGGCCG GAAGACTTGC CAACATT                           1200
```

(2) INFORMATION FOR SEQ ID NO: 92:

    (i) SEQUENCE CHARACTERISTICS:
        (A) LENGTH: 371 amino acids
        (B) TYPE: amino acid
        (C) STRANDEDNESS: single
        (D) TOPOLOGY: linear

(ii) MOLECULE TYPE: protein
(v) FRAGMENT TYPE: internal

(xi) SEQUENCE DESCRIPTION: SEQ ID NO: 92:

```
Met Ile Thr Met Leu Trp His Ala Met Pro Pro Glu Leu Asn Thr Ala
 1               5                  10                  15

Arg Leu Met Ala Gly Ala Gly Pro Ala Pro Met Leu Ala Ala Ala Ala
            20                  25                  30

Gly Trp Gln Thr Leu Ser Ala Ala Leu Asp Ala Gln Ala Val Glu Leu
        35                  40                  45

Thr Ala Arg Leu Asn Ser Leu Gly Glu Ala Trp Thr Gly Gly Gly Ser
    50                  55                  60

Asp Lys Ala Leu Ala Ala Ala Thr Pro Met Val Val Trp Leu Gln Thr
65                  70                  75                  80

Ala Ser Thr Gln Ala Lys Thr Arg Ala Met Gln Ala Thr Ala Gln Ala
            85                  90                  95

Ala Ala Tyr Thr Gln Ala Met Ala Thr Thr Pro Ser Leu Pro Glu Ile
            100                 105                 110

Ala Ala Asn His Ile Thr Gln Ala Val Leu Thr Ala Thr Asn Phe Phe
        115                 120                 125

Gly Ile Asn Thr Ile Pro Ile Ala Leu Thr Glu Met Asp Tyr Phe Ile
    130                 135                 140

Arg Met Trp Asn Gln Ala Ala Leu Ala Met Glu Val Tyr Gln Ala Glu
145                 150                 155                 160

Thr Ala Val Asn Thr Leu Phe Glu Lys Leu Glu Pro Met Ala Ser Ile
                165                 170                 175

Leu Asp Pro Gly Ala Ser Gln Ser Thr Thr Asn Pro Ile Phe Gly Met
            180                 185                 190

Pro Ser Pro Gly Ser Ser Thr Pro Val Gly Gln Leu Pro Pro Ala Ala
        195                 200                 205

Thr Gln Thr Leu Gly Gln Leu Gly Glu Met Ser Gly Pro Met Gln Gln
    210                 215                 220

Leu Thr Gln Pro Leu Gln Gln Val Thr Ser Leu Phe Ser Gln Val Gly
225                 230                 235                 240

Gly Thr Gly Gly Gly Asn Pro Ala Asp Glu Glu Ala Ala Gln Met Gly
            245                 250                 255

Leu Leu Gly Thr Ser Pro Leu Ser Asn His Pro Leu Ala Gly Gly Ser
            260                 265                 270

Gly Pro Ser Ala Gly Ala Gly Leu Leu Arg Ala Glu Ser Leu Pro Gly
        275                 280                 285

Ala Gly Gly Ser Leu Thr Arg Thr Pro Leu Met Ser Gln Leu Ile Glu
```

```
        290                  295                  300
Lys Pro Val Ala Pro Ser Val Met Pro Ala Ala Ala Ala Gly Ser Ser
305                 310                 315                 320

Ala Thr Gly Gly Ala Ala Pro Val Gly Ala Gly Ala Met Gly Gln Gly
            325                 330                 335

Ala Gln Ser Gly Gly Ser Thr Arg Pro Gly Leu Val Ala Pro Ala Pro
        340                 345                 350

Leu Ala Gln Glu Arg Glu Glu Asp Asp Glu Asp Asp Trp Asp Glu Glu
    355                 360                 365

Asp Asp Trp
    370
```

(2) INFORMATION FOR SEQ ID NO: 93:

    (i) SEQUENCE CHARACTERISTICS:
        (A) LENGTH: 1000 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 46...969
        (D) OTHER INFORMATION:

    (xi) SEQUENCE DESCRIPTION: SEQ ID NO: 93:

```
GACGCGACAC AGAAATCCTT AAGGCCGGCG GCCAAGGGGC CGAAG GTG AAG AAG GTG      57
                                              Met Lys Lys Val
                                               1

AAG CCC CAG AAA CCG AAG GCC ACG AAG CCG CCC AAA GTG GTG TCG CAG      105
Lys Pro Gln Lys Pro Lys Ala Thr Lys Pro Pro Lys Val Val Ser Gln
 5                  10                  15                  20

CGC GGC TGG CGA CAT TGG GTG CAT GCG TTG ACG CGA ATC AAC CTG GGC      153
Arg Gly Trp Arg His Trp Val His Ala Leu Thr Arg Ile Asn Leu Gly
                25                  30                  35

CTG TCA CCC GAC GAG AAG TAC GAG CTG GAC CTG CAC GCT CGA GTC CGC      201
Leu Ser Pro Asp Glu Lys Tyr Glu Leu Asp Leu His Ala Arg Val Arg
            40                  45                  50

CGC AAT CCC CGC GGG TCG TAT CAG ATC GCC GTC GTC GGT CTC AAA GGT      249
Arg Asn Pro Arg Gly Ser Tyr Gln Ile Ala Val Val Gly Leu Lys Gly
        55                  60                  65

GGG GCT GGC AAA ACC ACG CTG ACA GCA GCG TTG GGG TCG ACG TTG GCT      297
Gly Ala Gly Lys Thr Thr Leu Thr Ala Ala Leu Gly Ser Thr Leu Ala
        70                  75                  80

CAG GTG CGG GCC GAC CGG ATC CTG GCT CTA GAC GCG GAT CCA GGC GCC      345
Gln Val Arg Ala Asp Arg Ile Leu Ala Leu Asp Ala Asp Pro Gly Ala
85                  90                  95                  100
```

GGA AAC CTC GCC GAT CGG GTA GGG CGA CAA TCG GGC GCG ACC ATC GCT          393
Gly Asn Leu Ala Asp Arg Val Gly Arg Gln Ser Gly Ala Thr Ile Ala
            105             110             115

GAT GTG CTT GCA GAA AAA GAG CTG TCG CAC TAC AAC GAC ATC CGC GCA          441
Asp Val Leu Ala Glu Lys Glu Leu Ser His Tyr Asn Asp Ile Arg Ala
            120             125             130

CAC ACT AGC GTC AAT GCG GTC AAT CTG GAA GTG CTG CCG GCA CCG GAA          489
His Thr Ser Val Asn Ala Val Asn Leu Glu Val Leu Pro Ala Pro Glu
            135             140             145

TAC AGC TCG GCG CAG CGC GCG CTC AGC GAC GCC GAC TGG CAT TTC ATC          537
Tyr Ser Ser Ala Gln Arg Ala Leu Ser Asp Ala Asp Trp His Phe Ile
            150             155             160

GCC GAT CCT GCG TCG AGG TTT TAC AAC CTC GTC TTG GCT GAT TGT GGG          585
Ala Asp Pro Ala Ser Arg Phe Tyr Asn Leu Val Leu Ala Asp Cys Gly
165             170             175             180

GCC GGC TTC TTC GAC CCG CTG ACC CGC GGC GTG CTG TCC ACG GTG TCC          633
Ala Gly Phe Phe Asp Pro Leu Thr Arg Gly Val Leu Ser Thr Val Ser
                185             190             195

GGT GTC GTG GTC GTG GCA AGT GTC TCA ATC GAC GGC GCA CAA CAG GCG          681
Gly Val Val Val Val Ala Ser Val Ser Ile Asp Gly Ala Gln Gln Ala
            200             205             210

TCG GTC GCG TTG GAC TGG TTG CGC AAC AAC GGT TAC CAA GAT TTG GCG          729
Ser Val Ala Leu Asp Trp Leu Arg Asn Asn Gly Tyr Gln Asp Leu Ala
            215             220             225

AGC CGC GCA TGC GTG GTC ATC AAT CAC ATC ATG CCG GGA GAA CCC AAT          777
Ser Arg Ala Cys Val Val Ile Asn His Ile Met Pro Gly Glu Pro Asn
            230             235             240

GTC GCA GTT AAA GAC CTG GTG CGG CAT TTC GAA CAG CAA GTT CAA CCC          825
Val Ala Val Lys Asp Leu Val Arg His Phe Glu Gln Gln Val Gln Pro
245             250             255             260

GGC CGG GTC GTG GTC ATG CCG TGG GAC AGG CAC ATT GCG GCC GGA ACC          873
Gly Arg Val Val Val Met Pro Trp Asp Arg His Ile Ala Ala Gly Thr
            265             270             275

GAG ATT TCA CTC GAC TTG CTC GAC CCT ATC TAC AAG CGC AAG GTC CTC          921
Glu Ile Ser Leu Asp Leu Leu Asp Pro Ile Tyr Lys Arg Lys Val Leu
            280             285             290

GAA TTG GCC GCA GCG CTA TCC GAC GAT TTC GAG AGG GCT GGA CGT CGT T        970
Glu Leu Ala Ala Ala Leu Ser Asp Asp Phe Glu Arg Ala Gly Arg Arg
            295             300             305

GAGCGCACCT GCTGTTGCTG CTGGTCCTAC                                        1000

(2) INFORMATION FOR SEQ ID NO: 94:

    (i) SEQUENCE CHARACTERISTICS:
        (A) LENGTH: 308 amino acids
        (B) TYPE: amino acid
        (C) STRANDEDNESS: single

144

(D) TOPOLOGY: linear

(ii) MOLECULE TYPE: protein
(v) FRAGMENT TYPE: internal

(xi) SEQUENCE DESCRIPTION: SEQ ID NO: 94:

```
Met Lys Lys Val Lys Pro Gln Lys Pro Lys Ala Thr Lys Pro Pro Lys
 1           5               10              15

Val Val Ser Gln Arg Gly Trp Arg His Trp Val His Ala Leu Thr Arg
            20              25              30

Ile Asn Leu Gly Leu Ser Pro Asp Glu Lys Tyr Glu Leu Asp Leu His
        35              40              45

Ala Arg Val Arg Arg Asn Pro Arg Gly Ser Tyr Gln Ile Ala Val Val
        50              55              60

Gly Leu Lys Gly Gly Ala Gly Lys Thr Thr Leu Thr Ala Ala Leu Gly
 65              70              75              80

Ser Thr Leu Ala Gln Val Arg Ala Asp Arg Ile Leu Ala Leu Asp Ala
                85              90              95

Asp Pro Gly Ala Gly Asn Leu Ala Asp Arg Val Gly Arg Gln Ser Gly
            100             105             110

Ala Thr Ile Ala Asp Val Leu Ala Glu Lys Glu Leu Ser His Tyr Asn
        115             120             125

Asp Ile Arg Ala His Thr Ser Val Asn Ala Val Asn Leu Glu Val Leu
        130             135             140

Pro Ala Pro Glu Tyr Ser Ser Ala Gln Arg Ala Leu Ser Asp Ala Asp
145             150             155             160

Trp His Phe Ile Ala Asp Pro Ala Ser Arg Phe Tyr Asn Leu Val Leu
            165             170             175

Ala Asp Cys Gly Ala Gly Phe Phe Asp Pro Leu Thr Arg Gly Val Leu
            180             185             190

Ser Thr Val Ser Gly Val Val Val Val Ala Ser Val Ser Ile Asp Gly
        195             200             205

Ala Gln Gln Ala Ser Val Ala Leu Asp Trp Leu Arg Asn Asn Gly Tyr
        210             215             220

Gln Asp Leu Ala Ser Arg Ala Cys Val Val Ile Asn His Ile Met Pro
225             230             235             240

Gly Glu Pro Asn Val Ala Val Lys Asp Leu Val Arg His Phe Glu Gln
            245             250             255

Gln Val Gln Pro Gly Arg Val Val Val Met Pro Trp Asp Arg His Ile
            260             265             270

Ala Ala Gly Thr Glu Ile Ser Leu Asp Leu Leu Asp Pro Ile Tyr Lys
            275             280             285
```

```
Arg Lys Val Leu Glu Leu Ala Ala Ala Leu Ser Asp Asp Phe Glu Arg
    290             295             300

Ala Gly Arg Arg
  305
```

(2) INFORMATION FOR SEQ ID NO: 95:

    (i) SEQUENCE CHARACTERISTICS:
        (A) LENGTH: 34 base pairs
        (B) TYPE: nucleic acid
        (C) STRANDEDNESS: single
        (D) TOPOLOGY: linear

    (xi) SEQUENCE DESCRIPTION: SEQ ID NO: 95:

AAGAGTAGAT CTATGATGGC CGAGGATGTT CGCG           34

(2) INFORMATION FOR SEQ ID NO: 96:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (xi) SEQUENCE DESCRIPTION: SEQ ID NO: 96:

CGGCGACGAC GGATCCTACC GCGTCGG           27

(2) INFORMATION FOR SEQ ID NO: 97:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (xi) SEQUENCE DESCRIPTION: SEQ ID NO: 97:

CCTTGGGAGA TCTTTGGACC CCGGTTGC           28

(2) INFORMATION FOR SEQ ID NO: 98:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (xi) SEQUENCE DESCRIPTION: SEQ ID NO: 98:

GACGAGATCT TATGGGCTTA CTGAC                                25

(2) INFORMATION FOR SEQ ID NO: 99:

    (i) SEQUENCE CHARACTERISTICS:
        (A) LENGTH: 33 base pairs
        (B) TYPE: nucleic acid
        (C) STRANDEDNESS: single
        (D) TOPOLOGY: linear


    (xi) SEQUENCE DESCRIPTION: SEQ ID NO: 99:

CCCCCCAGAT CTGCACCACC GGCATCGGCG GGC                        33

(2) INFORMATION FOR SEQ ID NO: 100

    (i) SEQUENCE CHARACTERISTICS:
        (A) LENGTH: 24 base pairs
        (B) TYPE: nucleic acid
        (C) STRANDEDNESS: single
        (D) TOPOLOGY: linear


    (xi) SEQUENCE DESCRIPTION: SEQ ID NO: 100:

GCGGCGGATC CGTTGCTTAG CCGG                                  24

(2) INFORMATION FOR SEQ ID NO: 101:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear


    (xi) SEQUENCE DESCRIPTION: SEQ ID NO: 101:

CCGGCTGAGA TCTATGACAG AATACGAAGG GC                         32

(2) INFORMATION FOR SEQ ID NO: 102:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear


    (xi) SEQUENCE DESCRIPTION: SEQ ID NO: 102:

CCCCGCCAGG GAACTAGAGG CGGC                                  24

(2) INFORMATION FOR SEQ ID NO: 103:

    (i) SEQUENCE CHARACTERISTICS:
        (A) LENGTH: 38 base pairs
        (B) TYPE: nucleic acid
        (C) STRANDEDNESS: single
        (D) TOPOLOGY: linear

(xi) SEQUENCE DESCRIPTION: SEQ ID NO: 103:

CTGCCGAGAT CTACCACCAT TGTCGCGCTG AAATACCC                    38

(2) INFORMATION FOR SEQ ID NO: 104:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (xi) SEQUENCE DESCRIPTION: SEQ ID NO: 104:

CGCCATGGCC TTACGCGCCA ACTCG                                  25

(2) INFORMATION FOR SEQ ID NO: 105:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear

    (xi) SEQUENCE DESCRIPTION: SEQ ID NO: 105:

GGCGGAGATC TGTGAGTTTT CCGTATTTCA TC                          32

(2) INFORMATION FOR SEQ ID NO: 106:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (xi) SEQUENCE DESCRIPTION: SEQ ID NO: 106:

CGCGTCGAGC CATGGTTAGG CGCAG                                  25

(2) INFORMATION FOR SEQ ID NO: 107:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear

    (xi) SEQUENCE DESCRIPTION: SEQ ID NO: 107:

GAGGAAGATC TATGACAACT TCACCCGACC CG                          32

(2) INFORMATION FOR SEQ ID NO: 108:

    (i) SEQUENCE CHARACTERISTICS:

                (A)  LENGTH: 28 base pairs
                (B)  TYPE: nucleic acid
                (C)  STRANDEDNESS: single
                (D)  TOPOLOGY: linear


        (xi) SEQUENCE DESCRIPTION: SEQ ID NO: 108:

CATGAAGCCA TGGCCCGCAG GCTGCATG                                    28

(2) INFORMATION FOR SEQ ID NO: 109:

        (i) SEQUENCE CHARACTERISTICS:
                (A)  LENGTH: 33 base pairs
                (B)  TYPE: nucleic acid
                (C)  STRANDEDNESS: single
                (D)  TOPOLOGY: linear


        (xi) SEQUENCE DESCRIPTION: SEQ ID NO: 109:

GGCCGAGATC TGTGACCCAC TATGACGTCG TCG                              33

(2) INFORMATION FOR SEQ ID NO: 110:

        (i) SEQUENCE CHARACTERISTICS:
                (A)  LENGTH: 36 base pairs
                (B)  TYPE: nucleic acid
                (C)  STRANDEDNESS: single
                (D)  TOPOLOGY: linear


        (xi) SEQUENCE DESCRIPTION: SEQ ID NO: 110:

GGCGCCCATG GTCAGAAATT GATCATGTGG CCAACC                           36

(2) INFORMATION FOR SEQ ID NO: 111:

        (i) SEQUENCE CHARACTERISTICS:
                (A)  LENGTH: 33 base pairs
                (B)  TYPE: nucleic acid
                (C)  STRANDEDNESS: single
                (D)  TOPOLOGY: linear


        (xi) SEQUENCE DESCRIPTION: SEQ ID NO: 111:

CCGGGAGATC TATGGCAAAG CTCTCCACCG ACG                              33

(2) INFORMATION FOR SEQ ID NO: 112:

        (i) SEQUENCE CHARACTERISTICS:
                (A)  LENGTH: 32 base pairs
                (B)  TYPE: nucleic acid
                (C)  STRANDEDNESS: single
                (D)  TOPOLOGY: linear


        (xi) SEQUENCE DESCRIPTION: SEQ ID NO: 112:

CGCTGGGCAG AGCTACTTGA CGGTGACGGT GG                               32

(2) INFORMATION FOR SEQ ID NO: 113:

    (i) SEQUENCE CHARACTERISTICS:
        (A) LENGTH: 36 base pairs
        (B) TYPE: nucleic acid
        (C) STRANDEDNESS: single
        (D) TOPOLOGY: linear


    (xi) SEQUENCE DESCRIPTION: SEQ ID NO: 113:

GGCCCAGATC TATGGCCATT GAGGTTTCGG TGTTGC        36

(2) INFORMATION FOR SEQ ID NO: 114:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear


    (xi) SEQUENCE DESCRIPTION: SEQ ID NO: 114:

CGCCGTGTTG CATGGCAGCG CTGAGC        26


(2) INFORMATION FOR SEQ ID NO: 115:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear


    (xi) SEQUENCE DESCRIPTION: SEQ ID NO: 115:

GGACGTTCAA GCGACACATC GCCG        24

(2) INFORMATION FOR SEQ ID NO: 116:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear


    (xi) SEQUENCE DESCRIPTION: SEQ ID NO: 116:

CAGCACGAAC GCGCCGTCGA TGGC        24

(2) INFORMATION FOR SEQ ID NO: 117:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

(xi) SEQUENCE DESCRIPTION: SEQ ID NO: 117:

ACAGATCTGT GACGGACATG AACCCG                    26

(2) INFORMATION FOR SEQ ID NO: 118:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

(xi) SEQUENCE DESCRIPTION: SEQ ID NO: 118:

TTTTCCATGG TCACGGGCCC CCGGTACT                 28

(2) INFORMATION FOR SEQ ID NO: 119:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

(xi) SEQUENCE DESCRIPTION: SEQ ID NO: 119:

ACAGATCTGT GCCCATGGCA CAGATA                   26

(2) INFORMATION FOR SEQ ID NO: 120:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

(xi) SEQUENCE DESCRIPTION: SEQ ID NO: 120:

TTTAAGCTTC TAGGCGCCCA GCGCGGC                  27

(2) INFORMATION FOR SEQ ID NO: 121:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

(xi) SEQUENCE DESCRIPTION: SEQ ID NO: 121:

ACAGATCTGC GCATGCGGAT CCGTGT                   26

(2) INFORMATION FOR SEQ ID NO: 122:

    (i) SEQUENCE CHARACTERISTICS:

```
          (A) LENGTH: 28 base pairs
          (B) TYPE: nucleic acid
          (C) STRANDEDNESS: single
          (D) TOPOLOGY: linear


     (xi) SEQUENCE DESCRIPTION: SEQ ID NO: 122:

TTTTCCATGG TCATCCGGCG TGATCGAG                              28

(2) INFORMATION FOR SEQ ID NO: 123:

     (i) SEQUENCE CHARACTERISTICS:
          (A) LENGTH: 26 base pairs
          (B) TYPE: nucleic acid
          (C) STRANDEDNESS: single
          (D) TOPOLOGY: linear


     (xi) SEQUENCE DESCRIPTION: SEQ ID NO: 123:

ACAGATCTGT AATGGCAGAC TGTGAT                                26

(2) INFORMATION FOR SEQ ID NO: 124:

     (i) SEQUENCE CHARACTERISTICS:
          (A) LENGTH: 28 base pairs
          (B) TYPE: nucleic acid
          (C) STRANDEDNESS: single
          (D) TOPOLOGY: linear


     (xi) SEQUENCE DESCRIPTION: SEQ ID NO: 124:

TTTTCCATGG TCAGGAGATG GTGATCGA                              28

(2) INFORMATION FOR SEQ ID NO: 125:

     (i) SEQUENCE CHARACTERISTICS:
          (A) LENGTH: 26 base pairs
          (B) TYPE: nucleic acid
          (C) STRANDEDNESS: single
          (D) TOPOLOGY: linear


     (xi) SEQUENCE DESCRIPTION: SEQ ID NO: 125:

ACAGATCTGC CGGCTACCCC GGTGCC                                26

(2) INFORMATION FOR SEQ ID NO: 126:

     (i) SEQUENCE CHARACTERISTICS:
          (A) LENGTH: 28 base pairs
          (B) TYPE: nucleic acid
          (C) STRANDEDNESS: single
          (D) TOPOLOGY: linear


     (xi) SEQUENCE DESCRIPTION: SEQ ID NO: 126:

TTTTCCATGG CTATTGCAGC TTTCCGGC                              28
```

(2) INFORMATION FOR SEQ ID NO: 127:

    (i) SEQUENCE CHARACTERISTICS:
        (A) LENGTH: 50 amino acids
        (B) TYPE: amino acid
        (C) STRANDEDNESS: single
        (D) TOPOLOGY: linear

    (ii) MOLECULE TYPE: None

    (xi) SEQUENCE DESCRIPTION: SEQ ID NO: 127:

```
Ala Glu Asp Val Arg Ala Glu Ile Val Ala Ser Val Leu Glu Val Val
 1               5                  10                  15

Val Asn Glu Gly Asp Gln Ile Asp Lys Gly Asp Val Val Val Leu Leu
            20                  25                  30

Glu Ser Met Tyr Met Glu Ile Pro Val Leu Ala Glu Ala Ala Gly Thr
        35                  40                  45

Val Ser
    50
```

(2) INFORMATION FOR SEQ ID NO: 128:

    (i) SEQUENCE CHARACTERISTICS:
        (A) LENGTH: 49 amino acids
        (B) TYPE: amino acid
        (C) STRANDEDNESS: single
        (D) TOPOLOGY: linear

    (ii) MOLECULE TYPE: None

    (xi) SEQUENCE DESCRIPTION: SEQ ID NO: 128:

```
Ala Glu Asp Val Arg Ala Glu Ile Val Ala Ser Val Leu Glu Val Val
 1               5                  10                  15

Val Asn Glu Gly Asp Gln Ile Asp Lys Gly Asp Val Val Val Leu Leu
            20                  25                  30

Glu Ser Met Met Glu Ile Pro Val Leu Ala Glu Ala Ala Gly Thr Val
        35                  40                  45

Ser
```

(2) INFORMATION FOR SEQ ID NO: 129:

    (i) SEQUENCE CHARACTERISTICS:
        (A) LENGTH: 50 amino acids
        (B) TYPE: amino acid
        (C) STRANDEDNESS: single
        (D) TOPOLOGY: linear

    (ii) MOLECULE TYPE: None

    (xi) SEQUENCE DESCRIPTION: SEQ ID NO: 129:

```
Ala Glu Asp Val Arg Ala Glu Ile Val Ala Ser Val Leu Glu Val Val
```

```
        1            5              10              15

Val Asn Glu Gly Asp Gln Ile Asp Lys Gly Asp Val Val Val Leu Leu
                20                  25                  30

Glu Ser Met Lys Met Glu Ile Pro Val Leu Ala Glu Ala Ala Gly Thr
            35                  40                  45

Val Ser
     50
```

(2) INFORMATION FOR SEQ ID NO: 130:

    (i) SEQUENCE CHARACTERISTICS:
        (A) LENGTH: 33 base pairs
        (B) TYPE: nucleic acid
        (C) STRANDEDNESS: single
        (D) TOPOLOGY: linear

    (xi) SEQUENCE DESCRIPTION: SEQ ID NO: 130:

CCGGGAGATC TATGGCAAAG CTCTCCACCG ACG           33

(2) INFORMATION FOR SEQ ID NO: 131:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear

    (xi) SEQUENCE DESCRIPTION: SEQ ID NO: 131:

CGCTGGGCAG AGCTACTTGA CGGTGACGGT GG           32

(2) INFORMATION FOR SEQ ID NO: 132:

    (i) SEQUENCE CHARACTERISTICS:
        (A) LENGTH: 36 base pairs
        (B) TYPE: nucleic acid
        (C) STRANDEDNESS: single
        (D) TOPOLOGY: linear

    (xi) SEQUENCE DESCRIPTION: SEQ ID NO: 132:

GGCGCCGGCA AGCTTGCCAT GACAGAGCAG CAGTGG        36

(2) INFORMATION FOR SEQ ID NO: 133:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

    (xi) SEQUENCE DESCRIPTION: SEQ ID NO: 133:

CGAACTCGCC GGATCCCGTG TTTCGC                                                  26

(2) INFORMATION FOR SEQ ID NO: 134:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear


    (xi) SEQUENCE DESCRIPTION: SEQ ID NO: 134:

GGCAACCGCG AGATCTTTCT CCCGGCCGGG GC                                           32

(2) INFORMATION FOR SEQ ID NO: 135:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear


    (xi) SEQUENCE DESCRIPTION: SEQ ID NO: 135:

GGCAAGCTTG CCGGCGCCTA ACGAACT                                                 27

(2) INFORMATION FOR SEQ ID NO: 136:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear


    (xi) SEQUENCE DESCRIPTION: SEQ ID NO: 136:

GGACCCAGAT CTATGACAGA GCAGCAGTGG                                              30

(2) INFORMATION FOR SEQ ID NO: 137:

    (i) SEQUENCE CHARACTERISTICS:
        (A) LENGTH: 47 base pairs
        (B) TYPE: nucleic acid
        (C) STRANDEDNESS: single
        (D) TOPOLOGY: linear


    (xi) SEQUENCE DESCRIPTION: SEQ ID NO: 137:

CCGGCAGCCC CGGCCGGGAG AAAAGCTTTG CGAACATCCC AGTGACG                           47

(2) INFORMATION FOR SEQ ID NO: 138:

    (i) SEQUENCE CHARACTERISTICS:
        (A) LENGTH: 44 base pairs
        (B) TYPE: nucleic acid
        (C) STRANDEDNESS: single

(D) TOPOLOGY: linear

(xi) SEQUENCE DESCRIPTION: SEQ ID NO: 138:

GTTCGCAAAG CTTTTCTCCC GGCCGGGGCT GCCGGTCGAG TACC                    44

(2) INFORMATION FOR SEQ ID NO: 139:

     (i) SEQUENCE CHARACTERISTICS:
          (A) LENGTH: 20 base pairs
          (B) TYPE: nucleic acid
          (C) STRANDEDNESS: single
          (D) TOPOLOGY: linear

     (xi) SEQUENCE DESCRIPTION: SEQ ID NO: 139:

CCTTCGGTGG ATCCCGTCAG                                               20

(2) INFORMATION FOR SEQ ID NO: 140:

     (i) SEQUENCE CHARACTERISTICS:
          (A) LENGTH: 450 base pairs
          (B) TYPE: nucleic acid
          (C) STRANDEDNESS: single
          (D) TOPOLOGY: linear

     (ix) FEATURE:

          (A) NAME/KEY: Coding Sequence
          (B) LOCATION: 68...346
          (D) OTHER INFORMATION:

     (xi) SEQUENCE DESCRIPTION: SEQ ID NO: 140:

TGGCGCTGTC ACCGAGGAAC CTGTCAATGT CGTCGAGCAG TACTGAACCG TTCCGAGAAA    60

GGCCAGC ATG AAC GTC ACC GTA TCC ATT CCG ACC ATC CTG CGG CCC CAC    109
       Met Asn Val Thr Val Ser Ile Pro Thr Ile Leu Arg Pro His
        1          5             10

ACC GGC GGC CAG AAG AGT GTC TCG GCC AGC GGC GAT ACC TTG GGT GCC    157
Thr Gly Gly Gln Lys Ser Val Ser Ala Ser Gly Asp Thr Leu Gly Ala
 15          20          25            30

GTC ATC AGC GAC CTG GAG GCC AAC TAT TCG GGC ATT TCC GAG CGC CTG    205
Val Ile Ser Asp Leu Glu Ala Asn Tyr Ser Gly Ile Ser Glu Arg Leu
        35          40            45

ATG GAC CCG TCT TCC CCA GGT AAG TTG CAC CGC TTC GTG AAC ATC TAC    253
Met Asp Pro Ser Ser Pro Gly Lys Leu His Arg Phe Val Asn Ile Tyr
       50          55          60

GTC AAC GAC GAG GAC GTG CGG TTC TCC GGC GGC TTG GCC ACC GCG ATC    301
Val Asn Asp Glu Asp Val Arg Phe Ser Gly Gly Leu Ala Thr Ala Ile
       65          70          75

GCT GAC GGT GAC TCG GTC ACC ATC CTC CCC GCC GTG GCC GGT GGG TGAGC    351
Ala Asp Gly Asp Ser Val Thr Ile Leu Pro Ala Val Ala Gly Gly

156

80                    85                    90

GGAGCACATG ACACGATACG ACTCGCTGTT GCAGGCCTTG GGCAACACGC CGCTGGTTGG    411

CCTGCAGCGA TTGTCGCCAC GCTGGGATGA CGGGCGAGA                            450


(2) INFORMATION FOR SEQ ID NO: 141:

        (i) SEQUENCE CHARACTERISTICS:
            (A) LENGTH: 93 amino acids
            (B) TYPE: amino acid
            (C) STRANDEDNESS: single
            (D) TOPOLOGY: linear

        (ii) MOLECULE TYPE: protein
        (v) FRAGMENT TYPE: internal

        (xi) SEQUENCE DESCRIPTION: SEQ ID NO: 141:

Met Asn Val Thr Val Ser Ile Pro Thr Ile Leu Arg Pro His Thr Gly
 1               5                   10                  15

Gly Gln Lys Ser Val Ser Ala Ser Gly Asp Thr Leu Gly Ala Val Ile
            20                  25                  30

Ser Asp Leu Glu Ala Asn Tyr Ser Gly Ile Ser Glu Arg Leu Met Asp
            35                  40                  45

Pro Ser Ser Pro Gly Lys Leu His Arg Phe Val Asn Ile Tyr Val Asn
        50                  55                  60

Asp Glu Asp Val Arg Phe Ser Gly Gly Leu Ala Thr Ala Ile Ala Asp
    65                  70                  75                  80

Gly Asp Ser Val Thr Ile Leu Pro Ala Val Ala Gly Gly
                85                  90


(2) INFORMATION FOR SEQ ID NO: 142:

        (i) SEQUENCE CHARACTERISTICS:
            (A) LENGTH: 480 base pairs
            (B) TYPE: nucleic acid
            (C) STRANDEDNESS: single
            (D) TOPOLOGY: linear

        (ix) FEATURE:

            (A) NAME/KEY: Coding Sequence
            (B) LOCATION: 88...381
            (D) OTHER INFORMATION:

        (xi) SEQUENCE DESCRIPTION: SEQ ID NO: 142:

GGTGTTCCCG CGGCCGGCTA TGACAACAGT CAATGTGCAT GACAAGTTAC AGGTATTAGG    60

TCCAGGTTCA ACAAGGAGAC AGGCAAC ATG GCA ACA CGT TTT ATG ACG GAT CCG    114
                                Met Ala Thr Arg Phe Met Thr Asp Pro
                                 1                   5

157

```
CAC GCG ATG CGG GAC ATG GCG GGC CGT TTT GAG GTG CAC GCC CAG ACG    162
His Ala Met Arg Asp Met Ala Gly Arg Phe Glu Val His Ala Gln Thr
 10              15                  20                  25

GTG GAG GAC GAG GCT CGC CGG ATG TGG GCG TCC GCG CAA AAC ATC TCG    210
Val Glu Asp Glu Ala Arg Arg Met Trp Ala Ser Ala Gln Asn Ile Ser
                30                  35                  40

GGC GCG GGC TGG AGT GGC ATG GCC GAG GCG ACC TCG CTA GAC ACC ATG    258
Gly Ala Gly Trp Ser Gly Met Ala Glu Ala Thr Ser Leu Asp Thr Met
                45                  50                  55

GCC CAG ATG AAT CAG GCG TTT CGC AAC ATC GTG AAC ATG CTG CAC GGG    306
Ala Gln Met Asn Gln Ala Phe Arg Asn Ile Val Asn Met Leu His Gly
            60                  65                  70

GTG CGT GAC GGG CTG GTT CGC GAC GCC AAC AAC TAC GAG CAG CAA GAG    354
Val Arg Asp Gly Leu Val Arg Asp Ala Asn Asn Tyr Glu Gln Gln Glu
 75                  80                  85

CAG GCC TCC CAG CAG ATC CTC AGC AGC TAACGTCAGC CGCTGCAGCA CAATACT    408
Gln Ala Ser Gln Gln Ile Leu Ser Ser
 90                  95

TTTACAAGCG AAGGAGAACA GGTTCGATGA CCATCAACTA TCAGTTCGGT GATGTCGACG    468

CTCATGGCGC CA    480
```

(2) INFORMATION FOR SEQ ID NO: 143:

    (i) SEQUENCE CHARACTERISTICS:
        (A) LENGTH: 98 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 143:

```
Met Ala Thr Arg Phe Met Thr Asp Pro His Ala Met Arg Asp Met Ala
 1               5                   10                  15

Gly Arg Phe Glu Val His Ala Gln Thr Val Glu Asp Glu Ala Arg Arg
                20                  25                  30

Met Trp Ala Ser Ala Gln Asn Ile Ser Gly Ala Gly Trp Ser Gly Met
                35                  40                  45

Ala Glu Ala Thr Ser Leu Asp Thr Met Ala Gln Met Asn Gln Ala Phe
        50                  55                  60

Arg Asn Ile Val Asn Met Leu His Gly Val Arg Asp Gly Leu Val Arg
 65                  70                  75                  80

Asp Ala Asn Asn Tyr Glu Gln Gln Glu Gln Ala Ser Gln Gln Ile Leu
                85                  90                  95

Ser Ser
```

(2) INFORMATION FOR SEQ ID NO: 144:

    (i) SEQUENCE CHARACTERISTICS:
        (A) LENGTH: 940 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 86...868
        (D) OTHER INFORMATION:

    (xi) SEQUENCE DESCRIPTION: SEQ ID NO: 144:

```
GCCCCAGTCC TCGATCGCCT CATCGCCTTC ACCGGCCGCC AGCCGACCGC AGGCCACGTG        60

TCCGCCACCT AACGAAAGGA TGATC ATG CCC AAG AGA AGC GAA TAC AGG CAA       112
                            Met Pro Lys Arg Ser Glu Tyr Arg Gln
                              1           5

GGC ACG CCG AAC TGG GTC GAC CTT CAG ACC ACC GAT CAG TCC GCC GCC       160
Gly Thr Pro Asn Trp Val Asp Leu Gln Thr Thr Asp Gln Ser Ala Ala
 10              15              20              25

AAA AAG TTC TAC ACA TCG TTG TTC GGC TGG GGT TAC GAC GAC AAC CCG       208
Lys Lys Phe Tyr Thr Ser Leu Phe Gly Trp Gly Tyr Asp Asp Asn Pro
            30              35              40

GTC CCC GGA GGC GGT GGG GTC TAT TCC ATG GCC ACG CTG AAC GGC GAA       256
Val Pro Gly Gly Gly Gly Val Tyr Ser Met Ala Thr Leu Asn Gly Glu
            45              50              55

GCC GTG GCC GCC ATC GCA CCG ATG CCC CCG GGT GCA CCG GAG GGG ATG       304
Ala Val Ala Ala Ile Ala Pro Met Pro Pro Gly Ala Pro Glu Gly Met
            60              65              70

CCG CCG ATC TGG AAC ACC TAT ATC GCG GTG GAC GAC GTC GAT GCG GTG       352
Pro Pro Ile Trp Asn Thr Tyr Ile Ala Val Asp Asp Val Asp Ala Val
            75              80              85

GTG GAC AAG GTG GTG CCC GGG GGC GGG CAG GTG ATG ATG CCG GCC TTC       400
Val Asp Lys Val Val Pro Gly Gly Gly Gln Val Met Met Pro Ala Phe
 90              95              100             105

GAC ATC GGC GAT GCC GGC CGG ATG TCG TTC ATC ACC GAT CCG ACC GGC       448
Asp Ile Gly Asp Ala Gly Arg Met Ser Phe Ile Thr Asp Pro Thr Gly
            110             115             120

GCT GCC GTG GGC CTA TGG CAG GCC AAT CGG CAC ATC GGA GCG ACG TTG       496
Ala Ala Val Gly Leu Trp Gln Ala Asn Arg His Ile Gly Ala Thr Leu
            125             130             135

GTC AAC GAG ACG GGC ACG CTC ATC TGG AAC GAA CTG CTC ACG GAC AAG       544
Val Asn Glu Thr Gly Thr Leu Ile Trp Asn Glu Leu Leu Thr Asp Lys
            140             145             150

CCG GAT TTG GCG CTA GCG TTC TAC GAG GCT GTG GTT GGC CTC ACC CAC       592
```

```
Pro Asp Leu Ala Leu Ala Phe Tyr Glu Ala Val Val Gly Leu Thr His
    155             160             165

TCG AGC ATG GAG ATA GCT GCG GGC CAG AAC TAT CGG GTG CTC AAG GCC          640
Ser Ser Met Glu Ile Ala Ala Gly Gln Asn Tyr Arg Val Leu Lys Ala
170             175             180             185

GGC GAC GCG GAA GTC GGC GGC TGT ATG GAA CCG CCG ATG CCC GGC GTG          688
Gly Asp Ala Glu Val Gly Gly Cys Met Glu Pro Pro Met Pro Gly Val
                190             195             200

CCG AAT CAT TGG CAC GTC TAC TTT GCG GTG GAT GAC GCC GAC GCC ACG          736
Pro Asn His Trp His Val Tyr Phe Ala Val Asp Asp Ala Asp Ala Thr
            205             210             215

GCG GCC AAA GCC GCC GCA GCG GGC GGC CAG GTC ATT GCG GAA CCG GCT          784
Ala Ala Lys Ala Ala Ala Ala Gly Gly Gln Val Ile Ala Glu Pro Ala
        220             225             230

GAC ATT CCG TCG GTG GGC CGG TTC GCC GTG TTG TCC GAT CCG CAG GGC          832
Asp Ile Pro Ser Val Gly Arg Phe Ala Val Leu Ser Asp Pro Gln Gly
        235             240             245

GCG ATC TTC AGT GTG TTG AAG CCC GCA CCG CAG CAA TAGGGAGCAT CCCGGG        884
Ala Ile Phe Ser Val Leu Lys Pro Ala Pro Gln Gln
250             255             260

CAGGCCCGCC GGCCGGCAGA TTCGGAGAAT GCTAGAAGCT GCCGCCGGCG CCGCCG           940
```

(2) INFORMATION FOR SEQ ID NO: 145:

    (i) SEQUENCE CHARACTERISTICS:
        (A) LENGTH: 261 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 145:

```
Met Pro Lys Arg Ser Glu Tyr Arg Gln Gly Thr Pro Asn Trp Val Asp
1               5               10              15

Leu Gln Thr Thr Asp Gln Ser Ala Ala Lys Lys Phe Tyr Thr Ser Leu
            20              25              30

Phe Gly Trp Gly Tyr Asp Asp Asn Pro Val Pro Gly Gly Gly Gly Val
        35              40              45

Tyr Ser Met Ala Thr Leu Asn Gly Glu Ala Val Ala Ala Ile Ala Pro
    50              55              60

Met Pro Pro Gly Ala Pro Glu Gly Met Pro Pro Ile Trp Asn Thr Tyr
65              70              75              80

Ile Ala Val Asp Asp Val Asp Ala Val Val Asp Lys Val Val Pro Gly
            85              90              95

Gly Gly Gln Val Met Met Pro Ala Phe Asp Ile Gly Asp Ala Gly Arg
```

```
                 100                 105                 110
Met Ser Phe Ile Thr Asp Pro Thr Gly Ala Ala Val Gly Leu Trp Gln
        115                 120                 125

Ala Asn Arg His Ile Gly Ala Thr Leu Val Asn Glu Thr Gly Thr Leu
        130                 135                 140

Ile Trp Asn Glu Leu Leu Thr Asp Lys Pro Asp Leu Ala Leu Ala Phe
145                 150                 155                 160

Tyr Glu Ala Val Val Gly Leu Thr His Ser Ser Met Glu Ile Ala Ala
                165                 170                 175

Gly Gln Asn Tyr Arg Val Leu Lys Ala Gly Asp Ala Glu Val Gly Gly
                180                 185                 190

Cys Met Glu Pro Pro Met Pro Gly Val Pro Asn His Trp His Val Tyr
        195                 200                 205

Phe Ala Val Asp Asp Ala Asp Ala Thr Ala Ala Lys Ala Ala Ala Ala
        210                 215                 220

Gly Gly Gln Val Ile Ala Glu Pro Ala Asp Ile Pro Ser Val Gly Arg
225                 230                 235                 240

Phe Ala Val Leu Ser Asp Pro Gln Gly Ala Ile Phe Ser Val Leu Lys
                245                 250                 255

Pro Ala Pro Gln Gln
            260
```

(2) INFORMATION FOR SEQ ID NO: 146:

    (i) SEQUENCE CHARACTERISTICS:
        (A) LENGTH: 280 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 47...247
        (D) OTHER INFORMATION:

    (xi) SEQUENCE DESCRIPTION: SEQ ID NO: 146:

```
CCGAAAGGCG GTGCACCGCA CCCAGAAGAA AAGGAAAGAT CGAGAA ATG CCA CAG      55
                                                 Met Pro Gln
                                                  1

GGA ACT GTG AAG TGG TTC AAC GCG GAG AAG GGG TTC GGC TTT ATC GCC    103
Gly Thr Val Lys Trp Phe Asn Ala Glu Lys Gly Phe Gly Phe Ile Ala
         5                  10                  15

CCC GAA GAC GGT TCC GCG GAT GTA TTT GTC CAC TAC ACG GAG ATC CAG    151
Pro Glu Asp Gly Ser Ala Asp Val Phe Val His Tyr Thr Glu Ile Gln
 20                  25                  30                  35

GGA ACG GGC TTC CGC ACC CTT GAA GAA AAC CAG AAG GTC GAG TTC GAG    199
```

```
Gly Thr Gly Phe Arg Thr Leu Glu Glu Asn Gln Lys Val Glu Phe Glu
            40                  45              50

ATC GGC CAC AGC CCT AAG GGC CCC CAG GCC ACC GGA GTC CGC TCG CTC T    248
Ile Gly His Ser Pro Lys Gly Pro Gln Ala Thr Gly Val Arg Ser Leu
            55                  60              65

GAGTTACCCC CGCGAGCAGA CGCAAAAAGC CC                                  280
```

(2) INFORMATION FOR SEQ ID NO: 147:

    (i) SEQUENCE CHARACTERISTICS:
        (A) LENGTH: 67 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 147:

```
Met Pro Gln Gly Thr Val Lys Trp Phe Asn Ala Glu Lys Gly Phe Gly
 1               5               10              15

Phe Ile Ala Pro Glu Asp Gly Ser Ala Asp Val Phe Val His Tyr Thr
            20                  25              30

Glu Ile Gln Gly Thr Gly Phe Arg Thr Leu Glu Glu Asn Gln Lys Val
        35                  40              45

Glu Phe Glu Ile Gly His Ser Pro Lys Gly Pro Gln Ala Thr Gly Val
    50                  55              60

Arg Ser Leu
 65
```

(2) INFORMATION FOR SEQ ID NO: 148:

    (i) SEQUENCE CHARACTERISTICS:
        (A) LENGTH: 540 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 105...491
        (D) OTHER INFORMATION:

    (xi) SEQUENCE DESCRIPTION: SEQ ID NO: 148:

```
ATCGTGTCGT ATCGAGAACC CCGGCCGGTA TCAGAACGCG CCAGAGCGCA AACCTTTATA    60

ACTTCGTGTC CCAAATGTGA CGACCATGGA CCAAGGTTCC TGAG ATG AAC CTA CGG     116
                                                 Met Asn Leu Arg
                                                  1

CGC CAT CAG ACC CTG ACG CTG CGA CTG CTG GCG GCA TCC GCG GGC ATT     164
Arg His Gln Thr Leu Thr Leu Arg Leu Leu Ala Ala Ser Ala Gly Ile
```

```
         5                    10                    15                    20
CTC AGC GCC GCG GCC TTC GCC GCG CCA GCA CAG GCA AAC CCC GTC GAC          212
Leu Ser Ala Ala Ala Phe Ala Ala Pro Ala Gln Ala Asn Pro Val Asp
                25                    30                    35

GAC GCG TTC ATC GCC GCG CTG AAC AAT GCC GGC GTC AAC TAC GGC GAT          260
Asp Ala Phe Ile Ala Ala Leu Asn Asn Ala Gly Val Asn Tyr Gly Asp
                40                    45                    50

CCG GTC GAC GCC AAA GCG CTG GGT CAG TCC GTC TGC CCG ATC CTG GCC          308
Pro Val Asp Ala Lys Ala Leu Gly Gln Ser Val Cys Pro Ile Leu Ala
                55                    60                    65

GAG CCC GGC GGG TCG TTT AAC ACC GCG GTA GCC AGC GTT GTG GCG CGC          356
Glu Pro Gly Gly Ser Phe Asn Thr Ala Val Ala Ser Val Val Ala Arg
        70                    75                    80

GCC CAA GGC ATG TCC CAG GAC ATG GCG CAA ACC TTC ACC AGT ATC GCG          404
Ala Gln Gly Met Ser Gln Asp Met Ala Gln Thr Phe Thr Ser Ile Ala
85                    90                    95                    100

ATT TCG ATG TAC TGC CCC TCG GTG ATG GCA GAC GTC GCC AGC GGC AAC          452
Ile Ser Met Tyr Cys Pro Ser Val Met Ala Asp Val Ala Ser Gly Asn
                105                   110                   115

CTG CCG GCC CTG CCA GAC ATG CCG GGG CTG CCC GGG TCC TAGGCGTGCG CG        503
Leu Pro Ala Leu Pro Asp Met Pro Gly Leu Pro Gly Ser
                120                   125

GCTCCTAGCC GGTCCCTAAC GGATCGATCG TGGATGC                                 540
```

(2) INFORMATION FOR SEQ ID NO: 149:

    (i) SEQUENCE CHARACTERISTICS:
        (A) LENGTH: 129 amino acids
        (B) TYPE: amino acid
        (C) STRANDEDNESS: single
        (D) TOPOLOGY: linear

    (ii) MOLECULE TYPE: protein
    (v) FRAGMENT TYPE: internal

    (xi) SEQUENCE DESCRIPTION: SEQ ID NO: 149:

```
Met Asn Leu Arg Arg His Gln Thr Leu Thr Leu Arg Leu Leu Ala Ala
1                   5                   10                  15

Ser Ala Gly Ile Leu Ser Ala Ala Ala Phe Ala Ala Pro Ala Gln Ala
                20                  25                  30

Asn Pro Val Asp Asp Ala Phe Ile Ala Ala Leu Asn Asn Ala Gly Val
            35                  40                  45

Asn Tyr Gly Asp Pro Val Asp Ala Lys Ala Leu Gly Gln Ser Val Cys
        50                  55                  60

Pro Ile Leu Ala Glu Pro Gly Gly Ser Phe Asn Thr Ala Val Ala Ser
65                  70                  75                  80

Val Val Ala Arg Ala Gln Gly Met Ser Gln Asp Met Ala Gln Thr Phe
```

```
                  85                    90                    95
Thr Ser Ile Ala Ile Ser Met Tyr Cys Pro Ser Val Met Ala Asp Val
             100                 105                 110

Ala Ser Gly Asn Leu Pro Ala Leu Pro Asp Met Pro Gly Leu Pro Gly
         115             120                 125

Ser
```

(2) INFORMATION FOR SEQ ID NO: 150:

    (i) SEQUENCE CHARACTERISTICS:
        (A) LENGTH: 400 base pairs
        (B) TYPE: nucleic acid
        (C) STRANDEDNESS: single
        (D) TOPOLOGY: linear

    (ix) FEATURE:

        (A) NAME/KEY: Coding Sequence
        (B) LOCATION: 25...354
        (D) OTHER INFORMATION:
    (ix) FEATURE:
        (A) NAME/KEY: mat_peptide
        (B) LOCATION: 109..357

    (xi) SEQUENCE DESCRIPTION: SEQ ID NO: 150:

```
ATAGTTTGGG GAAGGTGTCC ATAA ATG AGG CTG TCG TTG ACC GCA TTG AGC       51
                           Met Arg Leu Ser Leu Thr Ala Leu Ser
                           -28         -25                 -20

GCC GGT GTA GGC GCC GTG GCA ATG TCG TTG ACC GTC GGG GCC GGG GTC      99
Ala Gly Val Gly Ala Val Ala Met Ser Leu Thr Val Gly Ala Gly Val
             -15             -10                 -5

GCC TCC GCA GAT CCC GTG GAC GCG GTC ATT AAC ACC ACC TGC AAT TAC     147
Ala Ser Ala Asp Pro Val Asp Ala Val Ile Asn Thr Thr Cys Asn Tyr
             1           5                 10

GGG CAG GTA GTA GCT GCG CTC AAC GCG ACG GAT CCG GGG GCT GCC GCA     195
Gly Gln Val Val Ala Ala Leu Asn Ala Thr Asp Pro Gly Ala Ala Ala
    15                  20                  25

CAG TTC AAC GCC TCA CCG GTG GCG CAG TCC TAT TTG CGC AAT TTC CTC     243
Gln Phe Asn Ala Ser Pro Val Ala Gln Ser Tyr Leu Arg Asn Phe Leu
  30              35                  40                  45

GCC GCA CCG CCA CCT CAG CGC GCT GCC ATG GCC GCG CAA TTG CAA GCT     291
Ala Ala Pro Pro Pro Gln Arg Ala Ala Met Ala Ala Gln Leu Gln Ala
                50                  55                  60

GTG CCG GGG GCG GCA CAG TAC ATC GGC CTT GTC GAG TCG GTT GCC GGC     339
Val Pro Gly Ala Ala Gln Tyr Ile Gly Leu Val Glu Ser Val Ala Gly
                65                  70                  75

TCC TGC AAC AAC TAT TAAGCCCATG CGGGCCCCAT CCCGCGACCC GGCATCGTCG      394
Ser Cys Asn Asn Tyr
```

## EP 1 484 405 A1

80

CCGGGG     400

(2) INFORMATION FOR SEQ ID NO: 151:

    (i) SEQUENCE CHARACTERISTICS:
        (A) LENGTH: 110 amino acids
        (B) TYPE: amino acid
        (D) TOPOLOGY: linear

    (ii) MOLECULE TYPE: protein

    (xi) SEQUENCE DESCRIPTION: SEQ ID NO: 151:

Met Arg Leu Ser Leu Thr Ala Leu Ser Ala Gly Val Gly Ala Val Ala
-28      -25         -20          -15

Met Ser Leu Thr Val Gly Ala Gly Val Ala Ser Ala Asp Pro Val Asp
    -10         -5          1

Ala Val Ile Asn Thr Thr Cys Asn Tyr Gly Gln Val Val Ala Ala Leu
  5        10        15          20

Asn Ala Thr Asp Pro Gly Ala Ala Ala Gln Phe Asn Ala Ser Pro Val
       25         30         35

Ala Gln Ser Tyr Leu Arg Asn Phe Leu Ala Ala Pro Pro Pro Gln Arg
      40         45        50

Ala Ala Met Ala Ala Gln Leu Gln Ala Val Pro Gly Ala Ala Gln Tyr
      55         60        65

Ile Gly Leu Val Glu Ser Val Ala Gly Ser Cys Asn Asn Tyr
    70         75        80

(2) INFORMATION FOR SEQ ID NO: 152:

    (i) SEQUENCE CHARACTERISTICS:
        (A) LENGTH: 990 base pairs
        (B) TYPE: nucleic acid
        (C) STRANDEDNESS: single
        (D) TOPOLOGY: linear

    (ii) MOLECULE TYPE: cDNA
    (ix) FEATURE:

        (A) NAME/KEY: Coding Sequence
        (B) LOCATION: 93...890
        (D) OTHER INFORMATION:

    (xi) SEQUENCE DESCRIPTION: SEQ ID NO: 152:

AATAGTAATA TCGCTGTGCG GTTGCAAAAC GTGTGACCGA GGTTCCGCAG TCGAGCGCTG     60
CGGGCCGCCT TCGAGGAGGA CGAACCACAG TC ATG ACG AAC ATC GTG GTC CTG     113
                                 Met Thr Asn Ile Val Val Leu
                                  1           5

ATC AAG CAG GTC CCA GAT ACC TGG TCG GAG CGC AAG CTG ACC GAC GGC     161

```
Ile Lys Gln Val Pro Asp Thr Trp Ser Glu Arg Lys Leu Thr Asp Gly
        10                  15                  20

GAT TTC ACG CTG GAC CGC GAG GCC GCC GAC GCG GTG CTG GAC GAG ATC    209
Asp Phe Thr Leu Asp Arg Glu Ala Ala Asp Ala Val Leu Asp Glu Ile
        25                  30                  35

AAC GAG CGC GCC GTG GAG GAA GCG CTA CAG ATT CGG GAG AAA GAG GCC    257
Asn Glu Arg Ala Val Glu Glu Ala Leu Gln Ile Arg Glu Lys Glu Ala
    40                  45                  50                  55

GCC GAC GGC ATC GAA GGG TCG GTA ACC GTG CTG ACG GCG GGC CCC GAG    305
Ala Asp Gly Ile Glu Gly Ser Val Thr Val Leu Thr Ala Gly Pro Glu
                    60                  65                  70

CGC GCC ACC GAG GCG ATC CGC AAG GCG CTG TCG ATG GGT GCC GAC AAG    353
Arg Ala Thr Glu Ala Ile Arg Lys Ala Leu Ser Met Gly Ala Asp Lys
            75                  80                  85

GCC GTC CAC CTA AAG GAC GAC GGC ATG CAC GGC TCG GAC GTC ATC CAA    401
Ala Val His Leu Lys Asp Asp Gly Met His Gly Ser Asp Val Ile Gln
        90                  95                  100

ACC GGG TGG GCT TTG GCG CGC GCG TTG GGC ACC ATC GAG GGC ACC GAG    449
Thr Gly Trp Ala Leu Ala Arg Ala Leu Gly Thr Ile Glu Gly Thr Glu
        105                 110                 115

CTG GTG ATC GCA GGC AAC GAA TCG ACC GAC GGG GTG GGC GGT GCG GTG    497
Leu Val Ile Ala Gly Asn Glu Ser Thr Asp Gly Val Gly Gly Ala Val
120                 125                 130                 135

CCG GCC ATC ATC GCC GAG TAC CTG GGC CTG CCG CAG CTC ACC CAC CTG    545
Pro Ala Ile Ile Ala Glu Tyr Leu Gly Leu Pro Gln Leu Thr His Leu
                140                 145                 150

CGC AAA GTG TCG ATC GAG GGC GGC AAG ATC ACC GGC GAG CGT GAG ACC    593
Arg Lys Val Ser Ile Glu Gly Gly Lys Ile Thr Gly Glu Arg Glu Thr
            155                 160                 165

GAT GAG GGC GTA TTC ACC CTC GAG GCC ACG CTG CCC GCG GTG ATC AGC    641
Asp Glu Gly Val Phe Thr Leu Glu Ala Thr Leu Pro Ala Val Ile Ser
        170                 175                 180

GTG AAC GAG AAG ATC AAC GAG CCG CGC TTC CCG TCC TTC AAA GGC ATC    689
Val Asn Glu Lys Ile Asn Glu Pro Arg Phe Pro Ser Phe Lys Gly Ile
        185                 190                 195

ATG GCC GCC AAG AAG AAG GAA GTT ACC GTG CTG ACC CTG GCC GAG ATC    737
Met Ala Ala Lys Lys Lys Glu Val Thr Val Leu Thr Leu Ala Glu Ile
200                 205                 210                 215

GGT GTC GAG AGC GAC GAG GTG GGG CTG GCC AAC GCC GGA TCC ACC GTG    785
Gly Val Glu Ser Asp Glu Val Gly Leu Ala Asn Ala Gly Ser Thr Val
                220                 225                 230

CTG GCG TCG ACG CCC AAA CCG GCC AAG ACT GCC GGG GAG AAG GTC ACC    833
Leu Ala Ser Thr Pro Lys Pro Ala Lys Thr Ala Gly Glu Lys Val Thr
            235                 240                 245

GAC GAG GGT GAA GGC GGC AAC CAG ATC GTG CAG TAC CTG GTT GCC CAG    881
Asp Glu Gly Glu Gly Gly Asn Gln Ile Val Gln Tyr Leu Val Ala Gln
```

```
                250                 255                 260

AAA ATC ATC TAAGACATAC GCACCTCCCA AAGACGAGAG CGATATAACC CATGGCTGA    939
Lys Ile Ile
    265


AGTACTGGTG CTCGTTGAGC ACGCTGAAGG CGCGTTAAAG AAGGTCAGCG C             990
```

(2) INFORMATION FOR SEQ ID NO: 153:

    (i) SEQUENCE CHARACTERISTICS:
        (A) LENGTH: 266 amino acids
        (B) TYPE: amino acid
        (C) STRANDEDNESS: single
        (D) TOPOLOGY: linear

    (ii) MOLECULE TYPE: protein
    (v) FRAGMENT TYPE: internal
    (xi) SEQUENCE DESCRIPTION: SEQ ID NO: 153:

```
Met Thr Asn Ile Val Val Leu Ile Lys Gln Val Pro Asp Thr Trp Ser
  1               5                  10                  15

Glu Arg Lys Leu Thr Asp Gly Asp Phe Thr Leu Asp Arg Glu Ala Ala
            20                  25                  30

Asp Ala Val Leu Asp Glu Ile Asn Glu Arg Ala Val Glu Glu Ala Leu
            35                  40                  45

Gln Ile Arg Glu Lys Glu Ala Ala Asp Gly Ile Glu Gly Ser Val Thr
      50                  55                  60

Val Leu Thr Ala Gly Pro Glu Arg Ala Thr Glu Ala Ile Arg Lys Ala
  65                  70                  75                  80

Leu Ser Met Gly Ala Asp Lys Ala Val His Leu Lys Asp Asp Gly Met
                85                  90                  95

His Gly Ser Asp Val Ile Gln Thr Gly Trp Ala Leu Ala Arg Ala Leu
            100                 105                 110

Gly Thr Ile Glu Gly Thr Glu Leu Val Ile Ala Gly Asn Glu Ser Thr
            115                 120                 125

Asp Gly Val Gly Gly Ala Val Pro Ala Ile Ile Ala Glu Tyr Leu Gly
      130                 135                 140

Leu Pro Gln Leu Thr His Leu Arg Lys Val Ser Ile Glu Gly Gly Lys
145                 150                 155                 160

Ile Thr Gly Glu Arg Glu Thr Asp Glu Gly Val Phe Thr Leu Glu Ala
                165                 170                 175

Thr Leu Pro Ala Val Ile Ser Val Asn Glu Lys Ile Asn Glu Pro Arg
            180                 185                 190

Phe Pro Ser Phe Lys Gly Ile Met Ala Ala Lys Lys Lys Glu Val Thr
            195                 200                 205

Val Leu Thr Leu Ala Glu Ile Gly Val Glu Ser Asp Glu Val Gly Leu
      210                 215                 220
```

```
Ala Asn Ala Gly Ser Thr Val Leu Ala Ser Thr Pro Lys Pro Ala Lys
225                 230             235                 240

Thr Ala Gly Glu Lys Val Thr Asp Glu Gly Glu Gly Gly Asn Gln Ile
            245             250             255

Val Gln Tyr Leu Val Ala Gln Lys Ile Ile
            260             265
```

(2) INFORMATION FOR SEQ ID NO: 154:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (xi) SEQUENCE DESCRIPTION: SEQ ID NO: 154:

CTGAGATCTA TGAACCTACG GCGCC               25

(2) INFORMATION FOR SEQ ID NO: 155:

    (i) SEQUENCE CHARACTERISTICS:
        (A) LENGTH: 35 base pairs
        (B) TYPE: nucleic acid
        (C) STRANDEDNESS: single
        (D) TOPOLOGY: linear

    (xi) SEQUENCE DESCRIPTION: SEQ ID NO: 155:

CTCCCATGGT ACCCTAGGAC CCGGGCAGCC CCGGC        35

(2) INFORMATION FOR SEQ ID NO: 156:

    (i) SEQUENCE CHARACTERISTICS:
        (A) LENGTH: 29 base pairs
        (B) TYPE: nucleic acid
        (C) STRANDEDNESS: single
        (D) TOPOLOGY: linear

    (xi) SEQUENCE DESCRIPTION: SEQ ID NO: 156:

CTGAGATCTA TGAGGCTGTC GTTGACCGC          29

(2) INFORMATION FOR SEQ ID NO: 157:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (xi) SEQUENCE DESCRIPTION: SEQ ID NO: 157:

CTCCCCGGGC TTAATAGTTG TTGCAGGAGC         30

(2) INFORMATION FOR SEQ ID NO: 158:

    (i) SEQUENCE CHARACTERISTICS:
        (A) LENGTH: 33 base pairs
        (B) TYPE: nucleic acid
        (C) STRANDEDNESS: single
        (D) TOPOLOGY: linear

    (xi) SEQUENCE DESCRIPTION: SEQ ID NO: 158:

GCTTAGATCT ATGATTTTCT GGGCAACCAG GTA            33

(2) INFORMATION FOR SEQ ID NO: 159:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (xi) SEQUENCE DESCRIPTION: SEQ ID NO: 159:

GCTTCCATGG GCGAGGCACA GGCGTGGGAA            30

(2) INFORMATION FOR SEQ ID NO: 160:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (xi) SEQUENCE DESCRIPTION: SEQ ID NO: 160:

CTGAGATCTA GAATGCCACA GGGAACTGTG            30

(2) INFORMATION FOR SEQ ID NO: 161:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (xi) SEQUENCE DESCRIPTION: SEQ ID NO: 161:

TCTCCCGGGG GTAACTCAGA GCGAGCGGAC            30

(2) INFORMATION FOR SEQ ID NO: 162:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

(xi) SEQUENCE DESCRIPTION: SEQ ID NO: 162:

CTGAGATCTA TGAACGTCAC CGTATCC                                    27

(2) INFORMATION FOR SEQ ID NO: 163:

    (i) SEQUENCE CHARACTERISTICS:
       (A) LENGTH: 27 base pairs
       (B) TYPE: nucleic acid
       (C) STRANDEDNESS: single
       (D) TOPOLOGY: linear

    (xi) SEQUENCE DESCRIPTION: SEQ ID NO: 163:

TCTCCCGGGG CTCACCCACC GGCCACG                                    27

(2) INFORMATION FOR SEQ ID NO: 164:

    (i) SEQUENCE CHARACTERISTICS:
       (A) LENGTH: 30 base pairs
       (B) TYPE: nucleic acid
       (C) STRANDEDNESS: single
       (D) TOPOLOGY: linear

    (xi) SEQUENCE DESCRIPTION: SEQ ID NO: 164:

CTGAGATCTA TGGCAACACG TTTTATGACG                                 30

(2) INFORMATION FOR SEQ ID NO: 165:

    (i) SEQUENCE CHARACTERISTICS:
       (A) LENGTH: 30 base pairs
       (B) TYPE: nucleic acid
       (C) STRANDEDNESS: single
       (D) TOPOLOGY: linear

    (xi) SEQUENCE DESCRIPTION: SEQ ID NO: 165:

CTCCCCGGGT TAGCTGCTGA GGATCTGCTH                                 30

(2) INFORMATION FOR SEQ ID NO: 166:

    (i) SEQUENCE CHARACTERISTICS:
       (A) LENGTH: 31 base pairs
       (B) TYPE: nucleic acid
       (C) STRANDEDNESS: single
       (D) TOPOLOGY: linear

    (xi) SEQUENCE DESCRIPTION: SEQ ID NO: 166:

CTGAAGATCT ATGCCCAAGA GAAGCGAATA C                               31

(2) INFORMATION FOR SEQ ID NO: 167:

    (i) SEQUENCE CHARACTERISTICS:
       (A) LENGTH: 31 base pairs

(B) TYPE: nucleic acid
(C) STRANDEDNESS: single
(D) TOPOLOGY: linear

(xi) SEQUENCE DESCRIPTION: SEQ ID NO: 167:

CGGCAGCTGC TAGCATTCTC CGAATCTGCC G                                      31

(2) INFORMATION FOR SEQ ID NO: 168:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: None

    (xi) SEQUENCE DESCRIPTION: SEQ ID NO: 168:

Pro Gln Gly Thr Val Lys Trp Phe Asn Ala Glu Lys Gly Phe Gly
  1           5             10           15

(2) INFORMATION FOR SEQ ID NO: 169:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: None

    (ix) FEATURE:
        (A) NAME/KEY: Other
        (B) LOCATION: 15
        (D) OTHER INFORMATION: Xaa is unknown

    (xi) SEQUENCE DESCRIPTION: SEQ ID NO: 169:

Asn Val Thr Val Ser Ile Pro Thr Ile Leu Arg Pro Xaa Xaa Xaa
  1           5             10           15

(2) INFORMATION FOR SEQ ID NO: 170:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: None

    (ix) FEATURE:

        (A) NAME/KEY: Other
        (B) LOCATION: 1
        (D) OTHER INFORMATION: Thr Could also be Ala

    (xi) SEQUENCE DESCRIPTION: SEQ ID NO: 170:

```
Thr Arg Phe Met Thr Asp Pro His Ala Met Arg Asp Met Ala Gly
 1               5                  10                  15
```

(2) INFORMATION FOR SEQ ID NO: 171:

    (i) SEQUENCE CHARACTERISTICS:
      (A) LENGTH: 15 amino acids
      (B) TYPE: amino acid
      (C) STRANDEDNESS: single
      (D) TOPOLOGY: linear

    (ii) MOLECULE TYPE: None

    (xi) SEQUENCE DESCRIPTION: SEQ ID NO: 171:

```
Pro Lys Arg Ser Glu Tyr Arg Gln Gly Thr Pro Asn Trp Val Asp
1         5           10          15
```

(2) INFORMATION FOR SEQ ID NO:172:

    (i) SEQUENCE CHARACTERISTICS:
      (A) LENGTH: 404 amino acids
      (B) TYPE: amino acid
      (C) STRANDEDNESS: single
      (D) TOPOLOGY: linear

    (xi) SEQUENCE DESCRIPTION: SEQ ID NO:172:

```
Met Ala Thr Val Asn Arg Ser Arg His His His His His His His His
 1               5                  10                  15
Ile Glu Gly Arg Ser Phe Ser Arg Pro Gly Leu Pro Val Glu Tyr Leu
            20                  25                  30
Gln Val Pro Ser Pro Ser Met Gly Arg Asp Ile Lys Val Gln Phe Gln
            35                  40                  45
Ser Gly Gly Asn Asn Ser Pro Ala Val Tyr Leu Leu Asp Gly Leu Arg
         50                  55                  60
Ala Gln Asp Asp Tyr Asn Gly Trp Asp Ile Asn Thr Pro Ala Phe Glu
65                  70                  75                  80
Trp Tyr Tyr Gln Ser Gly Leu Ser Ile Val Met Pro Val Gly Gly Gln
                85                  90                  95
Ser Ser Phe Tyr Ser Asp Trp Tyr Ser Pro Ala Cys Gly Lys Ala Gly
            100                 105                 110
Cys Gln Thr Tyr Lys Trp Glu Thr Phe Leu Thr Ser Glu Leu Pro Gln
            115                 120                 125
Trp Leu Ser Ala Asn Arg Ala Val Lys Pro Thr Gly Ser Ala Ala Ile
    130                 135                 140
Gly Leu Ser Met Ala Gly Ser Ser Ala Met Ile Leu Ala Ala Tyr His
145                 150                 155                 160
Pro Gln Gln Phe Ile Tyr Ala Gly Ser Leu Ser Ala Leu Leu Asp Pro
                165                 170                 175
Ser Gln Gly Met Gly Pro Ser Leu Ile Gly Leu Ala Met Gly Asp Ala
            180                 185                 190
Gly Gly Tyr Lys Ala Ala Asp Met Trp Gly Pro Ser Ser Asp Pro Ala
            195                 200                 205
Trp Glu Arg Asn Asp Pro Thr Gln Gln Ile Pro Lys Leu Val Ala Asn
    210                 215                 220
Asn Thr Arg Leu Trp Val Tyr Cys Gly Asn Gly Thr Pro Asn Glu Leu
225                 230                 235                 240
Gly Gly Ala Asn Ile Pro Ala Glu Phe Leu Glu Asn Phe Val Arg Ser
```

```
                    245                    250                    255
     Ser Asn Leu Lys Phe Gln Asp Ala Tyr Asn Ala Ala Gly Gly His Asn
                 260                    265                    270
     Ala Val Phe Asn Phe Pro Pro Asn Gly Thr His Ser Trp Glu Tyr Trp
                 275                    280                    285
     Gly Ala Gln Leu Asn Ala Met Lys Gly Asp Leu Gln Ser Ser Leu Gly
                 290                    295                    300
     Ala Gly Lys Leu Ala Met Thr Glu Gln Gln Trp Asn Phe Ala Gly Ile
     305                    310                    315                    320
     Glu Ala Ala Ala Ser Ala Ile Gln Gly Asn Val Thr Ser Ile His Ser
                    325                    330                    335
     Leu Leu Asp Glu Gly Lys Gln Ser Leu Thr Lys Leu Ala Ala Ala Trp
                 340                    345                    350
     Gly Gly Ser Gly Ser Glu Ala Tyr Gln Gly Val Gln Gln Lys Trp Asp
                 355                    360                    365
     Ala Thr Ala Thr Glu Leu Asn Asn Ala Leu Gln Asn Leu Ala Arg Thr
                 370                    375                    380
     Ile Ser Glu Ala Gly Gln Ala Met Ala Ser Thr Glu Gly Asn Val Thr
     385                    390                    395                    400
     Gly Met Phe Ala
```

(2) INFORMATION FOR SEQ ID NO:173:

    (i) SEQUENCE CHARACTERISTICS:
      (A) LENGTH: 403 amino acids
      (B) TYPE: amino acid
      (C) STRANDEDNESS: single
      (D) TOPOLOGY: linear


    (xi) SEQUENCE DESCRIPTION: SEQ ID NO:173:

```
     Met Ala Thr Val Asn Arg Ser Arg His His His His His His His His
     1                  5                      10                     15
     Ile Glu Gly Arg Ser Met Thr Glu Gln Gln Trp Asn Phe Ala Gly Ile
                 20                     25                     30
     Glu Ala Ala Ala Ser Ala Ile Gln Gly Asn Val Thr Ser Ile His Ser
                 35                     40                     45
     Leu Leu Asp Glu Gly Lys Gln Ser Leu Thr Lys Leu Ala Ala Ala Trp
              50                     55                     60
     Gly Gly Ser Gly Ser Glu Ala Tyr Gln Gly Val Gln Gln Lys Trp Asp
     65                     70                     75                     80
     Ala Thr Ala Thr Glu Leu Asn Asn Ala Leu Gln Asn Leu Ala Arg Thr
                    85                     90                     95
     Ile Ser Glu Ala Gly Gln Ala Met Ala Ser Thr Glu Gly Asn Val Thr
                 100                    105                    110
     Gly Met Phe Ala Lys Leu Phe Ser Arg Pro Gly Leu Pro Val Glu Tyr
                 115                    120                    125
     Leu Gln Val Pro Ser Pro Ser Met Gly Arg Asp Ile Lys Val Gln Phe
                 130                    135                    140
     Gln Ser Gly Gly Asn Asn Ser Pro Ala Val Tyr Leu Leu Asp Gly Leu
     145                    150                    155                    160
     Arg Ala Gln Asp Asp Tyr Asn Gly Trp Asp Ile Asn Thr Pro Ala Phe
                 165                    170                    175
     Glu Trp Tyr Tyr Gln Ser Gly Leu Ser Ile Val Met Pro Val Gly Gly
                 180                    185                    190
     Gln Ser Ser Phe Tyr Ser Asp Trp Tyr Ser Pro Ala Cys Gly Lys Ala
                 195                    200                    205
     Gly Cys Gln Thr Tyr Lys Trp Glu Thr Phe Leu Thr Ser Glu Leu Pro
                 210                    215                    220
```

```
Gln Trp Leu Ser Ala Asn Arg Ala Val Lys Pro Thr Gly Ser Ala Ala
225                 230                 235                 240
Ile Gly Leu Ser Met Ala Gly Ser Ser Ala Met Ile Leu Ala Ala Tyr
                245                 250                 255
His Pro Gln Gln Phe Ile Tyr Ala Gly Ser Leu Ser Ala Leu Leu Asp
            260                 265                 270
Pro Ser Gln Gly Met Gly Pro Ser Leu Ile Gly Leu Ala Met Gly Asp
        275                 280                 285
Ala Gly Gly Tyr Lys Ala Ala Asp Met Trp Gly Pro Ser Ser Asp Pro
    290                 295                 300
Ala Trp Glu Arg Asn Asp Pro Thr Gln Gln Ile Pro Lys Leu Val Ala
305                 310                 315                 320
Asn Asn Thr Arg Leu Trp Val Tyr Cys Gly Asn Gly Thr Pro Asn Glu
                325                 330                 335
Leu Gly Gly Ala Asn Ile Pro Ala Glu Phe Leu Glu Asn Phe Val Arg
            340                 345                 350
Ser Ser Asn Leu Lys Phe Gln Asp Ala Tyr Asn Ala Ala Gly Gly His
        355                 360                 365
Asn Ala Val Phe Asn Phe Pro Pro Asn Gly Thr His Ser Trp Glu Tyr
    370                 375                 380
Trp Gly Ala Gln Leu Asn Ala Met Lys Gly Asp Leu Gln Ser Ser Leu
385                 390                 395                 400
Gly Ala Gly
```

(2) INFORMATION FOR SEQ ID NO:174:

          (i) SEQUENCE CHARACTERISTICS:
              (A) LENGTH: 291 base pairs
              (B) TYPE: nucleic acid
              (C) STRANDEDNESS: single
              (D) TOPOLOGY: linear

          (ix) FEATURE:

              (A) NAME/KEY: Coding Sequence
              (B) LOCATION: 1...288
              (D) OTHER INFORMATION:

          (xi) SEQUENCE DESCRIPTION: SEQ ID NO:174:

```
ATG TCG CAG ATT ATG TAC AAC TAT CCG GCG ATG ATG GCT CAT GCC GGG        48
Met Ser Gln Ile Met Tyr Asn Tyr Pro Ala Met Met Ala His Ala Gly
 1               5                  10                  15

GAC ATG GCC GGT TAT GCG GGC ACG CTG CAG AGC TTG GGG GCC GAT ATC        96
Asp Met Ala Gly Tyr Ala Gly Thr Leu Gln Ser Leu Gly Ala Asp Ile
            20                  25                  30

GCC AGT GAG CAG GCC GTG CTG TCC AGT GCT TGG CAG GGT GAT ACC GGG       144
Ala Ser Glu Gln Ala Val Leu Ser Ser Ala Trp Gln Gly Asp Thr Gly
        35                  40                  45

ATC ACG TAT CAG GGC TGG CAG ACC CAG TGG AAC CAG GCC CTA GAG GAT       192
Ile Thr Tyr Gln Gly Trp Gln Thr Gln Trp Asn Gln Ala Leu Glu Asp
        50                  55                  60

CTG GTG CGG GCC TAT CAG TCG ATG TCT GGC ACC CAT GAG TCC AAC ACC       240
Leu Val Arg Ala Tyr Gln Ser Met Ser Gly Thr His Glu Ser Asn Thr
65                  70                  75                  80

ATG GCG ATG TTG GCT CGA GAT GGG GCC GAA GCC GCC AAG TGG GGC GGC T     289
```

```
Met Ala Met Leu Ala Arg Asp Gly Ala Glu Ala Ala Lys Trp Gly Gly
            85                  90                  95

AG                                                              291
```

(2) INFORMATION FOR SEQ ID NO:175:

    (i) SEQUENCE CHARACTERISTICS:
     (A) LENGTH: 96 amino acids
     (B) TYPE: amino acid
     (C) STRANDEDNESS: single
     (D) TOPOLOGY: linear

    (ii) MOLECULE TYPE: protein
    (v) FRAGMENT TYPE: internal

    (xi) SEQUENCE DESCRIPTION: SEQ ID NO:175:

```
Met Ser Gln Ile Met Tyr Asn Tyr Pro Ala Met Met Ala His Ala Gly
1               5               10              15
Asp Met Ala Gly Tyr Ala Gly Thr Leu Gln Ser Leu Gly Ala Asp Ile
            20              25              30
Ala Ser Glu Gln Ala Val Leu Ser Ser Ala Trp Gln Gly Asp Thr Gly
        35              40              45
Ile Thr Tyr Gln Gly Trp Gln Thr Gln Trp Asn Gln Ala Leu Glu Asp
        50              55              60
Leu Val Arg Ala Tyr Gln Ser Met Ser Gly Thr His Glu Ser Asn Thr
65              70              75              80
Met Ala Met Leu Ala Arg Asp Gly Ala Glu Ala Ala Lys Trp Gly Gly
            85              90              95
```

    (2) INFORMATION FOR SEQ ID NO:176:

    (i) SEQUENCE CHARACTERISTICS:
     (A) LENGTH: 363 base pairs
     (B) TYPE: nucleic acid
     (C) STRANDEDNESS: single
     (D) TOPOLOGY: linear

    (ii) MOLECULE TYPE: cDNA
    (ix) FEATURE:

     (A) NAME/KEY: Coding Sequence
     (B) LOCATION: 1...360
     (D) OTHER INFORMATION:

    (xi) SEQUENCE DESCRIPTION: SEQ ID NO:176:

```
GTG TCG CAG AGT ATG TAC AGC TAC CCG GCG ATG ACG GCC AAT GTC GGA    48
Met Ser Gln Ser Met Tyr Ser Tyr Pro Ala Met Thr Ala Asn Val Gly
1               5               10              15

GAC ATG GCC GGT TAT ACG GGC ACG ACG CAG AGC TTG GGG GCC GAT ATC    96
Asp Met Ala Gly Tyr Thr Gly Thr Thr Gln Ser Leu Gly Ala Asp Ile
            20              25              30

GCC AGT GAG CGC ACC GCG CCG TCG CGT GCT TGC CAA GGT GAT CTC GGG   144
Ala Ser Glu Arg Thr Ala Pro Ser Arg Ala Cys Gln Gly Asp Leu Gly
            35              40              45

ATG AGT CAT CAG GAC TGG CAG GCC CAG TGG AAT CAG GCC ATG GAG GCT   192
```

```
            Met Ser His Gln Asp Trp Gln Ala Gln Trp Asn Gln Ala Met Glu Ala
                50              55              60

CTC GCG CGG GCC TAC CGT CGG TGC CGG CGA GCA CTA CGC CAG ATC GGG       240
Leu Ala Arg Ala Tyr Arg Arg Cys Arg Arg Ala Leu Arg Gln Ile Gly
65              70              75              80

GTG CTG GAA AGG CCG GTA GGC GAT TCG TCA GAC TGC GGA ACG ATT AGG       288
Val Leu Glu Arg Pro Val Gly Asp Ser Ser Asp Cys Gly Thr Ile Arg
                85              90              95

GTG GGG TCG TTC CGG GGT CGG TGG CTG GAC CCG CGC CAT GCG GGT CCA       336
Val Gly Ser Phe Arg Gly Arg Trp Leu Asp Pro Arg His Ala Gly Pro
                100             105             110

GCC ACG GCC GCC GAC GCC GGA GAC TAA                                   363
Ala Thr Ala Ala Asp Ala Gly Asp
                115             120
```

(2) INFORMATION FOR SEQ ID NO:177:

    (i) SEQUENCE CHARACTERISTICS:
      (A) LENGTH: 120 amino acids
      (B) TYPE: amino acid
      (C) STRANDEDNESS: single
      (D) TOPOLOGY: linear

    (ii) MOLECULE TYPE: protein
    (v) FRAGMENT TYPE: internal

    (xi) SEQUENCE DESCRIPTION: SEQ ID NO:177:

```
Met Ser Gln Ser Met Tyr Ser Tyr Pro Ala Met Thr Ala Asn Val Gly
1               5               10              15
Asp Met Ala Gly Tyr Thr Gly Thr Thr Gln Ser Leu Gly Ala Asp Ile
                20              25              30
Ala Ser Glu Arg Thr Ala Pro Ser Arg Ala Cys Gln Gly Asp Leu Gly
        35              40              45
Met Ser His Gln Asp Trp Gln Ala Gln Trp Asn Gln Ala Met Glu Ala
    50              55              60
Leu Ala Arg Ala Tyr Arg Arg Cys Arg Arg Ala Leu Arg Gln Ile Gly
65              70              75              80
Val Leu Glu Arg Pro Val Gly Asp Ser Ser Asp Cys Gly Thr Ile Arg
                85              90              95
Val Gly Ser Phe Arg Gly Arg Trp Leu Asp Pro Arg His Ala Gly Pro
                100             105             110
Ala Thr Ala Ala Asp Ala Gly Asp
        115             120
```

(2) INFORMATION FOR SEQ ID NO:178:

    (i) SEQUENCE CHARACTERISTICS:
      (A) LENGTH: 297 base pairs
      (B) TYPE: nucleic acid
      (C) STRANDEDNESS: single
      (D) TOPOLOGY: linear

    (ix) FEATURE:

      (A) NAME/KEY: Coding Sequence

(B) LOCATION: 1...294
(D) OTHER INFORMATION:

(xi) SEQUENCE DESCRIPTION: SEQ ID NO:178:

```
ATG GCC TCG CGT TTT ATG ACG GAT CCG CAC GCG ATG CGG GAC ATG GCG      48
Met Ala Ser Arg Phe Met Thr Asp Pro His Ala Met Arg Asp Met Ala
 1               5                  10                  15

GGC CGT TTT GAG GTG CAC GCC CAG ACG GTG GAG GAC GAG GCT CGC CGG      96
Gly Arg Phe Glu Val His Ala Gln Thr Val Glu Asp Glu Ala Arg Arg
            20                  25                  30

ATG TGG GCG TCC GCG CAA AAC ATC TCG GGC GCG GGC TGG AGT GGC ATG     144
Met Trp Ala Ser Ala Gln Asn Ile Ser Gly Ala Gly Trp Ser Gly Met
        35                  40                  45

GCC GAG GCG ACC TCG CTA GAC ACC ATG ACC CAG ATG AAT CAG GCG TTT     192
Ala Glu Ala Thr Ser Leu Asp Thr Met Thr Gln Met Asn Gln Ala Phe
    50                  55                  60

CGC AAC ATC GTG AAC ATG CTG CAC GGG GTG CGT GAC GGG CTG GTT CGC     240
Arg Asn Ile Val Asn Met Leu His Gly Val Arg Asp Gly Leu Val Arg
65                  70                  75                  80

GAC GCC AAC AAC TAC GAA CAG CAA GAG CAG GCC TCC CAG CAG ATC CTC     288
Asp Ala Asn Asn Tyr Glu Gln Gln Glu Gln Ala Ser Gln Gln Ile Leu
                85                  90                  95

AGC AGC TGA                                                         297
Ser Ser
```

(2) INFORMATION FOR SEQ ID NO:179:

(i) SEQUENCE CHARACTERISTICS:
    (A) LENGTH: 98 amino acids
    (B) TYPE: amino acid
    (C) STRANDEDNESS: single
    (D) TOPOLOGY: linear

(ii) MOLECULE TYPE: protein
(v) FRAGMENT TYPE: internal

(xi) SEQUENCE DESCRIPTION: SEQ ID NO:179:

```
Met Ala Ser Arg Phe Met Thr Asp Pro His Ala Met Arg Asp Met Ala
 1               5                  10                  15
Gly Arg Phe Glu Val His Ala Gln Thr Val Glu Asp Glu Ala Arg Arg
            20                  25                  30
Met Trp Ala Ser Ala Gln Asn Ile Ser Gly Ala Gly Trp Ser Gly Met
        35                  40                  45
Ala Glu Ala Thr Ser Leu Asp Thr Met Thr Gln Met Asn Gln Ala Phe
    50                  55                  60
Arg Asn Ile Val Asn Met Leu His Gly Val Arg Asp Gly Leu Val Arg
65                  70                  75                  80
Asp Ala Asn Asn Tyr Glu Gln Gln Glu Gln Ala Ser Gln Gln Ile Leu
                85                  90                  95
Ser Ser
```

(2) INFORMATION FOR SEQ ID NO:180:

(i) SEQUENCE CHARACTERISTICS:
    (A) LENGTH: 297 base pairs
    (B) TYPE: nucleic acid
    (C) STRANDEDNESS: single
    (D) TOPOLOGY: linear

(ix) FEATURE:

    (A) NAME/KEY: Coding Sequence
    (B) LOCATION: 1...294
    (D) OTHER INFORMATION:

(xi) SEQUENCE DESCRIPTION: SEQ ID NO:180:

```
ATG GCC TCA CGT TTT ATG ACG GAT CCG CAC GCG ATG CGG GAC ATG GCG      48
Met Ala Ser Arg Phe Met Thr Asp Pro His Ala Met Arg Asp Met Ala
 1               5                  10                  15

GGC CGT TTT GAG GTG CAC GCC CAG ACG GTG GAG GAC GAG GCT CGC CGG      96
Gly Arg Phe Glu Val His Ala Gln Thr Val Glu Asp Glu Ala Arg Arg
            20                  25                  30  .

ATG TGG GCG TCC GCG CAA AAC ATT TCC GGT GCG GGC TGG AGT GGC ATG     144
Met Trp Ala Ser Ala Gln Asn Ile Ser Gly Ala Gly Trp Ser Gly Met
        35                  40                  45

GCC GAG GCG ACC TCG CTA GAC ACC ATG GCC CAG ATG AAT CAG GCG TTT     192
Ala Glu Ala Thr Ser Leu Asp Thr Met Ala Gln Met Asn Gln Ala Phe
    50                  55                  60

CGC AAC ATC GTG AAC ATG CTG CAC GGG GTG CGT GAC GGG CTG GTT CGC     240
Arg Asn Ile Val Asn Met Leu His Gly Val Arg Asp Gly Leu Val Arg
65                  70                  75                  80

GAC GCC AAC AAC TAC GAG CAG CAA GAG CAG GCC TCC CAG CAG ATC CTC     288
Asp Ala Asn Asn Tyr Glu Gln Gln Glu Gln Ala Ser Gln Gln Ile Leu
                85                  90                  95

AGC AGC TAA                                                         297
Ser Ser
```

(2) INFORMATION FOR SEQ ID NO:181:

(i) SEQUENCE CHARACTERISTICS:
    (A) LENGTH: 98 amino acids
    (B) TYPE: amino acid
    (C) STRANDEDNESS: single
    (D) TOPOLOGY: linear

(ii) MOLECULE TYPE: protein
(v) FRAGMENT TYPE: internal

(xi) SEQUENCE DESCRIPTION: SEQ ID NO:181:

```
Met Ala Ser Arg Phe Met Thr Asp Pro His Ala Met Arg Asp Met Ala
 1               5                  10                  15
```

178

```
Gly Arg Phe Glu Val His Ala Gln Thr Val Glu Asp Glu Ala Arg Arg
        20                  25                  30
Met Trp Ala Ser Ala Gln Asn Ile Ser Gly Ala Gly Trp Ser Gly Met
        35                  40                  45
Ala Glu Ala Thr Ser Leu Asp Thr Met Ala Gln Met Asn Gln Ala Phe
        50                  55                  60
Arg Asn Ile Val Asn Met Leu His Gly Val Arg Asp Gly Leu Val Arg
65                  70                  75                  80
Asp Ala Asn Asn Tyr Glu Gln Gln Glu Gln Ala Ser Gln Gln Ile Leu
                85                  90                  95
Ser Ser
```

(2) INFORMATION FOR SEQ ID NO:182:

    (i) SEQUENCE CHARACTERISTICS:
      (A) LENGTH: 297 base pairs
      (B) TYPE: nucleic acid
      (C) STRANDEDNESS: single
      (D) TOPOLOGY: linear

    (ix) FEATURE:

    (xi) SEQUENCE DESCRIPTION: SEQ ID NO:182:

```
ATGGCCTCAC GTTTTATGAC GGATCCGCAT GCGATGCGGG ACATGGCGGG CCGTTTTGAG    60
GTGCACGCCC AGACGGTGGA GGACGAGGCT CGCCGGATGT GGGCGTCCGC GCAAAACATT   120
TCCGGTGCGG GCTGGAGTGG CATGGCCGAG GCGACCTCGC TAGACACCAT GACCTAGATG   180
AATCAGGCGT TTCGCAACAT CGTGAACATG CTGCACGGGG TGCGTGACGG GCTGGTTCGC   240
GACGCCAACA ACTACGAACA GCAAGAGCAG GCCTCCCAGC AGATCCTGAG CAGCTAG      297
```

(2) INFORMATION FOR SEQ ID NO:183:

    (i) SEQUENCE CHARACTERISTICS:
      (A) LENGTH: 98 amino acids
      (B) TYPE: amino acid
      (C) STRANDEDNESS: single
      (D) TOPOLOGY: linear

    (ii) MOLECULE TYPE: peptide

    (xi) SEQUENCE DESCRIPTION: SEQ ID NO:183:

```
Met Ala Ser Arg Phe Met Thr Asp Pro His Ala Met Arg Asp Met Ala
1               5                   10                  15
Gly Arg Phe Glu Val His Ala Gln Thr Val Glu Asp Glu Ala Arg Arg
        20                  25                  30
Met Trp Ala Ser Ala Gln Asn Ile Ser Gly Ala Gly Trp Ser Gly Met
        35                  40                  45
Ala Glu Ala Thr Ser Leu Asp Thr Met Thr Gln Met Asn Gln Ala Phe
        50                  55                  60
Arg Asn Ile Val Asn Met Leu His Gly Val Arg Asp Gly Leu Val Arg
65                  70                  75                  80
Asp Ala Asn Asn Tyr Glu Gln Gln Glu Gln Ala Ser Gln Gln Ile Leu
                85                  90                  95
Ser Ser
```

(2) INFORMATION FOR SEQ ID NO:184:

    (i) SEQUENCE CHARACTERISTICS:

(A) LENGTH: 297 base pairs
(B) TYPE: nucleic acid
(C) STRANDEDNESS: single
(D) TOPOLOGY: linear

(ii) MOLECULE TYPE: cDNA
(ix) FEATURE:

(A) NAME/KEY: Coding Sequence
(B) LOCATION: 1...294
(D) OTHER INFORMATION:

(xi) SEQUENCE DESCRIPTION: SEQ ID NO:184:

```
ATG ACC TCG CGT TTT ATG ACG GAT CCG CAC GCG ATG CGG GAC ATG GCG      48
Met Thr Ser Arg Phe Met Thr Asp Pro His Ala Met Arg Asp Met Ala
 1               5                  10                  15

GGC CGT TTT GAG GTG CAC GCC CAG ACG GTG GAG GAC GAG GCT CGC CGG      96
Gly Arg Phe Glu Val His Ala Gln Thr Val Glu Asp Glu Ala Arg Arg
                20                  25                  30

ATG TGG GCG TCC GCG CAA AAC ATT TCC GGC GCG GGC TGG AGT GGC ATG     144
Met Trp Ala Ser Ala Gln Asn Ile Ser Gly Ala Gly Trp Ser Gly Met
            35                  40                  45

GCC GAG GCG ACC TCG CTA GAC ACC ATG ACC CAG ATG AAT CAG GCG TTT     192
Ala Glu Ala Thr Ser Leu Asp Thr Met Thr Gln Met Asn Gln Ala Phe
        50                  55                  60

CGC AAC ATC GTG AAC ATG CTG CAC GGG GTG CGT GAC GGG CTG GTT CGC     240
Arg Asn Ile Val Asn Met Leu His Gly Val Arg Asp Gly Leu Val Arg
65                  70                  75                  80

GAC GCC AAC AAC TAC GAA CAG CAA GAG CAG GCC TCC CAG CAG ATC CTC     288
Asp Ala Asn Asn Tyr Glu Gln Gln Glu Gln Ala Ser Gln Gln Ile Leu
                85                  90                  95

AGC AGC TGA                                                         297
Ser Ser
```

(2)  INFORMATION FOR SEQ ID NO:185:

(i) SEQUENCE CHARACTERISTICS:
(A) LENGTH: 98 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:185:

```
Met Thr Ser Arg Phe Met Thr Asp Pro His Ala Met Arg Asp Met Ala
 1               5                  10                  15
Gly Arg Phe Glu Val His Ala Gln Thr Val Glu Asp Glu Ala Arg Arg
                20                  25                  30
Met Trp Ala Ser Ala Gln Asn Ile Ser Gly Ala Gly Trp Ser Gly Met
```

```
                  35                40                45
Ala Glu Ala Thr Ser Leu Asp Thr Met Thr Gln Met Asn Gln Ala Phe
          50                55                60
Arg Asn Ile Val Asn Met Leu His Gly Val Arg Asp Gly Leu Val Arg
  65                    70                75                80
Asp Ala Asn Asn Tyr Glu Gln Gln Glu Gln Ala Ser Gln Gln Ile Leu
              85                90                95
Ser Ser
```

(2) INFORMATION FOR SEQ ID NO:186:

    (i) SEQUENCE CHARACTERISTICS:
      (A) LENGTH: 20 base pairs
      (B) TYPE: nucleic acid
      (C) STRANDEDNESS: single
      (D) TOPOLOGY: linear

    (xi) SEQUENCE DESCRIPTION: SEQ ID NO:186:

GGAATGAAAA GGGGTTTGTG         20

(2) INFORMATION FOR SEQ ID NO:187:

    (i) SEQUENCE CHARACTERISTICS:
      (A) LENGTH: 20 base pairs
      (B) TYPE: nucleic acid
      (C) STRANDEDNESS: single
      (D) TOPOLOGY: linear

    (xi) SEQUENCE DESCRIPTION: SEQ ID NO:187:

GACCACGCCC GCGCCGTGTG         20

(2) INFORMATION FOR SEQ ID NO:188:

    (i) SEQUENCE CHARACTERISTICS:
      (A) LENGTH: 27 base pairs
      (B) TYPE: nucleic acid
      (C) STRANDEDNESS: single
      (D) TOPOLOGY: linear

    (xi) SEQUENCE DESCRIPTION: SEQ ID NO:188:

GCAACACCCG GGATGTCGCA GATTATG         27

(2) INFORMATION FOR SEQ ID NO:189:

    (i) SEQUENCE CHARACTERISTICS:
      (A) LENGTH: 30 base pairs
      (B) TYPE: nucleic acid
      (C) STRANDEDNESS: single
      (D) TOPOLOGY: linear

    (xi) SEQUENCE DESCRIPTION: SEQ ID NO:189:

CTAAGCTTGG ATCCCTAGCC GCCCCACTTG         30

(2) INFORMATION FOR SEQ ID NO:190:

(i) SEQUENCE CHARACTERISTICS:
 (A) LENGTH: 22 base pairs
 (B) TYPE: nucleic acid
 (C) STRANDEDNESS: single
 (D) TOPOLOGY: linear

(xi) SEQUENCE DESCRIPTION: SEQ ID NO:190:

GAATATTTGA AAGGGATTCG TG                                                  22

(2) INFORMATION FOR SEQ ID NO:191:

(i) SEQUENCE CHARACTERISTICS:
 (A) LENGTH: 30 base pairs
 (B) TYPE: nucleic acid
 (C) STRANDEDNESS: single
 (D) TOPOLOGY: linear

(xi) SEQUENCE DESCRIPTION: SEQ ID NO:191:

CTACTAAGCT TGGATCCTTA GTCTCCGGCG                                          30

(2) INFORMATION FOR SEQ ID NO:192:

(i) SEQUENCE CHARACTERISTICS:
 (A) LENGTH: 27 base pairs
 (B) TYPE: nucleic acid
 (C) STRANDEDNESS: single
 (D) TOPOLOGY: linear

(ii) MOLECULE TYPE: cDNA

(xi) SEQUENCE DESCRIPTION: SEQ ID NO:192:

GCAACACCCG GGGTGTCGCA GAGTATG                                             27

(2) INFORMATION FOR SEQ ID NO:193:

(i) SEQUENCE CHARACTERISTICS:
 (A) LENGTH: 30 base pairs
 (B) TYPE: nucleic acid
 (C) STRANDEDNESS: single
 (D) TOPOLOGY: linear

(ii) MOLECULE TYPE: cDNA

(xi) SEQUENCE DESCRIPTION: SEQ ID NO:193:

CTACTAAGCT TGGATCCTTA GTCTCCGGCG                                          30

20486PC1 seqlst
      Page 140 of 140

**Claims**

1. A substantially pure polypeptide fragment which

   a) comprises an amino acid sequence shown in SEQ ID NO: 92,

   b) comprises a subsequence of the amino acid sequence shown in SEQ ID NO: 92 which has a length of at least 6 amino acid residues, said subsequence being immunologically equivalent to the amino acid sequence shown in SEQ ID NO: 92 with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex, or

   c) comprises an amino acid sequence having a sequence identity amino acid sequence shown in SEQ ID NO: 92 or the subsequence of the amino acid sequence shown in SEQ ID NO: 92 defined in b) of at least 70% and at the same time being immunologically equivalent to the amino acid sequence shown in SEQ ID NO: 92 with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex.

2. The polypeptide fragment according to claim 1 in essentially pure form.

3. The polypeptide fragment according to claim 1 or 2, which comprises an epitope for a T-helper cell.

4. The polypeptide fragment according to any of the preceding claims, which has a length of at least 7 amino acid residues, such as at least 8, at least 9, at least 10, at least 12, at least 14, at least 16, at least 18, at least 20, at least 22, at least 24, and at least 30 amino acid residues.

5. The polypeptide fragment according to any of the preceding claims which is free from any signal sequence.

6. The polypeptide fragment according to any of the preceding claims which

   1) induces a release of IFN-$\gamma$ from primed memory T-lymphocytes withdrawn from a mouse within 2 weeks of primary infection or within 4 days after the mouse has been rechallenge infected with mycobacteria belonging to the tuberculosis complex, the induction performed by the addition of the polypeptide to a suspension comprising about 200.000 spleen cells per ml, the addition of the polypeptide resulting in a concentration of 1-4 $\mu$g polypeptide per ml suspension, the release of IFN-$\gamma$ being assessable by determination of IFN-$\gamma$ in supernatant harvested 2 days after the addition of the polypeptide to the suspension, and/or

   2) induces a release of IFN-$\gamma$ of at least 300 pg above background level from about 1000,000 human PBMC (peripheral blood mononuclear cells) per ml isolated from TB patients in the first phase of infection, or from healthy BCG vaccinated donors, or from healthy contacts to TB patients, the induction being performed by the addition of the polypeptide to a suspension comprising the about 1,000,000 PBMC per ml, the addition of the polypeptide resulting in a concentration of 1-4 $\mu$g polypeptide per ml suspension, the release of IFN-$\gamma$ being assessable by determination of IFN-$\gamma$ in supernatant harvested 2 days after the addition of the polypeptide to the suspension; and/or

   3) induces an IFN-$\gamma$ release from bovine PBMC derived from animals previously sensitized with mycobacteria belonging to the tuberculosis complex, said release being at least two times the release observed from bovine PBMC derived from animals not previously sensitized with mycobacteria belonging to the tuberculosis complex.

7. A polypeptide fragment according to any of the preceding claims, wherein the sequence identity in c) is at least 80%, such as at least 85%, at least 90%, at least 91%, at least 92%, at least 93%, at least 94%, at least 95%, at least 96%, at least 97%, at least 98%, at least 99%, and at least 99.5%.

8. A fusion polypeptide comprising at least one polypeptide fragment according to any of the preceding claims and at least one fusion partner.

**9.** A fusion polypeptide according to claim 8, wherein the fusion partner is selected from the group consisting of a polypeptide fragment as defined in any of claims 1-8, and an other polypeptide fragment derived from a bacterium belonging to the tuberculosis complex, such as ESAT-6 or at least one T-cell epitope thereof, MPB64 or at least one T-cell epitope thereof, MPT64 or at least one T-cell epitope thereof, and MPB59 or at least one T-cell epitope thereof.

**10.** A polypeptide according to any of the preceding claims which is lipidated so as to allow a self-adjuvating effect of the polypeptide.

**11.** A substantially pure polypeptide according to any of claims 1-10 for use as a pharmaceutical.

**12.** The use of a substantially pure polypeptide according to any of claims 1-11 in the preparation of a pharmaceutical composition for the diagnosis of tuberculosis caused by *Mycobacterium tuberculosis, Mycobacterium africanum* or *Mycobacterium bovis.*

**13.** The use of a substantially pure polypeptide according to any of claims 1-11 in the preparation of a pharmaceutical composition for the vaccination against tuberculosis caused by *Mycobacterium tuberculosis, Mycobacterium africanum* or *Mycobacterium bovis.*

**14.** A nucleic acid fragment in isolated form which

1) comprises a nucleic acid sequence which encodes a polypeptide as defined in any of claims 1-10, or comprises a nucleic acid sequence complementary thereto,

2) has a length of at least 10 nucleotides and hybridizes readily under stringent hybridization conditions with a nucleic acid fragment which has a nucleotide sequence selected shown in SEQ ID NO: 91 or a sequence complementary thereto.

**15.** A nucleic acid fragment according to claim 14, which is a DNA fragment.

**16.** A nucleic acid fragment according to claims 14 or 15 for use as a pharmaceutical.

**17.** The use of a nucleic acid fragment according to any of claims 14-16 in the preparation of a pharmaceutical composition for the vaccination against tuberculosis caused by *Mycobacterium tuberculosis, Mycobacterium africanum* or *Mycobacterium bovis.*

**18.** The use of a nucleic acid fragment according to any of claims 14-16 in the preparation of a pharmaceutical composition for the diagnosis of tuberculosis caused by *Mycobacterium tuberculosis, Mycobacterium africanum* or *Mycobacterium bovis.*

**19.** A vaccine comprising a nucleic acid fragment according to any of claims 14-16, the vaccine effecting *in vivo* expression of antigen by an animal, including a human being, to whom the vaccine has been administered, the amount of expressed antigen being effective to confer substantially increased resistance to infections with mycobacteria of the tuberculosis complex in an animal, including a human being.

**20.** An immunologic composition comprising a polypeptide according to any of claims 1-11.

**21.** An immunologic composition according to claim 20, which further comprises an immunologically and pharmaceutically acceptable carrier, vehicle or adjuvant.

**22.** An immunologic composition according to claim 21, wherein the carrier is selected from the group consisting of a polymer to which the polypeptide(s) is/are bound by hydrophobic non-covalent interaction, such as a plastic, e.g. polystyrene, a polymer to which the polypeptide(s) is/are covalently bound, such as a polysaccharide, and a polypeptide, e.g. bovine serum albumin, ovalbumin or keyhole limpet hemocyanin; the vehicle is selected from the group consisting of a diluent and a suspending agent; and the adjuvant is selected from the group consisting of dimethyldioctadecylammonium bromide (DDA), Quil A, poly I:C, Freund's incomplete adjuvant, IFN-γ, IL-2, IL-12, monophosphoryl lipid A (MPL), and muramyl dipeptide (MDP).

**23.** An immunologic composition according to any of claims 28-30, comprising at least two different polypeptide fragments, each different polypeptide fragment being a polypeptide according to any of claims 1-11.

**24.** An immunologic composition according to claim 23, comprising 3-20 different polypeptide fragments, each different polypeptide fragment being according to any of claims 1-11.

**25.** An immunologic composition according to any of claims 20-24, which is in the form of a vaccine.

**26.** An immunologic composition according to any of claims 20-24, which is in the form of a skin test reagent.

**27.** A vaccine for immunizing an animal, including a human being, against tuberculosis caused by mycobacteria belonging to the tuberculosis complex, comprising as the effective component a non-pathogenic microorganism, wherein at least one copy of a DNA fragment comprising a DNA sequence encoding a polypeptide according to any of claims 1-11 has been incorporated into the genome of the microorganism in a manner allowing the microorganism to express and optionally secrete the polypeptide.

**28.** A vaccine according to claim 27, wherein the microorganism is a bacterium.

**29.** A vaccine according to claim 28, wherein the bacterium is selected from the group consisting of the genera *Mycobacterium, Salmonella, Pseudomonas* and *Eschericia.*

**30.** A vaccine according to claim 29, wherein the microorganism is *Mycobacterium bovis* BCG, such as *Mycobacterium bovis* BCG strain: Danish 1331.

**31.** A vaccine according to any of claims 27-30, wherein at least 2 copies of a DNA fragment encoding a polypeptide according to any of claims 1-9 are incorporated into the genome of the microorganism.

**32.** A vaccine according to claim 31, wherein the number of copies is at least 5.

**33.** A replicable expression vector, which comprises a nucleic acid fragment according to any of claims 14-16.

**34.** A vector according to claim 33, which is selected from the group consisting of a virus, a bacteriophage, a plasmid, a cosmid, and a microchromosome.

**35.** A transformed cell harbouring at least one vector according to claim 33 or 34.

**36.** A transformed cell according to claim 35, which is a bacterium belonging to the tuberculosis complex, such as a *M. tuberculosis bovis* BCG cell.

**37.** A transformed cell according to claim 35 or 36, which expresses a polypeptide according to any of claims 1-11.

**38.** A method for producing a polypeptide according to any of claims 1-11, comprising
inserting a nucleic acid fragment according to any of claims 22-24 into a vector which is able to replicate in a host cell, introducing the resulting recombinant vector into the host cell, culturing the host cell in a culture medium under conditions sufficient to effect expression of the polypeptide, and recovering the polypeptide from the host cell or culture medium; or
isolating the polypeptide from a short-term culture filtrate; or
isolating the polypeptide from whole mycobacteria of the tuberculosis complex or from lysates or fractions thereof, e.g. cell wall containing fractions; or
synthesizing the polypeptide by solid or liquid phase peptide synthesis.

**39.** A method for producing an immunologic composition according to any of claims 20-26 comprising
preparing, synthesizing or isolating a polypeptide according to any of claims 1-11, and
solubilizing or dispersing the polypeptide in a medium for a vaccine, and
optionally adding other *M. tuberculosis* antigens and/or a carrier, vehicle and/or adjuvant substance,
or

cultivating a cell according to any of claims 33-37, and
transferring the cells to a medium for a vaccine, and
optionally adding a carrier, vehicle and/or adjuvant substance.

**40.** A method for diagnosing ongoing or previous sensitization in an animal or a human being with bacteria belonging to the tuberculosis complex, the method comprising providing a blood sample from the animal or human being, and contacting the sample from the animal with the polypeptide according to any of claims 1-11, a significant release into the extracellular phase of at least one cytokine by mononuclear cells in the blood sample being indicative of the animal being sensitized.

**41.** A composition for diagnosing tuberculosis in an animal, including a human being, comprising a polypeptide according to any of claims 1-11, or a nucleic acid fragment according to any of claims 14-16, optionally in combination with a means for detection.

**42.** A monoclonal or polyclonal antibody, which is specifically reacting with a polypeptide according to any of claims 1-11 in an immuno assay, or a specific binding fragment of said antibody.

## Fig. 1A

## Fig. 1B

**Fig. 2**

```
  1  GGCCGCCGGT ACCTATGTGG CCGCCGATGC TGCGGNCGCG TCGACCTATA CCGGGTTCTG        60
                -35region                            -10 region
 61  ATCGAACCCT GCTGACCGAG AGGACTTGTG ATG TCG CAA ATC ATG TAC AAC TAC CCC GCG    120
                Shine Delgarno               M   S   Q   I   M   Y   N   Y   P   A
121  ATG TTG GGT CAC GCC GGG GAT ATG GCC GGA TAT GCC GGC ACG CTG CAG AGC TTG GGT GCC    180
     M   L   G   H   A   G   D   M   A   G   Y   A   G   T   L   Q   S   L   G   A
181  GAG ATC GCC GTG GAG CAG GCC GCG TTG CAG AGT GCG TGG CAG GGC GAT ACC GGG ATC ACG    240
     E   I   A   V   E   Q   A   A   L   Q   S   A   W   Q   G   D   T   G   I   T
241  TAT CAG GCG TGG CAG GCA CAG TGG AAC CAG GCC ATG GAA GAT TTG GTG CGG GCC TAT CAT    300
     Y   Q   A   W   Q   A   Q   W   N   Q   A   M   E   D   L   V   R   A   Y   H
301  GCG ATG TCC AGC ACC CAT GAA GCC AAC ACC ATG GCG ATG ATG GCC CGC GAC ACC GCC GAA    360
     A   M   S   S   T   H   E   A   N   T   M   A   M   M   A   R   D   T   A   E
361  GCC GCC AAA TGG GGC GGC TAG                                               381
     A   A   K   W   G   G   •
```

# Fig. 3

EP 1 484 405 A1

EP 1 484 405 A1

GGG*TAGCCG*G ACCACGGCTG GGCAAAGATG TGCAGGCCGC CATCAAGGCG GTCAAGGCCG    60
   -35 region

GCGACGGCGT CA*TAAACC*CG GACGGCACCT TGTTGGCGGG CCCCGCGGTG CTGACGCCCG    120
    -10 region

AC*GAGTACAA* CTCCCGGCTG GTG GCC GCC GAC CCG GAG TCC ACC GCG GCG    170
  Shine Delgarno     V   A   A   D   P   E   S   T   A   A

TTG CCC GAC GGC GCC GGG CTG GTC GTT CTG GAT GGC ACC GTC ACT GCC GAA CTC GAA GCC   230
L   P   D   G   A   G   L   V   V   L   D   G   T   V   T   A   E   L   E   A

GAG GGC TGG GCC AAA GAT CGC ATC CGC GAA CTG CAA GAG CTG CGT AAG TCG ACC GGG CTG   290
E   G   W   A   K   D   R   I   R   E   L   Q   E   L   R   K   S   T   G   L

GAC GTT TCC GAC CGC ATC CGG GTG GTG ATG TCG GTG CCT GCG GAA CGC GAA GAC TGG GCG   350
D   V   S   D   R   I   R   V   V   M   S   V   P   A   E   R   E   D   W   A

CGC ACC CAT CGC GAC CTC ATT GCC GGA GAA ATC TTG GCT ACC GAC TTC <u>GAA TTC GCC GAC</u>   410
R   T   H   R   D   L   I   A   G   E   I   L   A   T   D   F   E   F   A   D

<u>CTC GCC GAT GGT GTG GCC ATC GGC GAC GGC GTG CGG GTA AGC ATC GAA AAG ACC TGA</u>   467
L   A   D   G   V   A   I   G   D   G   V   R   V   S   I   E   K   T   *

# Fig. 4

```
  1  GAATTCGCCGGGTGCACACAGCCTTACACGACGGAGGTGGACACATGAAG      50
                                                         M   K
 51  GGTCGGTCGGCGCTGCTGCGGGCGCTCTGGATTGCCGCACTGTCATTCGG     100
      G   R   S   A   L   L   R   A   L   W   I   A   A   L   S   F   G
101  GTTGGGCGGTGTCGCGGTAGCCGCGGAACCCACCGCCAAGGCCGCCCCAT     150
      L   G   G   V   A   V   A   A   E   P   T   A   K   A   A   P
151  ACGAGAACCTGATGGTGCCGTCGCCCTCGATGGGCCGGGACATCCCGGTG     200
      Y   E   N   L   M   V   P   S   P   S   M   G   R   D   I   P   V
201  GCCTTCCTAGCCGGTGGGCCGCACGCGGTGTATCTGCTGGACGCCTTCAA     250
      A   F   L   A   G   G   P   H   A   V   Y   L   L   D   A   F   N
251  CGCCGGCCCGGATGTCAGTAACTGGGTCACCGCGGGTAACGCGATGAACA     300
      A   G   P   D   V   S   N   W   V   T   A   G   N   A   M   N
301  CGTTGGCGGGCAAGGGGATTTCGGTGGTGGCACCGGCCGGTGGTGCGTAC     350
      T   L   A   G   K   G   I   S   V   V   A   P   A   G   G   A   Y
351  AGCATGTACACCAACTGGGAGCAGGATGGCAGCAAGCAGTGGGACACCTT     400
      S   M   Y   T   N   W   E   Q   D   G   S   K   Q   W   D   T   F
401  CTTGTCCGCTGAGCTGCCCGACTGGCTGGCCGCTAACCGGGGCTTGGCCC     450
      L   S   A   E   L   P   D   W   L   A   A   N   R   G   L   A
451  CCGGTGGCCATGCGGCCGTTGGCGCCGCTCAGGGCGGTTACGGGGCGATG     500
      P   G   G   H   A   A   V   G   A   A   Q   G   G   Y   G   A   M
501  GCGCTGGCGGCCTTCCACCCCGACCGCTTCGGCTTCGCTGGCTCGATGTC     550
      A   L   A   A   F   H   P   D   R   F   G   F   A   G   S   M   S
551  GGGCTTTTTGTACCCGTCGAACACCACCACCAACGGTGCGATCGCGGCGG     600
      G   F   L   Y   P   S   N   T   T   T   N   G   A   I   A   A
601  GCATGCAGCAATTCGGCGGTGTGGACACCAACGGAATGTGGGGAGCACCA     650
      G   M   Q   Q   F   G   G   V   D   T   N   G   M   W   G   A   P
651  CAGCTGGGTCGGTGGAAGTGGCACGACCCGTGGGTGCATGCCAGCCTGCT     700
      Q   L   G   R   W   K   W   H   D   P   W   V   H   A   S   L   L
701  GGCGCAAAACAACACCCGGGTGTGGGTGTGGAGCCCGACCAACCCGGGAG     750
      A   Q   N   N   T   R   V   W   V   W   S   P   T   N   P   G
751  CCAGCGATCCCGCCGCCATGATCGGCCAAACCGCCGAGGCGATGGGTAAC     800
      A   S   D   P   A   A   M   I   G   Q   T   A   E   A   M   G   N
801  AGCCGCATGTTCTACAACCAGTATCGCAGCGTCGGCGGGCACAACGGACA     850
      S   R   M   F   Y   N   Q   Y   R   S   V   G   G   H   N   G   H
851  CTTCGACTTCCCAGCCAGCGGTGACAACGGCTGGGGCTCGTGGGCGCCCC     900
      F   D   F   P   A   S   G   D   N   G   W   G   S   W   A   P
901  AGCTGGGCGCTATGTCGGGCGATATCGTCGGTGCGATCCGCTAAGCGAAT     950
      Q   L   G   A   M   S   G   D   I   V   G   A   I   R   .
951  TC                                                     952
```

# Fig. 5

## 2-DE reference map of ST-CF

# Fig. 6

**European Patent Office**

**EUROPEAN SEARCH REPORT**

Application Number

EP 04 07 7071

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 97/09428 A (CORIXA CORP) 13 March 1997 (1997-03-13) *see SEQ ID NO: 112 and 114 (pp. 144-147)* * abstract; claims 1-34; examples 1,3 * * page 12 - page 18 * * page 21 - page 25 * ----- | 1-42 | C12N15/31 A61K39/04 C07K14/35 C12N15/62 A61K38/16 G01N33/569 C12Q1/68 C07K16/12 |
| X | WO 97/09429 A (CORIXA CORP) 13 March 1997 (1997-03-13) *see SEQ ID NO: 107 and 109 (pp. 157-161)* * abstract; examples 1,3 * * page 12 - page 15, paragraph 2 * * page 17, paragraph 4 - page 19, paragraph 2 * * page 24 - page 25 * ----- | 1-42 | |
| X | DATABASE EMBL EBI; 4 November 1995 (1995-11-04), ANDERSEN P ET AL: "Recall of long-lived immunity to Mycobacterium tuberculosis infection in mice" XP002300140 Database accession no. U34848 *see nucleotides 3100-4310* * abstract * ----- | 14-16, 33-37,41 | **TECHNICAL FIELDS SEARCHED (Int.Cl.7)** C12N A61K C07K G01N C12Q |
| P,X | WO 98/16646 A (CORIXA CORP) 23 April 1998 (1998-04-23) *see SEQ ID NO: 112 and 114 (pp. 135-138)* * abstract; claims 1-37; examples 1,3,7 * * page 4 - page 29 * ----- | 1-42 | |
| P,X | WO 98/16645 A (CORIXA CORP) 23 April 1998 (1998-04-23) *see SEQ ID NO: 107 and 109 (pp. 141-144) * abstract * * page 4 - page 28; claims 1-54; examples 1,3,7 * ----- | 1-42 | |

The present search report has been drawn up for all claims

| Place of search | Date of completion of the search | Examiner |
|---|---|---|
| The Hague | 11 October 2004 | Oderwald, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 07 7071

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2004

| Patent document cited in search report | | Publication date | Patent family member(s) | | Publication date |
|---|---|---|---|---|---|
| WO 9709428 | A | 13-03-1997 | AT | 252640 T | 15-11-2003 |
| | | | AU | 727602 B2 | 14-12-2000 |
| | | | AU | 7158696 A | 27-03-1997 |
| | | | BR | 9610262 A | 06-07-1999 |
| | | | CA | 2230885 A1 | 13-03-1997 |
| | | | CN | 1200147 A ,B | 25-11-1998 |
| | | | CZ | 9800628 A3 | 13-10-1999 |
| | | | DE | 69630457 D1 | 27-11-2003 |
| | | | DK | 851927 T3 | 26-01-2004 |
| | | | EP | 1203817 A2 | 08-05-2002 |
| | | | EP | 1347055 A2 | 24-09-2003 |
| | | | EP | 1398379 A2 | 17-03-2004 |
| | | | EP | 0851927 A2 | 08-07-1998 |
| | | | ES | 2210392 T3 | 01-07-2004 |
| | | | HU | 9900902 A2 | 28-07-1999 |
| | | | JP | 2001517069 T | 02-10-2001 |
| | | | NO | 980883 A | 27-04-1998 |
| | | | NZ | 319377 A | 28-01-2000 |
| | | | PL | 325373 A1 | 20-07-1998 |
| | | | PT | 851927 T | 31-03-2004 |
| | | | SI | 851927 T1 | 29-02-2004 |
| | | | TR | 9800411 T1 | 21-05-1998 |
| | | | US | 6592877 B1 | 15-07-2003 |
| | | | US | 2003143243 A1 | 31-07-2003 |
| | | | WO | 9709428 A2 | 13-03-1997 |
| | | | US | 6290969 B1 | 18-09-2001 |
| | | | ZA | 9607394 A | 05-05-1997 |
| | | | US | 6458366 B1 | 01-10-2002 |
| | | | US | 6338852 B1 | 15-01-2002 |
| WO 9709429 | A | 13-03-1997 | AU | 7158796 A | 27-03-1997 |
| | | | BR | 9610268 A | 06-07-1999 |
| | | | CA | 2230927 A1 | 13-03-1997 |
| | | | CN | 1200146 A | 25-11-1998 |
| | | | EP | 0850305 A2 | 01-07-1998 |
| | | | JP | 11514217 T | 07-12-1999 |
| | | | RU | 2206574 C2 | 20-06-2003 |
| | | | US | 6458366 B1 | 01-10-2002 |
| | | | WO | 9709429 A2 | 13-03-1997 |
| | | | US | 2003135026 A1 | 17-07-2003 |
| | | | US | 6338852 B1 | 15-01-2002 |
| | | | ZA | 9607395 A | 24-04-1997 |
| WO 9816646 | A | 23-04-1998 | US | 6290969 B1 | 18-09-2001 |
| | | | AU | 4814497 A | 11-05-1998 |
| | | | BR | 9712518 A | 24-10-2000 |

For more details about this annex : see Official Journal of the European Patent Office, No. 12/82

## ANNEX TO THE EUROPEAN SEARCH REPORT
## ON EUROPEAN PATENT APPLICATION NO.

EP 04 07 7071

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2004

| Patent document cited in search report | | Publication date | Patent family member(s) | | | Publication date |
|---|---|---|---|---|---|---|
| WO 9816646 | A | | CA | 2268008 | A1 | 23-04-1998 |
| | | | CZ | 9901265 | A3 | 17-11-1999 |
| | | | EP | 0932681 | A2 | 04-08-1999 |
| | | | JP | 2001501832 | T | 13-02-2001 |
| | | | KR | 2000049101 | A | 25-07-2000 |
| | | | NO | 991694 | A | 10-06-1999 |
| | | | PL | 332878 | A1 | 25-10-1999 |
| | | | TR | 9901571 | T2 | 21-10-1999 |
| | | | US | 2003143243 | A1 | 31-07-2003 |
| | | | WO | 9816646 | A2 | 23-04-1998 |
| | | | ZA | 9708969 | A | 20-04-1998 |
| | | | US | 6350456 | B1 | 26-02-2002 |
| | | | US | 6592877 | B1 | 15-07-2003 |
| | | | US | 2002009459 | A1 | 24-01-2002 |
| | | | US | 2003147911 | A1 | 07-08-2003 |
| WO 9816645 | A | 23-04-1998 | US | 6338852 | B1 | 15-01-2002 |
| | | | AU | 4750597 | A | 11-05-1998 |
| | | | BR | 9712298 | A | 24-10-2000 |
| | | | CA | 2268036 | A1 | 23-04-1998 |
| | | | CZ | 9901266 | A3 | 15-09-1999 |
| | | | EP | 0934415 | A2 | 11-08-1999 |
| | | | JP | 2001500383 | T | 16-01-2001 |
| | | | KR | 2000049100 | A | 25-07-2000 |
| | | | NO | 991693 | A | 09-06-1999 |
| | | | PL | 333304 | A1 | 22-11-1999 |
| | | | TR | 9901569 | T2 | 21-12-2000 |
| | | | US | 6458366 | B1 | 01-10-2002 |
| | | | WO | 9816645 | A2 | 23-04-1998 |
| | | | US | 2003135026 | A1 | 17-07-2003 |
| | | | ZA | 9708967 | A | 20-04-1998 |

EPO FORM P0459

For more details about this annex : see Official Journal of the European Patent Office, No. 12/82